# EUROPEAN PATENT SPECIFICATION

(11) **EP 1 041 985 B1**
(45) Date of publication and mention of the grant of the patent: **15.02.2006**
(21) Application number: 98963829.1
(22) Date of filing: 21.12.1998
(51) Int. Cl.: A61K 31/44, A61K 31/495, A61P 35/00, A61P 35/02, A61P 35/04, A61P 43/00

(54) **COMBINATION OF BENZOCYCLOHEPTAPYRIDINE COMPOUNDS AND ANTINEOPLASTIC DRUGS FOR TREATING PROLIFERATIVE DISEASES**
BENZOCYCLOHEPTAPYRIDINEZUSAMMENSETZUNGEN UND ANTINEOPLASTISCHE ARZNEIMITTEL ALS KOMBINATIONSTHERAPEUTIKA FÜR THERAPIE DER ZELLPROLIFERATION
COMBINAISON DE COMPOSES BENZOCYCLOHEPTAPYRIDINES ET MEDICAMENTS ANTINEOPLASIQUES POUR LE TRAITEMENT DE MALADIES PROLIFERANTES

(30) Priority: 22.12.1997 US 996027; 28.08.1998 US 143529; 29.10.1998 US 181969
(43) Date of publication of application: 11.10.2000
(73) Proprietor: SCHERING CORPORATION, Kenilworth, New Jersey 07033-0530 (US)
(72) Inventor: BISHOP, Walter, R., Pompton Plains, NJ 07444 (US); CATINO, Joseph, J., Guilford, CT 06437 (US); DOLL, Ronald, J., Maplewood, NJ 07040 (US); GANGULY, Ashit, Upper Montclair, NJ 07043 (US); GIRIJAVALLABHAN, Viyyoor, Parsippany, NJ 07054 (US); KIRSCHMEIER, Paul, Basking Ridge, NJ 07920 (US); LIU, Ming, Fanwood, NJ 07023 (US); NIELSEN, Loretta, L., Millington, NJ 07946 (US); CUTLER, David, L., Moorestown, NJ 08057 (US)
(74) Representative: Gross, Ulrich-Maria
(86) International application number: PCT/US1998/026224
(87) International publication number: WO 1999/032114

(56) References cited:
- EP-A- 0 856 315
- WO-A-92/11034
- WO-A-97/23478
- WO-A-97/38664
- WO-A-97/45412
- WO-A-98/35554
- LIU M ET AL: "Antitumor activity of SCH 66336, an orally bioavailable tricyclic inhibitor of farnesyl protein transferase, in huma tumor xenograft models and wap-ras transgenic mice" CANCER RESEARCH, vol. 58, no. 21, 1 November 1998, pages 4947-4956, XP002096891
- MOASSER M.M. L. ET AL: "Pharnesyl transferase inhibitor cause enhanced mitotic sensitivity to taxol and epothilones" PROC NATL ACAD SCI USA, vol. 95, February 1998, pages 1369-1374, XP002102823
- "SCH66336 plus gemictabine for all advanced malignancies" CANCERTHERAPY,1999, XP002104517 149.142.240.3/cancertherapy/sch_66336.html
- SHI B_(A) M: "Enhanced efficacy of the farnesyl protein transferase inhibitor SCH66336 in combination with paclitaxel." PROCEEDINGS OF THE AMERICAN ASSOCIATION FOR CANCER RESEARCH ANNUAL MEETING, 1999, XP002102820
- "Hemmstoffe der Farnesysltransferase: einer neuer Ansatz zur Entwicklung potentieller Krebstherapeutika" PHARMAZIE IN UNSERER ZEIT, vol. 27, no. 6, November 1998, pages 278-288, XP002104518
- OMER C A ET AL: "CA1A2X-competitive inhibitors of farnesyltransferase as anti -cancer agents" TRENDS IN PHARMACOLOGICAL SCIENCES, vol. 18, no. 11, 1 November 1997, page 437-445 XP004099346

## Description

This invention describes novel uses of an FPT inhibitor for the manufacture of a medicament for treating subjects afflicted with proliferative diseases such as cancers, tumors, or metastatic disease. In particular, this invention provides uses for the manufacture of a medicament for inhibiting the proliferation of cells, more specifically cancer cells, comprising the combined use of (1) a farnesyl protein transferase ("FPT") inhibitor and (2) an antineoplastic agent and/or radiation therapy.

### BACKGROUND OF THE INVENTION

Oncogenes frequently encode protein components of signal transduction pathways which lead to stimulation of cell growth and mitogenesis. Mutation and/or overexpression of certain oncogenes is frequently associated with cellular tranformation and human cancer. To acquire transforming potential, the precursor of the *ras* oncoprotein must undergo farnesylation of the cysteine residue located in a carboxyl-terminal tetrapeptide. Inhibitors of the enzyme that catalyzes this modification, farnesyl protein transferase, have therefore been suggested as anticancer agents for tumors. Mutated, oncogenic forms of *ras* are frequently found in many human cancers, most notably in more than 50% of colon and pancreatic carcinomas (Kohl *et al*., Science, Vol. 260, 1934 to 1937, (1993)). Proteins other than *ras* may play a part in tumorigenicity and may also require farnesylation for biological activity.

International Patent Publication Number W092/ 11034 (published July 9, 1992) discloses a method of increasing the sensitivity of a tumor to an antineoplastic agent (in cases where the tumor is resistant to the antineoplastic agent), by the concurrent administration of the antineoplastic agent and (*inter alia*) a potentiating agent of the formula: 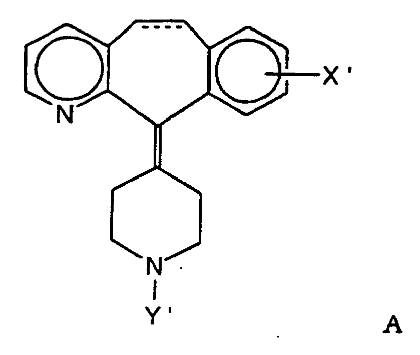
wherein the dotted line represents an optional double bond, X' is hydrogen or halo, and Y' is hydrogen, substituted carboxylate or substituted sulfonyl. For example, Y' can be, amongst others, -COOR' wherein R' is C-1 to C-6 alkyl or substituted alkyl, phenyl, substituted phenyl, C-7 to C-12 aralkyl or substituted aralkyl, 2-, 3- or 4-piperidyl or N-substituted piperidyl. Y' can also be, amongst others, SO₂R' wherein R' is C-1 to C-6 alkyl, phenyl, substituted phenyl, C-7 to C-12 aralkyl or substituted aralkyl. Examples of such potentiating agents include 11-(4-piperidylidene)-5H-benzo[5,6]cyclohepta[1.2-b]pyridines such as Loratadine. Antineoplastic agents exemplified are: vinca alkaloids, epipodophyllotoxins, anthracycline antibiotics, actinomycin D, plicamycin, puromycin, gramicidin D, taxol, colchicine, cytochalasin B, emetine, maytansine, and amsacrine. The WO92/11034 publication focuses on potentiating the antineoplastic agents through a specific mechanism of action: inhibition of multiple drug resistance.

In view of the need for improved treatments for proliferative diseases, particularly cancers, novel methods of treatment would be a welcome contribution to the art. The present invention provides uses of an FPT inhibitor for the manufacture of a medicament for such treatments.

### SUMMARY OF THE INVENTION

The present invention provides uses of the FPT inhibitor 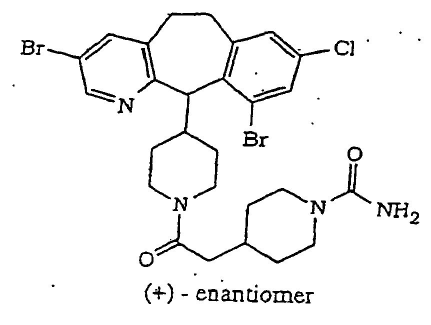
for the manufacture of a medicament for use concurrently or sequentially with an antineoplastic agent and/or radiation therapy, in the treatment of proliferative disease in a patient in need of such treatment The medicaments prepared by the uses of the present invention are particularly useful for the treatment of various cancers, especially epithelial cancers, *e*.*g*., prostate cancer, lung cancer, breast cancer, colorectal cancer, and pancreatic cancer. In preferred embodiments, the FPT inhibitor is combined with one of the following antineoplastic agents: gemcitabine, paclitaxel (Taxol®), 5-Fluorouracil (5-FU), cyclophosphamide (Cytoxan®), temozolomide, or Vincristine.

For instance, in a preferred embodiment, the antineoplastic agent is gemcitabine. In a particularly preferred embodiment, the cancer to be treated is a pancreatic cancer.

In another preferred embodiment, the present invention provides uses of the FPT inhibitor 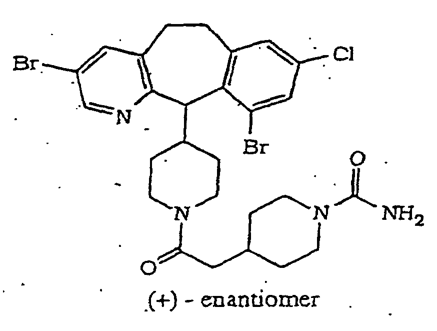
for the manufacture of a medicament for use concurrently or sequentially with a microtubule affecting agent (e.g., paclitaxel) in the treatment of proliferative disease in a patient in need of such treatment.

### BRIEF DESCRIPTION OF THE DRAWINGS

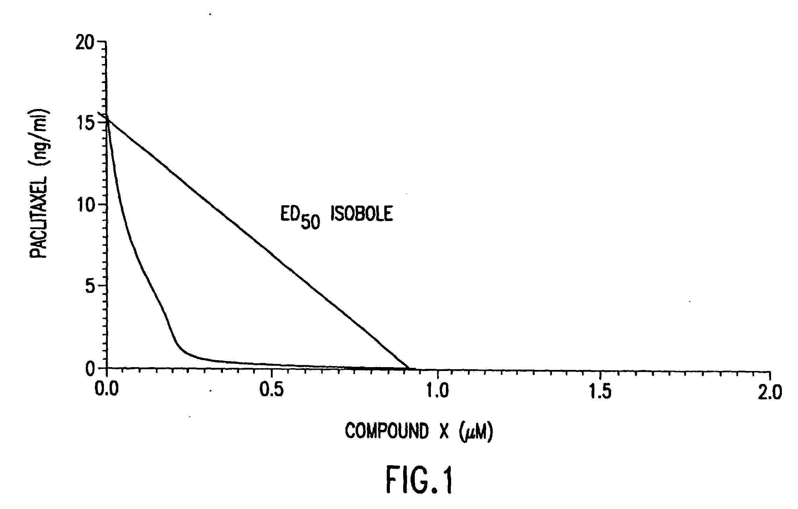

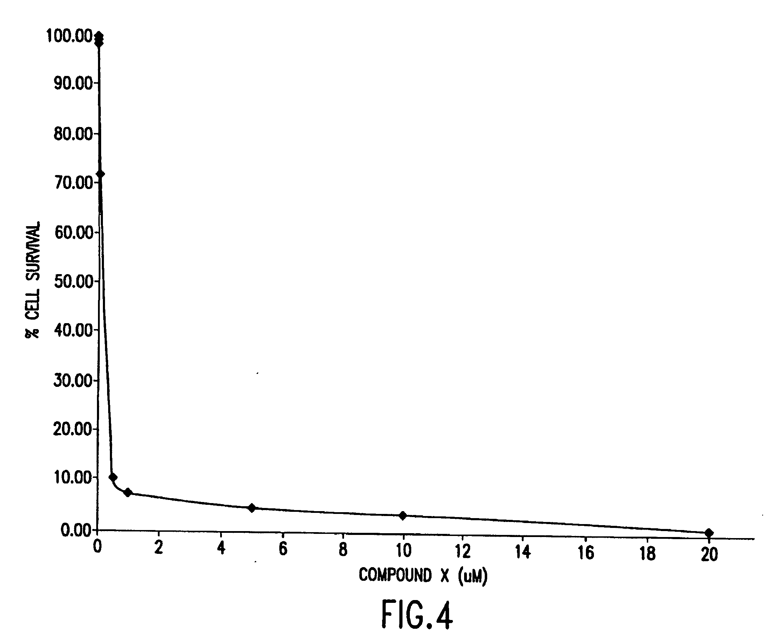

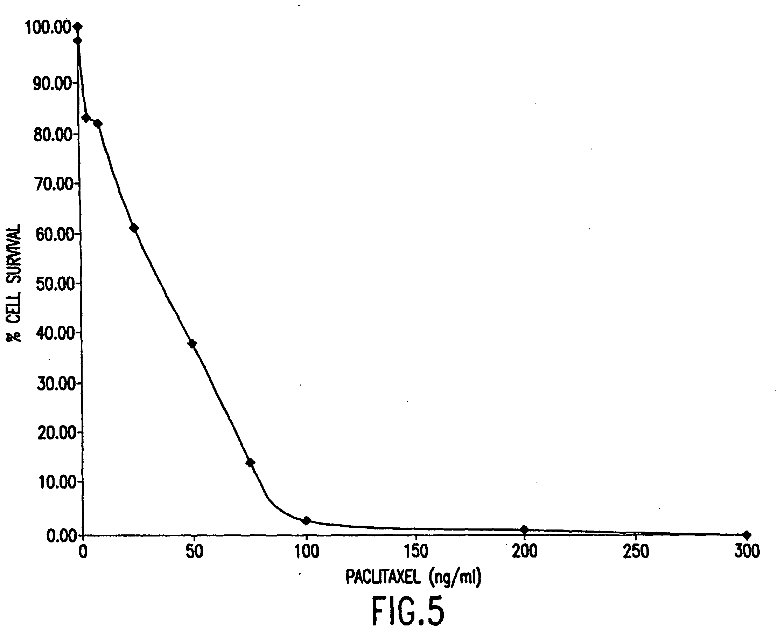

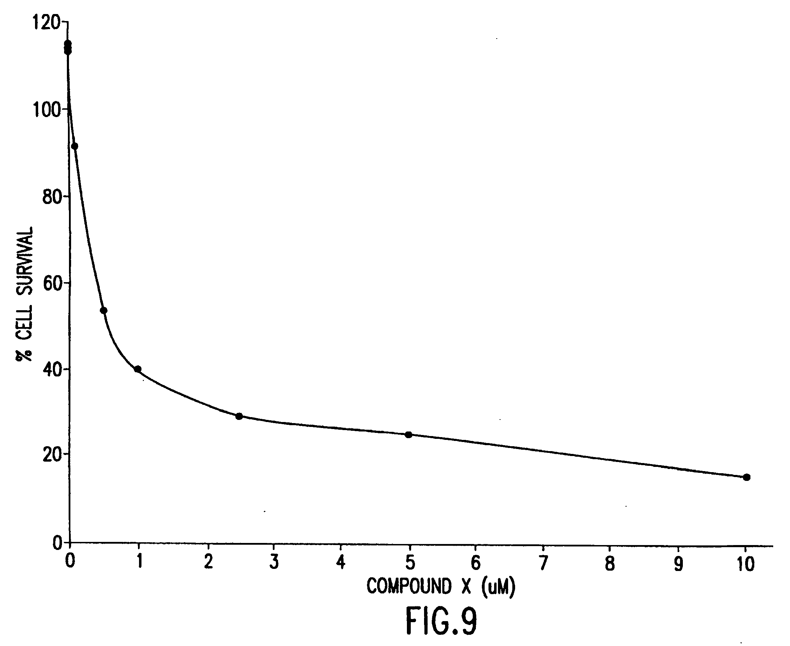

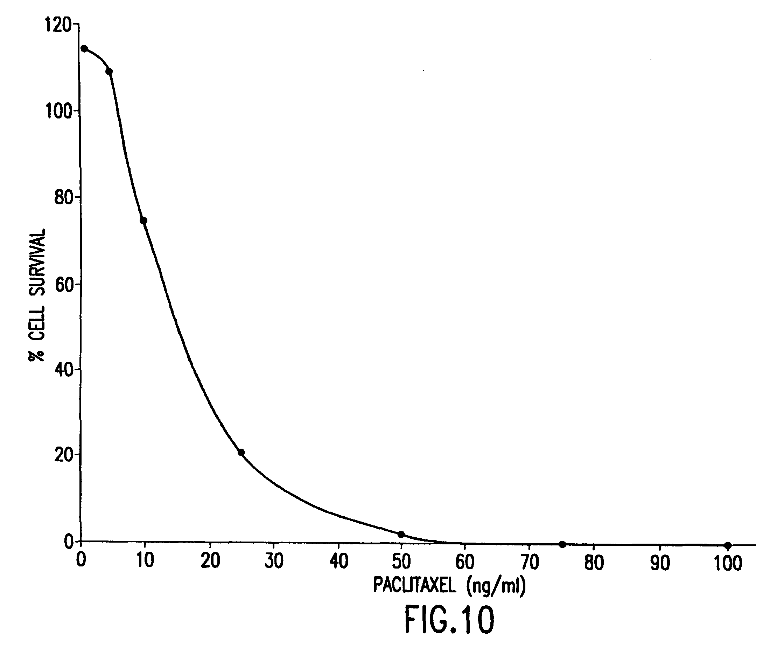

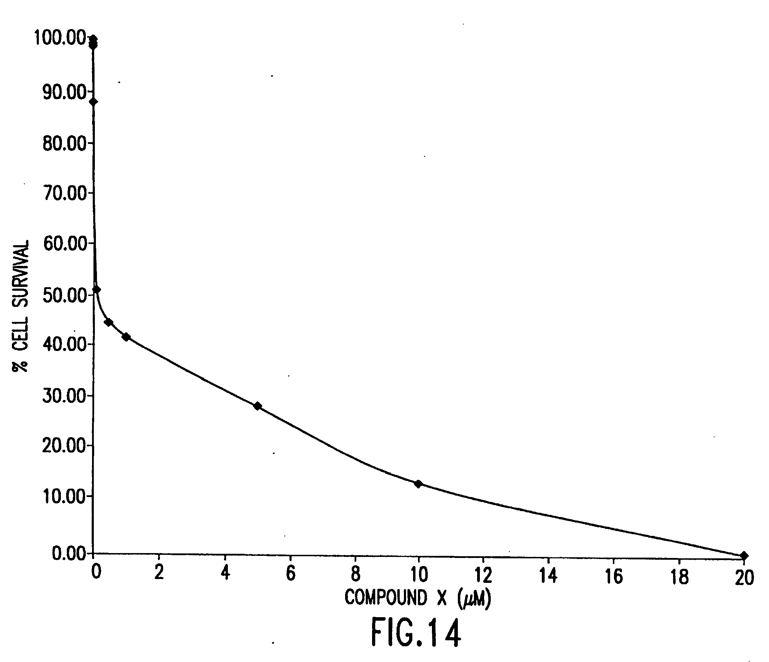

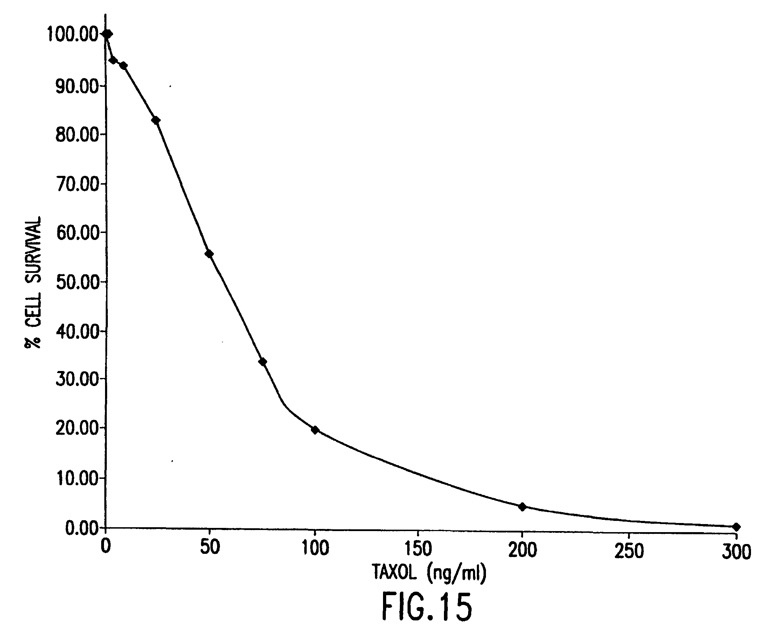

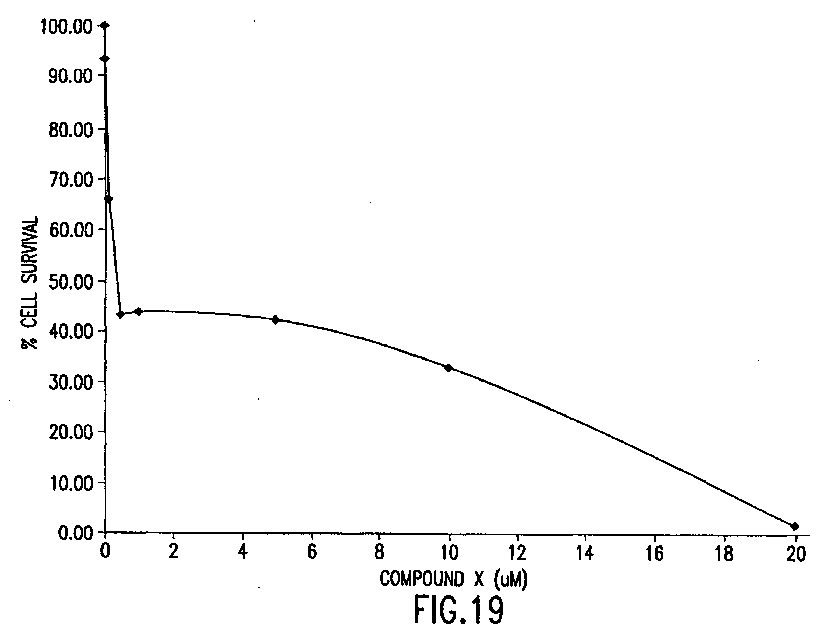

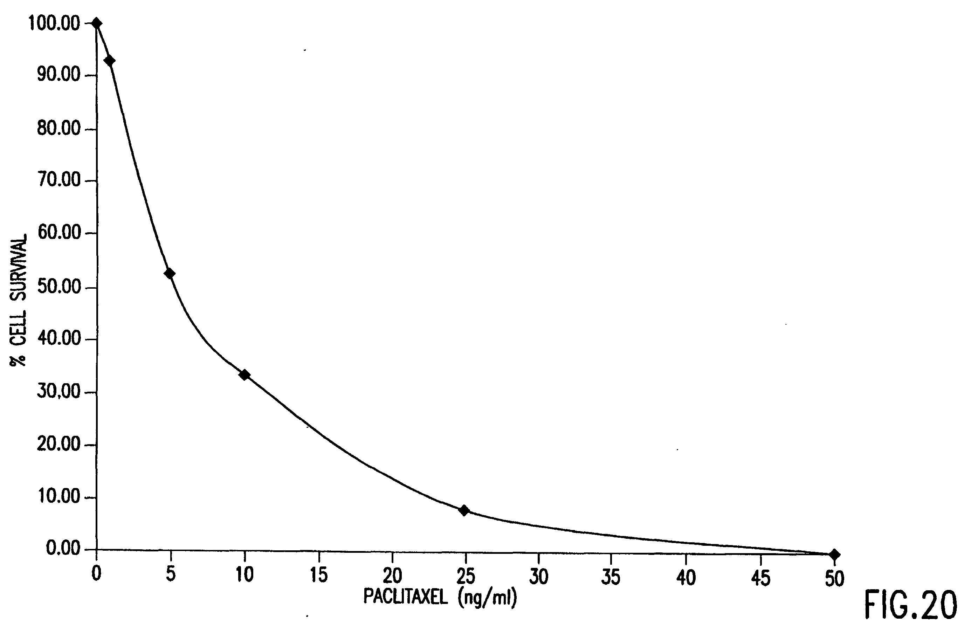

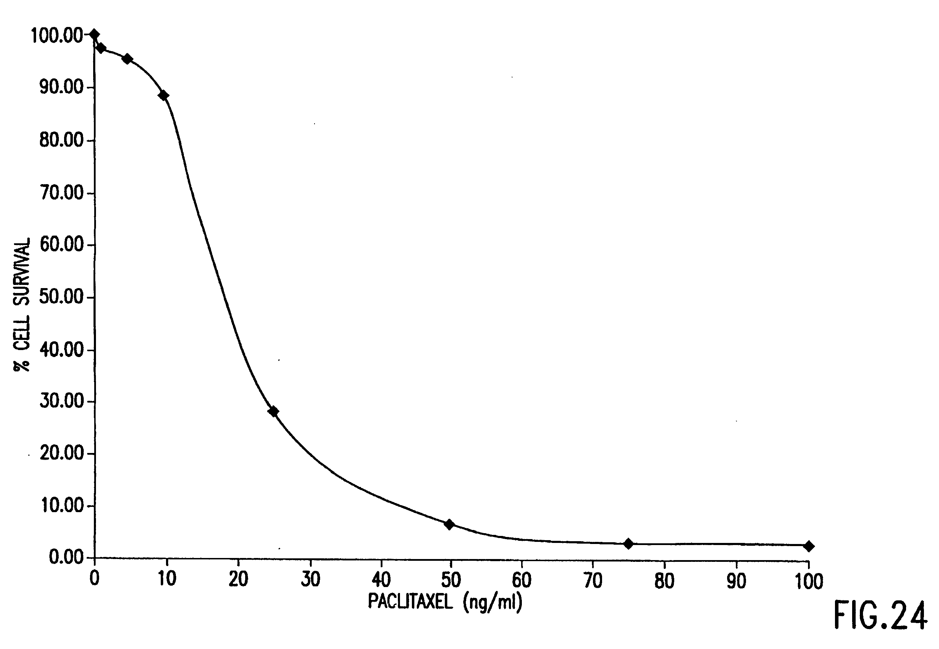

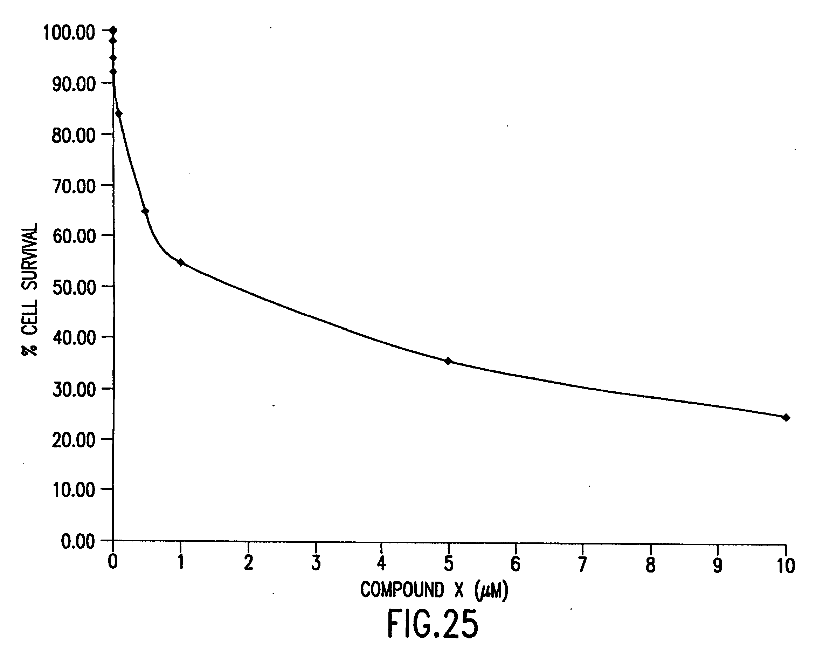

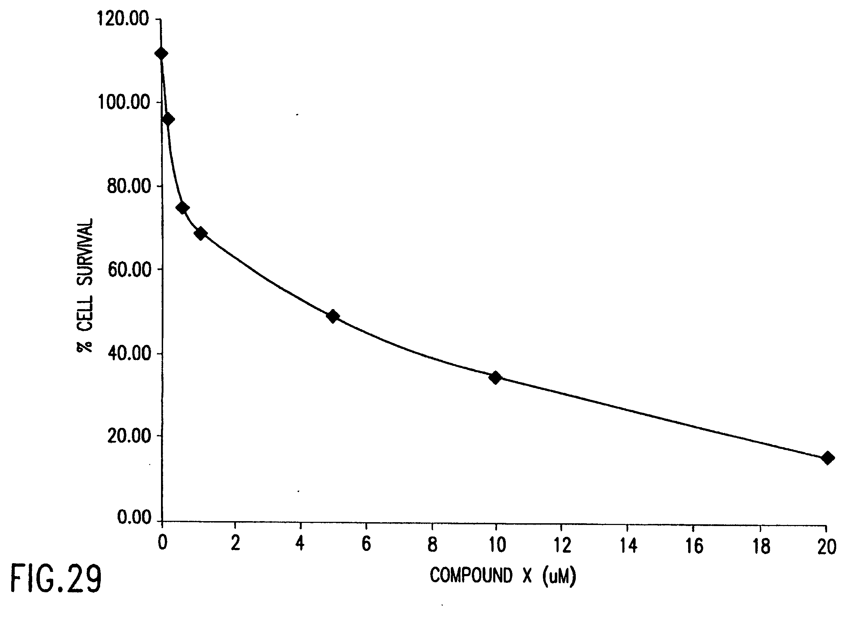

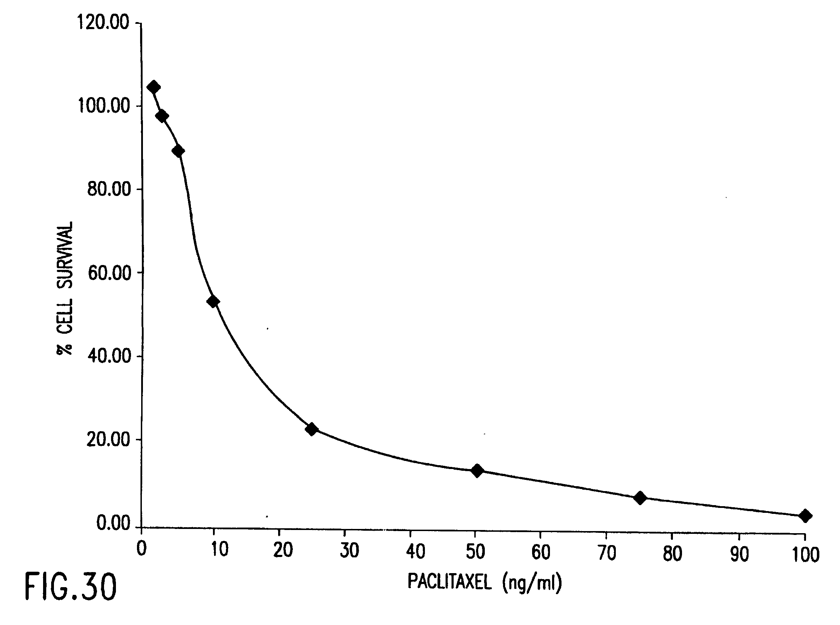

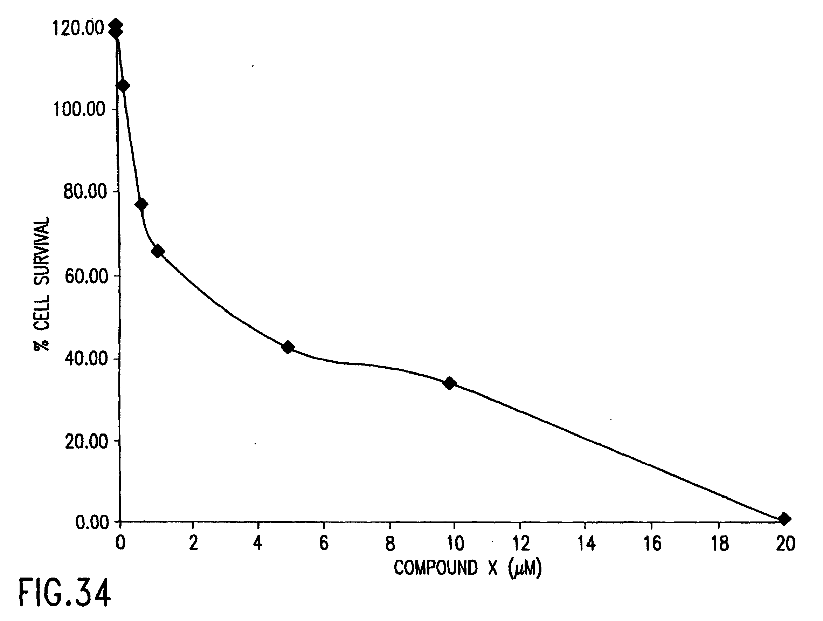

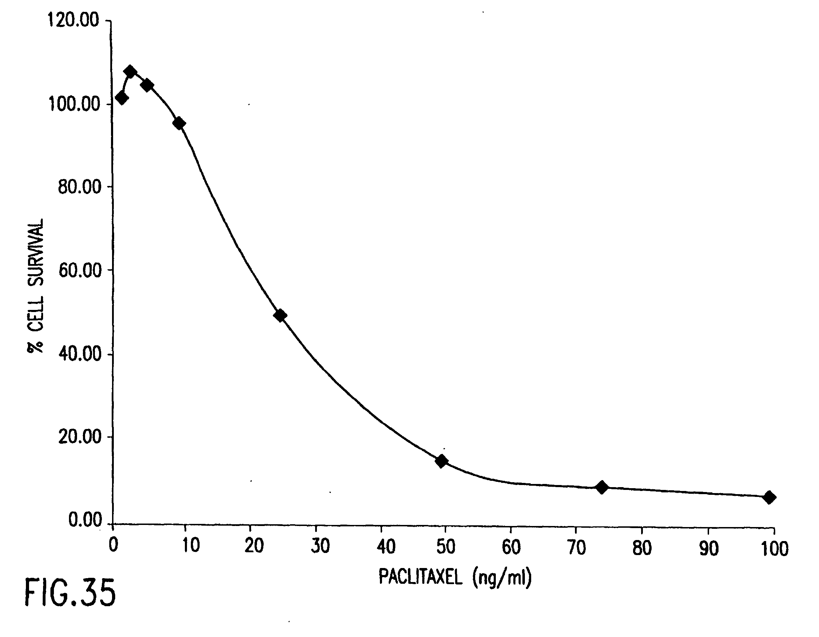

The FPT Inhibitory Compound in Figures 1 through 38 (sometimes referred to as "Compound X") is as follows:

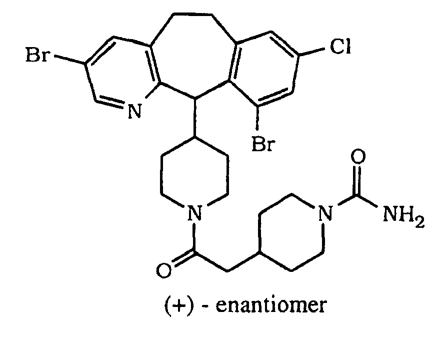

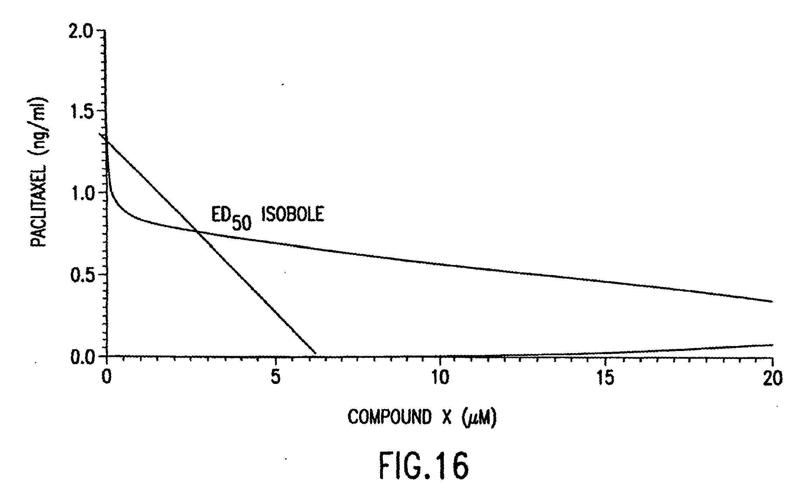

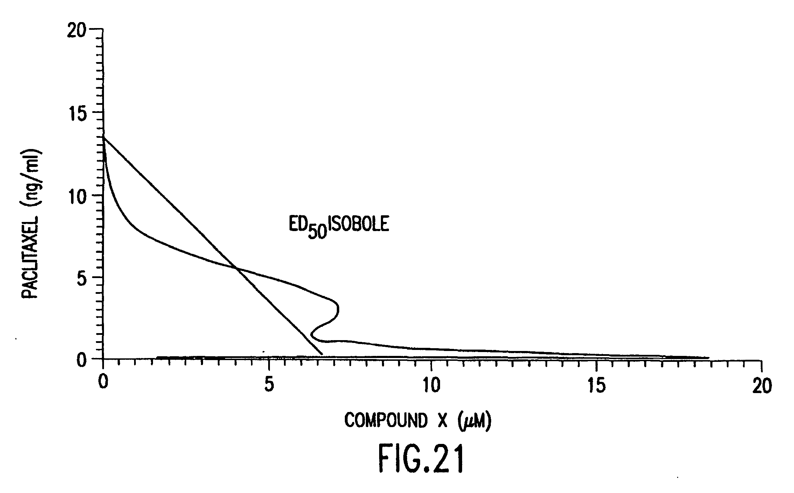

Figures 1 through 15 show three examples where clear synergy was observed. Similar results were observed in the DLD-1 colon, HTB177 lung, PA-1 ovarian, LNCaP prostate, AsPC-1 pancreatic and PANC-1 pancreatic models (data not shown). Clear Antagonism was observed in one cell line MDA-MB-231(Figs. 16-20). Mixed results were seen in MDA-MB-468 (Figs. 21-35).
More specifically, figure 1 shows an isobologram for MiaPaCa2 pancreatic tumor cells treated with an FPT inhibitory compound and paclitaxel in vitro. The x-axis indicates the amount of paclitaxel expressed as ng/ml. The y-axis indicates the amount of Compound X in µM units. When the experimental curve falls below the straight diagonal line, as is the case here, this represents a synergistic interaction. (*See*, *e*.*g*., O'Connell, M.A., and Wolfinger, R.D., J. Computational and Graphical Statistics *6*: 224-241, 1997, and Berenbaum, M.C., Pharmacol. Rev. *41*: 93-141, 1989)
Figure 2 shows a 3-dimensional cell proliferation model from which Figure 1 was derived.
Figure 3 shows a dose response curve before statistical analysis for drug interactions of paclitaxel (dosages expressed as ng/ml along the x-axis) and Compound X (dosages in µM units as indicated in the box to the right of the curves) in MiaPaCa2 cells. The y-axis indicates percentage of cell survival.
Figure 4 shows a dose response curve before statistical analysis for Compound X (dosages in µM units as indicated along the x-axis) in MiaPaCa2 cells, with the y-axis indicating percentage of cell survival.
Figure 5 shows a dose response curve before statistical analysis for paclitaxel (dosages expressed as ng/ml as indicated along the x-axis) in MiaPaCa2 cells, with the y-axis indicating percentage of cell survival.
Figure 6 shows an isobologram for DU-145 prostate tumor cells treated with an FPT inhibitory compound and paclitaxel in vitro. The x-axis indicates the amount of paclitaxel expressed as ng/ml. The y-axis indicates the amount of Compound X in µM units.
Figure 7 shows a 3-dimensional model from which figure 6 was generated.
Figure 8 shows a dose response curve before statistical analysis for drug interactions of paclitaxel (dosages expressed as ng/ml along the x-axis) and Compound X (dosages in µM units as indicated in the box to the right of the curves) in DU-145 cells. The y-axis indicates percentage of cell survival.
Figure 9 shows a dose response curve before statistical analysis for Compound X (dosages in µM units as indicated along the x-axis) in DU-145 cells, with the y-axis indicating percentage of cell survival.
Figure 10 shows a dose response curve before statistical analysis for paclitaxel (dosages expressed as ng/ml as indicated along the x-axis) in DU-145 cells, with the y-axis indicating percentage of cell survival.
Figure 11 shows an isobologram for MidT#2-1 mouse mammary tumor cells treated with an FPT inhibitory compound and paclitaxel in vitro. The x-axis indicates the amount of paclitaxel expressed as ng/ml. The y-axis indicates the amount of Compound X in µM units.
Figure 12 shows a 3-dimensional cell proliferation model from which Figure 11 is derived.
Figure 13 shows a dose response curve before statistical analysis for drug interactions of paclitaxel (dosages expressed as ng/ml along the x-axis) and Compound X (dosages in µM units as indicated in the box to the right of the curves) in MidT#2-1 cells. The y-axis indicates percentage of cell survival.
Figure 14 shows a dose response curve before statistical analysis for Compound X (dosages in µM units as indicated along the x-axis) in MidT#2-1 cells, with the y-axis indicating percentage of cell survival.
Figure 15 shows a dose response curve before statistical analysis for paclitaxel (dosages expressed as ng/ml as indicated along the x-axis) in MidT#2-1 cells, with the y-axis indicating percentage of cell survival.
Figure 16 shows an isobologram for MDA-MB-231 breast tumor cells treated with a FPT inhibitory compound and paclitaxel in vitro. The x-axis indicates the amount of paclitaxel expressed as ng/ml. The y-axis indicates the amount of Compound X in µM units.
Figure 17 shows a 3-dimensional cell proliferation model from which Figure 16 was generated.
Figure 18 shows a dose response curve before statistical analysis for drug interactions of paclitaxel (dosages expressed as ng/ml along the x-axis) and Compound X (dosages in µM units as indicated in the box to the right of the curves) in MDA-MB-231 cells. The y-axis indicates percentage of cell survival.
Figure 19 shows a dose response curve before statistical analysis for Compound X (dosages in µM units as indicated along the x-axis) in MDA-MB-231 cells, with the y-axis indicating percentage of cell survival.
Figure 20 shows a dose response curve before statistical analysis for paclitaxel (dosages expressed as ng/ml as indicated along the x-axis) in MDA-MB-231 cells, with the y-axis indicating percentage of cell survival.
Figure 21 shows an isobologram for Study #1 of MDA-MB-468 breast tumor cells treated with a FPT inhibitory compound and paclitaxel in vitro. (Example E herein). The x-axis indicates the amount of paclitaxel expressed as ng/ml. The y-axis indicates the amount of Compound X in µM units.
Figure 22 shows a 3-dimensional cell proliferation model from which Figure 21 was generated.
Figure 23 shows a dose response curve before statistical analysis for drug interactions of paclitaxel (dosages expressed as ng/ml along the x-axis) and Compound X (dosages in µM units as indicated in the box to the right of the curves) in MDA-MB-468 cells. The y-axis indicates percentage of cell survival.
Figure 24 shows a dose response curve before statistical analysis for paclitaxel (dosages expressed as ng/ml as indicated along the x-axis) in MDA-MB-468 cells, with the y-axis indicating percentage of cell survival.
Figure 25 shows a dose response curve before statistical analysis for Compound X (dosages in µM units as indicated along the x-axis) in MDA-MB-468 cells, with the y-axis indicating percentage of cell survival.
Figure 26 shows an isobologram for Study #2 of MDA-MB-468 breast tumor cells treated with a FPT inhibitory compound and paclitaxel in vitro. (Example F herein). The x-axis indicates the amount of paclitaxel expressed as ng/ml. The y-axis indicates the amount of Compound X in µM units.
Figure 27 shows a 3-dimensional cell proliferation model from which Figure 26 was generated.
Figure 28 shows a dose response curve before statistical analysis for drug interactions of paclitaxel (dosages expressed as ng/ml along the x-axis) and Compound X (dosages in µM units as indicated in the box to the right of the curves) in MDA-MB-468 cells. The y-axis indicates percentage of cell survival.
Figure 29 shows a dose response curve before statistical analysis for Compound X (dosages in µM units as indicated along the x-axis) in MDA-MB-468 cells, with the y-axis indicating percentage of cell survival.
Figure 30 shows a dose response curve before statistical analysis for paclitaxel (dosages expressed as ng/ml as indicated along the x-axis) in MDA-MB-468 cells, with the y-axis indicating percentage of cell survival.
Figure 31 shows an isobologram for Study #3 of MDA-MB-468 breast tumor cells treated with a FPT inhibitory compound and paclitaxel in vitro. (Example G herein). The x-axis indicates the amount of paclitaxel expressed as ng/ml. The y-axis indicates the amount of Compound X in µM units.
Figure 32 shows a 3-dimensional cell proliferation model from which Figure 31 was generated.
Figure 33 shows a dose response curve before statistical analysis for drug interactions of paclitaxel (dosages expressed as ng/ml along the x-axis) and Compound X (dosages in µM units as indicated in the box to the right of the curves) in MDA-MB-468 cells. The y-axis indicates percentage of cell survival.
Figure 34 shows a dose response curve before statistical analysis for Compound X (dosages in µM units as indicated along the x-axis) in MDA-MB-468 cells, with the y-axis indicating percentage of cell survival.
Figure 35 shows a dose response curve before statistical analysis for paclitaxel (dosages expressed as ng/ml as indicated along the x-axis) in MDA-MB-468 cells, with the y-axis indicating percentage of cell survival.

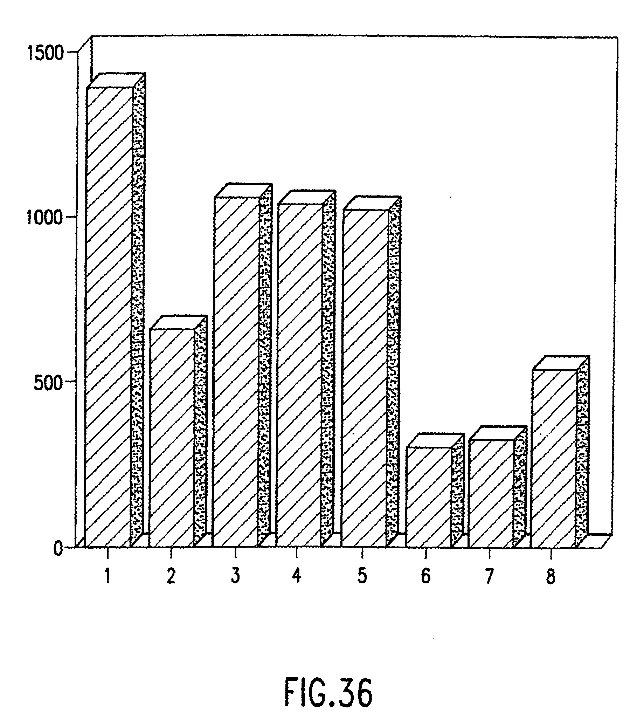

Figure 36 graphically represents the data provided in Table 2, which is set forth later on in the present specification. The Y-axis (vertical axis, numbers 0-1500) represents the tumor volume, in cubic millimeters (mm³), on day 25. The X-axis (horizontal axis, numbers 1-8) represents the substances administered. Numbers 1-8 represent: 1 is the vehicle; 2 is the FPT Inhibitory Compound (identified as "Compound X" in Table 2); 3 is Cytoxan; 4 is 5-FU; 5 is Vincristine; 6 is the FPT Inhibitory Compound plus Cytoxan; 7 is the FPT Inhibitory Compound plus 5-FU; and 8 is the FPT Inhibitory Compound plus Vincristine. Figure 36 represents experiments wherein HTB 177 cells were implanted subcutaneously in nude mice on day 0. The FPT Inhibitory Compound, dosed orally at 40 mpk, four times a day on days 1-26, resulted in 68% tumor growth inhibition. Chemo-therapeutic agents were administered intraperitoneally on day 13. When used as single agents the cytotoxics yielded 9%, 28% and 7% inhibition for Cytoxan (Cyclophosphamide) (200 mpk), 5-Fluorouracil (5-FU)(50 mpk), and Vincristine (1 mpk), respectively. When the FPT Inhibitory Compound X (40 mpk) was used in combination with the cytotoxics, tumor growth inhibition of 81%, 80% and 80% was observed for the combination with Cytoxan, 5-FU and Vincristine, respectively. These results indicate that enhanced efficacy is observed when the FPT Compound is combined with the cytotoxic chemotherapeutic agents compared to treatment with single agents. Similar results were observed when the FPT compound was dosed using a twice a day schedule. The data in Figure 36 represented by bars 6, 7 and 8 had a P value <0.05 compared to each single agent treatment.

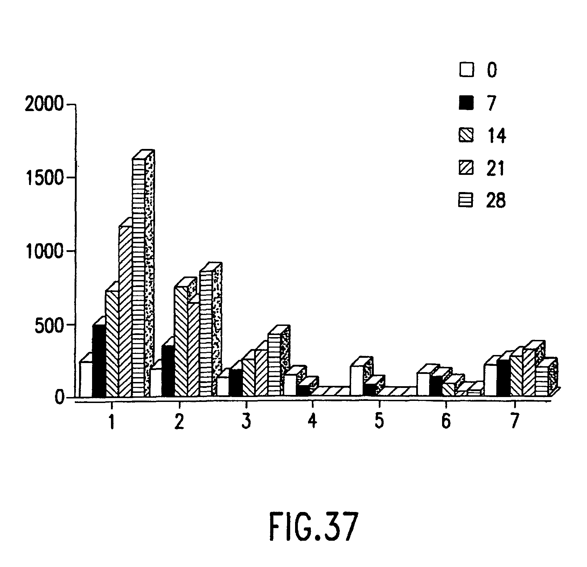

Figure 37 graphically represents tumor regression data. The Y-axis (vertical axis, numbers 0-2000) represents the tumor volume in cubic millimeters (mm³). The X-axis (horizontal axis, numbers 1-7) represents the substances administered. Numbers 1-7 represent: 1 is 20%HPBCD (vehicle); 2 is the FPT Inhibitory Compound X dosed at 2.5 mpk; 3 is the FPT Inhibitory Compound X dosed at 10 mpk; 4 is the FPT Inhibitory Compound X dosed at 20 mpk; 5 is the FPT Inhibitory Compound X dosed at 30 mpk; 6 is the FPT Inhibitory Compound X dosed at 10 mpk and Cytoxan dosed at 200 mpk; and 7 is Cytoxan dosed at 200 mpk. The number of days (0, 7, 14, 21 or 28) represented by a particular bar in the graph is indicated one of the patterns set forth in the legend in the upper right portion of Figure 37. The data in Figure 37 was obtained from the evaluation of the FPT Inhibitory compound in a transgenic mouse model in which an activated H-Ras oncogene is expressed from whey acidic promoter (Nielsen et al., Evaluation of the *wap-ras* Transgenic Mouse as a Model System for Testing Anticancer Drugs, Cancer Research 52, 3733-3738, July 1, 1992). Since the transgene is carried on the Y chromosome, male transgenics reproducibly develop tumors (breast and salivary gland) at about 1.5 to 2 months of age. This model has been run in a therapeutic mode in which treatment was initiated after mice had developed palpable tumors (mean tumor size at the start of dosing was 200 mm³). Mice were dosed orally four times a day with 2.5, 10, 20 or 30 mpk of the FPT Inhibitory Compound for 4 weeks. Some mice were also treated by weekly intraperitoneal injection with Cytoxan (Cyclophosphamide) at 200 mpk. Growth curves for the various treatment groups are shown in the bar graph in Figure 37. In the vehicle-treated control group, tumors grew throughout the course of the experiment to a volume of > 1500 mm³ by the end of the study. Cytoxan alone resulted in near complete inhibition of tumor growth but no significant tumor regression. The FPT Inhibitory Compound at the 20 and 30 mpk dose levels resulted in significant tumor regression. The FPT Inhibitory Compound at 2.5 or 10 mpk slowed the rate of tumor growth but did not result in tumor regression. Surprisingly, while Cytoxan or 10 mpk of the FPT Inhibitory Compound as single agents did not result in tumor regressions, the combination of these treatments resulted in significant tumor regression. This indicates that, when used in combination with a standard chemotherapeutic agent such as Cytoxan, greater tumor responses can be obtained at lower doses of the FPT Inhibitory Compound.
Figure 38 shows data for combination therapy (in the Wap-ras transgenic model) with Compound X and paclitaxel relative to mean tumor volume (expressed in mm³ along the y-axis) over time. The curve with white boxes represents paclitaxel alone (5 mpk), the white circles represent Compound X alone (20 mpk), the cross-hatched boxes represent the combination of paclitaxel (5 mpk) plus Compound X (20 mpk), and the black boxes represent vehicle without drug.

### DETAILED DESCRIPTION OF THE INVENTION

The present invention provides uses of the FPT inhibitor 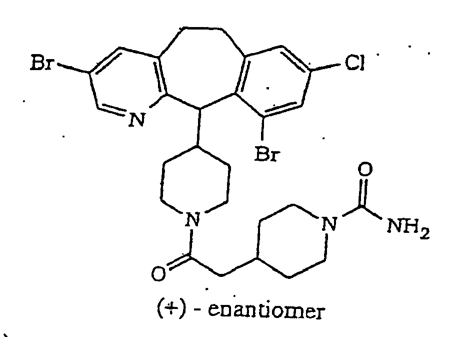
for the manufacture of a medicament for use concurrently or sequentially with an antineoplastic agent and/or radiation therapy, in the treatment of proliferative disease in a patient in need of such treatment.

FPT inhibitors are compounds which: (i) potently inhibit FPT (but preferably not geranylgeranyl protein transferase I, *in vitro*); (ii) block the phenotypic change induced by a form of transforming *H*-*ras* which is a farnesyl acceptor (but preferably not by a form of transforming *H*-*ras* engineered to be a geranylgeranyl acceptor); (iii) block intracellular farnesylation of *ras*; and (iv) block abnormal cell growth.

The use for the manufacture of a medicament for treating proliferative diseases, according to this invention, includes a use for treating (inhibiting) the abnormal growth of cells, including transformed cells, in a patient in need of such treatment (e.g., a mammal such as a human), by administering, concurrently or sequentially, an effective amount of an FPT inhibitor and an effective amount of a chemotherapeutic agent and/or radiation. Abnormal growth of cells means cell growth independent of normal regulatory mechanisms (e.g., loss of contact inhibition), including the abnormal growth of: (1) tumor cells (tumors) expressing an activated *ras* oncogene; (2) tumor cells in which the *ras* protein is activated as a result of oncogenic mutation in another gene; and (3) benign and malignant cells of other proliferative diseases.

In preferred embodiments, the uses of the present invention include uses for the manufacture of a medicament for treating or inhibiting tumor growth in a patient in need of such treatment (*e*.*g*., a mammal such as a human) by administering, concurrently or sequentially, (1) an effective amount of the FPT inhibitor and (2) an effective amount of an antineoplastic agent and/or radiation therapy. Examples of tumors which may be treated include, but are not limited to, epithelial cancers, *e.g.*, prostate cancer, lung cancer (*e*.*g*., lung adenocarcinoma), pancreatic cancers (*e.g.*, pancreatic carcinoma such as, for example, exocrine pancreatic carcinoma), breast cancers, colon cancers (*e.g*., colorectal carcinomas, such as, for example, colon adenocarcinoma and colon adenoma), ovarian cancer, and bladder carcinoma. Other cancers that can be treated include melanoma, myeloid leukemias (for example, acute myelogenous leukemia), sarcomas, thyroid follicular cancer, and myelodysplastic syndrome.

The uses for the manufacture of a medicament for treating proliferative diseases, according to this invention; also include a use for treating (inhibiting) proliferative diseases, both benign and malignant, wherein *ras* proteins are aberrantly activated as a result of oncogenic mutation in other genes - *i*.*e*., the *ras* gene itself is not activated by mutation to an oncogenic form. This use comprises administering, concurrently or sequentially, an effective amount of an FPT inhibitor and an effective amount of an antineoplastic agent (and/or radiation therapy) to a patient in need of such treatment (*e.g.,* a mammal such as a human). Examples of such proliferative diseases which may be treated include: the benign proliferative disorder neurofibromatosis, or tumors in which *ras* is activated due to mutation or overexpression of tyrosine kinase oncogenes (*e.g*., neu, src, abl, lck, lyn, fyn).

As used herein the following terms have the following meanings unless indicated otherwise:
antineoplastic agent - a chemotherapeutic agent effective against cancer;
concurrently - (1) simultaneously in time, or (2) at different times during the course of a common treatment schedule; and
sequentially - (1) administration of one component of the method ((a) FPT inhibitor, or (b) antineoplastic agent and/or radiation therapy) followed by administration of the other component; after adminsitration of one component, the second component can be administered substantially immediately after the first component, or the second component can be administered after an effective time period after the first component; the effective time period is the amount of time given for realization of maximum benefit from the administration of the first component.

### CHEMOTHERAPEUTIC AGENTS

Classes of compounds that can be used as the chemotherapeutic agent (antineoplastic agent) include: alkylating agents, antimetabolites, natural products and their derivatives, hormones and steroids (including synthetic analogs), and synthetics. Examples of compounds within these classes are given below.

Alkylating agents (including nitrogen mustards, ethylenimine derivatives, alkyl sulfonates, nitrosoureas and triazenes): Uracil mustard, Chlormethine, Cyclophosphamide (Cytoxan®), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide.

Antimetabolites (including folic acid antagonists, pyrimidine analogs, purine analogs and adenosine deaminase inhibitors): Methotrexate, 5-Fluorouracil, Floxuridine, Cytarabine, 6-Mercaptopurine, 6-Thioguanine, Fludarabine phosphate, Pentostatine, and Gemcitabine.

Natural products and their derivatives (including vinca alkaloids, antitumor antibiotics, enzymes, lymphokines and epipodophyllotoxins): Vinblastine, Vincristine, Vindesine, Bleomycin, Dactinomycin, Daunorubicin, Doxorubicin, Epirubicin, Idarubicin, paclitaxel (paclitaxel is commercially available as Taxol® and is described in more detail below in the subsection entitled "Microtubule Affecting Agents"), Mithramycin, Deoxycoformycin, Mitomycin-C, L-Asparaginase, Interferons (especially IFN-a), Etoposide, and Teniposide.

Hormones and steroids (including synthetic analogs): 17α-Ethinylestradiol, Diethylstilbestrol, Testosterone. Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, Zoladex.

Synthetics (including inorganic complexes such as platinum coordination complexes): Cisplatin, Carboplatin, Hydroxyurea, Amsacrine, Procarbazine, Mitotane, Mitoxantrone, Levamisole, and Hexamethylmelamine.

Methods for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, NJ 07645-1742, USA).

### MICROTUBULE AFFECTING AGENTS

As explained above, the present invention also provides methods of treating diseased cells by contacting the cells with a FPT inhibitor and a microtubule affecting agent (*e.g.*, paclitaxel, a paclitaxel derivative or a paclitaxel-like compound). As used herein, a microtubule affecting agent is a compound that interferes with cellular mitosis, *i*.*e*., having an anti-mitotic effect, by affecting microtubule formation and/or action. Such agents can be, for instance, microtubule stabilizing agents or agents which disrupt microtubule formation.

Microtubule affecting agents useful in the invention are well known to those of skill in the art and include allocolchicine (NSC 406042), Halichondrin B (NSC 609395), colchicine (NSC 757), colchicine derivatives (e.g., NSC 33410), dolastatin 10 (NSC 376128), maytansine (NSC 153858), rhizoxin (NSC 332598), paclitaxel (Taxol®, NSC 125973), Taxol® derivatives (*e.g*., derivatives (*e.g*., NSC 608832), thiocolchicine (NSC 361792), trityl cysteine (NSC 83265), vinblastine sulfate (NSC 49842), vincristine sulfate (NSC 67574), epothilone A, epothilone, and discodermolide (*see* Service, (1996) *Science.* 274:2009) estramustine, nocodazole, MAP4. Examples of such agents are also described in the scientific and patent literature, see, *e.g.,* Bulinski (1997) *J. Cell Sci*. 110:3055-3064; Panda (1997) *Proc. Natl. Acad. Sci.* USA 94:10560-10564; Muhlradt (1997) Cancer Res. 57:3344-3346; Nicolaou (1997) *Nature* 387:268-272; Vasquez (1997) *Mol. Biol. Cell.* 8:973-985; Panda (1996) *J*. *Biol.* Chem. 271:29807-29812.

Particularly preferred agents are compounds with paclitaxel-like activity. These include paclitaxel and paclitaxel derivatives (paclitaxel-like compounds) and analogues. Paclitaxel and its derivatives are available commercially. In addition, methods of making paclitaxel and paclitaxel derivatives and analogues are well known to those of skill in the art (*see, e.g.,* U.S. Patent Nos: 5,569,729; 5,565,478; 5,530,020; 5,527,924; 5,508,447; 5,489,589; 5,488,116; 5,484,809; 5,478,854; 5,478,736; 5,475,120; 5,468,769; 5,461,169; 5,440,057; 5,422,364; 5,411,984; 5,405,972; and 5,296,506).

More specifically, the term "paclitaxel" as used herein refers to the drug commercially available as Taxol® (NSC number: 125973). Taxol® inhibits eukaryotic cell replication by enhancing polymerization of tubulin moieties into stabilized microtubule bundles that are unable to reorganize into the proper structures for mitosis. Of the many available chemotherapeutic drugs, paclitaxel has generated interest because of its efficacy in clinical trials against drug-refractory tumors, including ovarian and mammary gland tumors (Hawkins (1992) *Oncology,* 6: 17-23, Horwitz (1992) *Trends Pharmacol. Sci*. 13: 134-146. Rowinsky (1990) *J. Natl. Canc. Inst*. 82: 1247-1259).

Additional microtubule affecting agents can be assessed using one of many such assays known in the art, *e.g*., a semiautomated assay which measures the tubulin-polymerizing activity of paclitaxel analogs in combination with a cellular assay to measure the potential of these compounds to block cells in mitosis (see *Lopes* (1997) *Cancer Chemother. Pharmacol.* 41:37-47).

Generally, activity of a test compound is determined by contacting a cell with that compound and determining whether or not the cell cycle is disrupted, in particular, through the inhibition of a mitotic event. Such inhibition may be mediated by disruption of the mitotic apparatus, *e*.*g*., disruption of normal spindle formation. Cells in which mitosis is interrupted may be characterized by altered morphology (*e.g*., microtubule compaction, increased chromosome number, etc.).

In a preferred embodiment, compounds with possible tubulin polymerization activity are screened in vitro. In a preferred embodiment, the compounds are screened against cultured WR21 cells (derived from line 69-2 wap-ras mice) for inhibition of proliferation and/or for altered cellular morphology, in particular for microtubule compaction. *In vivo* screening of positive-testing compounds can then be performed using nude mice bearing the WR21 tumor cells. Detailed protocols for this screening method are described by Porter (1995) *Lab. Anim. Sci*., 45(2):145-150.

Other methods of screening compounds for desired activity are well known to those of skill in the art. Typically such assays involve assays for inhibition of microtubule assembly and/or disassembly. Assays for microtubule assembly are described, for example, by Gaskin *et al*. (1974) *J*. *Molec. Biol*., 89: 737-758. U.S. Patent No. 5,569,720 also provides *in vitro* and *in vivo* assays for compounds with paclitaxel-like activity.

Methods for the safe and effective administration of the above-mentioned microtubule affecting agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company. Montvale, NJ 07645-1742, USA).

### FPT INHIBITORS

Generally, classes of compounds that can be used as FPT inhibitor include: oligopeptides, peptido-mimetic compounds, farnesylated peptido-mimetic compounds, fused-ringed tricyclic benzocycloheptapyridines, carbonyl piperazinyl compounds, carbonyl piperidinyl compounds, farnesyl derivatives, and natural products and derivatives.

Some of the compounds are oligopeptides, especially tetrapeptides, or derivatives thereof, based on the formula Cys-Xaa₁-Xaa₂-Xaa₃, where Xaa₃ represents a serine, methionine or glutamine residue, and Xaa₁ and Xaa₂ can represent a wide variety of amino acid residues, but especially those with an aliphatic side-chain. Their derivatives may or may not have three peptide bonds; thus it has been found that reduction of a peptide bond -CO-NH- to a secondary amine grouping, or even replacement of the nitrogen atoms in the peptide chain with carbon atoms (provided that certain factors such as general shape of the molecule and separation of the ends are largely conserved) affords compounds that are frequently more stable than the oligopeptides and, if active, have longer activity. Such compounds are referred to herein as peptido-mimetic compounds.

Examples of compounds that are FPT inhibitors and the documents directed to those compounds are given below.

Oligopeptides (mostly tetrapeptides but also pentapeptides) including the formula Cys-Xaa₁-Xaa₂-Xaa₃: EPA 461,489; EPA 520,823; EPA 528,486; and WO 95/11917.

Peptido-mimetic compounds - especially Cys-Xaa-Xaa-Xaa-mimetics: EPA 535,730; EPA 535,731; EPA 618,221; WO 94/09766; WO 94/10138; WO 94/07966; US 5,326,773; US 5,340,828; US 5,420,245; WO 95/20396; US 5,439,918; and WO 95/20396.

Farnesylated peptido-mimetic compounds - specifically farnesylated Cys-Xaa-Xaa-Xaa-mimetic: GB-A 2,276,618.

Other peptido-mimetic compounds: US 5,352,705; WO 94/00419; WO 95/00497; WO 95/09000; WO 95/09001; WO 95/12612; WO 95/25086; EPA 675, 112; and FR-A 2,718,149.

With regard to the use of peptido-mimetic compounds as FPT inhibitors, it is noted that data based on combining a particular peptido-mimetic FTI compound with several different chemotherapeutic agents (*e*.*g*., taxol (paclitaxel), doxorubicin, cisplatin, and vinblastine) in breast cancer cell lines MDA-MB-468 and MCF-7 are presented in Moasser, MM, *et al.* Proc. Natl. Acad. Sci. USA *95:* 1369-1374, 1998. (See also Kohl *et al,* Nature Medicine *1*: 792 (1995) for identification of the subject compound as a peptido-mimetic compound). Fused-ring tricyclic benzocycloheptapyridines: WO 95/10514; WO 95/10515; WO 95/10516; WO 96/30363; WO 96/30018; WO 96/30017; WO 96/30362; WO 96/31111; WO 96/31478; WO 96/31477; WO 96/31505; WO 97/23478; International Patent Application No. PCT/US97/ 17314 (WO 98/ 15556); International Patent Application No. PCT/US97/15899 (WO 98/11092); International Patent Application No. PCT/US97/ 15900 (WO 98/11096); International Patent Application No. PCT/US97/ 15801 (WO 98/11106); International Patent Application No. PCT/US97/15902 (WO 98/11097); International Patent Application No. PCT/US97/ 15903 (WO 98/11098); International Patent Application No. PCT/US97/15904; International Patent Application No. PCT/US97/15905 (WO 98/11099); International Patent Application No. PCT/US97/ 15906 (WO 98/11100); International Patent Application No. PCT/US97/ 15907 (WO 98/11093); International Patent Application No. PCT/US97/ 19976 (WO 98/11091); U.S. Application Serial No. 08/877049; U.S. Application Serial No. 08/877366; U.S. Application Serial No. 08/877399; U.S. Application Serial No. 08/877336; U.S. Application Serial No. 08/877269; U.S. Application Serial No. 08/877050; U.S. Application Serial No. 08/877052; U.S. Application Serial No. 08/877051; U.S. Application Serial No. 08/877498; U.S. Application Serial No. 08/877057; U.S. Application Serial No. 08/877739; U.S. Application Serial No. 08/877677; U.S. Application Serial No. 08/877741; U.S. Application Serial No. 08/877743; U.S. Application Serial No. 08/877457; U.S. Application Serial No. 08/877673; and U.S. Application Serial No. 08/876507.

Farnesyl derivatives: EPA 534,546; WO 94/19357; WO 95/08546; EPA 537,007; and WO 95/13059.

Natural products and derivatives: WO 94/18157; US 5,430,055; GB-A 2,261,373; GB-A 2,261,374, GB-A 2,261,375; US 5,420,334; US 5,436,263.

Other compounds: WO 94/26723; WO 95/08542; US 5,420,157; WO 95/21815; WO 96/31501; WO 97/16443; WO 97/21701; U.S. 5,578,629; U.S. 5,627,202; WO 96/39137: WO 97/18813; WO 97/27752WO 97/27852; WO 97/27853: WO 97/27854; WO 97/36587; WO 97/36901; WO 97/36900; WO 97/36898; WO 97/36897; WO 97/36896; WO 97/36892; WO 97/36891; WO 97/36890; WO 97/36889; WO 97/36888; WO 97/36886; WO 97/36881; WO 97/36879; WO 97/36877; WO 97/36876; WO 97/36875; WO 97/36605; WO 97/36593; WO 97/36592; WO 97/36591; WO 97/36585; WO 97/36584; and WO 97/36583.

A plasmid encoding an α- and a β-unit of an FPT, and describing an assay therefor: WO 94/10184.

A review of many such compounds is given by Graham in *Exp. Opin. Ther. Patents* (1995) 5(12): 1269-1285.

It will be understood that the breadth of a chemical formula in a patent specification may not enable one to classify all compounds therein under one of the headings above. For example, the monoterpenyl chain in the farnesyl derivatives may be extended, e.g. by a number of methylene groups or even another isoprene residue.

The tetrapeptides of the formula Cys-Xaa₁-Xaa₂-Xaa₃ have an amino-terminal cysteine residue. A tetrapeptide of that type forms the carboxyl-terminal of *ras*. Such tetrapeptides are capable of binding with FPT and competing with *ras*. Compounds of similar structure but having at least one of the carbonyl groups of the tetrapeptide replaced by a hydrocarbyl group such as a methylene group and classified above as peptido-mimetic compounds are also capable of binding with FPT and competing with *ras*, but are generally more resistant to enzymatic degradation *in vivo*.

### FPT INHIBITORS-EXEMPLIFIED COMPOUNDS

The following documents disclose compounds that are FPT inhibitors. The documents also disclose methods of inhibiting abnormal cell growth (e.g., tumors) using the compounds disclosed in the document. The radicals and formulae designations defined herein for a particular document apply only to the compounds described in that document.

WO 95/10516 published April 20, 1995 and WO 96/30363 published October 3, 1996 disclose compounds of formula 1.0: 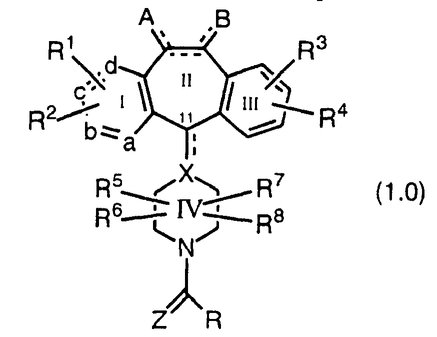
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d is independently selected from CR¹ and C_{R}2;
each R¹ and each R² is independently selected from H, halo, -CF₃, -OR¹⁰, -COR¹⁰, -SR¹⁰, -S(O)ₜR¹ (wherein t is 0, 1 or 2), -SCN, -N(R¹⁰)₂, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, -SR¹¹C(O)OR¹¹, 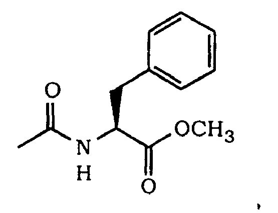
   -SR¹¹N(R⁷⁵)₂ (wherein each R⁷⁵ is independently selected from H and -C(O)OR¹¹), benzotriazol-1-yloxy, tetrazol-5-ylthio, substituted tetrazol-5-ylthio, alkynyl, alkenyl and alkyl, said alkyl or alkenyl group optionally being substituted with halo, -OR¹⁰ or -CO₂R¹⁰;
R³ and R⁴ are the same or different and each independently represents H or any of the substituents of R¹ and R², or R³ and R⁴ taken together represent a saturated or unsaturated C₅-C₇ ring fused to the benzene ring;
each of R⁵, R⁶, R⁷ and R⁸ independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, or OPO₃R¹⁰, or one of R⁵, R6, R⁷ and R⁸ can be taken in combination with R⁴⁰ as defined below to represent -(CH₂)ᵣ - wherein r is 1 to 4 which can be substituted with lower alkyl, lower alkoxy, -CF₃ or aryl, or R⁵ is combined with R⁶ to represent =O or =S and/or R⁷ is combined with R⁸ to represent =O or =S;
R¹⁰ represents H, alkyl, aryl, or aralkyl;
R¹¹ represents alkyl or aryl;
X represents N, CH or C, which C may contain an optional double bond, represented by the dotted line, to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, each of A and B independently represents H₂, -(OR¹¹)₂, (H and halo), dihalo, (alkyl and H), (alkyl)₂, (H and -OC(O)R¹⁰), (H and -OR¹⁰), =O, (aryl and H), =NOR¹⁰, or -O-(CH₂)ₚ-O- wherein p is 2, 3 or 4;
R represents R⁴⁰, R⁴², R⁴⁴, or R⁵⁴, as defined below;
R⁴⁰ represents H, aryl, alkyl, cycloalkyl, alkenyl, alkynyl or -D wherein -D represents 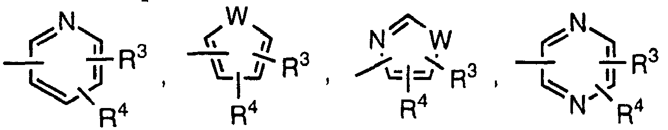 or 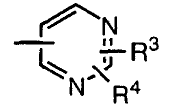 wherein R³ and R⁴ are as previously defined and W is O, S or NR¹⁰ wherein R¹⁰ is as defined above; said R⁴⁰ cycloalkyl, alkenyl and alkynyl groups being optionally substituted with from 1-3 groups selected from halo, -CON(R¹⁰)₂, aryl, -CO₂R¹⁰, -OR¹², -SR¹², -N(R¹⁰)₂, -N(R¹⁰)CO₂R¹¹, -COR¹², -NO₂ or D, wherein -D, R¹⁰ and R¹¹ are as defined above and R¹² represents R¹⁰, -(CH₂)ₘOR¹⁰ or -(CH₂)_{q}CO₂R¹⁰ wherein R¹⁰ is as previously defined, m is 1 to 4 and q is 0 to 4; said alkenyl and alkynyl R⁴⁰ groups not containing -OH, -SH or -N(R¹⁰)₂ on a carbon containing a double or triple bond respectively; or
R⁴⁰ represents phenyl substituted with a group selected from -SO₂NH₂, -NHSO₂CH₃, -SO₂NHCH₃, -SO₂CH₃, -SOCH₃, -SCH₃, and -NHSO₂CF₃, which group is preferably located in the para position of the phenyl ring; or
R⁴⁰ represents a group selected from 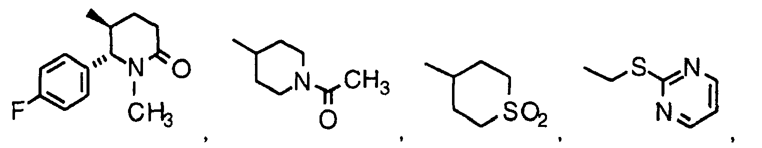 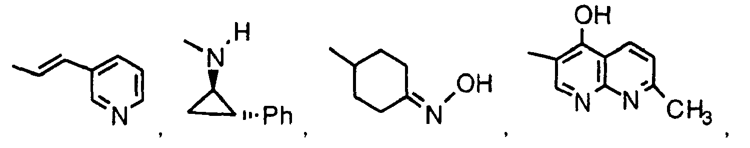 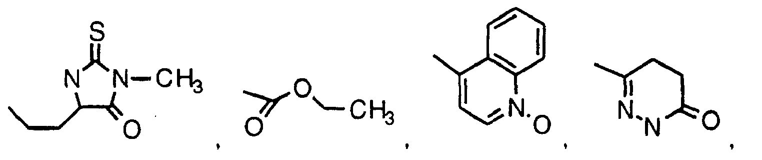 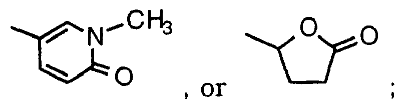
R⁴² represents 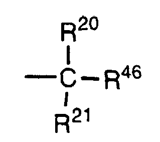
   wherein R²⁰, R²¹ and R⁴⁶ are each independently selected from the group consisting of:
   (1) H;
   (2) -(CH₂)_{q}SC(O)CH₃ wherein q is 1 to 3;
   (3) -(CH₂)_{q}OSO₂CH₃ wherein q is 1 to 3;
   (4) -OH;
   (5) -CS-(CH₂)_{w}-(substituted phenyl) wherein w is 1 to 3 and the substitutents on said substituted phenyl group are the same substitutents as described under (12) below for substituted phenyl;
   (6) -NH₂;
   (7) -NHCBZ;
   (8) -NHC(O)OR²² wherein R²² is an alkyl group having from 1 to 5 carbon atoms, or R²² represents phenyl substituted with 1 to 3 alkyl groups;
   (9) alkyl;
   (10) -(CH₂)ₖ-phenyl wherein k is 1 to 6;
   (11) phenyl;
   (12) substituted phenyl wherein the substituents are selected from the group consisting of: halo, NO₂, -OH, -OCH₃, -NH₂, -NHR²², -N(R²²)₂, alkyl, -O(CH₂)ₜ-phenyl (wherein t is from 1 to 3), and -O(CH₂)ₜ-substituted phenyl (wherein t is from 1 to 3);
   (13) naphthyl;
   (14) substituted naphthyl, wherein the substituents are as defined for substituted phenyl under (12) above;
   (15) bridged polycyclic hydrocarbons having from 5 to 10 carbon atoms;
   (16) cycloalkyl having from 5 to 7 carbon atoms;
   (17) heteroaryl;
   (18) hydroxyalkyl;
   (19) substituted pyridyl or substituted pyridyl N-oxide wherein the substituents are selected from methylpyridyl, morpholinyl, imidazolyl, 1-piperidinyl, 1-(4-methylpiperazinyl), -S(O)ₜR¹¹, and any of the substituents given under (12) above for substituted phenyl, and said substitutents are bound to a ring carbon by replacement of the hydrogen bound to said carbon; 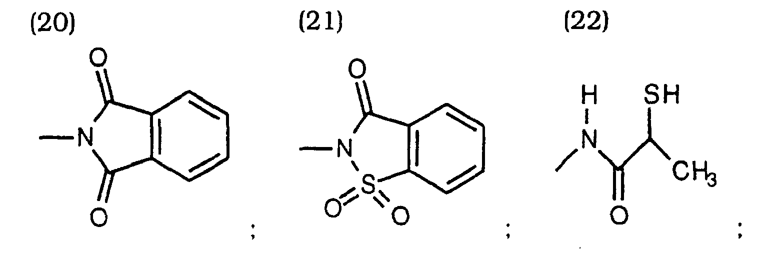
   (23) -NHC(O)-(CH₂)ₖ-phenyl or -NH(O)-(CH₂)ₖ₋(substituted phenyl), wherein said k is as defined under (10) above;
   (24) piperidine Ring V: 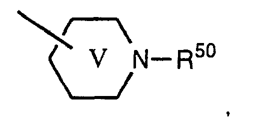
      wherein R⁵⁰ represents H, alkyl, alkylcarbonyl, alkoxycarbonyl, haloalkyl, or -C(O)NH(R¹⁰) wherein R¹⁰ is H or alkyl;
   (25) -NHC(O)CH₂C₆H₅ or -NHC(O)CH₂-(substituted C₆H₅);
   (26) -NHC(O)OC₆H₅; 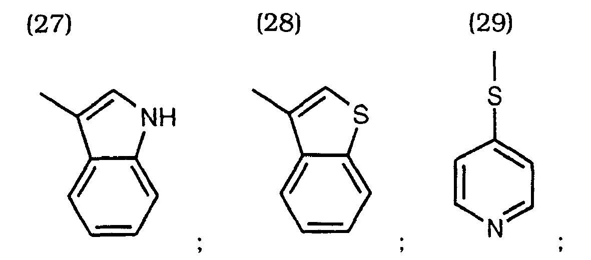
   (30) -OC(O)-heteroaryl (for example pyridine-4-carbonyloxy) ;
   (31) -O-alkyl (e.g., -OCH₃);
   (32) -CF₃;
   (33) -CN;
   (34) a heterocycloalkyl group of the formula 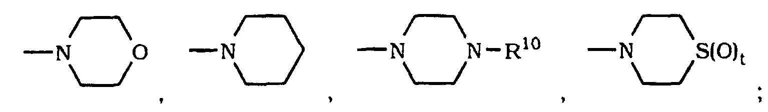 and
   (35) a piperidinyl group of the formula

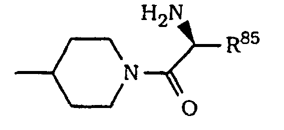

   wherein R⁸⁵ is H, alkyl, or alkyl substituted by -OH or -SCH₃; or
R²⁰ and R²¹ taken together form an =O group and the remaining R⁴⁶ is as defined above; or
two of R²⁰, R²¹ and R⁴⁶ taken together form piperidine Ring V 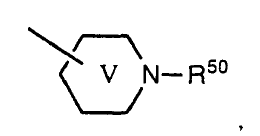
   wherein R⁵⁰ is as defined under (24) above;
with the proviso that R⁴⁶, R²⁰ and R²¹ are selected such that the carbon atom to which they are bound is not bonded to more than one heteroatom;
R⁴⁴ represents -NR²⁵R⁴⁸ wherein R²⁵ represents heteroaryl, N-methylpiperidinyl or aryl, and R⁴⁸ represents H or alkyl;
R⁵⁴ represents an N-oxide heterocyclic group of the formula (i), (ii), (iii) or (iv): 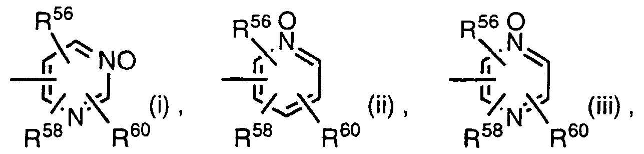 or 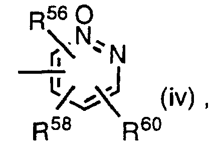 wherein R⁵⁶, R⁵⁸, and R⁶⁰ are the same or different and each is independently selected from H, halo, -CF₃, -OR¹⁰, -C(O)R¹⁰, -SR¹⁰, -S(O)ₑR¹¹ (wherein e is 1 or 2), -N(R¹⁰)₂, -NO₂, -CO₂R¹⁰, -OCO₂R¹¹, -OCOR¹⁰, alkyl, aryl, alkenyl and alkynyl, which alkyl may be substituted with -OR¹⁰, -SR¹⁰ or -N(R¹⁰)₂ and which alkenyl may be substituted with OR¹¹ or SR¹¹; or
R⁵⁴ represents an N-oxide heterocyclic group of the formula (ia), (iia), (iiia) or (iva): 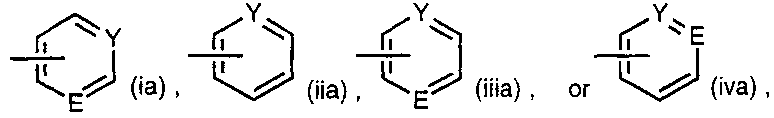
   wherein Y represents N⁺-O⁻ and E represents N; or
R⁵⁴ represents an alkyl group substituted with one of said N-oxide heterocyclic groups (i), (ii), (iii), (iv), (ia), (iia), (iiia) or (iva); and
Z represents O or S such that R can be taken in combination with R⁵, R⁶, R⁷ or R⁸ as defined above, or R represents R⁴⁰, R⁴², R⁴⁴ or R⁵⁴.

WO 95/10516 and WO 96/30363 also disclose compounds of the formulas: 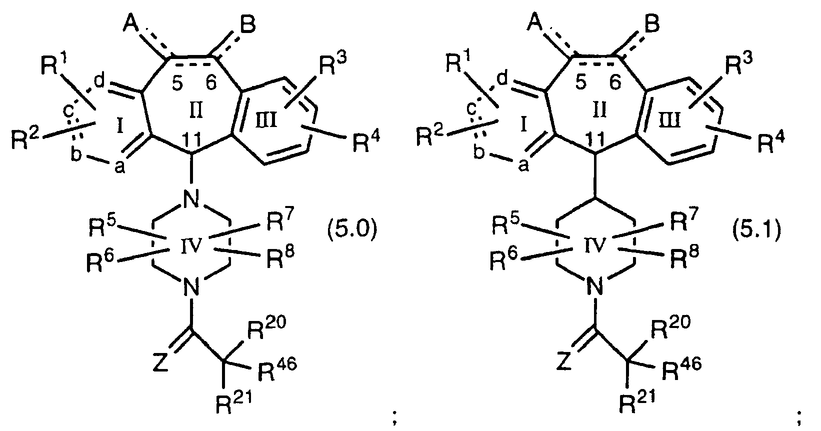 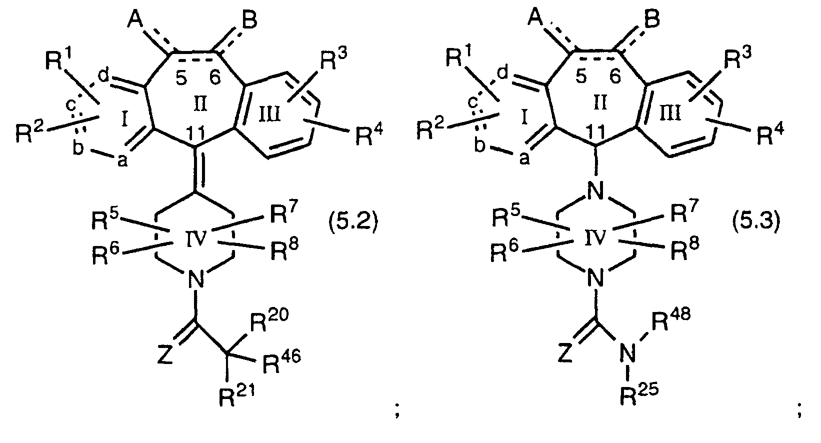 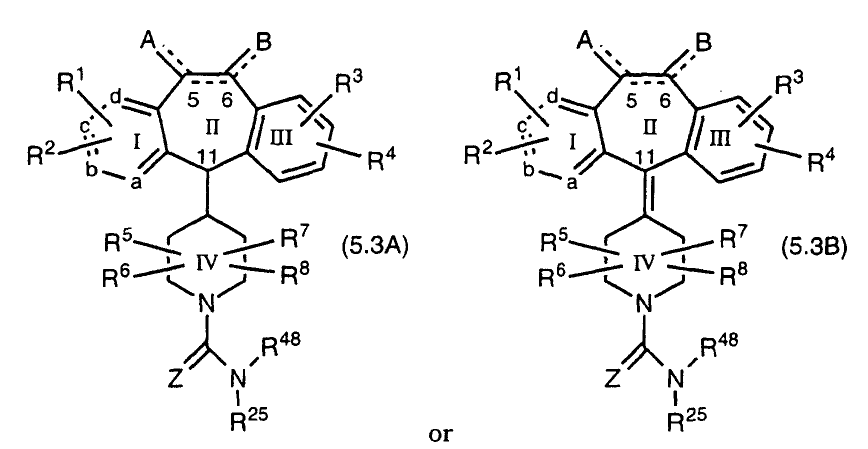
or a pharmaceutically acceptable salt or solvate thereof, wherein all the substituents are as defined for formula 1.0 of WO 95/10516 and WO 96/30363, and wherein for the compounds of Formula 5.2 the substituents R²⁰, R²¹, and R⁴⁶ are selected such that when one of said substituents R²⁰, R²¹, and R⁴⁶ is selected from the group consisting of: (1) H, (2) -OH, (3) -NH₂, (4) -NHC(O)OR²², (5) alkyl, (6) phenyl, (7) heteroaryl, (8) hydroxyalkyl, (9) substituted pyridyl, (10) substituted phenyl and (11) -O-alkyl, then the remaining two of said substituents R²⁰, R²¹ and R⁴⁶ cannot both be H when: (a) R¹ and R² are both H, and (b) the double bond between C-5 and C-6 is absent, and (c) both A and B are H₂, and (d) R⁴ is H, and (e) R³ is H or Cl at C-8.

WO 96/30363 also disclose the compounds: 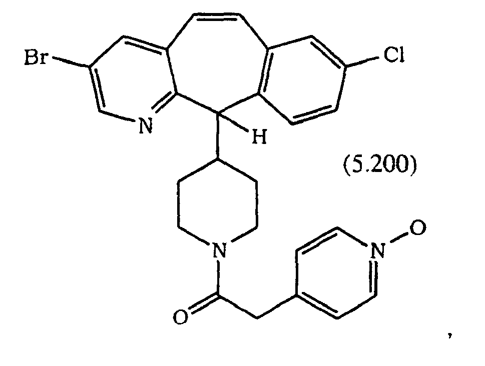 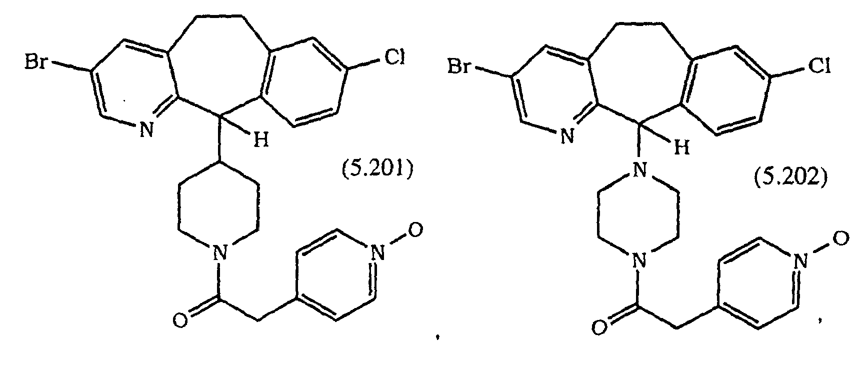 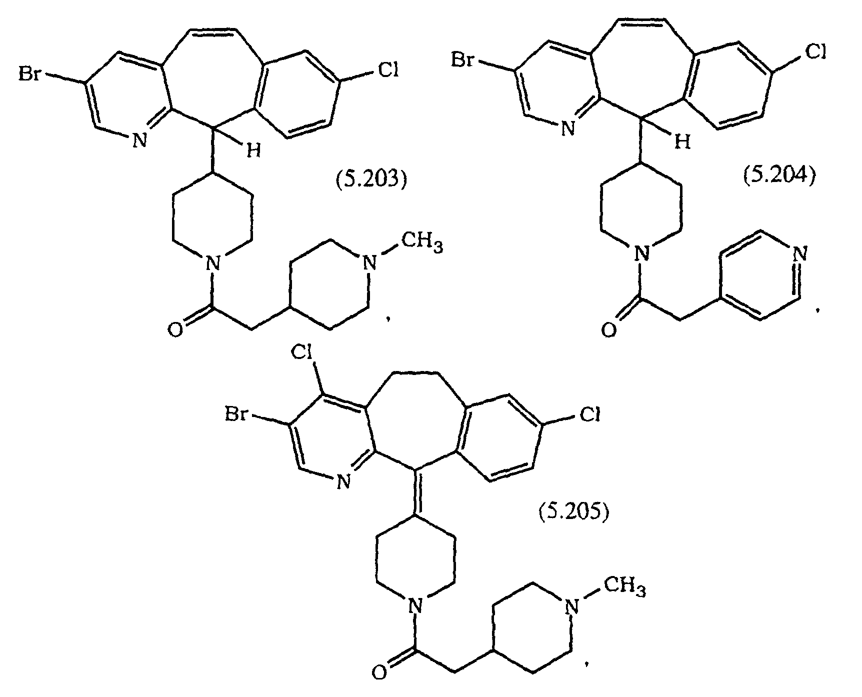 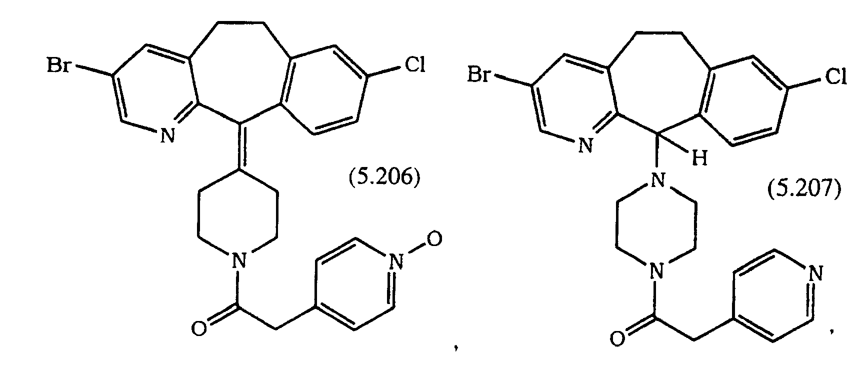 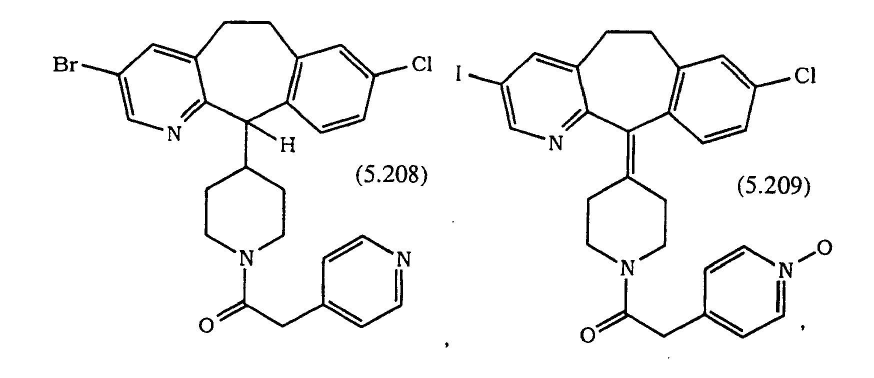 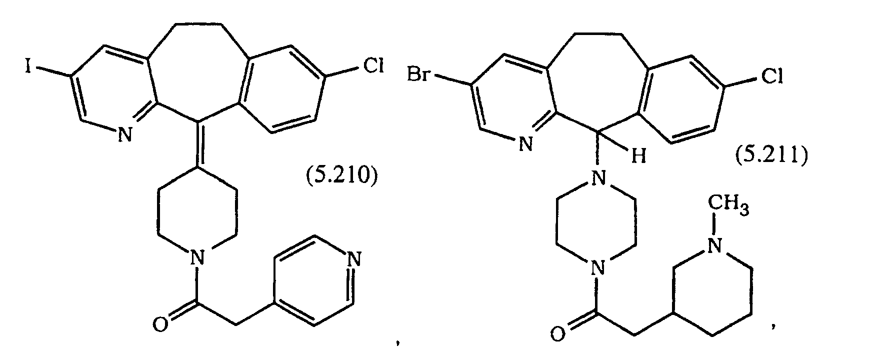 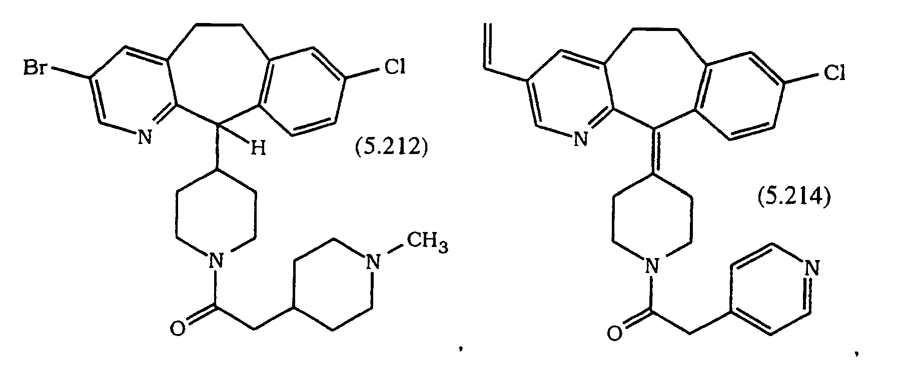 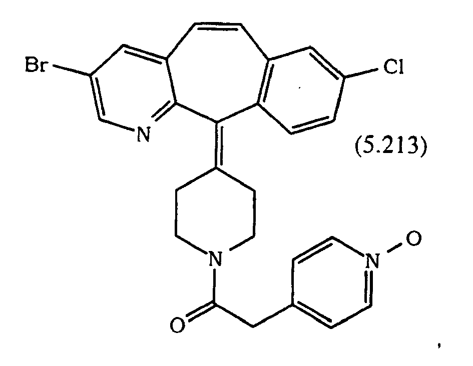 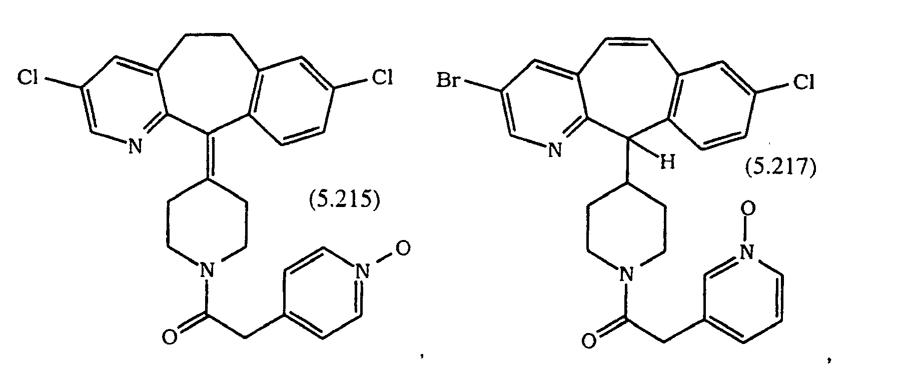 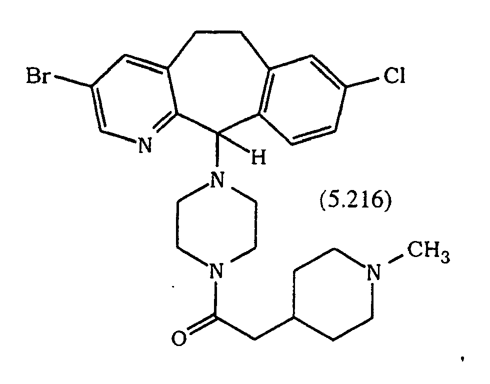 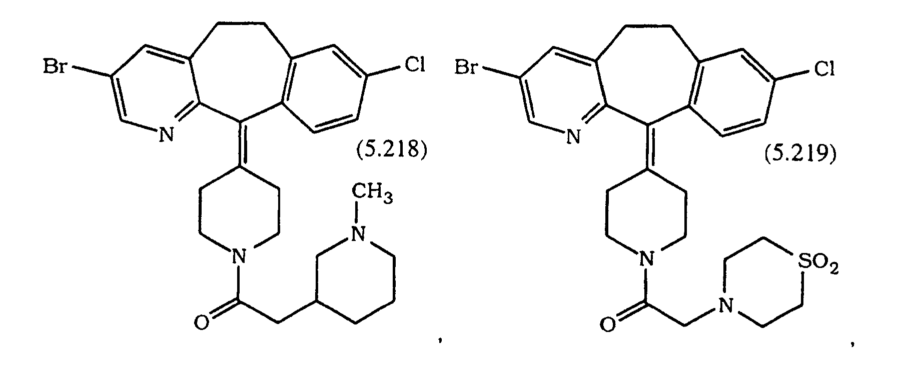
and

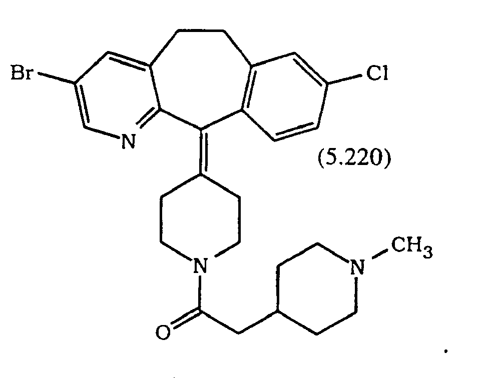

WO 95/10515 published April 20, 1995 discloses compounds of formula 1.0: 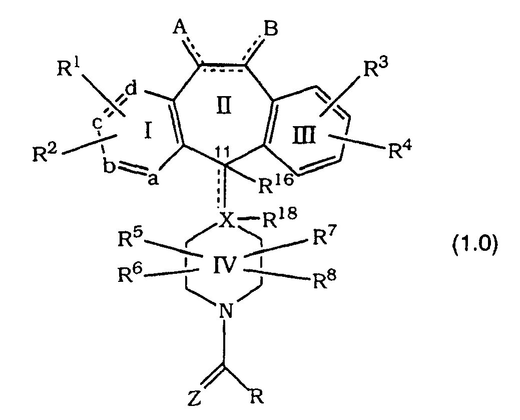
or a pharmaceutically acceptable salt or solvate thereof, wherein:
one of a, b, c and d represents N or NR⁹ wherein R⁹ is O⁻ (i.e., NR⁹ is an N-oxide group), -CH₃ or -(CH₂)ₙCO₂H wherein n is 1 to 3, and the remaining a, b, c and d groups represent CR¹ or CR²;
each R¹ and each R² is independently selected from H, halo, -CF₃, -OR¹⁰, -COR¹⁰, -SR¹⁰, -N(R¹⁰)₂, -NO₂, -OC(O)R¹⁰, -CO₂R¹⁰, -OCO₂R¹¹, benzotriazol-1-yloxy, CN, alkynyl, alkenyl and alkyl, said alkyl or alkenyl group optionally being substituted with halo, -OR¹⁰ or -CO₂R¹⁰;
R³ and R⁴ are the same or different and each independently represents H or any of the substituents of R¹ and R², or R³ and R⁴ together can represent a saturated or unsaturated C₅-C₇ ring fused to the benzene ring (Ring III);
each of R⁵, R⁶, R⁷ and R⁸ independently represents H, -CF₃, alkyl or aryl, said alkyl or aryl optionally being substituted with -OR¹⁰, -SR¹⁰, -N(R¹⁰)₂, -NO₂, -COR¹⁰, -OCOR¹⁰, -OCO₂R¹¹, -CO₂R¹⁰, or OPO₃R¹⁰, or one of R⁵, R⁶, R⁷ and R⁸ can be taken in combination with R as defined below to represent -(CH₂)ᵣ -, wherein r is 1 to 4, which can be substituted with lower alkyl, lower alkoxy, -CF₃ or aryl;
R¹⁰ represents H, alkyl, aryl, or aralkyl;
R¹¹ represents alkyl or aryl;
R¹⁶ and R¹⁸ represent H and F respectively, or F and H respectively, when the bond to X is a single bond and X is carbon; or
each of R¹⁶ and R¹⁸ represents H when the bond to X is a single bond;
X represents N or C, which C may contain an optional double bond (represented by the dotted line) to carbon atom 11:
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, each of A and B independently represents H₂, -(OR¹¹)₂, (H and halo), dihalo, (alkyl and H), (alkyl)₂, (H and -OC(O)R¹⁰), (H and -OR¹⁰), =O, (aryl and H), =NOR¹⁰ or -O-(CH₂)ₚ-O- wherein p is 2, 3 or 4;
Z represents O; and
R represents -SR⁶⁵ wherein R⁶⁵ is alkyl, aryl, heteroaryl, 2-, 3-, or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl; or
R represents -OR²⁰ wherein R²⁰ is C₁ to C₁₂ alkyl, substituted C₁ to C₁₂ alkyl, phenyl, substituted phenyl, C₇ to C₁₂ phenylalkyl, C₇ to C₁₂ phenylalkyl wherein the phenyl moiety is substituted, heteroaryl, or R²⁰ is 2-, 3-, or 4-piperidyl or N-substituted piperidyl, wherein the substituents on said substituted C₁ to C₁₂ alkyl are selected from amino and substituted amino, with the proviso that said amino or said substituted amino for said C₁ to C₁₂ alkyl is not on C₁, and the substitutents on said substituted amino are selected from C₁ to C₆ alkyl, the substituents on said substituted phenyl and on said substituted phenyl moiety of the C₇ to C₁₂ phenylalkyl are selected from C₁ to C₆ alkyl and halo, and the substituent on said N-substituted piperidyl is C₁ to C₄ alkyl, alkylcarbonyl or -C(O)NH(R¹⁰) wherein R¹⁰ is H or alkyl.
WO 95/10515 also disclose compounds of formulas 1.1, 1.2, and 1.3:

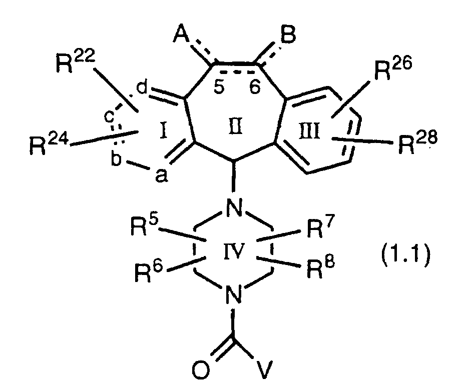

or a pharmaceutically acceptable salt or solvate thereof, wherein:
a, b, c, d, A, B, R⁵, R⁶, R⁷, and R⁸ are as defined for Formula 1.0 of WO 95/10515;
R²² and R²⁴ are the same or different and each independently represents any of the substituents of R¹ and R² for Formula 1.0 of WO 95/10515;
R²⁶ and R²⁸ are the same or different and each independently represents any of the substituents of R³ and R⁴ for Formula 1.0 of WO 95/10515;
V represents -OR³⁰ or -SR⁷⁰;
R³⁰ represents aralkyl, aryl, heteroaryl, alkyl, 2-, 3-, or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl;
R⁷⁰ represents aryl, heteroaryl, 2-, 3-, or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl; and
the dotted line between carbons 5 and 6 represents an optional double bond;

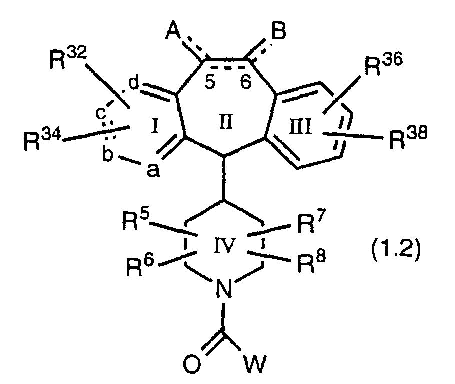

or a pharmaceutically acceptable salt or solvate thereof, wherein:
a, b, c, d, A, B, R⁵, R⁶, R⁷, and R⁸ are as defined for Formula 1.0 of WO 95/10515;
R³² and R³⁴ are the same or different and each independently represents any of the substituents of R¹ and R² for Formula 1.0 of WO 95/10515;
R³⁶ and R³⁸ are the same or different and each independently represents any of the substituents of R³ and R⁴ for Formula 1.0 of WO 95/10515;
W represents -OR⁴⁰ or -SR⁷⁰;
R⁴⁰ represents alkyl, aryl, heteroaryl, or 2-, 3-, or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl;
R⁷⁰ represents aryl, heteroaryl, 2-, 3-, or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl; and
the dotted line between carbons 5 and 6 represents an optional double bond; and

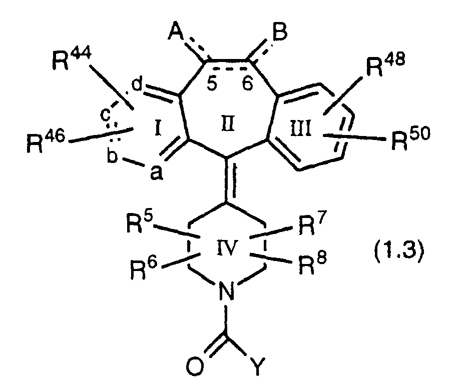

or a pharmaceutically acceptable salt or solvate thereof, wherein:
a, b, c, d, A, B, R⁵, R⁶, R⁷, and R⁸ are as defined for Formula 1.0 of WO 95/10515;
R⁴⁴ and R⁴⁶ are the same or different and each independently represents any of the substituents of R¹ and R² of Formula 1.0 of WO 95/10515;
R⁴⁸ and R⁵⁰ are the same or different and each independently represents any of the substituents of R³ and R⁴ of Formula 1.0 of WO 95/10515;
Y represents -OR⁵² or -SR⁷⁰;
R⁵² represents aralkyl, aryl, heteroaryl, alkyl, or 2-, 3-, or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl;
R⁷⁰ represents aryl, heteroarylalkyl, 2-, 3-, or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl; and
the dotted line between carbons 5 and 6 represents an optional double bond; and
with the provisos that: (a) when Y represents -OR⁵², and when there is a single bond between carbon atoms 5 and 6, and when both R⁴⁴ and R⁴⁶ are hydrogen, and when both R⁴⁸ and R⁵⁰ are H, then R⁵² is not phenyl; and (b) when Y represents -OR⁵², and when there is a single bond between carbon atoms 5 and 6, and when both R⁴⁴ and R⁴⁶ are hydrogen, and when R⁴⁸ is Cl at the C-8 position and R⁵⁰ is H, then R⁵² is not ethyl.

WO 96/30018 published October 3, 1996 discloses compounds of the formulas:

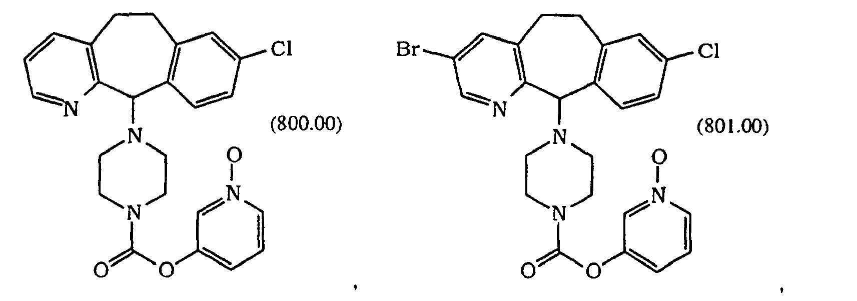

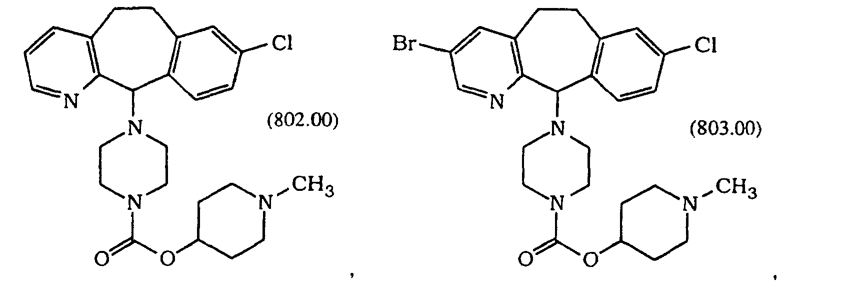

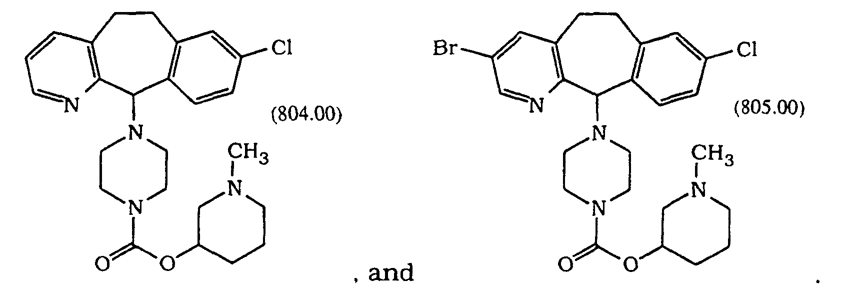

WO 96/30017 published October 3, 1996 discloses compounds of formulas (Ia), (Ib) and (Ic): 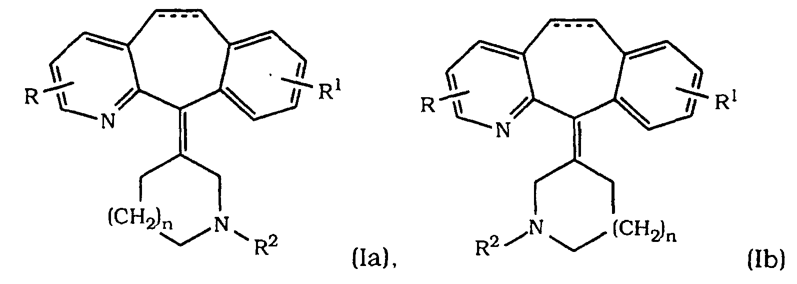 and 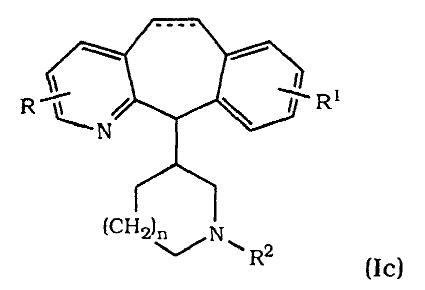
wherein:
R and R¹ are independently selected from H, (C₁-C₆)alkyl, halo, OH, (C₁-C₆)alkoxy, NH₂, (C₁-C₆)alkylamino, di(C₁-C₆)alkylamino, CF₃, SO₃H, CO₂R³, NO₂, SO₂NH₂, and CONHR⁴;
R² is R⁵C(O)-, R⁵CH₂C(O)-, R⁵C(R⁶)₂C(O)-, R⁵SO₂-, R⁵CH₂SO₂-, R⁵SCH₂C(O)-, R⁵OC(O)-, R⁵NHC(O)-, R⁵C(O)C(O)- or R⁵SC(O)-;
R³ is (C₁-C₆)alkyl or aryl;
R⁴ is (C₁-C₆)alkyl;
R⁵ is (C₁-C₆)alkyl, aryl, aryl(C₁-C₆)alkyl, aryl(C₂-C₆)alkenyl, heteroaryl, heteroaryl(C₁-C₆)alkyl, heteroaryl(C₂-C₆)alkenyl or heterocycloalkyl;
each R⁶ independently represents (C₁-C₆)alkyl, or both R⁶ groups together with the carbon atom to which they are attached comprise a (C₃-C₇)-carbocyclic ring;
n is 0 or 1; and
the dotted line represents an optional double bond;
and pharmaceutically acceptable salts thereof.

WO 96/30362 published October 3, 1996 discloses a compound of formula (7.0a), (7.0b) or (7.0c): 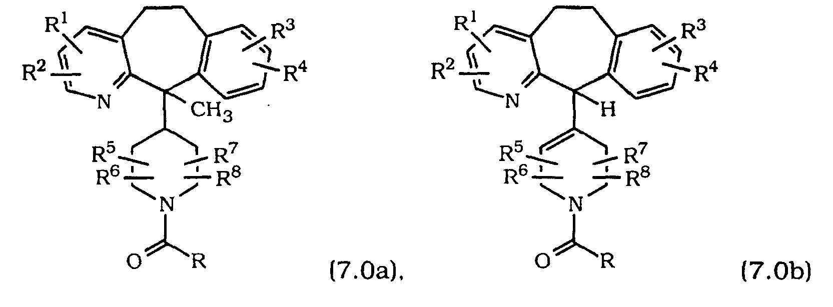 or 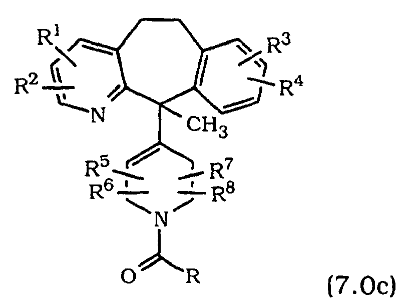
or a pharmaceutically acceptable salt or solvate thereof, wherein:
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 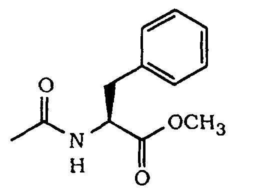 -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or one of R⁵, R⁶, R⁷ and R⁸ can be taken in combination with R⁴⁰ as defined below to represent -(CH₂)ᵣ- wherein r is 1 to 4 which can be substituted with lower alkyl, lower alkoxy, -CF₃ or aryl, or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
R represents R⁴⁰, R⁴², R⁴⁴, or R⁵⁴, as defined below;
R⁴⁰ represents H, aryl, alkyl, cycloalkyl, alkenyl, alkynyl or -D wherein -D represents 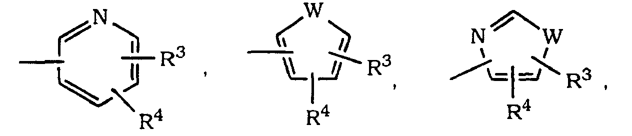 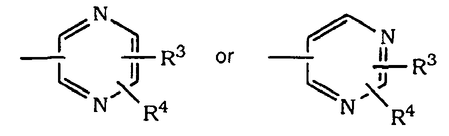
   wherein R³ and R⁴ are as previously defined and W is O, S or NR¹⁰ wherein R¹⁰ is as defined above; said R⁴⁰ cycloalkyl, alkenyl and alkynyl groups being optionally substituted with from 1-3 groups selected from halo, -CON(R¹⁰)₂, aryl, -CO₂R¹⁰, -OR¹², -SR¹², -N(R¹⁰)₂, -N(R¹⁰)CO₂R¹¹, -COR¹², -NO₂ or D, wherein -D, R¹⁰ and R¹¹ are as defined above and R¹² represents R¹⁰, -(CH₂)ₘOR¹⁰ or -(CH₂)_{q}CO₂R¹⁰ wherein R¹⁰ is as previously defined, m is 1 to 4 and q is 0 to 4; said alkenyl and alkynyl R⁴⁰ groups not containing -OH, -SH or
   -N(R¹⁰)₂ on a carbon containing a double or triple bond respectively; or
R⁴⁰ represents phenyl substituted with a group selected from -SO₂NH₂, -NHSO₂CH₃, -SO₂NHCH₃, -SO₂CH₃, -SOCH₃, -SCH₃, or -NHSO₂CF₃, preferably, said group is located in the para (p-) position of the phenyl ring; or
R⁴⁰ represents a group selected from 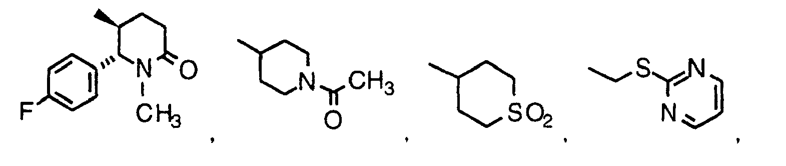 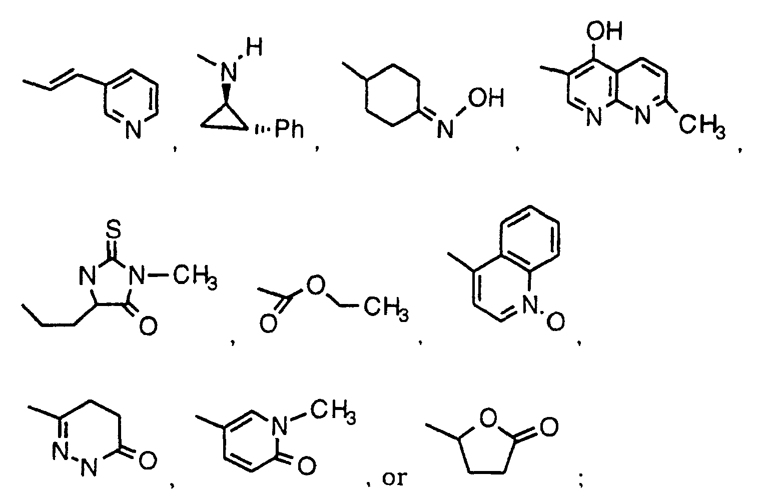 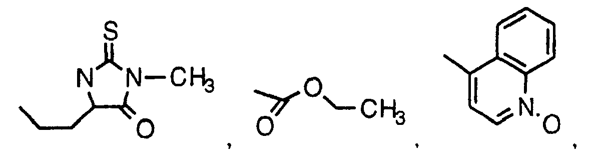 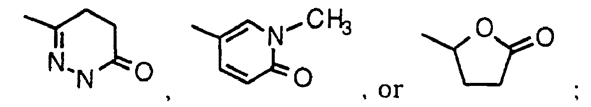
R⁴² represents 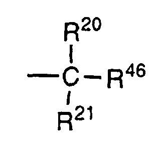
   wherein R²⁰, R²¹ and R⁴⁶ are each independently selected from the group consisting of:
   (1) H;
   (2) -(CH₂)_{q}SC(O)CH₃ wherein q is 1 to 3 (e.g., -CH₂SC(O)CH₃);
   (3) -(CH₂)_{q}OSO₂CH₃ wherein q is 1 to 3 (e.g., -CH₂OSO₂CH₃);
   (4) -OH;
   (5) -CS(CH₂)_{w}(substituted phenyl) wherein w is 1 to 3 and the substitutents on said substituted phenyl group are the same substitutents as described below for said substituted phenyl (e.g., -C-S-CH₂-4-methoxyphenyl);
   (6) -NH₂;
   (7) -NHCBZ (wherein CBZ stands for carbonylbenzyloxy-- i.e., CBZ represents -C(O)OCH₂C₆H₅);
   (8) -NHC(O)OR²² wherein R²² is an alkyl group having from 1 to 5 carbon atoms (e.g., R²² is t-butyl thus forming -NHBOC wherein BOC stands for tert-butyloxycarbonyl--i.e., BOC represents -C(O)OC(CH₃)₃), or R²² represents phenyl substituted with 1 to 3 alkyl groups (e.g., 4-methylphenyl);
   (9) alkyl (e.g., ethyl);
   (10) -(CH₂)ₖphenyl wherein k is 1 to 6, usually 1 to 4 and preferably 1 (e.g., benzyl);
   (11) phenyl;
   (12) substituted phenyl (i.e., phenyl substituted with from 1 to 3 substituents, preferably one) wherein the substituents are selected from the group consisting of: halo (e.g., Br, Cl, or I, with Br being preferred); NO₂; -OH; -OCH₃; -NH₂; -NHR²²; -N(R²²)₂; alkyl (e.g., alkyl having from 1 to 3 carbons with methyl being preferred); -O(CH₂)ₜphenyl (wherein t is from 1 to 3 with 1 being preferred); and -O(CH₂)ₜsubstituted phenyl (wherein t is from 1 to 3 with 1 being preferred); examples of substituted phenyls include, but are not limited to, p-bromophenyl, m-nitrophenyl, o-nitrophenyl, m-hydroxy-phenyl, o-hydroxyphenyl, methoxyphenyl, p-methylphenyl, m-methyl-phenyl, and -OCH₂C₆H₅;
   (13) naphthyl;
   (14) substituted naphthyl, wherein the substituents are as defined for substituted phenyl above;
   (15) bridged polycyclic hydrocarbons having from 5 to 10 carbon atoms (e.g., adamantyl and norbornyl);
   (16) cycloalkyl having from 5 to 7 carbon atoms (e.g., cyclopentyl, and cyclohexyl);
   (17) heteroaryl (e.g., pyridyl, and pyridyl N-oxide);
   (18) hydroxyalkyl (e.g., -(CH₂)ᵥOH wherein v is 1 to 3, such as, for example, -CH₂OH);
   (19) substituted pyridyl or substituted pyridyl N-oxide wherein the substituents are selected from methylpyridyl, morpholinyl, imidazolyl, 1-piperidinyl, 1-(4-methylpiperazinyl), -S(O)ₜR¹¹, or any of the substituents given above for said substituted phenyl, and said substitutents are bound to a ring carbon by replacement of the hydrogen bound to said carbon; 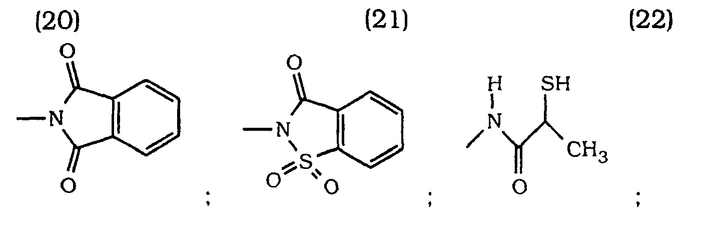
   (23) -NHC(O)-(CH₂)ₖ-phenyl or -NH(O)-(CH₂)ₖ-substitued phenyl, wherein said k is as defined above (i.e., 1-6, usually 1-4 and preferably 1);
   (24) piperidine Ring V: 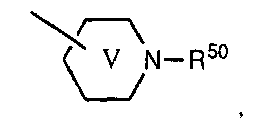
      wherein R⁵⁰ represents H, alkyl (e.g., methyl), alkylcarbonyl (e.g., CH₃C(O)-), alkyloxycarbonyl (e.g., -C(O)O-t-C₄H₉, -C(O)OC₂H₅, and -C(O)OCH₃), haloalkyl (e.g., trifluromethyl), or --C(O)NH(R¹⁰) wherein R¹⁰ is H or alkyl; Ring V includes 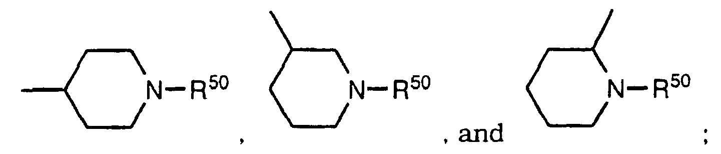 examples of Ring V include: 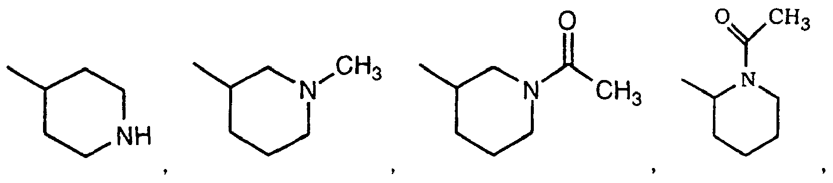 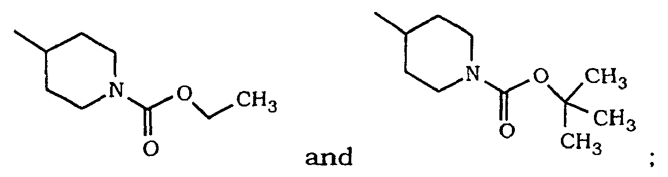
   (25) -NHC(O)CH₂C₆H₅ or -NHC(O)CH₂-substituted-C₆H₅, for example -NHC(O)CH₂-p-hydroxyphenyl, -NHC(O)CH₂-m-hydroxyphenyl, and -NHC(O)CH₂-o-hydroxyphenyl;
   (26) -NHC(O)OC₆H₅; 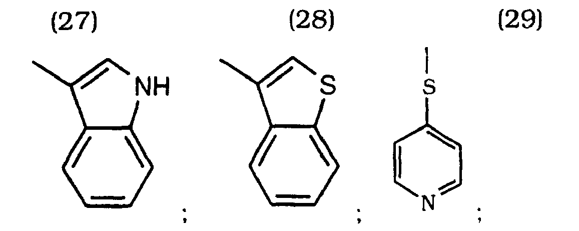
   (30) -OC(O)-heteroaryl, for example 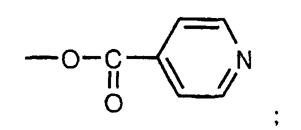
   (31) -O-alkyl (e.g., -OCH₃);
   (32) -CF₃;
   (33) -CN;
   (34) a heterocycloalkyl group of the formula 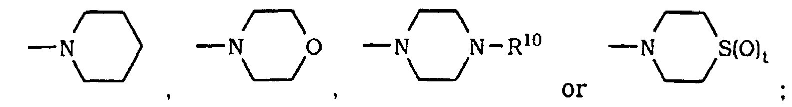 and
   (35) a piperidinyl group of the formula

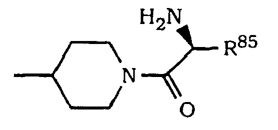

   wherein R⁸⁵ is H, alkyl, or alkyl substituted by -OH or -SCH₃ ; or
R²⁰ and R²¹ taken together form a =O group and the remaining R⁴⁶ is as defined above; or
two of R²⁰, R²¹ and R⁴⁶ taken together form piperidine Ring V 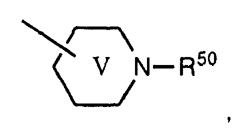
   wherein R⁵⁰ and Ring V are as defined above;
with the proviso R⁴⁶, R²⁰, and R²¹ are selected such that the carbon atom to which they are bound does not contain more than one heteroatom (i.e., R⁴⁶, R²⁰, and R²¹ are selected such that the carbon atom to which they are bound contains 0 or 1 heteroatom);
R44 represents 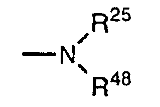
   wherein R²⁵ represents heteroaryl (e.g., pyridyl or pyridyl N-oxide) or aryl (e.g., phenyl and substituted phenyl); and R⁴⁸ represents H or alkyl (e.g., methyl);
R⁵⁴ represents an N-oxide heterocyclic group of the formula (i), (ii), (iii) or (iv): 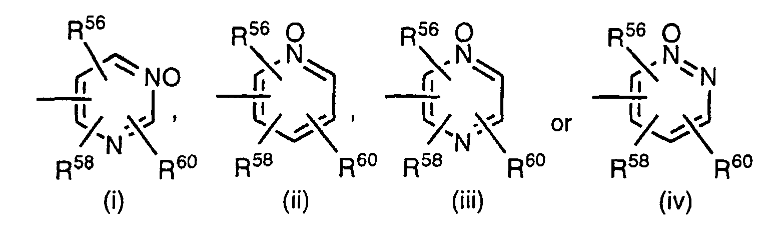
   wherein R⁵⁶, R⁵⁸, and R⁶⁰ are the same or different and each is independently selected from H, halo, -CF₃, -OR¹⁰, -C(O)R¹⁰, -SR¹⁰, -S(O)ₑR¹¹ (wherein e is 1 or 2), -N(R¹⁰)₂, -NO₂, -CO₂R¹⁰, -OCO₂R¹¹, -OCOR¹⁰, alkyl, aryl, alkenyl or alkynyl, which alkyl may be substituted with -OR¹⁰, -SR¹⁰ or -N(R¹⁰)₂ and which alkenyl may be substituted with OR¹¹ or SR¹¹; or
R⁵⁴ represents an N-oxide heterocyclic group of the formula (ia), (iia), (iiia) or (iva): 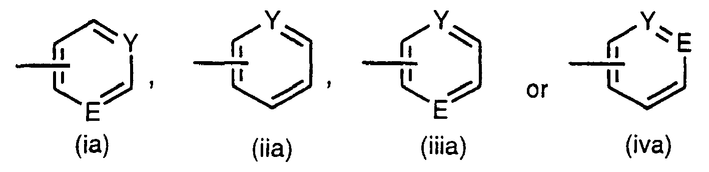
   wherein Y represents N⁺-O⁻ and E represents N; or
R⁵⁴ represents an alkyl group substituted with one of said N-oxide heterocyclic groups (i), (ii), (iii), (iv), (ia), (iia), (iiia) or (iva).

WO 96/31111 published October 10, 1996 discloses compounds of the formula: 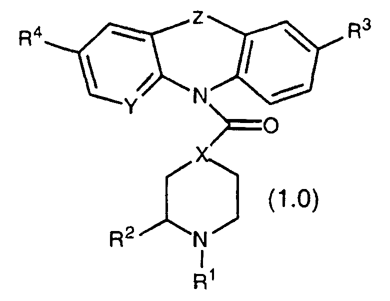
or a pharmaceutically acceptable salt or solvate thereof, wherein:
(1) R¹ is a group selected from: 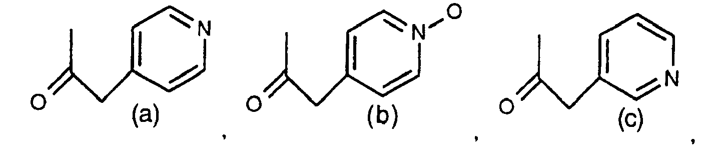 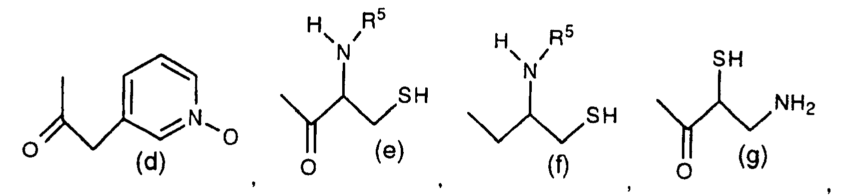 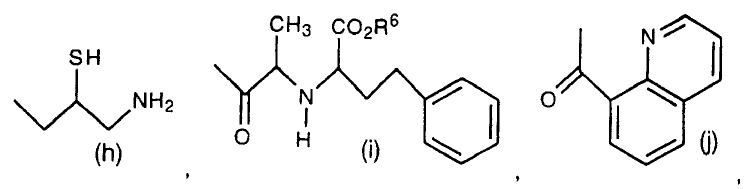 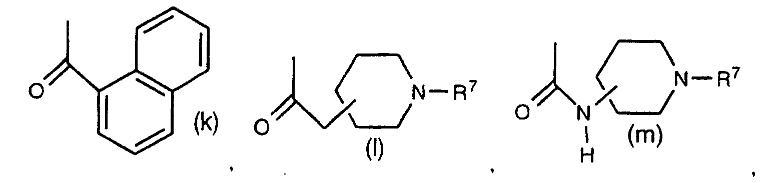 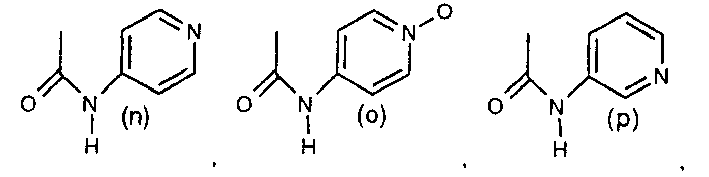 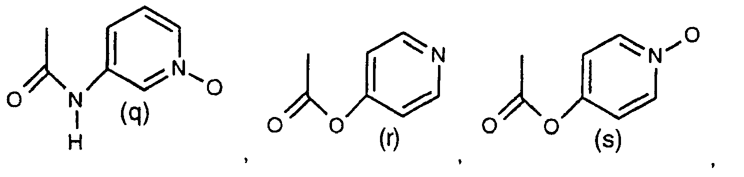 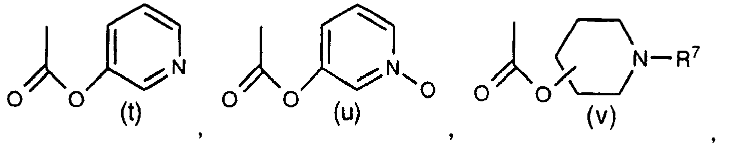 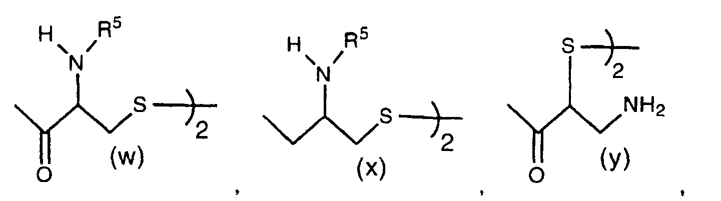 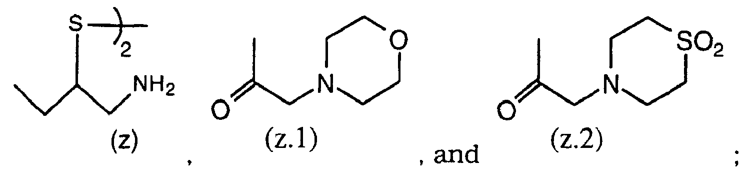
R² is selected from:
   (1) H;
   (2) C₁ to C₈ alkyl;
   (3) C₂ to C₈ alkenyl;
   (4) C₂ to C₈ alkynyl;
   (5) -CONR⁸R⁹; and
   (6) -COOR⁸;

   wherein said alkyl, alkenyl, or alkynyl is optionally substituted with one or more groups independently selected from:
   (a) aryl, aralkyl, heteroaryl, heteroarylalkyl, and heterocycloalkyl, said aryl, aralkyl, heteroarylalkyl, heteroaryl or heterocycloalkyl optionally being substituted with one or more of: C₁ to C₄ alkyl, (CH₂)ₜOR⁸ wherein t is 1 to 4, (CH₂)ₜNR⁸R⁹ wherein t is 1 to 4, halogen;
   (b) C₃ to C₆ cycloalkyl;
   (c) -OR⁸;
   (d) -SR⁸;
   (e) -S(O)R⁸;
   (f) -SO₂R⁸;
   (g) -NR⁸R⁹;
   (h) -NR⁸-CO-R⁹;
   (i) -NR⁸-CO-NR⁹R¹⁰;
   (j) -O-CO-NR⁸R⁹;
   (k) -O-CO-OR⁸;
   (l) -CO-NR⁸R⁹;
   (m) -SO₂-NR⁸R⁹;
   (n) -NR⁸-SO₂-OR⁹; and
   (o) -CO-R⁸;
R³ is selected from H, halogen and C₁ to C₆ alkyl;
R⁴ is selected from H, halogen and C₁ to C₆ alkyl;
R⁵ is selected from: H, C₁-C₆ alkyl, 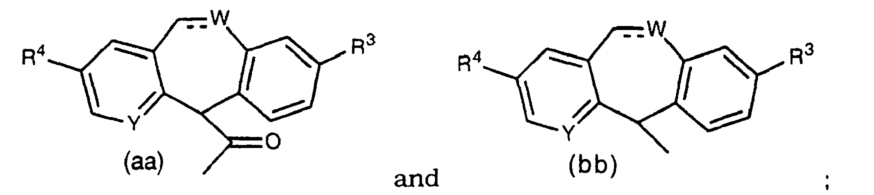
R⁶ is selected from H or C₁ to C₆ alkyl;
R⁷ is selected from H, C₁ to C₆ alkyl, haloalkyl, and -C(O)R¹¹ wherein R¹¹ is selected from C₁ to C₆ alkyl, C₁ to C₆ alkoxy or -NHR¹² (wherein R¹² is C₁ to C₆ alkyl or H), or R⁷ is an acyl radical of a naturally occurring amino acid;
R⁸, R⁹ and R¹⁰ are independently selected from H, C₁ to C₄ alkyl, C₃ to C₆ cycloalkyl, heteroaryl, heteroarylalkyl, heterocycloalkyl, aralkyl, and aryl; said alkyl, cycloalkyl, heteroaryl, heteroarylalkyl, heterocycloalkyl, aralkyl, or aryl optionally being substituted with C ₁ to C₄ alkoxy, aralkyl, aryl, heteroaryl, heteroarylalkyl, cyclopropyl, heterocycloalkyl, halogen, -OH, -C(O)R¹³, -SO₂R¹³, or -NR¹⁴R¹⁵ wherein R¹³ is selected from C₁ to C₄ alkyl and aralkyl, and wherein R¹⁴ and R¹⁵ are independently selected from H, C₁ to C₄ alkyl and aralkyl, with the provisos that:
   R⁸ may not be H in substituent (e), (f), or (k),
   R⁹ may not be H in substituent (h) or (n), and
   R⁸, R⁹, or R¹⁰ may not be CH₂OH or CH₂NR¹⁴R¹⁵ when R¹⁰ is directly attached to a heteroatom which is O, S, or N;
   R¹⁶ is selected from H, arylalkyl and C₁ to C₆ alkyl;
   optionally, when R⁸ and R⁹ are bound to the same nitrogen, R⁸ and R⁹, together with the nitrogen to which they are bound, form a 5 to 7 membered heterocycloalkyl ring;
   optionally, when R⁹ and R¹⁰ are bound to the same nitrogen, R⁹ and R¹⁰, together with the nitrogen to which they are bound, form a 5 to 7 membered heterocycloalkyl ring;
   - - - - represents an optional bond;
   W is CH when the optional bond is present, or CH₂, O, or S when the optional bond is absent;
   X is selected from CH and N;
   Y is selected from N and CH; and
   Z is selected from -CO-NR¹⁶-, -NR¹⁶-CO-, -CH₂-CH₂-, and -CH=CH-.

WO 96/31478 published October 10, 1996 discloses compounds of formula (1.0): 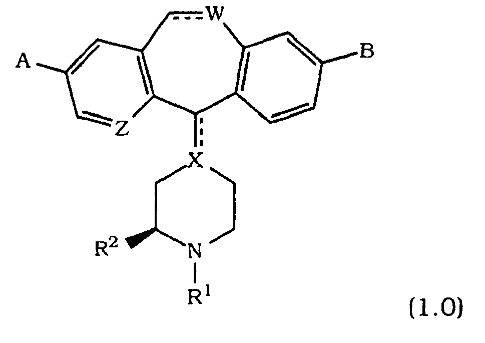
wherein:
A and B are independently selected from H, halo or C₁-C₆ alkyl;
Z is N or CH;
W is CH, CH₂, O or S, wherein the dotted line to W represents a double bond which is present when W is CH;
X is C, CH or N, wherein the dotted line connecting X to the tricyclic ring system represents a double bond which is present when X is C;
R¹ is selected from:
   1) a group of the formula: 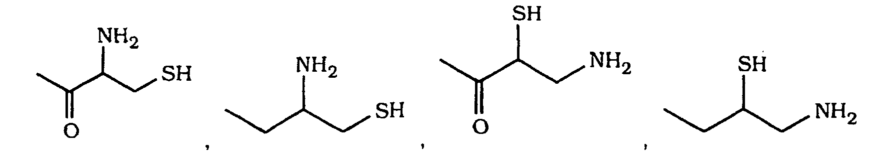
      or disulfide dimers thereof;
   2) a group of the formula: 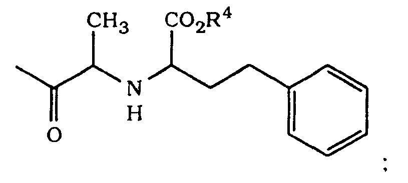
   3) a group of the formula: 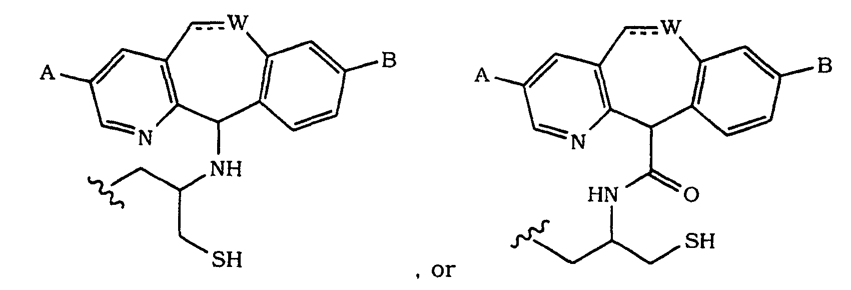
      wherein W, A and B are as defined above;
   4) a group of the formula: 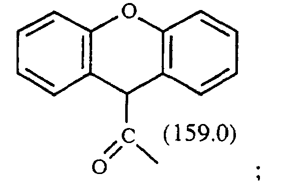
   5) a group of the formula: 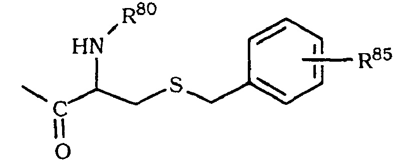
      wherein R⁸⁰ is selected from H or -C(O)OR⁹⁰ wherein R⁹⁰ is a C₁₋C₆ alkyl group (e.g., -C(CH₃)₃), and R⁸⁵ is a C₁-C₆ alkoxy group (e.g., p-OCH₃); and
   6) a group of the formula: 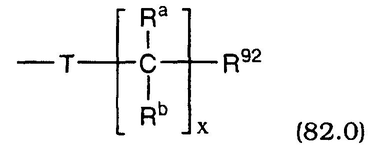
      wherein:
      (a) T is selected from: 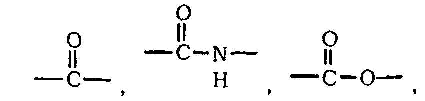 -SO₂-, or a single bond;
      (b) x is 0, 1, 2, 3, 4, 5 or 6;
      (c) each R^{a} and each R^{b} is independently selected from H, aryl, alkyl, alkoxy, aralkyl, amino, alkylamino, heterocyloalkyl, -COOR⁶⁰, -NH{C(O)}_{z}R⁶⁰ (wherein z is 0 or 1), or -(CH)_{w}S(O)ₘR⁶⁰ (wherein w is 0, 1, 2 or 3, and m is 0, 1 or 2); or R^{a} and R^{b} taken together can represent cycloalkyl, =N-O-alkyl, =O or heterocycloalkyl, with the proviso that for the same carbon, R^{a} is not selected from alkoxy, amino, alkylamino or -NH{C(O)}_{z}R⁶⁰ when R^{b} is selected from alkoxy, amino, alkylamino or -NH{C(O)}_{z}R⁶⁰; and with the proviso that when T is a single bond, for the first carbon containing R^{a} and R^{b}, R^{a} and R^{b} are not selected from alkoxy, alkylamino, amino or -NHR⁶⁰ (i.e., -NH{C(O)}_{z}R⁶⁰ wherein z is 0) (i.e., R^{a} and R^{b} on the first carbon bound to T, when T is a single bond, are not alkoxy, alkylamino, amino or -NHR⁶⁰); and
      (d) R⁹² can represent H, alkyl, aryl, aryloxy, arylthio, aralkoxy, aralkyl, heteroaryl or heterocycloalkyl;
R⁶⁰ represents H, alkyl, aryl or aralkyl;
R⁴ is H or C₁-C₆ alkyl;
R² is selected from: H, -C(O)OR⁶, -C(O)NR⁶R⁷, C₁-C₈ alkyl, C₂-C₈ alkenyl, C₂-C₈ alkynyl, substituted (C₁-C₈)alkyl, substituted (C₂-C₈)alkenyl, substituted (C₂-C₈)alkynyl, wherein said substituted groups have one or more substituents selected from:
   1) aryl, arylalkyl, heteroarylalkyl, heteroaryl, heterocycloalkyl, B-substituted aryl, B-substituted arylalkyl. B-substituted heteroarylalkyl, B-substituted heteroaryl or B-substituted heterocycloalkyl, wherein B is selected from C₁-C₄ alkyl, -(CH₂)ₙOR⁶, -(CH₂)ₙNR⁶R⁷ and halo;
   2) C₃-C₆ cycloalkyl;
   3) -OR⁶;
   4) -SH or -S(O)ₜR⁶;
   5) -NR⁶R⁷;
   6) -N(R⁶)-C(O)R⁷;
   7) -N(R⁶)-C(O)NR⁷R¹²;
   8) -O-C(O)NR⁶R⁷;
   9) -O-C(O)OR⁶;
   10) -SO₂NR⁶R⁷;
   11) -N(R⁶)-SO₂-R⁷;
   12) -C(O)NR⁶R⁷;
   13) -C(O)OR⁶; and

   provided where R¹ is D, R² is not H, and where R¹ is D and R² is C₁-C₈ alkyl, the substituents on said alkyl group are not substituents 3), 4), 5), 9), or 13); D is -C(O)-CH₂-R⁵, -C(O)-O-R⁵ or -C(O)-NH-R⁵, wherein R⁵ is pyridyl, pyridyl N-oxide, 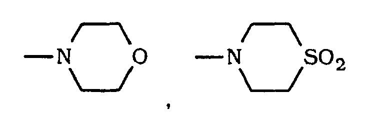
   or a piperidinyl group of the formula 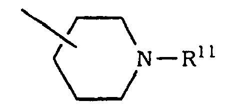
   wherein R¹¹ represents H, C₁-C₆ alkyl, haloalkyl or -C(O)-R⁹
   wherein R⁹ is C₁-C₆ alkyl, C₁-C₆ alkoxy or -NH(R¹⁰) wherein R¹⁰ is H or alkyl, or the group -C(O)-R⁹ represents an acyl radical of a naturally occurring amino acid;
R⁶, R⁷ and R¹² are independently selected from H, C₁-C₄ alkyl, (C₃-C₆)cycloalkyl, aryl, arylalkyl (i.e., aralkyl), heteroaryl, heteroarylalkyl, heterocycloalkyl, substituted (C₁-C₄)alkyl, substituted (C₃-C₆)cycloalkyl, substituted aryl, substituted arylalkyl, substituted heteroaryl, substituted heteroarylalkyl or substituted heterocycloalkyl, wherein said substituted groups have one or more substituents (e.g., 1-3) selected from: C₁-C₄ alkoxy, aralkyl, heteroarylalkyl, -NO₂, C₃-C₁₀-alkoxyalkoxy (e.g.,-O-(C₁₋C₄)alkyl-O-(C₁-C₄)alkyl), (C₃-C₆) cycloalkyl (e.g., cyclopropyl or cyclohexyl), aryl, -CN, nitrophenyl, methylenedioxy-phenyl, heteroaryl, heterocycloalkyl, halo, -OH, -C(O)R¹⁴, -C(O)NR⁶R⁷, -N(R⁶)C(O)R¹⁴, -S(O)ₜR¹⁴ (e.g., -S-(C₁₋C₄)alkyl and -SO₂R¹⁴) or -NR⁹⁵R¹⁵; provided that R⁶, R⁷ and R¹² are not -CH₂OH or -CH₂NR⁹⁵R¹⁵ when said R⁶, R⁷ or R¹² is directly bonded to a heteroatom, and further provided that R⁶ is not H for groups 4) and 9), and R⁷ is not H for group 6);
optionally, when R⁶ and R⁷ are bound to the same nitrogen, R⁶ and R⁷ together with the nitrogen to which they are bound, form a 5 to 7 membered heterocycloalkyl ring which optionally contains O, NR⁶, or S(O)ₜ wherein t is 0, 1 or 2;
optionally, when R⁷ and R¹² are bound to the same nitrogen, R⁷ and R¹² together with the nitrogen to which they are bound, form a 5 to 7 membered heterocycloalkyl ring which optionally contains O, NR⁶, or S(O)ₜ wherein t is 0, 1 or 2;
R⁹⁵ and R¹⁵ are independently H, C₁-C₄ alkyl or arylalkyl;
R¹⁴ is C₁-C₄ alkyl, aryl or arylalkyl;
n = 0, 1, 2, 3 or 4; and
t = 0, 1 or 2;

or pharmaceutically acceptable salts thereof.

WO 96/31477 published October 10, 1996 discloses compounds of formula (1.0): 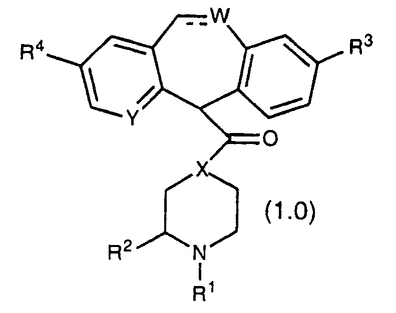
or a pharmaceutically acceptable salt or solvate thereof, wherein:
(1) R¹ is a group selected from: 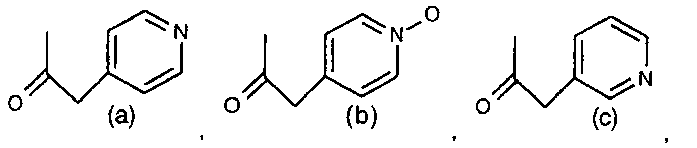 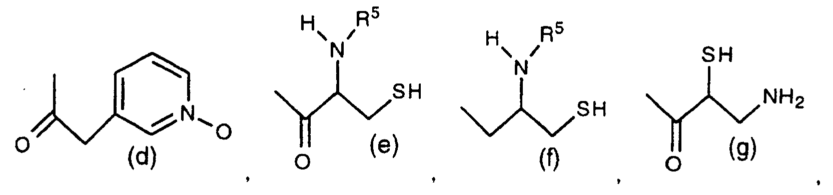 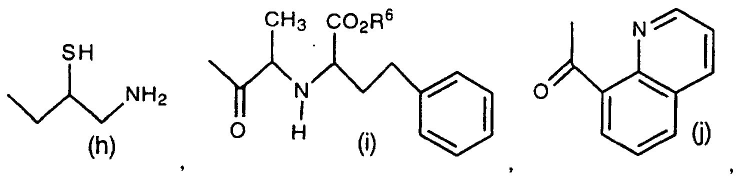 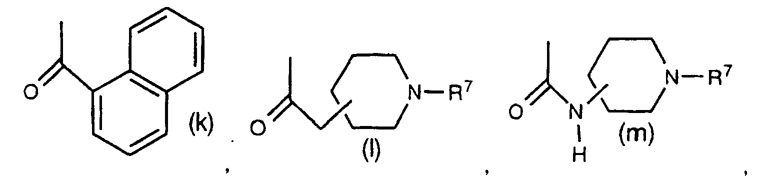 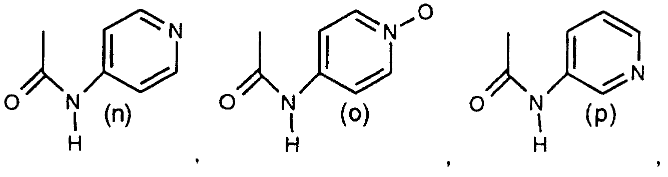 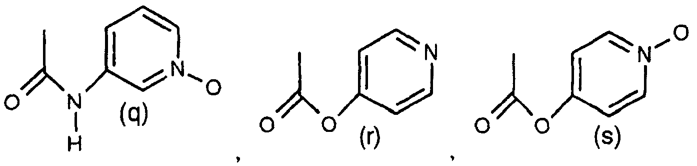 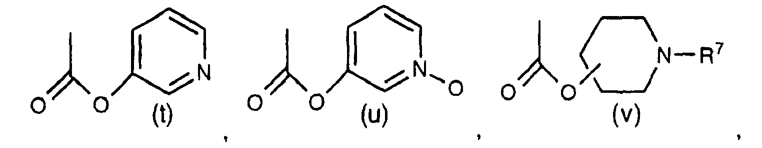 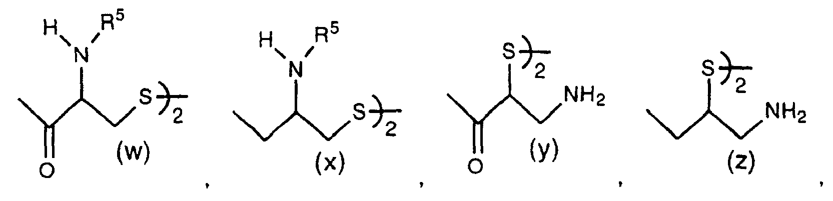 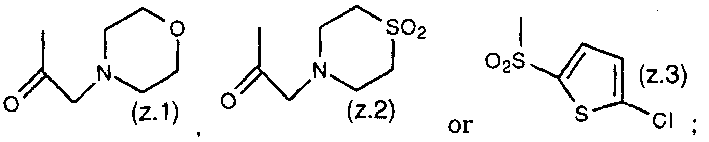
R² is selected from: (1) H, (2) C₁ to C₈ alkyl, (3) C₂ to C₈ alkenyl, (4) C₂ to C₈ alkynyl, 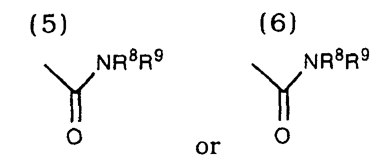
   wherein said alkyl, alkenyl, or alkynyl is optionally substituted with one or more groups independently selected from:
   (a) aryl, aralkyl, heteroaryl, heteroarylalkyl or heterocycloalkyl; said aryl, aralkyl, heteroaryl, heteroarylalkyl or heterocycloalkyl optionally substituted with one or more groups independently selected from:
      (1) C₁ to C₄ alkyl,
      (2) (CH₂)ₜOR⁸ wherein t is 1 to 4,
      (3) (CH₂)ₜNR⁸R⁹ wherein t is 1 to 4, or
      (4) halogen,
   (b) C₃ to C₆ cycloalkyl,
   (c) -OR⁸,
   (d) -SR⁸,
   (e) -S(O)R⁸,
   (f) -SO₂R⁸,
   (g) -NR⁸R⁹, 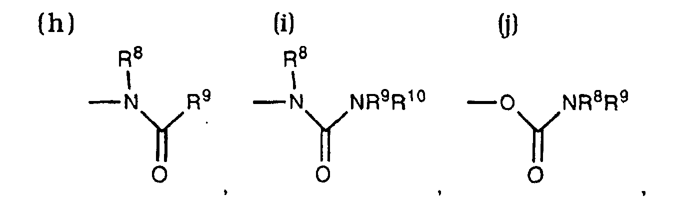 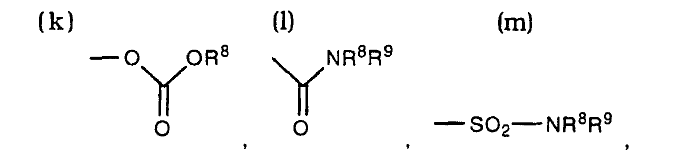 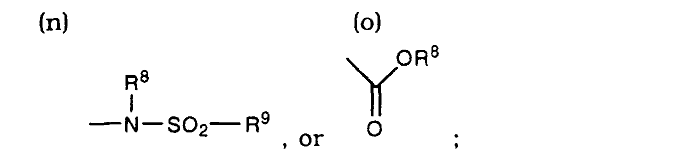
R³ is selected from H, halogen or C₁ to C₆ alkyl (e.g., methyl);
R⁴ is selected from H, halogen or C₁ to C₆ alkyl (e.g., methyl);
R⁵ is selected from: H, 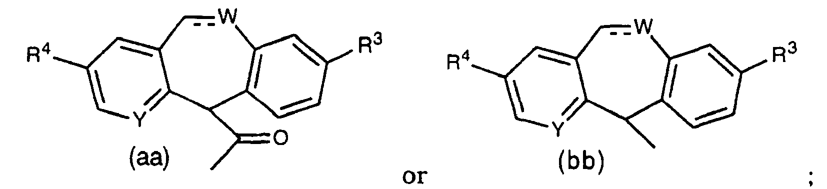
R⁶ is selected from H or C₁ to C₆ alkyl (preferably methyl or ethyl);
R⁷ is selected from H, C₁ to C₆ alkyl, haloalkyl, or -C(O)R¹¹ wherein R¹¹ is selected from C₁ to C₆ alkyl, C₁ to C₆ alkoxy or -NHR¹² (wherein R¹² is C₁ to C₆ alkyl or H), or R⁷ is an acyl radical of a naturally occurring amino acid;
R⁸, R⁹ and R¹⁰ are independently selected from H, C₁ to C₄ alkyl, C₃ to C₆ cycloalkyl, heteroaryl, heteroarylalkyl, heterocycloalkyl, aryl or aralkyl; said alkyl, cycloalkyl, heteroaryl, heteroarylalkyl, heterocycloalkyl, aryl or aralkyl are optionally substituted with C₁ to C₄ alkoxy, aryl, heteroaryl, heterocycloalkyl, cyclopropyl, halogen, -OH, -C(O)R¹³, -SO₂R¹³, or -NR¹⁴R¹⁵ wherein R¹³ is selected from C₁ to C₄ alkyl or aralkyl, and wherein R¹⁴ and R¹⁵ are independently selected from H, C₁ to C₄ alkyl or aralkyl; with the proviso that R⁸ is not H in substituents (e), (f) or (k), and with the proviso that R⁹ is not H in substituent (h) or (n), and with the proviso that R⁸, R⁹, or R¹⁰ is not -CH₂OH or -CH₂NR¹⁴R¹⁵ when R⁸, R⁹, or R¹⁰ is directly attached to a heteroatom (e.g., O, S or N);
   optionally, when R⁸ and R⁹ are bound to the same nitrogen, R⁸ and R⁹, together with the nitrogen to which they are bound, form a 5 to 7 membered heterocycloalkyl ring;
optionally, when R⁹ and R¹⁰ are bound to the same nitrogen, R⁹ and R¹⁰, together with the nitrogen to which they are bound, form a 5 to 7 membered heterocycloalkyl ring;
- - - - represents an optional bond;
W is selected from CH when the optional bond is present, or O, S or CH₂ when the optional bond is absent;
X is selected from CH or N; and
Y is selected from N or CH.

WO 96/31505 published October 10, 1996 discloses compounds of formula (1.0): 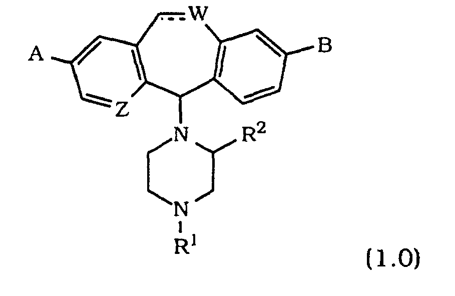
wherein:
A and B are independently selected from H, halo or C₁-C₆ alkyl;
Z is N or CH;
W is CH, CH₂, O or S, wherein the dotted line to W represents a double bond which is present when W is CH;
R¹ is selected from the group consisting of: 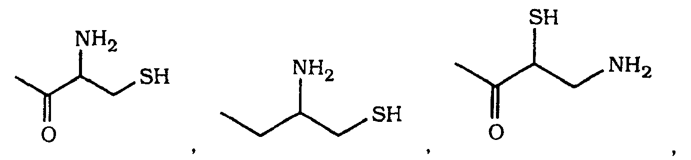 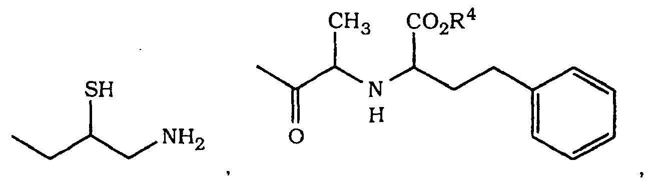 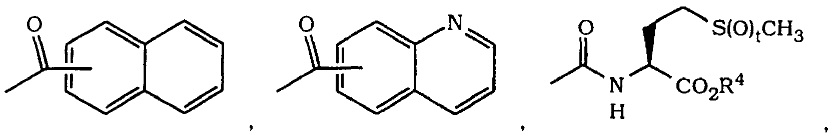 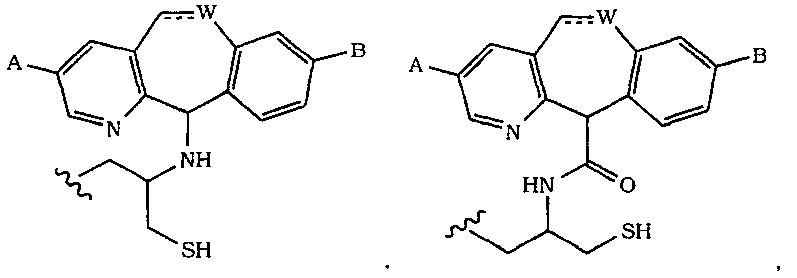 wherein W, A and B are as defined above; or
R¹ is a group D, wherein D is -C(O)-(CH₂)ₛ-R⁵, -C(O)O-(CH₂)ₘ-R⁵ or -C(O)NH-(CH₂)ₘ-R⁵, wherein R⁵ is aryl, (such as phenyl, B-substituted phenyl wherein B is as defined below), heteroaryl, (such as pyridyl or pyridyl N-oxide), heterocycloalkyl, or a group of the formula 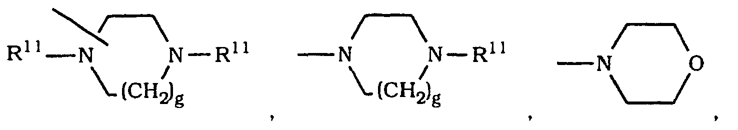 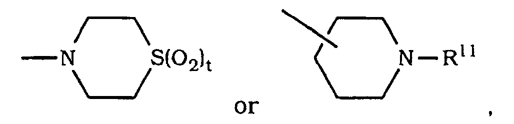 wherein g = 1 or 2, and R¹¹ represents H, C₁-C₆ alkyl, haloalkyl or -C(O)-R⁹ wherein R⁹ is C₁-C₆ alkyl, C₁-C₆ alkoxy or -NH(R^{10A}) wherein R^{10A} is H or alkyl, or the group -C(O)-R⁹ represents an acyl radical of a naturally occurring amino acid; or
R¹ is a group of the formula: 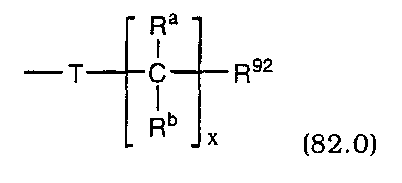
   wherein:
   (a) T is selected from: 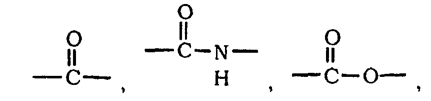 -SO₂-, or a single bond;
   (b) x is 0, 1, 2, 3, 4, 5 or 6;
   (c) each R^{a} and each R^{b} is independently selected from H, aryl, alkyl, alkoxy, aralkyl, amino, alkylamino, heterocyloalkyl, -COOR⁶⁰, -NH{C(O)}_{z}R⁶⁰ (wherein z is 0 or 1), or -(CH)_{w}S(O)ₜR⁶⁰ (wherein w is 0, 1, 2 or 3, and t is 0, 1 or 2); or R^{a} and R^{b} taken together can represent cycloalkyl, =N-O-alkyl, =O or heterocycloalkyl; with the proviso that for the same carbon, R^{a} is not selected from alkoxy, amino, alkylamino or -NH{C(O)}_{z}R⁶⁰ when R^{b} is selected from alkoxy, amino, alkylamino or -NH{C(O)}_{z}R⁶⁰; and with the proviso that when T is a single bond, for the first carbon containing R^{a} and R^{b}, R^{a} and R^{b} are not selected from alkoxy, alkylamino, amino or -NHR⁶⁰ (i.e., -NH{C(O)}_{z}R⁶⁰ wherein z is 0) (i.e., R^{a} and R^{b} on the first carbon bound to T, when T is a single bond, are not alkoxy, alkylamino, amino or -NHR⁶⁰); and
   (d) R⁹² can represent H, alkyl, aryl, aryloxy, arylthio, aralkoxy, aralkyl, heteroaryl or heterocycloalkyl;
R⁶⁰ represents H, alkyl, aryl or aralkyl;
R⁴ is H or C₁-C₆ alkyl;
R² is selected from: -C(O)OR⁶, -C(O)NR⁶R⁷, C₁-C₈ alkyl, C₂₋C₈ alkenyl, C₂-C₈ alkynyl, substituted (C₁-C₈)alkyl, substituted (C₂-C₈)alkenyl, substituted (C₂-C₈)alkynyl, wherein said substituted groups have one or more substituents selected from:
   1) aryl, heteroaryl, heterocycloalkyl, B-substituted aryl, B-substituted heteroaryl or B-substituted heterocycloalkyl, wherein B is selected from C₁-C₄ alkyl, phenyl, -(CH₂)ₙOR⁶, -(CH₂)ₙNR⁶R⁷ and halo;
   2) C₃-C₆ cycloalkyl;
   3) -OR⁶;
   4) -S(O)ₜR⁶;
   5) -NR⁶R⁷;
   6) -N(R⁶)-C(O)R⁷;
   7) -N(R⁶)-C(O)NR⁷R¹²;
   8) -O-C(O)NR⁶R⁷;
   9) -O-C(O)OR⁶;
   10) -SO₂NR⁶R⁷;
   11) -N(R⁶)-SO₂-R⁷;
   12) -C(O)NR⁶R⁷;
   13) -C(O)OR⁶; and

   provided that: where R¹ is D, R² is not H; where R¹ is D and R² is C₁-C₈ alkyl, the substituents on said alkyl group are not substituents 4), 5), 9) or 13); and where R¹ is D, and R² is C₁-C₈ alkyl substituted by the group -OR⁶, R⁶ is not H, alkyl, aryl, substituted aryl, aryl-substituted alkyl or nitro-phenylsubstituted alkyl;
R⁶, R⁷ and R¹² are independently selected from H, C₁-C₄ alkyl, (C₃-C₆)cycloalkyl, aryl, arylalkyl (i.e., aralkyl), heteroaryl, heteroarylalkyl, heterocycloalkyl, substituted (C₁-C₄)alkyl, substituted (C₃-C₆)cycloalkyl, substituted aryl, substituted arylalkyl, substituted heteroaryl, substituted heteroarylalky or substituted heterocycloalkyl, wherein said substituted groups have one or more substituents (e.g., 1-3) selected from: C₁-C₄ alkoxy, aralkyl, heteroarylalkyl, -NO₂, (C₃-C₁₀)alkoxyalkoxy (e.g., -O-(C₁-C₄)alkyl-O-(C₁-C₄)alkyl), (C₃-C₆)cycloalkyl (e.g., cyclopropyl or cyclohexyl), aryl, -CN, nitro-phenyl, methylenedioxyphenyl, heteroaryl, heterocycloalkyl, halo, -OH, -COOH, -C(O)R¹⁴, -C(O)OR¹⁴, -C(O)NR⁶R⁷ (e.g., -C(O)NR¹⁰R¹⁵), -N(R⁶)C(O)R¹⁴, -S(O)ₜR¹⁴ (e.g., -S-(C₁-C₄) and -SO₂R¹⁴) or -NR¹⁰R¹⁵; provided that R⁶, R⁷ and R¹² are not -CH₂OH or -CH₂NR¹⁰R¹⁵ when said R⁶, R⁷ or R¹² is directly bonded to a heteroatom, and further provided that R⁶ is not H for groups 4) and 9), and R⁷ is not H for group 6);
optionally, when R⁶ and R⁷ are bound to the same nitrogen, R⁶ and R⁷ together with the nitrogen to which they are bound, form a 5 to 7 membered heterocycloalkyl ring which optionally contains O, NR⁶ (e.g., NR⁸), or S(O)ₜ (e.g., S) wherein t is 0, 1 or 2;
optionally, when R⁷ and R¹² are bound to the same nitrogen, R⁷ and R¹² together with the nitrogen to which they are bound, form a 5 to 7 membered heterocycloalkyl ring which optionally contains O, NR⁶ (e.g., NR⁸), or S(O)ₜ (e.g., S) wherein t is 0, 1 or 2;
R⁸, R¹⁰ and R¹⁵ are independently H, C₁-C₄ alkyl or arylalkyl;
R¹⁴ is C₁-C₄ alkyl, aryl or arylalkyl;
m=0, 1, 2 or 3;
n = 0, 1, 2, 3 or 4;
s = 1, 2 or 3; and
t = 0, 1 or 2;

or pharmaceutically acceptable salts thereof.U.S.

WO 97/23478 published July 3, 1997 discloses the compounds: 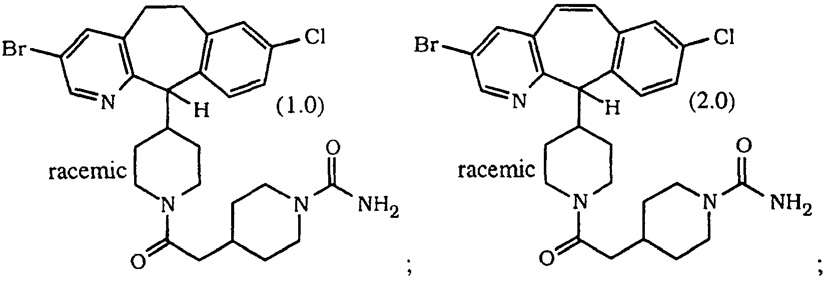 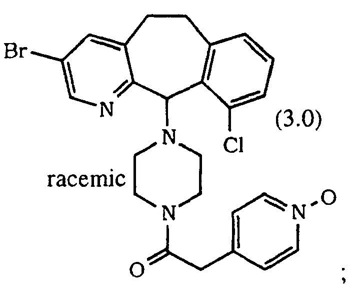 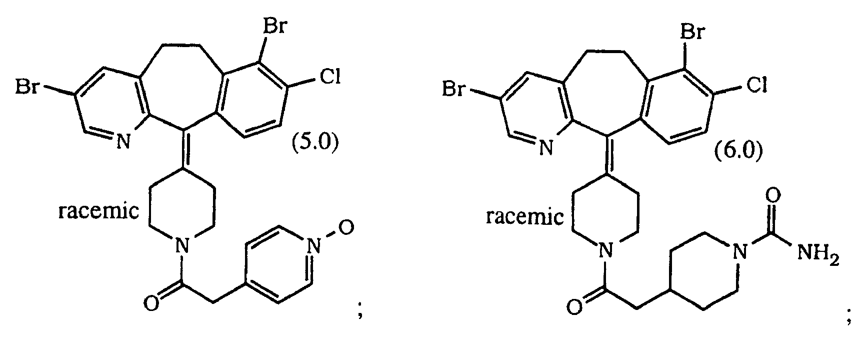 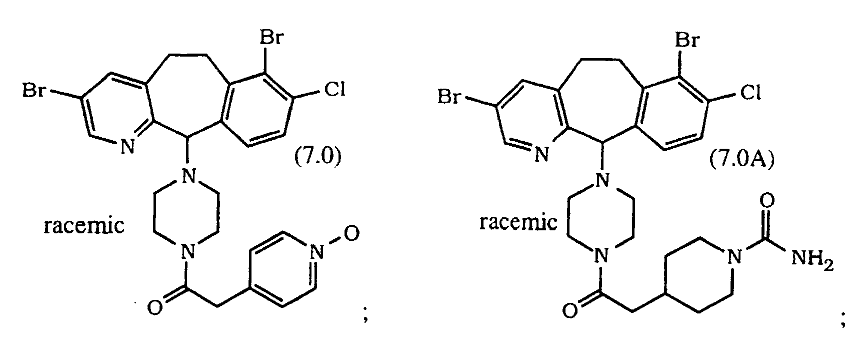 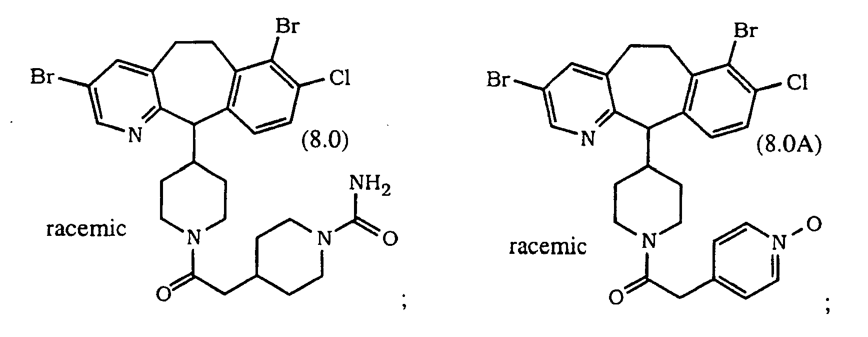 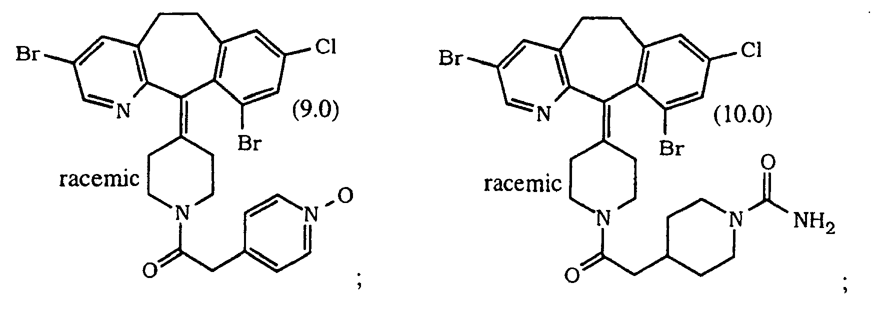 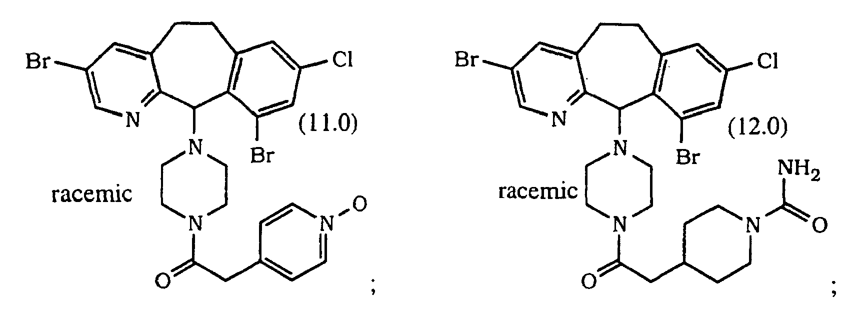 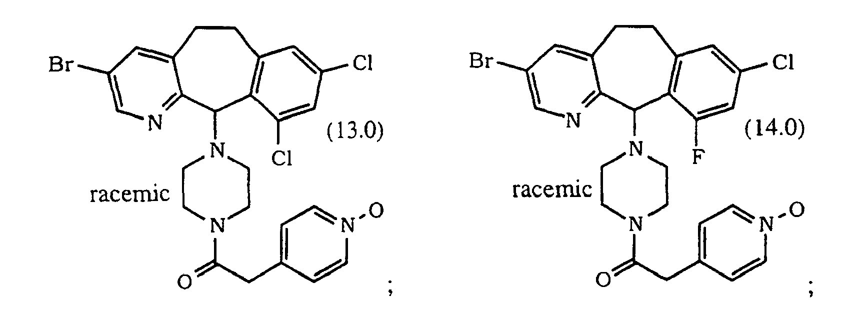 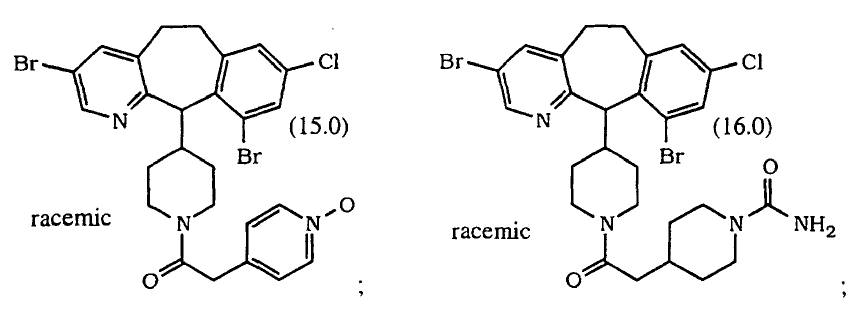 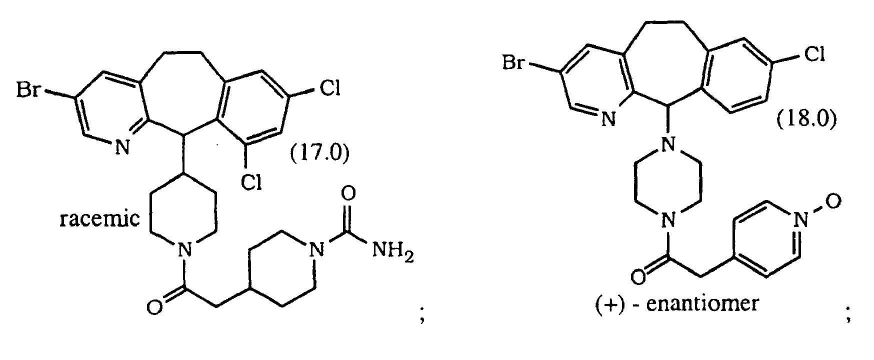 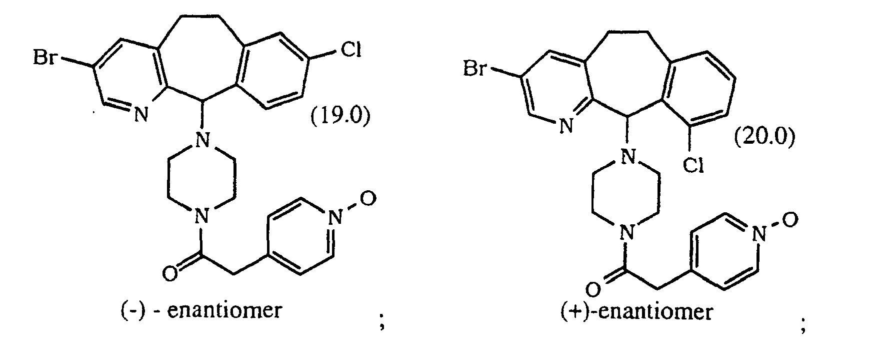 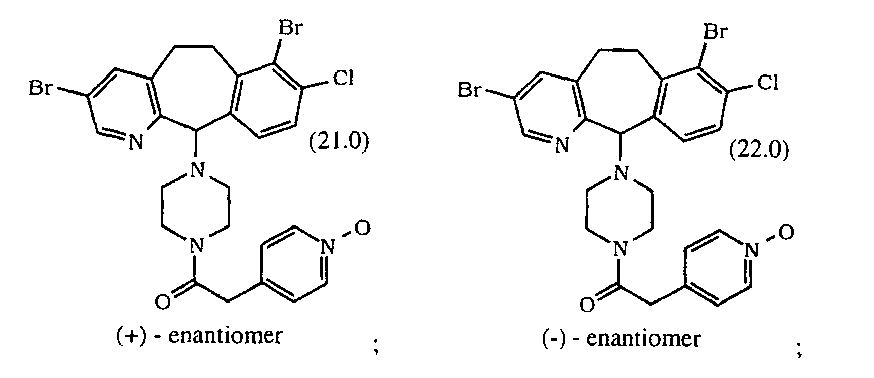 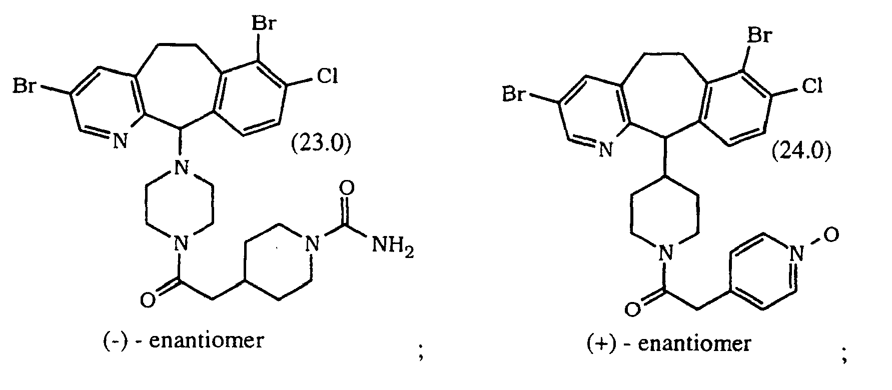 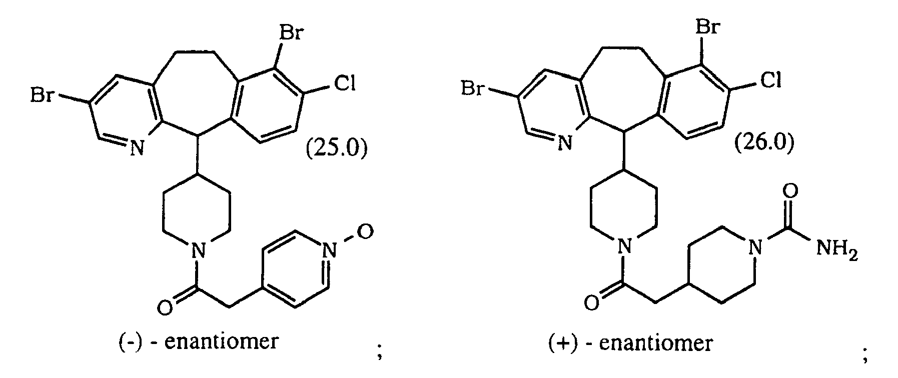 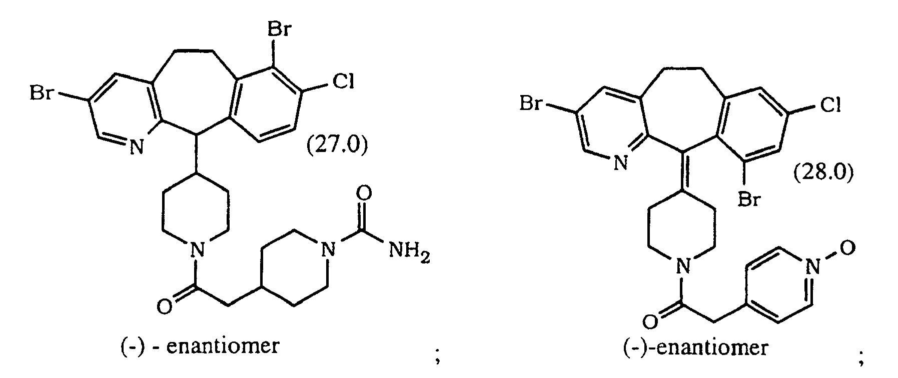 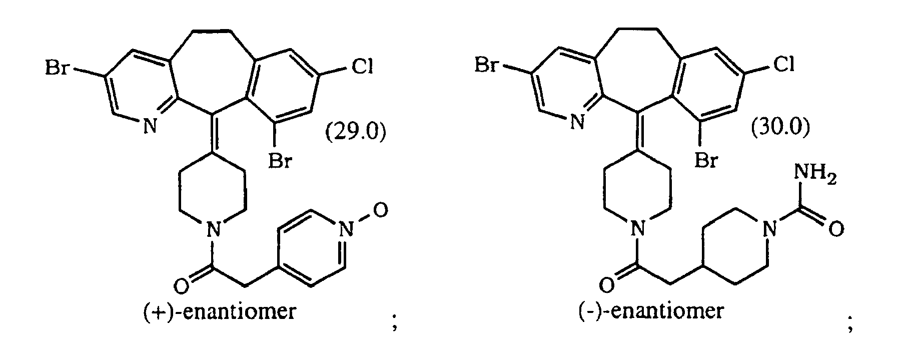 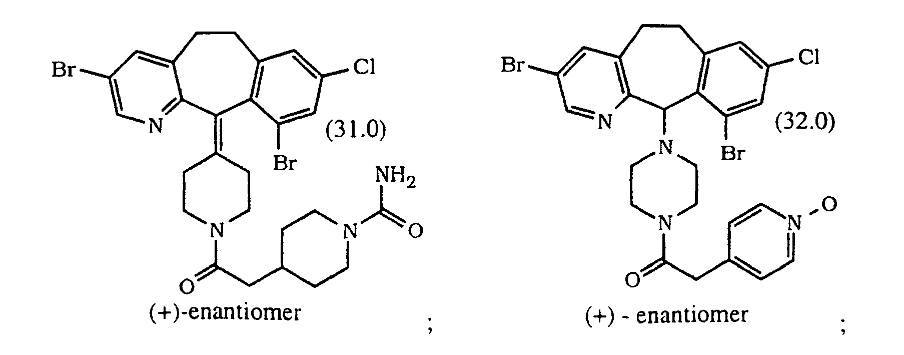 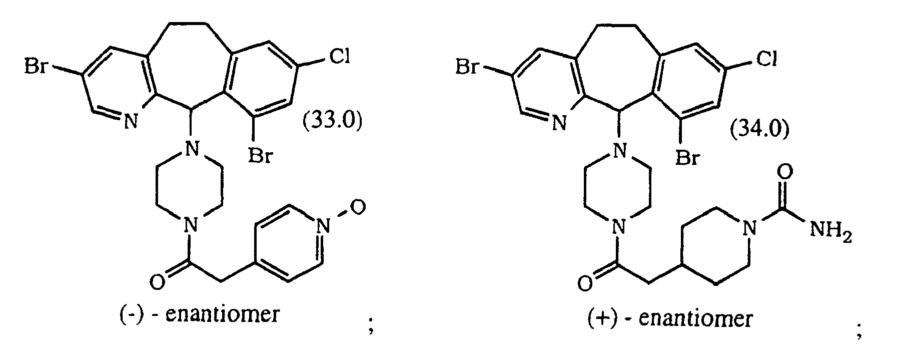 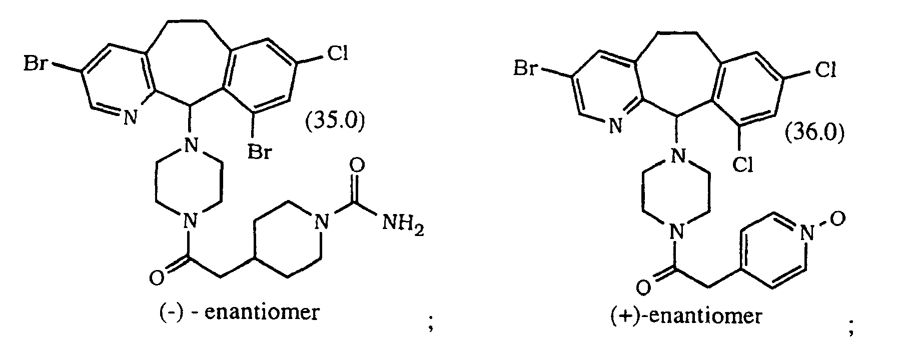 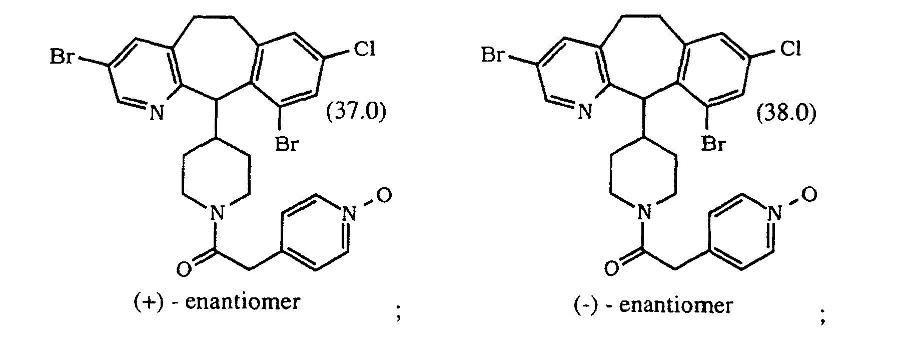 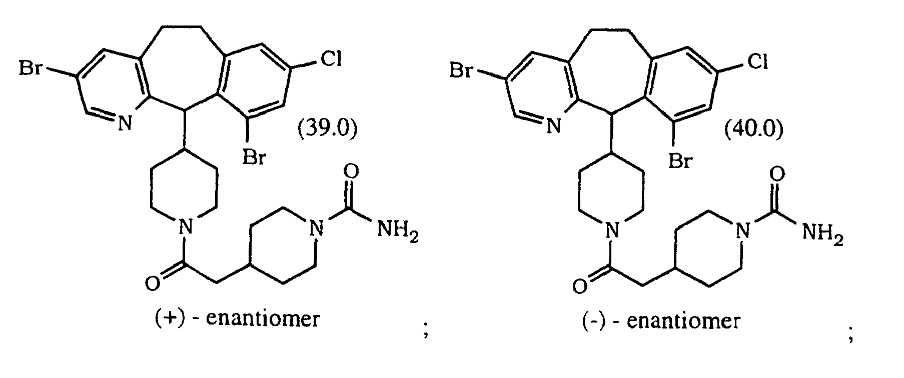 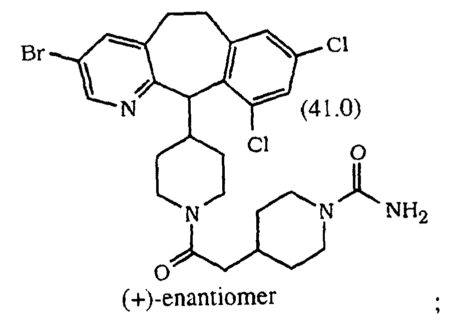 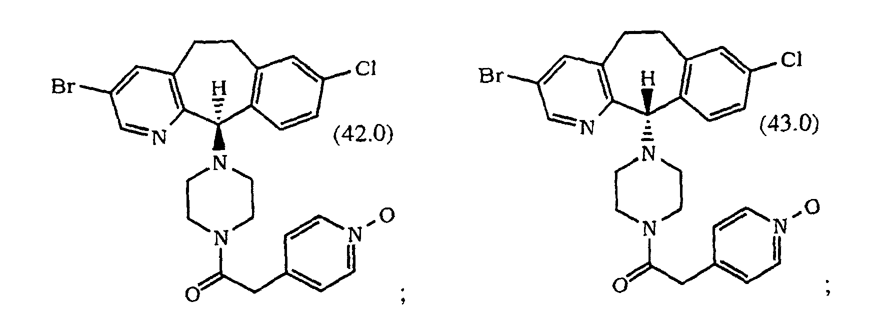 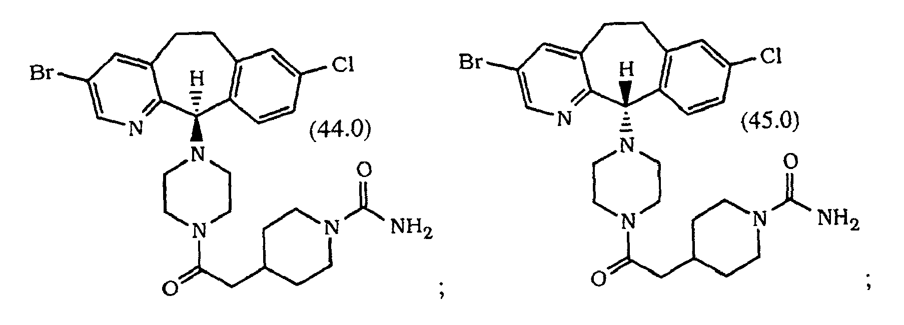 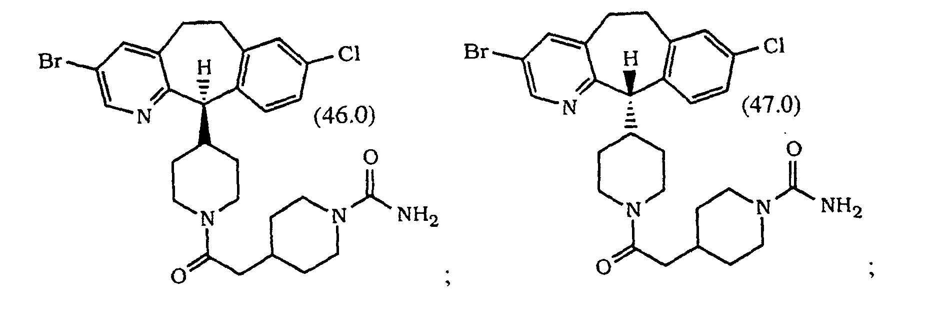 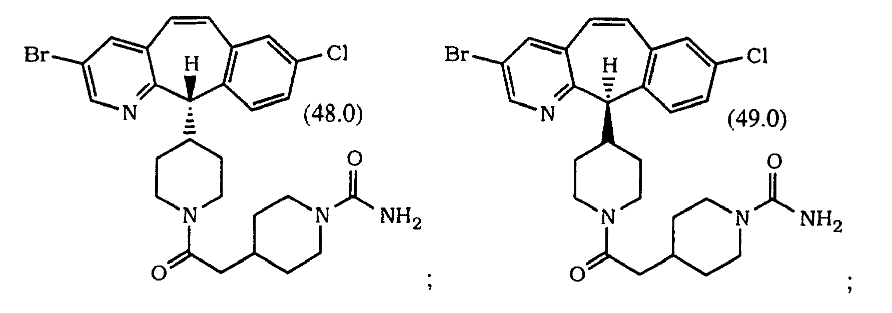 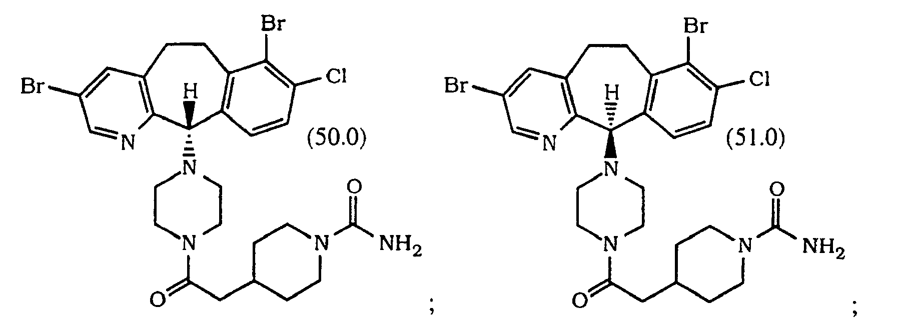 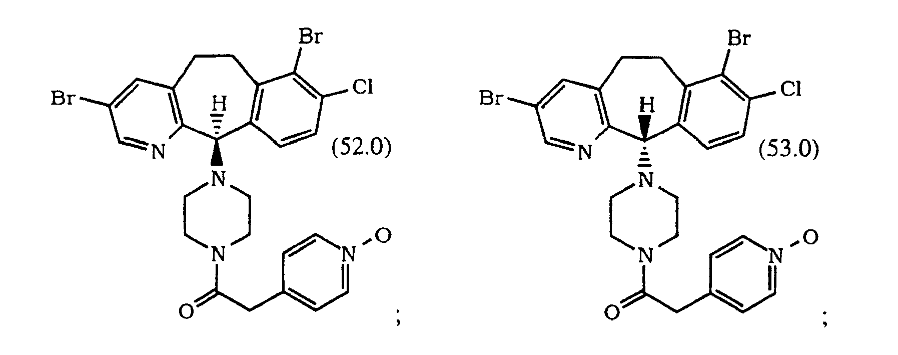 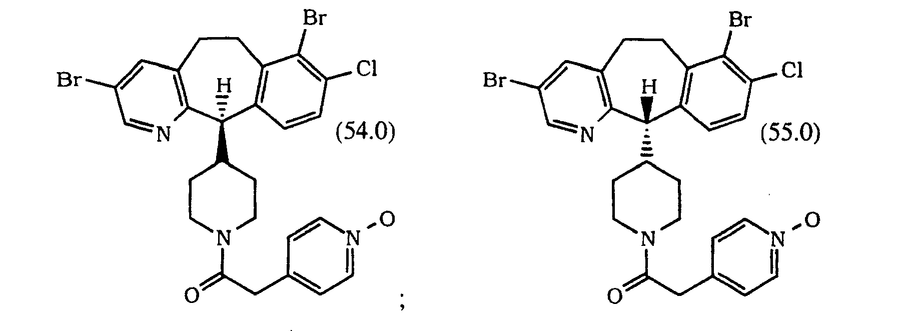 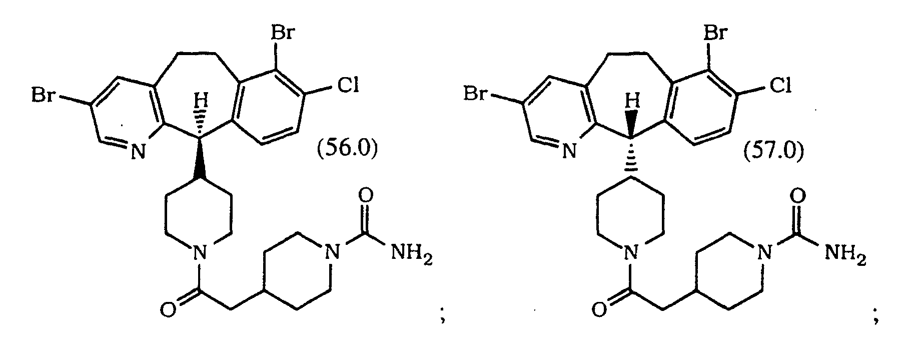 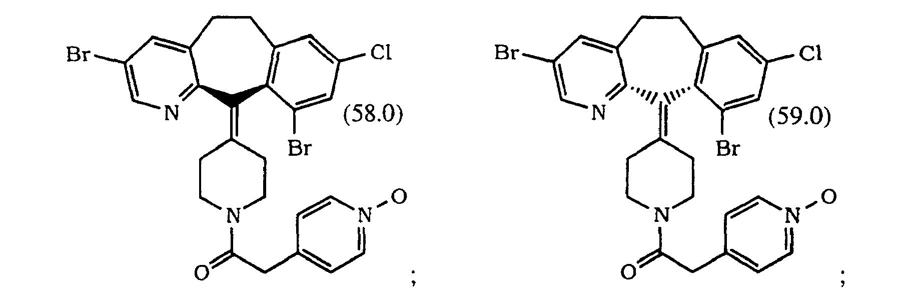 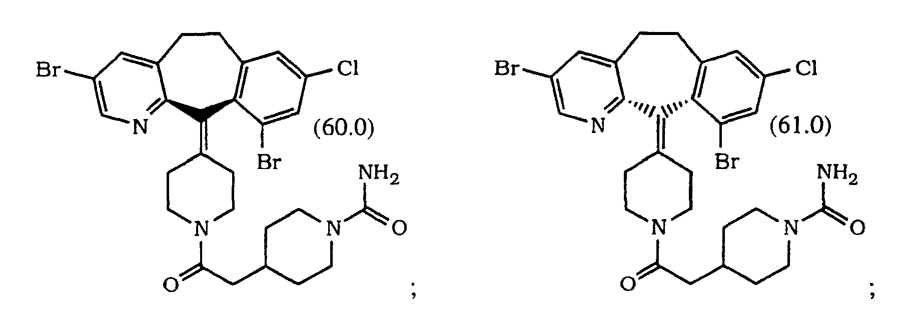 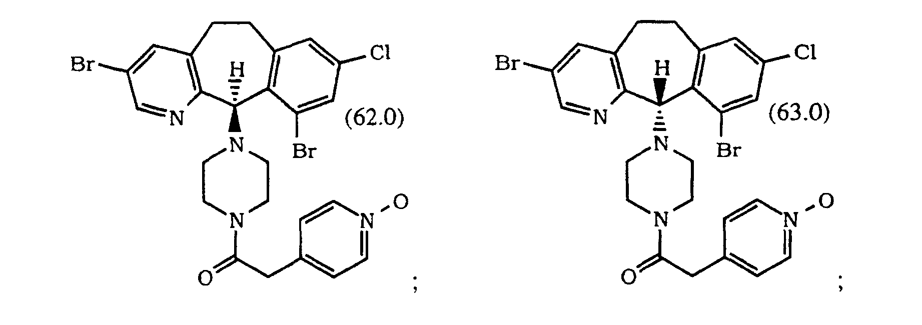 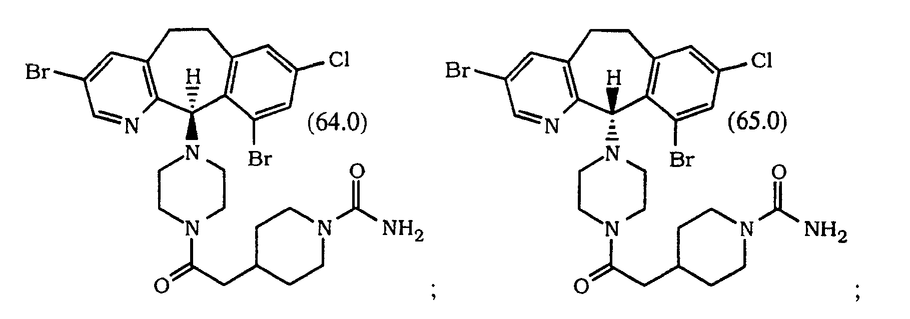 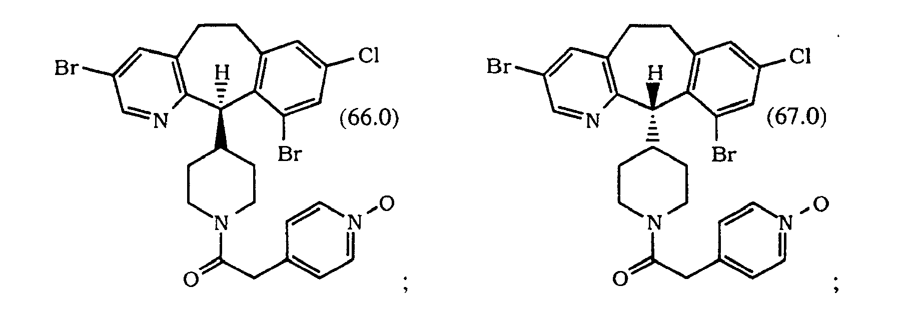 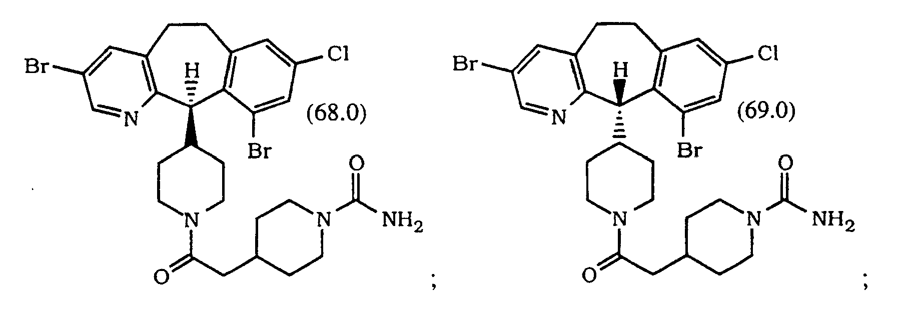 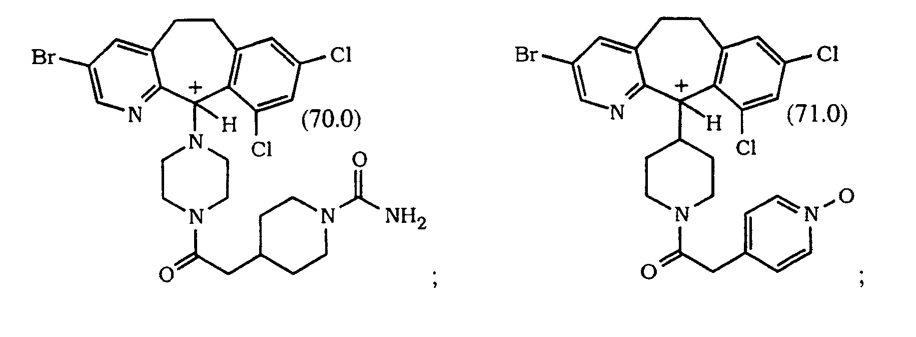 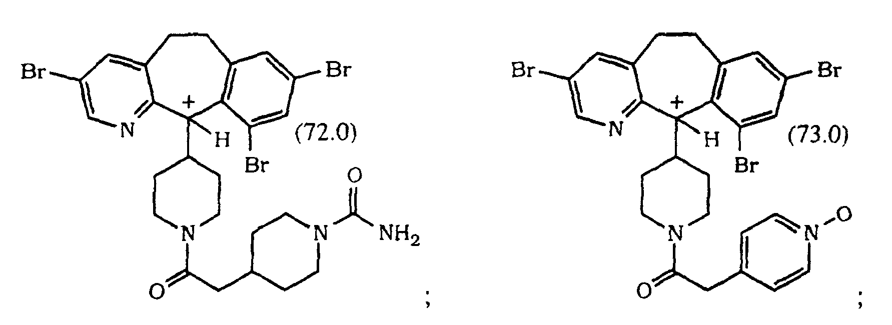 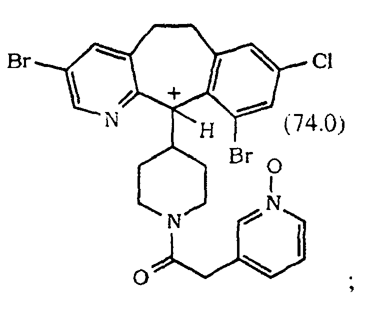 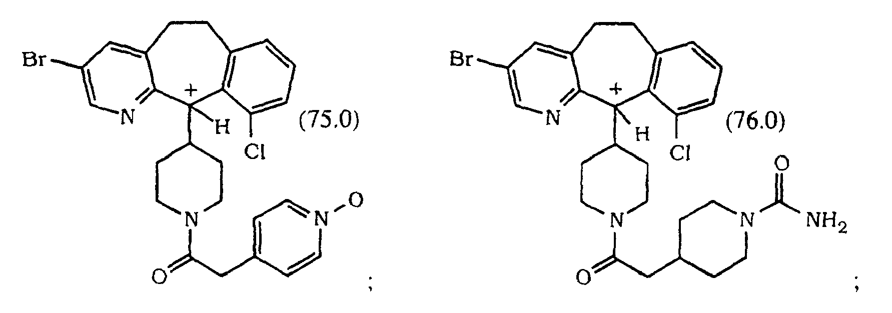 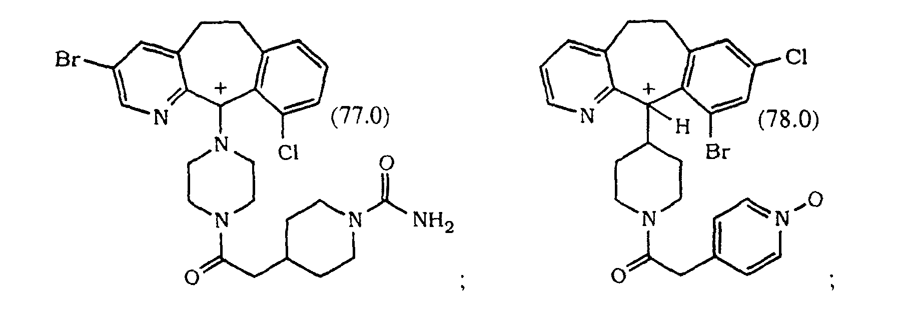 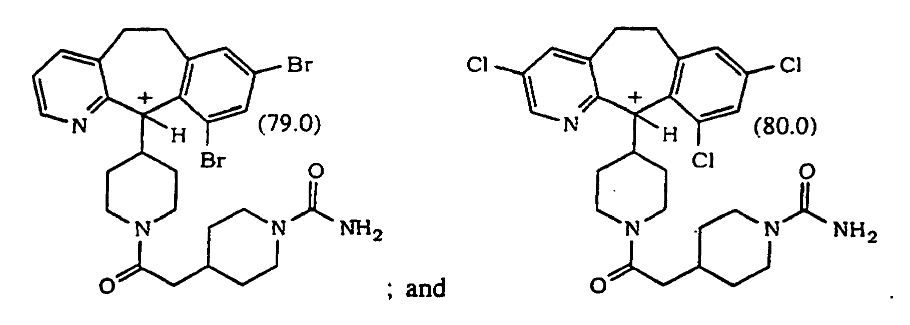
or pharmaceutically acceptable salts thereof.

The compound used as FPT inhibitor in the present invention has the formula: 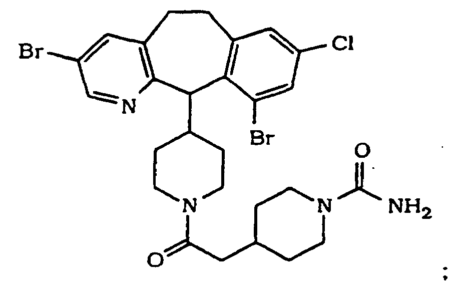
i.e., the compound 4-[2-14-[(8-chloro-3,10-dibromo-6.11-dihydro-5H-benzo[5,6]cyclohepta[1,2-b]pyridin-11-yl)-1-piperidinyl]-2-oxoethyl]-1-piperidinecarboxamide, in fact the (+)-isomer thereof, which has the structure 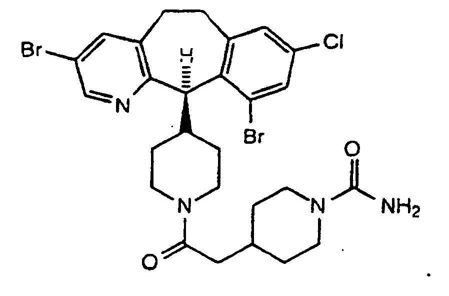
*See also* U.S. Patent No. 5.719.148, which issued on February 17, 1998.

WO 96/31501 published October 10, 1996 discloses compounds of formula (1.0): 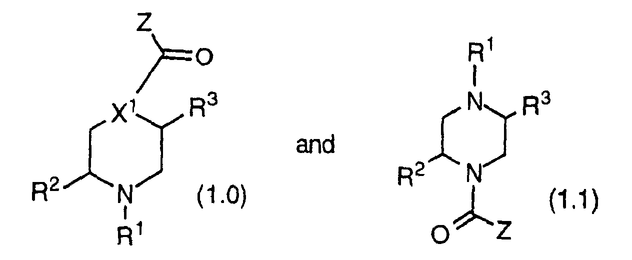
or a pharmaceutically acceptable salt or solvate thereof, wherein:
(1) Z is a group which is: 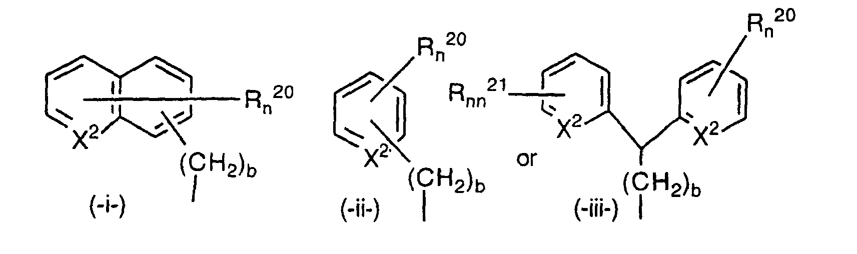
   wherein X¹ is CH or N;
   X² can be the same or different and can be CH, N or N-O;
   b is 0, 1, 2, 3 or 4;
   n and nn independently represent 0, 1, 2, 3, 4 or when X² is CH, n and nn can be 5;
   R²⁰ and R²¹ can be the same group or different groups when n or nn is 2, 3, 4 or 5, and can be:
   (a) hydrogen, C₁ to C₆ alkyl, aryl, aralkyl, heteroaryl, heteroarylalkyl or heterocycloalkyl, wherein each of said C₁ to C₆ alkyl, aryl, aralkyl, heteroaryl, heteroarylalkyl or heterocycloalkyl can be optionally substituted with one or more of the following:
      C₁ to C₄ alkyl, C₃-C₆ cycloalkyl,
      (CH₂)ₜOR⁸ wherein t is 0, 1, 2, 3 or 4,
      (CH₂)ₜNR⁸R⁹ wherein t is 0, 1, 2, 3 or 4, or
      halogen;
   (b) C₃ to C₆ (c) -OR⁸; (d) -SR⁸; (e) -S(O)R⁸; cycloalkyl;
   (f) -SO₂R⁸; (g) -NR⁸R⁹; (h) -CN; (i) -NO₂,
   (j) -CF₃ or (k) halogen (l) -CONR⁸R⁹ or (m) -COR¹³ wherein R⁸ and R⁹ can independently represent:
      H, C₁ to C₄ alkyl, C₃ to C₆ cycloalkyl, heteroaryl, heteroarylalkyl, heterocycloalkyl, aryl or aralkyl and each of said alkyl, cycloalkyl, heteroaryl, heteroarylalkyl, heterocycloalkyl, aryl or aralkyl can be optionally substituted with one to three of the following:
         C₁ to C₄ alkoxy, aryl, aralkyl, heteroaryl, heteroarylalkyl, heterocycloalkyl, halogen, -OH, -C(O)R¹³, -NR¹⁴R¹⁵;
         -CONR⁸R⁹ or -N(R⁸)COR¹³; -CN; C₃-C₆ cycloalkyl, S(O)_{q}R¹³; or C₃-C₁₀ alkoxyalkoxy wherein q is 0, 1 or 2;
         wherein R¹³ is selected from C₁ to C₄ alkyl, aryl or aralkyl, and
         R¹⁴ and R¹⁵ are independently selected from H, C₁ to C₄ alkyl or aralkyl;

      and optionally, when R⁸ and R⁹ are bound to the same nitrogen, R⁸ and R⁹, together with the nitrogen to which they are bound, can form a 5 to 7 membered heterocycloalkyl ring which may optionally contain O, NR⁸, S(O)q wherein q is 0, 1 or 2;
      with the proviso that R⁸ is not H in substituents (e) and (f), and with the proviso that R⁸ or R⁹ is not -CH₂OH or -CH₂NR¹⁴R¹⁵ when R⁸ or R⁹ is directly attached to a heteroatom;
(2) R¹ is a group which is: 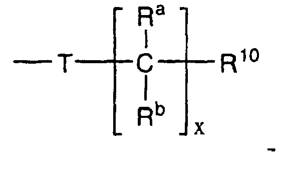
   wherein 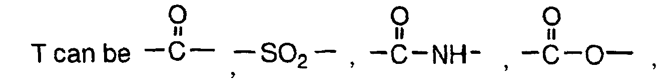 or a single bond,
   x=0, 1, 2, 3, 4, 5 or 6,
   R^{a} and R^{b} independently represent H, aryl, alkyl, amino, alkylamino, alkoxy, aralkyl, heterocyloalkyl, -COOR¹⁶, -NH(CO)_{z}R¹⁶ wherein z = 0 or 1, -(CH₂)_{w}S(O)ₘR¹⁶ wherein w=0, 1, 2 or 3 such that when x is greater than 1, then R^{a} and R^{b} can be independent of the substituents on an adjacent carbon atom provided R^{a} and R^{b} are not both selected from alkoxy, amino, alkylamino, and -NH(CO)_{z}R¹⁶;
   m = 0, 1 or 2 wherein
   R¹⁶ represent H, alkyl, aryl or aralkyl,
   or R^{a} and R^{b} taken together can represent cycloalkyl, =O, =N-O-alkyl or heterocycloalkyl, and
   R¹⁰ can represent H, alkyl, aryl, aryloxy, arylthio, aralkoxy, aralkthio, aralkyl, heteroaryl, heterocycloalkyl,
   or R¹ can also be 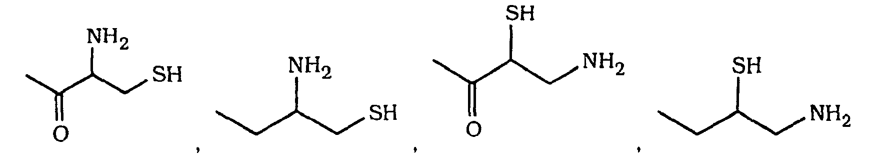
   or disulfide dimers thereof;
(3) R² and R³ are independently selected from the group which is:
   hydrogen, C₁ to C₈ alkyl, C₂ to C₈ alkenyl, C₂ to C₈ alkynyl, 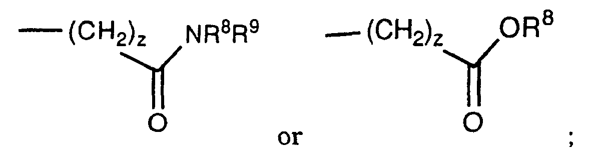
      wherein z is 0, 1, 2, 3 or 4; and said alkyl, alkenyl, or alkynyl group is optionally substituted with one or more groups which can independently represent:
      (a) aryl, aralkyl, heteroaryl, heteroarylalkyl or heterocycloalkyl, wherein each of said aryl, aralkyl, heteroaryl, heteroarylalkyl or heterocycloalkyl group can be optionally substituted with one or more of the following:
         C₁ to C₄ alkyl,
         (CH₂)ₜOR⁸ wherein t is 0, 1, 2, 3 or 4,
         (CH₂)ₜNR⁸R⁹ wherein t is 0, 1, 2, 3 or 4, or
         halogen:
      (b) C₃ to C₆ (c) -OR⁸; (d) -SR⁸; (e) -S(O)R⁸; cycloalkyl;
      (f) -SO₂R⁸; (g) -NR⁸R⁹;

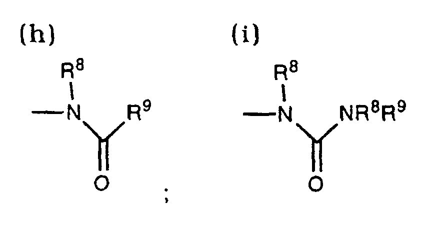

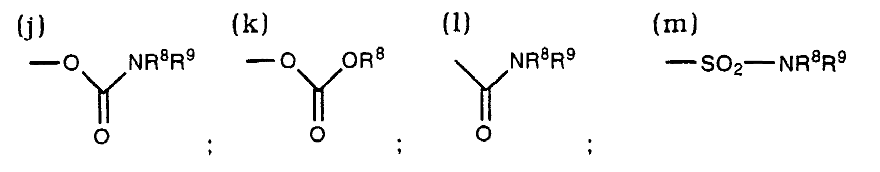

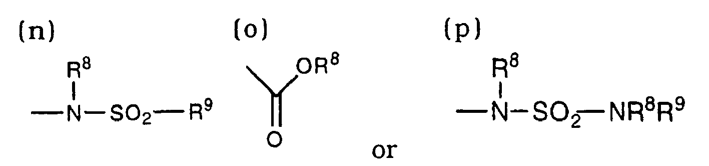

      wherein R⁸ and R⁹ are defined hereinbefore; and
      and optionally, when R⁸ and R⁹ are bound to the same nitrogen, R⁸ and R⁹, together with the nitrogen to which they are bound, can form a 5 to 7 membered heterocycloalkyl ring which may optionally contain O, NR⁸, S(O)q wherein q is 0, 1 or 2;
      with the proviso that for compound (1.0) when X¹ is CH, then R³ is hydrogen,
      and with the further proviso that R² and R³ cannot both be hydrogen;
      and with the provision that when X¹ is N, then R¹ is not

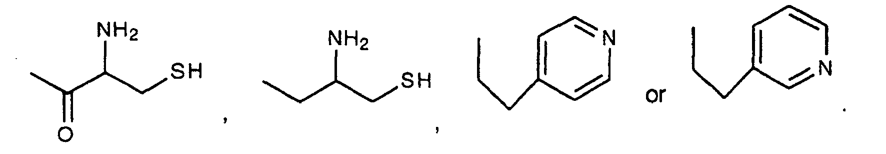

WO 95/10514 published April 20, 1995 discloses compounds of the formula: 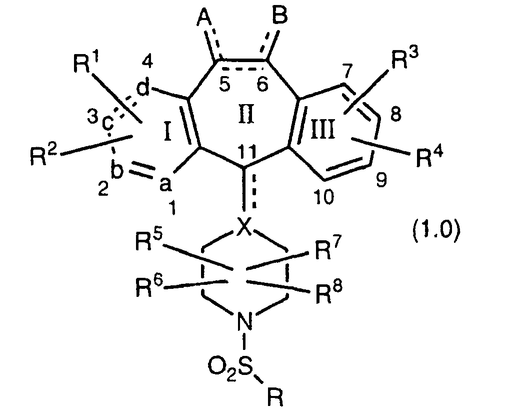
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²;
R¹ and R² are the same or different and each independently represents H, halo, benzotriazol-lyloxy, -CF₃, -OR¹⁰, -COR¹⁰, -SR¹⁰, -S(O)ₜR¹¹ (wherein t is 0, 1 or 2), -N(R¹⁰)₂, -NO₂, -OC(O)R¹⁰, -CO₂R¹⁰, -OCO₂R¹¹, -CN, -NR¹⁰COOR¹¹, alkynyl, alkenyl or alkyl, which alkyl or alkenyl group may be substituted with halo, -OR¹⁰ or -CO₂R¹⁰ ;
R³ and R⁴ are the same or different and each independently represents, any of the substituents of R¹ and R², or R³ and R⁴ together may represent a saturated or unsaturated C₅-C₇ ring fused to the benzene ring (Ring III);
R⁵, R⁶, R⁷ and R⁸ each independently represent H, -CF₃, -COR¹⁰, alkyl or aryl, which alkyl or aryl may be substituted with -OR¹⁰, -SR¹⁰, S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or one of R⁵, R⁶, R⁷ and R⁸ may be taken in combination with R as defined below to represent -(CH₂)ᵣ - wherein r is 1 to 4 which may be substituted with lower alkyl, lower alkoxy, -CF₃ or aryl;
R¹⁰ represents H, alkyl or aryl;
R¹¹ represents alkyl or aryl;
X represents N or C, which C may contain an optional double bond to carbon atom 11;
the dotted lines represent optional double bonds;
the dotted line between carbon atoms 5 and 6 represents an optional double bond, such that when a double bond is present, A and B independently represent -R¹⁰, -OR¹¹, OCO₂R¹¹ or -OC(O)R¹⁰, and when no double bond is present between carbon atoms 5 and 6, A and B each independently represent H₂, -(OR¹¹)₂, H and halo, dihalo, alkyl and H, (alkyl)₂, -H and -OC(O)R¹⁰, H and -OR¹⁰, =O, aryl and H, =NOR¹⁰ or -O-(CH₂)ₚ-O-wherein p is 2, 3 or 4;
R is selected from the group consisting of:
   (1) C₁ to C₄ alkyl (e.g., methyl, ethyl, and butyl);
   (2) phenyl substituted with 1 to 3 substituents selected from R¹, R² or C(O)OR²⁰, wherein R²⁰ is selected from the group consisting of: C₁ to C₆ alkyl (e.g., methyl) and H;
   (3) bridged polycyclic hydrocarbons having from 5 to 10 carbon atoms (*e.g*., adamantyl, norbornyl, norcamphoryl--i.e., the radical formed from 2-norbornaneone, and 2-norboranol);
   (4) substituted bridged polycyclic hydrocarbons, wherein the bridged unsubstituted polycyclic hydrocarbon contains 5 to 10 carbon atoms, wherein the substituents are selected from the group consisting of C₁ to C₆ alkyl (*e.g*., methyl), said substituted bridged polycyclic hydrocarbon having from 1 to 8 substituents with two being preferred, and each substituent being the same or different (with the same being preferred);
   (5) -CH₂R²¹ wherein R²¹ is aryl (e.g., phenyl or substituted phenyl--i.e., phenyl substituted with 1 to 3, preferably 1, group selected from halo, alkyl, haloalkyl or alkoxy), heteroaryl (e.g., thiophene, thiazole, pyridyl, such as 3- or 4- pyridyl, or pyridyl N-oxide, such as 3- or 4-pyridyl N-oxide), 2-,3- or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl, a bridged polycyclic hydrocarbon, or a substituted bridged polycyclic hydrocarbon as described above, e.g., 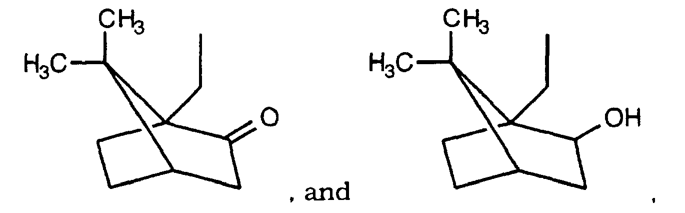
   (6) heteroaryl (e.g., thiophene, thiazole, pyridyl, such as 3- or 4- pyridyl, or pyridyl N-oxide, such as 3- or 4-pyridyl N-oxide);
   (7) substituted heteroaryl wherein said substituents are selected from the group consisting of: C₁ to C₆ alkyl (e.g., methyl) and -NHC(O)R²² wherein R²² is a C₁ to C₆ alkyl (e.g., methyl), e.g., 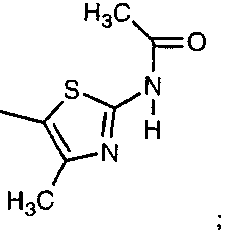
   (8) C₂ to C₆ alkenyl (e.g., -CH=CH₂);
   (9) benzyl; and
   (10) -N(R²³)₂ wherein each R²³ is independently selected from the group consisting of:C₁ to C₆ alkyl, H, aryl (e.g., phenyl and substituted phenyl), 2-,3- or 4-piperidyl or N-substituted piperidyl, wherein the substituent on said N-substituted piperidyl is C₁ to C₄ alkyl, alkylcarbonyl or -C(O)NH(R¹⁰) wherein R¹⁰ is H or alkyl (preferably, 3- or 4-N-substituted piperidyl wherein the substituent on the nitrogen is C₁ to C₄ alkyl, most preferably methyl), heteroaryl (e.g., pyridyl, such as 3- or 4-pyridyl, or 3- or 4- pyridyl N-oxide), preferably, each R²³ is selected such that there is no more than one H bound to the nitrogen (i.e., preferably there is 0 or 1 H attached to the nitrogen), most preferably one of the two R²³ substituents is H, more preferably one of the two R²³ substituents is H and the other R²³ substituent is other than H.

International Patent Application No. PCT/US97/17314 filed October 7, 1997 discloses compounds of the formula: 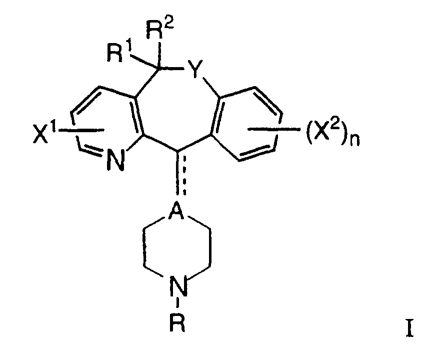
wherein:
X¹ is hydrogen, halogen, CF₃, nitro, NH₂ or lower alkyl;
each X² is independently selected from the group consisting of hydrogen, halogen, lower alkoxy and lower alkyl;
n is 1 or 2;
Y is selected from the group consisting of S(O)ₚ, O, and NR⁵, wherein p is 0, 1 or 2, and R⁵ is hydrogen, alkyl, aryl, cycloalkyl, loweralkoxycarbonyl, aminocarbonyl or acyl;
R¹ and R² are the same or different and are selected from the group consisting of hydrogen and lower alkyl groups, or taken together can form an oxygen atom when Y is NR⁵;
the dotted line indicates a single or double bond--i.e., the dotted line indicates that the bond from A to C-11 of the tricyclic ring can be a single bond or a double bond;
A is a C atom (when the dotted line indicates a double bond, i.e., when there is a double bond from A to the C-11 of the tricyclic ring) or CH or an N atom (when the dotted line indicates a single bond, i.e., when there is a single bond from A to the C-11 of the tricyclic ring);
R is -CZ-Y¹-Y²-R³, wherein:
Z is O, =CH-CN, or =N-CN;
one of Y¹ and Y² is a bond, -CO-, O, S, or -NR⁴-, and the other is (CH₂)ₘ, where m is 0 or an integer of 1 to 4, and R⁴ is H or alkyl, with the proviso that when Z is O and m is 0 then Y¹ or Y² is selected from -CO-, O, S, or -NR⁴;
R³ is aryl, heteroaryl or heterocycloalkyl, with the proviso that R³ can also be lower alkyl when Z is =N-CN;

and their pharmaceutically acceptable acid addition salts.

International Patent Application No. PCT/US97/ 15899 filed September 11. 1997 discloses compounds of the formula: 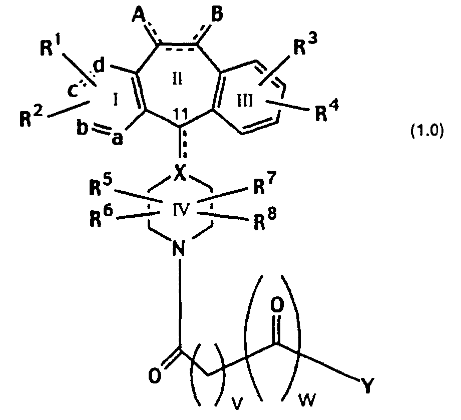
wherein:
X is N, CH, or C when the double bond is present at the C-11 position;
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 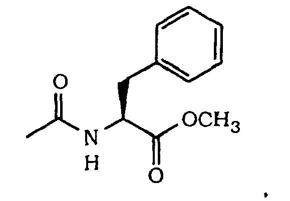
   -SR¹¹C(O)OR¹¹ (e.g., -SCH₂CO₂CH₃), -SR¹¹N(R¹²)₂ wherein each R¹² is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰, or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)p-O- wherein p is 2, 3 or 4;
v is 0 to 5;
w is 0 or 1;
Y is 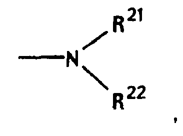 -O-C₁-C₆-alkyl or OM+, wherein M+ is an alkali metal cation;
R²¹ and R²² are each independently H, C₁-C₆ alkyl, -CH₂CONH₂, phenyl, benzyl, -SO₂-(C₁-C₆-alkyl), -NH-phenyl, acyl, C₃-C₆ cycloalkyl, pyridyl, chloro-phenyl, 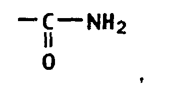 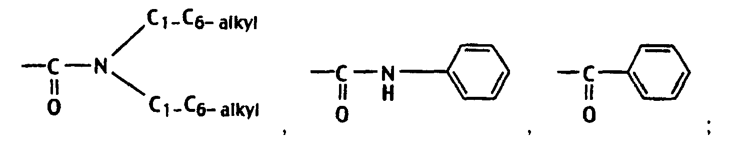
or R²¹ and R²² taken together with the nitrogen to which they are attached form 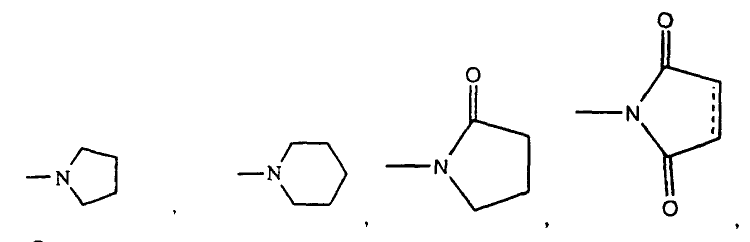 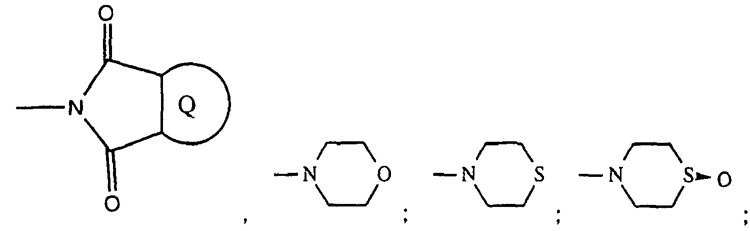 or 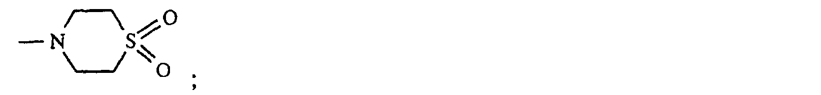
a dashed line means an optional chemical bond;

wherein Q is benzene, or a heterocyclic ring such as pyridine, pyrazine, or thiophene;
or a pharmaceutically acceptable salt thereof.

International Patent Application No. PCT/US97/ 15900 filed September 11, 1997 discloses compounds of formula: 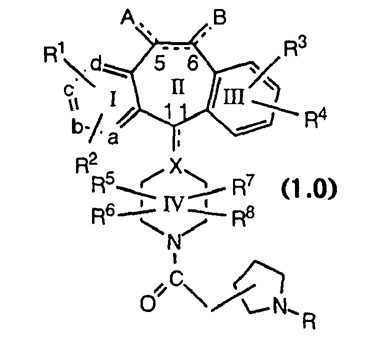
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo. -CF₃, -OR¹⁰ (e.g., -OCH₃), -COR¹⁰, -SR¹⁰ (e.g., -SCH₃ and -SCH₂C₆H₅), -S(O)ₜR¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 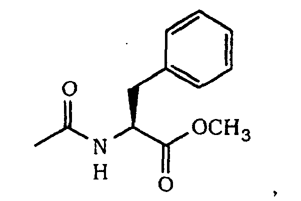
   -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are independently selected from the group consisting of H, R¹ and R², or R³ and R⁴ taken together represent a saturated or unsaturated C₅-C₇ fused ring to the benzene ring (Ring III);
R⁵, R⁶, R⁷ and R⁸ are independently selected from the group consisting of H, -CF₃, -COR¹⁰, alkyl and aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰ or OPO₃R¹⁰, or R⁵ is combined with R⁶ to represent =O or =S, or R⁷ is combined with R⁸ to represent =O or =S;
R¹⁰ represents H, alkyl, aryl, or aralkyl (e.g., benzyl);
R¹¹ represents alkyl or aryl;
X represents N, CH or C, which C may contain an optional double bond (represented by the dotted line) to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent (H, H), (-OR¹¹, -OR¹¹), (H, halo), (halo, halo), (alkyl, H), (alkyl, alkyl), (H, -OC(O)R¹⁰), (H, -OR¹⁰), =O, (aryl, H) or =NOR¹⁰, or A and B together are -O-(CH₂)ₚ-O- wherein p is 2, 3 or 4; and
R represents:
   (1) -C(O)N(R¹⁰)₂;
   (2) -CH₂C(O)N(R¹⁰)₂;
   (3) -SO₂-alkyl, -SO₂-aryl, -SO₂-aralkyl, -SO₂-heteroaryl or -SO₂ heterocycloalkyl;
   (4) cyano (i.e., CN);
   (5) an imidate represented by the formula: 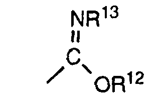
      wherein R¹³ is selected from the group consisting of H, CN, -SO₂₋alkyl (e.g., -SO₂CH₃), -C(O)-aryl (e.g., -C(O)C₆H₅, i.e., -C(O)phenyl), -SO₂NR¹⁰R¹⁴ (e.g., -SO₂NH₂), -C(O)NR¹⁰R¹⁴ (e.g., -C(O)NH₂) and -OR¹⁰ (e.g., OH and -OCH₃); R¹² is aryl; and R¹⁴ is independently selected from the group consisting of H, alkyl, aryl and aralkyl;
   (6) an imidamido group of the formula: 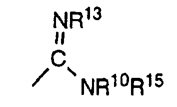
      wherein R¹⁰ and R¹³ are as defined above; R¹⁵ is alkyl, aryl, aralkyl, cycloalkyl, heteroaryl, heteroaralkyl or heterocycloalkyl;
   (7) a 1-amino-2-nitroethylene derivative of the formula: 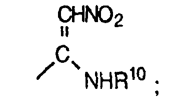
   (8) -C(O)R¹⁶, wherein R¹⁶ is alkyl, aryl, aralkyl or heteroaryl;
   (9) -C(O)-O-R¹⁶;
   (10) 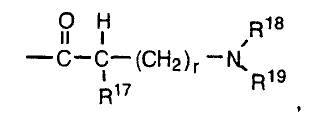
      wherein R¹⁷ is selected from the group consisting of H, alkyl, aralkyl (e.g., benzyl) and heteroaralkyl (e.g., -CH₂-imidazolyl); R¹⁸ and R¹⁹ are each independently selected from the group consisting of: H; -C(O)OR²⁰, wherein R²⁰ represents alkyl, aralkyl, and heteroaralkyl; -SO₂R²¹ wherein R²¹ is selected from the group consisting of alkyl (e.g., C₁₋₆ alkyl, such as methyl), aryl, aralkyl, heteroaryl and heteroaralkyl; -C(O)R²¹; C₁₋₆ alkyl; alkaryl; and C₃₋₆ cycloalkyl; and r is 0, 1 or 2;
   (11) alkyl, aryl, aralkyl, cycloalkyl, heterocycloalkyl or heteroaryl;
   (12) -SO₂NR¹⁰R¹⁴;
   (13) -P(O)(R¹⁰)₂;
   (14) a sugar group of the formula 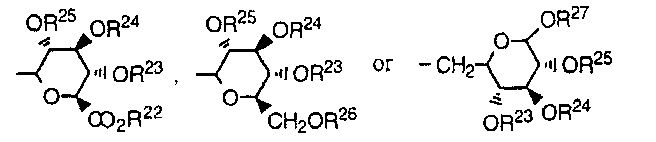
      wherein R²² and R²⁶ are independently selected from the group consisting of H, (C₁-C₆)alkyl, aryl and aryl(C₁-C₆)alkyl; and R²³, R²⁴, R²⁵ and R²⁷ are independently selected from the group consisting of H, (C₁-C₆)alkyl, aryl(C₁-C₆)alkyl, -C(O)(C₁-C₆)alkyl and -C(O)aryl; or
   (15) -CH₂C(O)OR²⁸, wherein R²⁸ is selected from the group consisting of H, alkyl (e.g., -C(CH₃)₃), aryl and heteroaryl.

International Patent Application No. PCT/US97/ 15901 filed September 11, 1997 discloses the compounds:
4-[8-Chloro-3,7-dibromo-5,6-dihydro-11H-benzo [5,6]-cyclohepta[1,2-b]pyridin-11-yl]-1-(4-thiomorpholinylacetyl)-piperidine
4-[8-Chloro-3,7-dibromo-6,11-dihydro-5H-benzo[5,6]cyclohepta[1,2-b]pyridin-11-yl]-1-(4-thiomorpholinylacetyl)piperidine S-oxide ;
(+,-)-1-(3-bromo-8,10-dichloro-5-ethyl-6,11-dihydro-5H-benzo[5,6]cyclohepta[1,2-b]pyridin-11-yl)-4-(4-pyridinylacetyl)-piperazine N4-oxide
(+)-4-(3-bromo-8,10-dichloro-6,11-dihydro-5H-benzo[5,6]-cyclohepta[1,2-b]pyridin-11-yl)-1-[2-(1,3-dihydro-1,3-dioxo-2H-isoindol-2-yl)-1-oxoethyl]piperidine; and
(+)-4-(3,10-Dibromo-8-chloro-6,11-dihydro-5H-benzo[5,6]-cyclohepta[1,2-b]pyridin-11(R)-yl)-1-[(1-oxopropyl-4-piperidinyl)acetyl]piperidine;

or a pharmaceutically acceptable salt or solvate thereof.

International Patent Application No. PCT/US97/ 15902 filed September 11, 1997 discloses compounds of the formula: 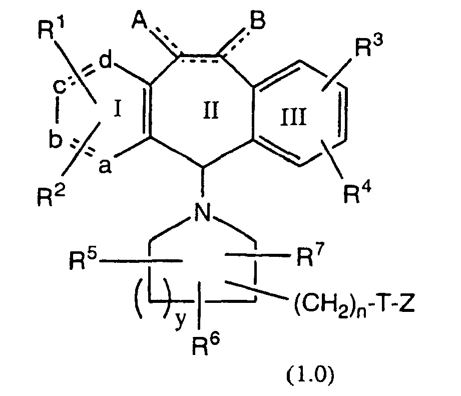
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵ and R⁶ (y=0) or R⁵, R⁶ and R⁷ (y=1) each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or one of R⁵, R⁶ and R⁷ can be taken in combination with R⁴⁰ as defined below to represent -(CH₂)ᵣ- wherein r is 1 to 4 which can be substituted with lower alkyl, lower alkoxy, -CF₃ or aryl, or R⁵ is combined with R⁶ or R⁷ to represent =O or =S;
R¹⁰ independently represents H, alkyl, cycloalkyl, cycloalkylalkyl, heteroaryl, aryl, aralkyl or -NR⁴⁰R⁴² wherein R⁴⁰ and R⁴² independently represent H, aryl, alkyl, aralkyl, heteroaryl, heteroarylalkyl, heterocycloalkyl, heterocycloalkylalkyl, heteroalkyl, cycloalkyl, cycloalkylalkyl, alkenyl and alkynyl;
R¹¹ represents alkyl or aryl;
the dotted line between carbon atoms 5 and 6 represents an optional double bond, such that when a double bond is present, A and B independently represent -NO₂, -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂, H and halo, dihalo, alkyl and H, (alkyl)₂, -H and -OC(O)R¹⁰, H and -OR¹⁰, oxy, aryl and H, =NOR¹⁰ or -O-(CH₂)ₚ-O- wherein p is 2, 3 or 4; and
y is 0 (zero) or 1;
n is 0, 1, 2, 3, 4, 5 or 6;
T is -CO-; -SO-; -SO₂-; or -CR³⁰R³¹- wherein R³⁰ and R³¹ independently represent H, alkyl, aryl, aralkyl, heteroalkyl, heteroaryl, heteroarylalkyl, heterocycloalkyl or heterocycloalkylalkyl;
Z represents alkyl, aryl, aralkyl, heteroalkyl, heteroaryl, heteroarylalkyl, heterocycloalkyl, heterocycloalkylalkyl, -OR⁴⁰, -SR⁴⁰, -CR⁴⁰R⁴² or -NR⁴⁰R⁴² wherein R⁴⁰ and R⁴² are defined hereinbefore

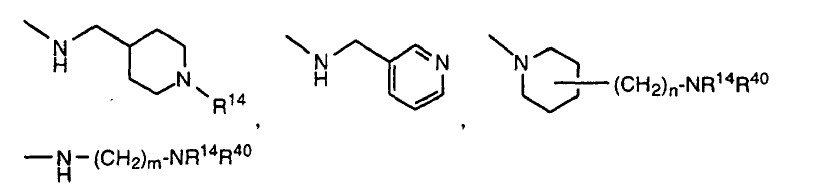

wherein n, R⁴⁰ and R⁴² are defined hereinbefore,
m is 2, 3 4, 5, 6, 7 or 8;
and R¹⁴ represents H, C₁₋₆ alkyl, aralkyl, acyl, carboxamido, cyano, alkoxycarbonyl, aralkyloxycarbonyl, D- and L-amino acids covalently bonded through the carboxyl group, imido, imidamido, sulfamoyl, sulfonyl, dialkylphosphinyl, N-glycosyl, 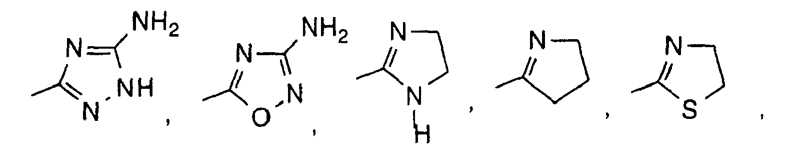 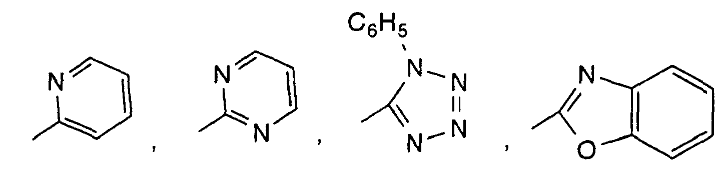 and
-C(NHCH₃)=CHNO₂,
with the proviso that when T is -SO-, Z is not -NR⁴⁰R⁴²

International Patent Application No. PCT/US97/ 15903 filed September 11, 1997 discloses compounds of formula 1.0: 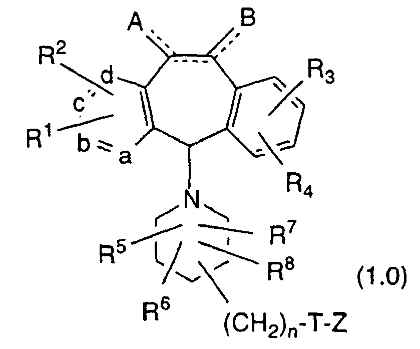
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²: or
each of a, b, c, and d are independently selected from CR¹ or CR²;
each R¹ and each R² is independently selected from H, halo, -CF₃, -OR¹⁰, -COR¹⁰, -SR¹⁰, -S(O)ₜR¹¹ (wherein t is 0, 1 or 2), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, -SR¹¹C(O)OR¹¹, -SR¹¹N(R⁷⁵)₂ wherein each R⁷⁵ is independently selected from H and -C(O)OR¹¹, benzotriazol-1-yloxy, tetrazol-5-ylthio, or substituted tetrazol-5-ylthio, alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰, or R⁵ is combined with R⁶ to represent =O or =S and/or R⁷ is combined with R⁸ to represent =O or =S;
R¹⁰ represents H, alkyl, cycloalkyl, cycloalkylalkyl, heteroaryl, aryl, aralkyl or -NR⁴⁰R⁴² wherein R⁴⁰ and R⁴² independently represent H, aryl, alkyl, aralkyl, heteroaryl, heteroarylalkyl, heterocycloalkyl, heterocycloalkylalkyl, heteroalkyl, cycloalkyl, cycloalkylalkyl, alkenyl and alkynyl;
R¹¹ represents alkyl or aryl;
the dotted line between carbon atoms 5 and 6 represents an optional double bond, such that when a double bond is present, A and B independently represent -NO₂, -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂, H and halo, dihalo, alkyl and H, (alkyl)₂, -H and -OC(O)R¹⁰, H and -OR¹⁰, oxy, aryl and H, =NOR¹⁰ or -O-(CH₂)ₚ-O- wherein p is 2, 3 or 4;
n is 0 (zero), 1, 2, 3, 4, 5 or 6;
T is -CO-; -SO-; -SO₂-; or -CR³⁰R³¹- wherein R³⁰ and R³¹ independently represent H, alkyl, aryl, aralkyl, heteroalkyl, heteroaryl, heteroarylalkyl, heterocycloalkyl or heterocycloalkylalkyl; and
Z represents alkyl, aryl, aralkyl, heteroalkyl, heteroaryl, heteroarylalkyl, heterocycloalkyl, heterocycloalkylalkyl, -OR⁴⁰, -SR⁴⁰, -CR⁴⁰R⁴², -NR⁴⁰R⁴²,

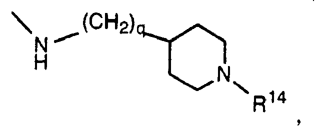

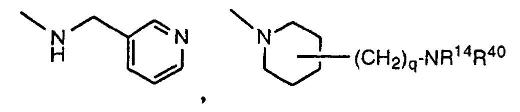

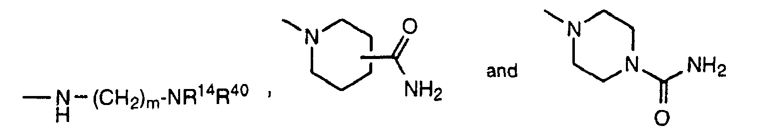

wherein n, R⁴⁰ and R⁴² are defined hereinbefore,
m is 2, 3 4, 5, 6, 7 or 8;
q is 0 (zero), 1 or 2;
and R¹⁴ represents H, C₁₋₆ alkyl, aralkyl, heteroaryl, acyl, carboxamido, carboxamidoalkyl, cyano, alkoxycarbonyl, aralkyloxycarbonyl, D- and L-amino acids covalently bonded through the carboxyl group, imido, imidamido, sulfamoyl, sulfonyl, dialkylphosphinyl, N-glycosyl, 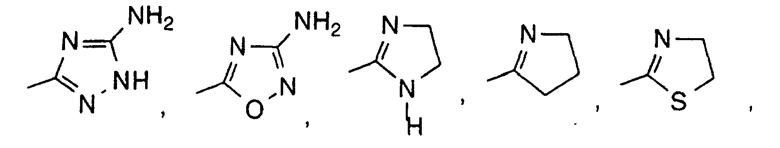 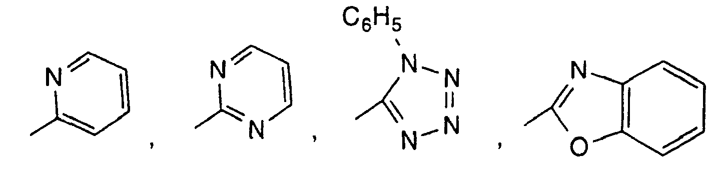 and -C(NHCH₃)=CHNO₂,
with the proviso that when T is -SO-, Z is not -NR⁴⁰R⁴².

International Patent Application No. PCT/US97/ 15904 filed September 11, 1997 discloses compounds of the formula: 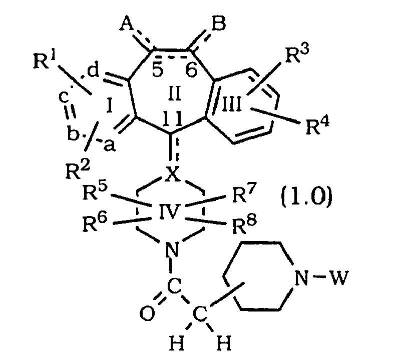
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 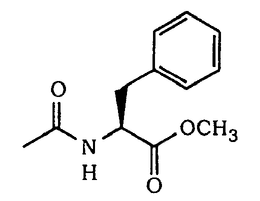
   -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰, or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
X represents N, CH or C, which C may contain an optional double bond (represented by the dotted line) to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)ₚ-O- wherein p is 2, 3 or 4; and
W represents a group selected from the group consisting of:

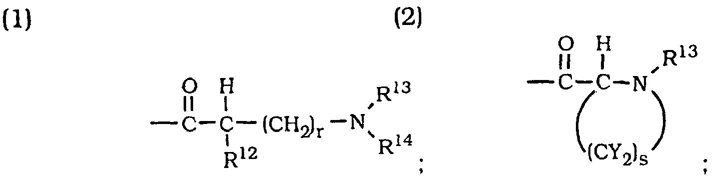

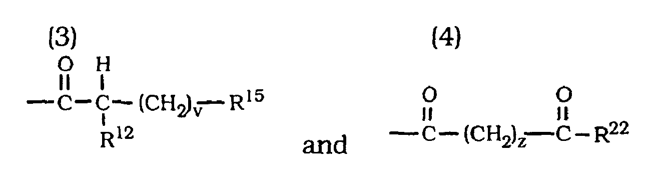

wherein:
R¹² is selected from the group consisting of: (a) H; (b) alkyl; (c) aralkyl (e.g., benzyl); and (d) heteroarylalkyl (heteroaralkyl) (e.g., -CH₂-imidazolyl);
R¹³ and R¹⁴ are each independently selected from the group consisting of: (a) H; (b) -C(O)OR¹⁶ wherein R¹⁶ represents alkyl, aralkyl, and heteroaralkyl; (c) -SO₂R¹⁷ wherein R¹⁷ is selected from the group consisting of: -NH₂, -N(alkyl)₂ wherein each alkyl is the same or different (e.g., -N(CH₃)₂), alkyl (e.g., C₁-₆ alkyl, such as methyl), aryl, aralkyl, heteroaryl and heteroaralkyl; (d) -C(O)R¹⁸ wherein R¹⁸ is selected from the group consisting of: aryl (e.g., phenyl), alkyl, aralkyl, heteroaryl, and heteroaralkyl; (e) C₁₋₆ alkyl; (f) alkaryl; and (g) C₃₋₆ cycloalkyl;
r is 0, 1 or 2;
s represents 1, 2, 3, 4, or 5 (preferably 3 or 4), and each Y for each -CY₂- group is independently selected from H or -OH, provided that both Y substituents of each -CY₂- group are not -OH, and provided that for the -CY₂- group alpha to the nitrogen both Y substituents are H, preferably each Y is H such that each -CY₂- group is a -CH₂- group, such that the group 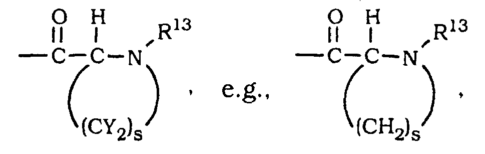
   forms a 3, 4, 5, 6, or 7 (preferably 5 or 6) membered ring (e.g., piperidyl or pyrrolidinyl), ;
v is 0, 1 or 2;
R¹⁵ is selected from the group consisting of:
   (a) heteroaryl (e.g., imidazolyl);
   (b) a group selected from:

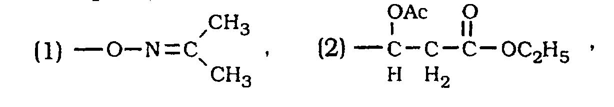

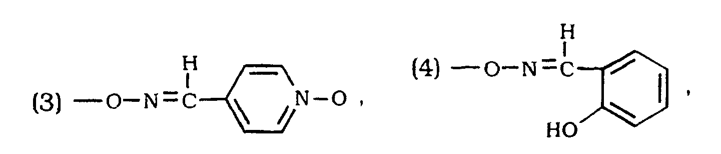

      (5) -CH(OCH₂CH₃)₂,
      (6) -OH, and
      (7) -CN; and
   (c) heterocycloalkyl selected from the group consisting of: 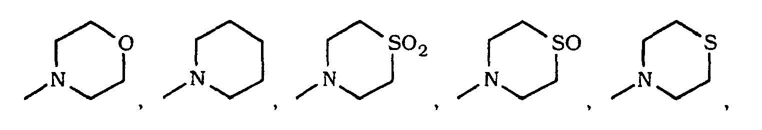 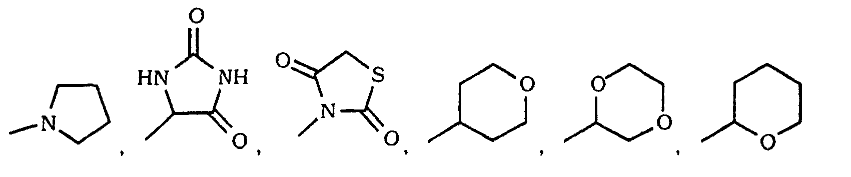 and 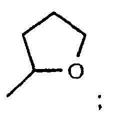
z is 0, 1, 2, 3, 4, or 5 wherein each -CH₂- group is optionally substituted with a -OH group, i.e., each H of each -CH₂- group can optionally be replaced with a -OH group and the optional substitution on each -CH₂- group is independent of the substitution on any other -CH₂- group, generally each -CH₂- is unsubstituted;
R²² represents a group selected from:

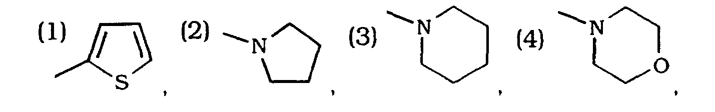

   (5) alkyl (e.g., -CH₃),
   (6) -OR²³ wherein R²³ is selected from the group consisting of: alkyl, aryl and H, and

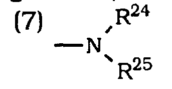

   wherein R²⁴ and R²⁵ are independently selected from the group consisting of: -NH₂, alkoxy (e.g., -OCH₃), -OH, -CH₂CO₂H, -OCH₂Ph (i.e., -OCH₂C₆H₅), -CH(OCH₃)CH(CH₃)₂ 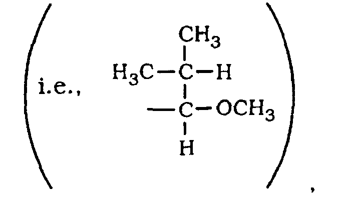 alkyl, aryl, H, aralkyl, and heteroaralkyl; or R²⁴ and R²⁵ taken together form a carbon chain having 4 or 5 (-CH₂-) groups such that R²⁴ and R²⁵ taken together with the nitrogen to which they are bound form a 5 or 6 membered heterocycloalkyl ring.

International Patent Application No. PCT/US97/ 15905 filed September 11, 1997 discloses compounds of the formula: 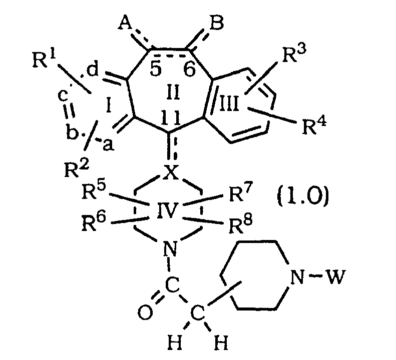
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 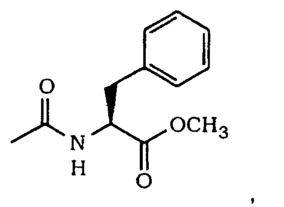
   -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
X represents N, CH or C, which C may contain an optional double bond (represented by the dotted line) to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)ₚ-O- wherein p is 2, 3 or 4; and
W represents a group selected from:

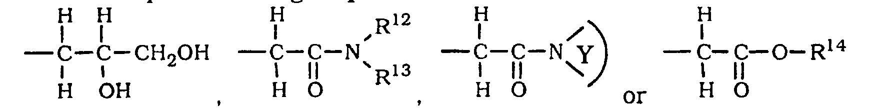

wherein:
R¹² is selected from the group consisting of: (1) H; (2) alkyl (e.g., methyl and ethyl); (3) aryl; (4) arylalkyl (aralkyl);
R¹³ is selected from the group consisting of: (1) H; (2) alkyl (e.g., methyl and ethyl); (3) alkoxy (e.g., methoxy); (4) heterocycloalkyl, e.g., (a) tetrahydopyranyl, and (b) substituted tetrahydropyranyl wherein said substituents are selected from hydroxy and hydroxyalkyl (e.g., hydroxymethyl), for example D-galactosyl, i.e., 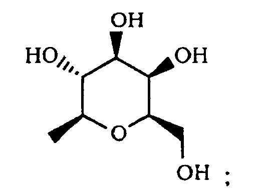
   (5) aryl; and (6) aralkyl, e.g., benzyl;
R¹⁴ is selected from the group consisting of: (1) H; (2) alkyl (e.g., -C(CH₃)₃); (3) aryl; and (4) heteroaryl; ring 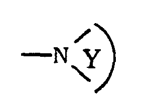
   represents a heterocycloalkyl ring wherein Y represents the remainder of the ring, said remainder comprising carbon atoms and optionally a hetero atom selected from the group consisting of: NH, NR¹⁵, O and S, and said remainder optionally having an aryl ring (e.g., phenyl) fused thereto; generally the heterocycloalkyl ring contains 4 or 5 carbon atoms and usually 4 carbon atoms, examples include: 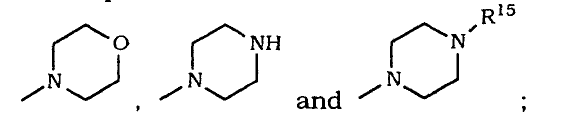
   examples of a heterocycloalkyl ring having a aryl ring fused to the remainder Y include 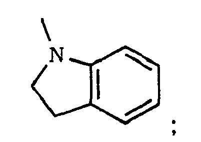
R¹⁵ represents -C(O)OR¹⁶; and
R¹⁶ represents alkyl, preferably -C(CH₃)₃.

International Patent Application No. PCT/US97/15906 filed September 11, 1997 discloses compounds of the formula: 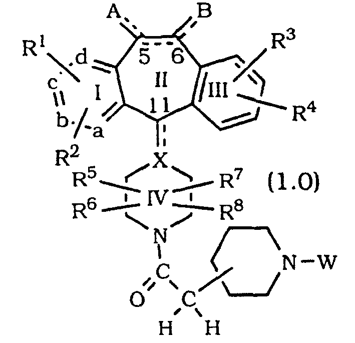
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 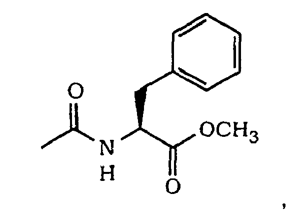
   -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
X represents N, CH or C, which C may contain an optional double bond (represented by the dotted line) to carbon atom 11;
the dotted line between carbon atoms 5 and 6 represents an optional double bond, such that when a double bond is present, A and B independently represent -R¹⁰, halo, -OR¹¹, -OCO₂R¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)p-O- wherein p is 2, 3 or 4; and
W represents a group selected from: -SO₂R¹² or -P(O)R¹³R¹⁴;
R¹² is selected from the group consisting of:
   (1) alkyl, e.g., methyl, ethyl and propyl (such as n-propyl and iso-propyl);
   (2) aralkyl, e.g., benzyl;
   (3) cycloalkyl;
   (4) aryl, e.g., phenyl;
   (5) heteroaryl, e.g., pyridyl, thienyl and imidazolyl (e.g., 4- or 5-imidazolyl);
   (6) substituted heteroaryl wherein said heteroaryl is as defined above and said substituents are selected from: (a) heteroaryl (e.g., pyridyl, and imidazolyl), (b) alkyl (e.g., methyl), (c) aryl (e.g., phenyl), (d) aralkyl (e.g., benzyl), (e) -OR¹⁰, and (f) N(R¹⁰)₂;
   (7) camphor, e.g., 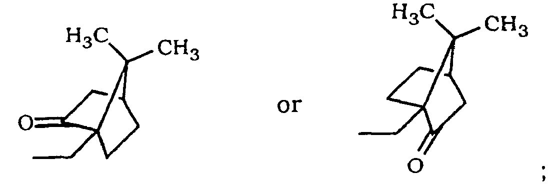 and
   (8) -NR¹⁵R¹⁶ wherein R¹⁵ and R¹⁶ are independently selected from the group consisting of: (a) H, (b) alkyl (e.g., methyl), (c) aryl (e.g., phenyl), (d) aralkyl (e.g., benzyl), (e) heteroaryl (e.g., pyridyl), and (f) heterocycloalkyl (e.g., piperidinyl), and preferably, R¹⁵ and R¹⁶ are the same; and
R¹³ and R¹⁴ are independently selected from the group consisting of:
   (1) H;
   (2) alkyl, e.g., methyl;
   (3) aryl, e.g., phenyl;
   (4) aralkyl, e.g., benzyl; and
   (5) -OR¹³ wherein R¹³ is as defined above;

   preferably R¹³ and R¹⁴ are the same.

International Patent Application No. PCT/US97/15907 filed September 11, 1997 discloses compounds of the formula: 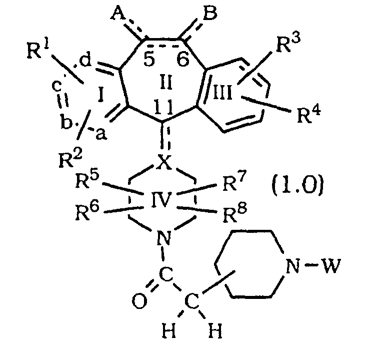
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 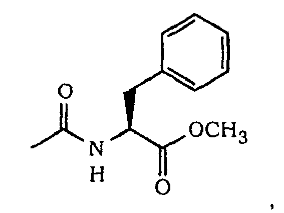
   -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g.. -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
X represents N, CH or C, which C may contain an optional double bond (represented by the dotted line) to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)ₚ-O- wherein p is 2, 3 or 4; and
W represents a heteroaryl, aryl, heterocyloalkyl or cycloalkyl group.

International Patent Application No. PCT/US97/19976 filed September 11, 1997 discloses compounds of the formula: 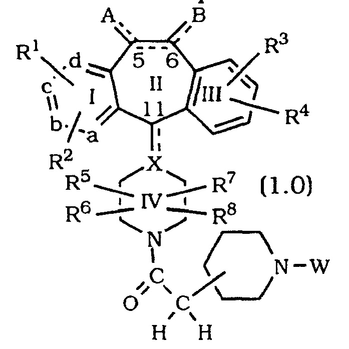
or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 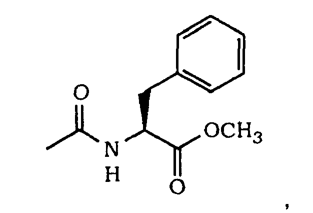
   -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-yl-thio, or substituted tetrazol-5-ylthio (e.g., alkyl substituted tetrazol-5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰, or R⁵ is combined with R⁶ to represent =O or =S and/or R⁷ is combined with R⁸ to represent =O or =S:

R¹⁰ represents H, alkyl, aryl, or aralkyl (e.g., benzyl);
R¹¹ represents alkyl or aryl;
X represents N, CH or C, which C may contain an optional double bond (represented by the dotted line) to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)ₚ-O- wherein p is 2, 3 or 4; and
W is selected from the group consisting of:
   (1) cyano (i.e., CN);
   (2) -C(O)R¹² wherein R¹² is selected from:
      (a) a heteroaryl group, for example, pyridyl (e.g., 3-pyridyl), indolyl (e.g., 2-indolyl), pyrrolyl (e.g., 2-pyrrolyl) and N-substituted pyrrolyl (e.g., N-alkylpyrrolyl such as N-alkylpyrrol-2-yl, such as, N-methylpyrrol-2-yl);
      (b) H;
      (c) alkyl (e.g., -CH₃); or
      (d) a substituent of the formula:

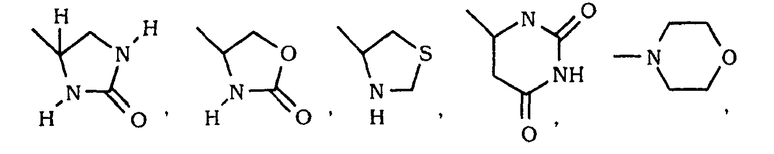

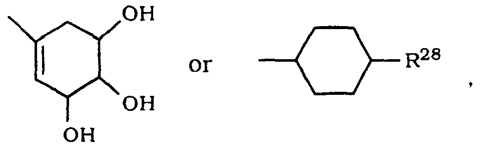

      wherein R²⁸ is selected from -OC(O)R²⁹, -OH, -OC(O)NHC(O)CCl₃, or -OC(O)NH₂, wherein R²⁹ is alkyl (e.g., -CH₃);
   (3) an imidate represented by the formula: 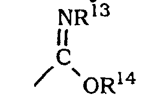
      wherein R¹³ is selected from the group consisting of: (a) H, (b) CN, (c) -SO₂-alkyl (e.g., -SO₂CH₃), (d) -C(O)-aryl (e.g., -C(O)C₆H₅, i.e., -C(O)phenyl), (e) -SO₂NR¹⁰R¹⁵ (e.g., -SO₂NH₂), (f) -C(O)NR¹⁰R¹⁵ (e.g., -C(O)NH₂), (g) -OR¹⁰ (e.g., -OH and -OCH₃); and (h) -C(O)NR¹⁰C(O)NR¹⁰R¹⁵ (e.g., -C(O)NHC(O)NH₂); R¹⁴ is aryl; and R¹⁰ and R¹⁵ are independently selected from the group consisting of: H, alkyl, aryl and aralkyl;
   (4) an imidamido (amidino) represented by the formula: 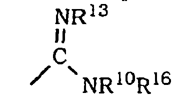
      wherein R¹³ is selected from the group consisting of (a)H, (b) CN, (c) -SO₂-alkyl (e.g., -SO₂CH₃), (d) -C(O)-aryl (e.g., -C(O)C₆H₅, i.e., -C(O)phenyl), (e) -SO₂NR¹⁰R¹⁵ (e.g., -SO₂NH₂), (f) -C(O)NR¹⁰R¹⁵ (e.g., -C(O)NH₂), (g) -OR¹⁰ (e.g., -OH and -OCH₃); and (h) -C(O)NR¹⁰C(O)NR¹⁰R¹⁵ (e.g., -C(O)NHC(O)NH₂); R¹⁶ is selected from the group consisting of: alkyl, aralkyl, aryl, cycloalkyl, heteroaryl, heteroaralkyl and heterocycloalkyl; R¹⁰ and R¹⁵ are as defined above; and R¹⁰ and R¹⁶ are independently selected from the above defined groups;
   (5) 1-amino-2-nitroethylene derivatives of the formula: 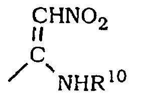
      wherein R¹⁰ is as defined above; and
   (6) a substituent of the formula:

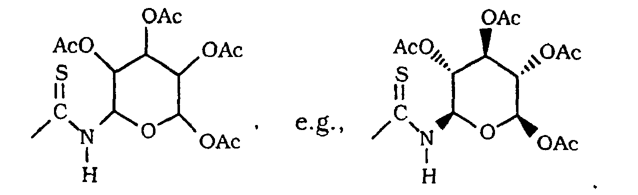

U.S. Application Serial No. 08/877049 filed June 17, 1997 discloses compounds of the formula: 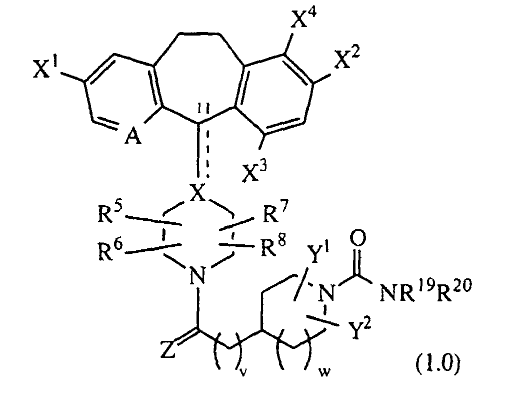
or a pharmaceutically acceptable salt or solvate thereof, wherein:
A represents N or N-oxide;
X represents N, CH or C, such that when X is N or CH, there is a single bond to carbon atom 11 as represented by the solid line; or when X is C, there is a double bond to carbon atom 11, as represented by the solid and dotted lines;
X¹ and X² are independently selected from bromo or chloro, and X³ and X⁴ are independently selected from hydrogen, bromo or chloro provided that at least one of X³ and X⁴ is hydrogen;
Y¹ and Y² are independently selected from hydrogen or alkyl;
Z is =O or =S;
R⁵, R⁶, R⁷ and R⁸ each independently represents hydrogen, -CF₃, -COR¹⁰, alkyl or aryl, and further wherein R⁵ may be combined with R⁶ to represent =O or =S and/or R⁷ may be combined with R⁸ to represent =O or =S;
R¹⁰, R¹⁹ and R²⁰ independently represent hydrogen, alkyl, alkoxy, aryl, aralkyl, heteroaryl, heteroarylalkyl, cycloalkyl, cycloalkylalkyl, heterocycloalkyl and heterocycloalkylalkyl, with the proviso that R¹⁹ and R²⁰ are not both hydrogen;
v is zero, 1, 2 or 3; and
w is zero or 1.

U.S. Application Serial No. 08/877366 filed June 17, 1997 discloses compounds of the formula: 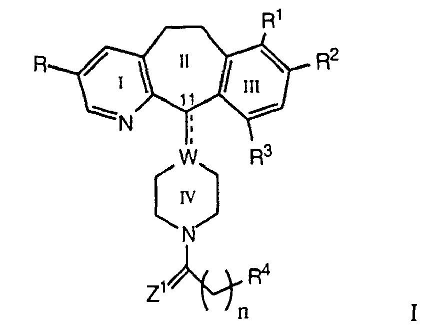 or an N-oxide thereof, or a pharmaceutically acceptable salt or solvate thereof, wherein:
R and R² are independently selected from halo;
R and R³ are independently selected from the group consisting of H and halo, provided that at least one of R¹ and R³ is H;
W is N, CH or C, when the double bond is present at the C-11 position;
R⁴ is 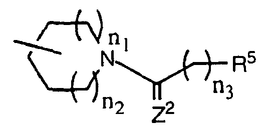 or R⁵;
R⁵ is 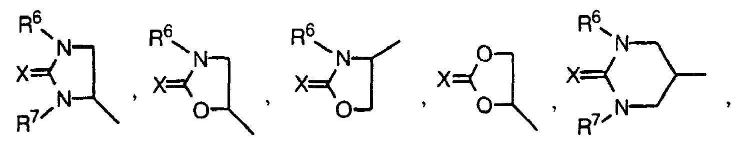 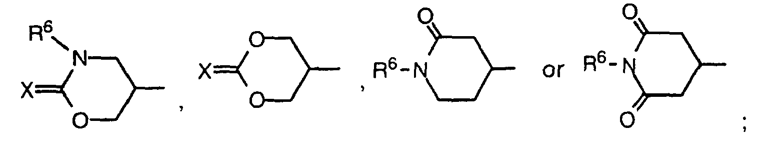
R⁶ and R⁷ are independently selected from the group consisting of H, alkyl, substituted alkyl, acyl, aryl, aralkyl, heterocycloalkyl and heteroaryl;
X is =O or =S;
Z¹ and Z² are independently =O or =S;
n and n₃ are independently 0, 1 or 2; and
n₁ and n₂ are independently 0 or 1.

U.S. Application Serial No. 08/877399 filed June 17, 1997 discloses compounds of the formula: 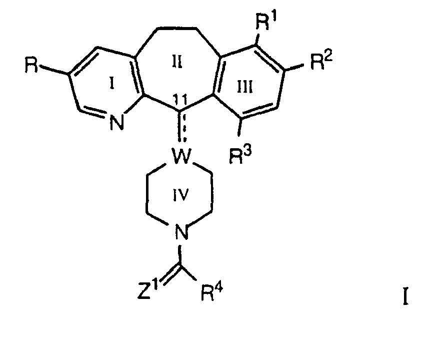 or an N-oxide thereof, or a pharmaceutically acceptable salt or solvate thereof, wherein:
R and R² are independently selected from halo;
R and R³ are independently selected from the group consisting of H and halo, provided that at least one of R¹ and R³ is H;
W is N, CH or C, when the double bond is present at the C-11 position;
R⁴ is 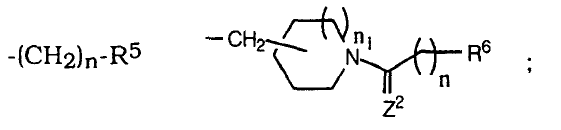 or
R⁵
   is 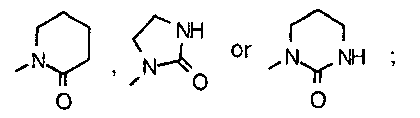
R⁶ is R⁵ or 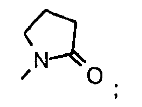
Z¹ and Z² are independently selected from the group consisting of =O and =S;
n is 1-6; and
n₁ is 0 or 1.

U.S. Application Serial No. 08/877336 filed June 17, 1997 discloses compounds of the formula: 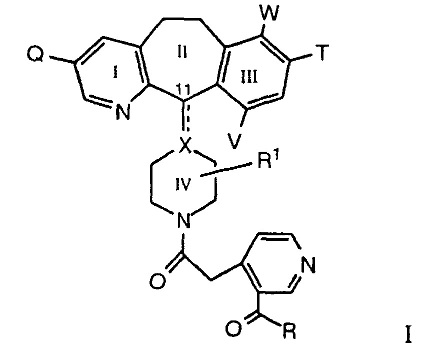
or an N-oxide thereof, or a pharmaceutically acceptable salt or solvate thereof, wherein:
Q and T are independently selected from halo;
W and V are independently selected from H and halo, provided that at least one of W and V is H;
R¹ is H or alkyl;
X represents N, CH, or C when the double bond is present at the C-11 position;
R is -OR³, -NR³R⁴ or -SR³; and
R³ and R⁴ are independently selected form the group consisting of H, alkyl, arylalkyl, substituted arylalkyl, heteroarylalkyl and substituted heteroarylalkyl.

U.S. Application Serial No. 08/877269 filed June 17, 1997 discloses compounds of the formula: 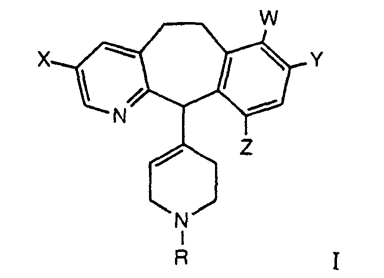
or an N-oxide thereof, or a pharmaceutically acceptable salt or solvate thereof, wherein:
W is H or halo;
X is H, halo, CH₃, isopropyl, t-butyl, cyclopropyl, 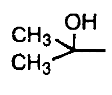 or 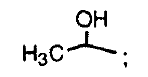
Y and Z are independently selected from the group consisting of halo, CH₃, OCH₃, CF₃, OCF₃ and CH₂OH;
R is R'-(CH₂)ₙC(O)-, R'-(CH₂)ₙSO₂- or R'-OC(O)-, wherein n is 0 to 2; and
R' is aryl, heteroaryl or heterocycloalkyl.

U.S. Application Serial No. 08/877050 filed June 17, 1997 discloses compounds of the formula: 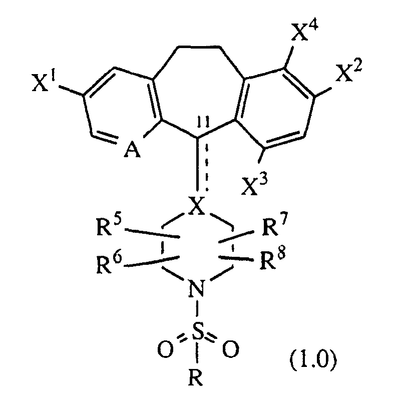
or a pharmaceutically acceptable salt or solvate thereof, wherein:
A represents N or N-oxide;
X represents N, CH or C, such that when X is N or CH, there is a single bond to carbon atom 11 as represented by the solid line; or when X is C, there is a double bond to carbon atom 11, as represented by the solid and dotted lines;
X¹ and X² are independently selected from bromo, iodo or chloro;
X³ and X⁴ are independently selected from bromo, iodo, chloro, fluro or hydrogen provided only one of X³ or X⁴ is hydrogen;
R can represent alkyl, aryl, arylalkyl, heteroaryl, heteroarylalkyl, cycloalkyl, cycloalkylalkyl, heterocycloalkyl, heterocycloalkylalkyl or -NR¹⁰R¹¹,

wherein R¹⁰ and R¹¹ can independently represent hydrogen, alkenyl, alkyl, aryl, aralkyl, heteroaryl, heteroarylalkyl, cycloalkyl, cycloalkylalkyl, heterocycloalkyl or heterocycloalkylalkyl.

U.S. Application Serial No. 08/877052 filed June 17, 1997 discloses compounds of the formula: 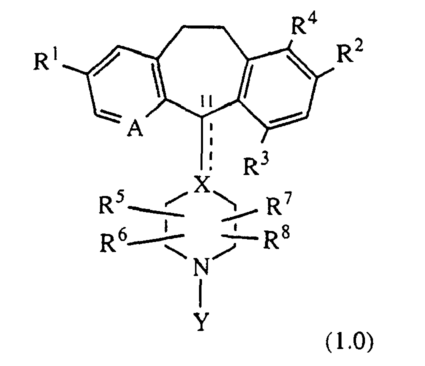
or a pharmaceutically acceptable salt or solvate thereof, wherein:
A represents N or N-oxide;
X represents N, CH or C, such that when X is N or CH, there is a single bond to carbon atom 11 as represented by the solid line; or when X is C, there is a double bond to carbon atom 11, as represented by the solid and dotted lines;
R¹ is hydrogen, bromo, chloro, trifluoromethyl, acyl, alkyl, cycloalkyl, amino, acylamino or alkoxy;
R² is hydrogen, halo, trifluoromethyl, alkyl, alkoxy, -OCF₃, hydroxy, amino or acylamino;
R³ is hydrogen, bromo, chloro, alkoxy, -OCF₃ or hydroxy;
R⁴ is hydrogen, halo, trifluoromethyl, alkyl or alkoxy;
provided that at least one of R² or R³ or R⁴ is alkyl or alkoxy and
provided that at least two of R¹, R², R³ or R⁴ are substituents other than hydrogen;
R⁵, R⁶, R⁷ and R⁸ independently represent hydrogen, alkyl or
-CONHR⁵⁰ wherein R⁵⁰ can be any of the values represented for R, below ;
Y is 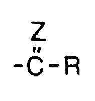 or -SO₂-R, wherein ;
Z is =O or =S; and
R is aryl, aralkyl, cycloalkyl, cycloalkylalkyl, heteroalkyl, heteroaryl, heteroarylalkyl, heterocycloalkyl or heterocycloalkylalkyl.

U.S. Application Serial No. 08/877051 filed June 17, 1997 discloses compounds of the formula: 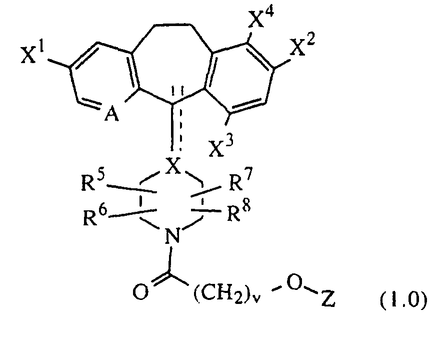
or a pharmaceutically acceptable salt or solvate thereof, wherein:
A represents N or N-oxide;
X represents N, CH or C, such that when X is N or CH, there is a single bond to carbon atom 11 as represented by the solid line; or when X is C, there is a double bond to carbon atom 11, as represented by the solid and dotted lines;
X¹ and X² are independently selected from bromo, iodo or chloro;
X³ and X⁴ are independently selected from bromo, iodo, chloro or hydrogen provided only one of X³ or X⁴ is hydrogen;
v is 1, 2, 3, 4, 5 or 6;
Z represents -NR¹⁹R²⁰ or -N=CR¹⁹R²⁰; wherein
R¹⁹ and R²⁰ are independently selected from hydrogen, alkyl, alkoxy, aryl, aralkyl, heteroaryl, heteroarylalkyl, cycloalkyl, cycloalkylalkyl, heterocycloalkyl, heterocycloalkylalkyl, -CONR¹⁰R¹², -COOR¹⁰, -COR¹⁰, -SO₂R¹⁰ and -SO₂NR¹⁰R¹², or
R¹⁹ and R²⁰ taken together can form a cycloalkyl or a heterocycloalkyl ring, wherein
R¹⁰ and R¹² are independently selected from hydrogen, alkyl, alkoxy, aryl, aralkyl, heteroaryl, heteroarylalkyl, cycloalkyl, cycloalkylalkyl, heterocycloalkyl or heterocycloalkylalkyl.

U.S. Application Serial No. 08/877498 filed June 17, 1997 discloses compounds of the formula: 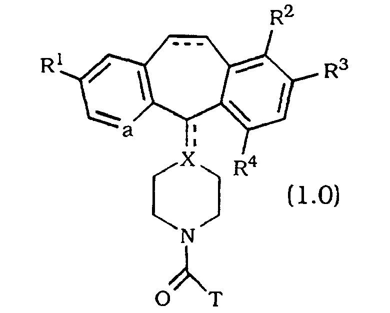
or a pharmaceutically acceptable salt or solvate thereof, wherein:
a represents N or NO-;
R¹ and R³ are the same or different halo atom;
R² and R⁴ are selected from H and halo, provided that at least one of R² and R⁴ is H;
the dotted line (- - -) represents an optional bond;
X is N, C when the optional bond is present, or CH when the optional bond is absent;
T is a substituent selected from: 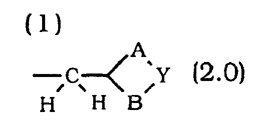
   wherein:
   A represents -(CH₂)_{b}-;
   B represents -(CH₂)_{d}-;
   b and d are independently selected from: 0, 1, 2, 3, or 4 such that the sum of b and d is 3 or 4; and
   Y is selected from: O, S, SO, or SO₂;

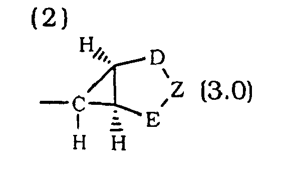

   wherein:
   D represents -(CH₂)ₑ-;
   B represents -(CH₂)_{f}-;
   e and f are independently selected from: 0, 1, 2, or3 such that the sum of e and f is 2 or 3; and
   Z is O;

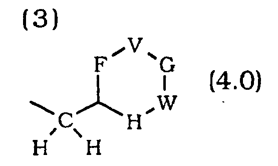

   wherein:
   F represents -(CH₂)_{g}-;
   G represents -(CH₂)ₕ-;
   H represents -(CH₂)ᵢ-;
   h represents 1, 2, or 3
   g and i are independently selected from: 0, 1 or 2 such that the sum of h, g and i is 2 or 3; and
   V and W are independently selected from O, S, SO, or SO₂;

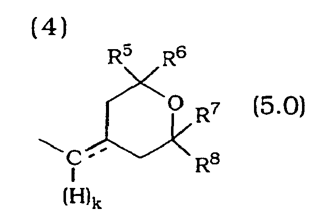

   wherein:
   the dotted line (- - -) represents an optional bound;
   k is 1 or 2 such that when the optional bond is present k represents 1, and when the optional double bond is absent then k represents 2;
   R⁵, R⁶, R⁷ and R⁸ are the same alkyl (preferably methyl); or
   R⁵ and R⁷ are the same alkyl (preferably methyl), and R⁶ and R⁸ are H;

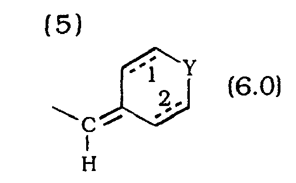

   wherein:
   the dotted lines (- - -) represent optional bonds 1 and 2 such that optional bonds 1 and 2 are both present, or optional bonds 1 and 2 are both absent;
   Y represents O, S, SO, or SO₂;

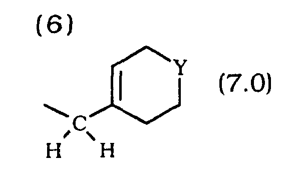

   wherein:
   Y represents O, S, SO, or SO₂;

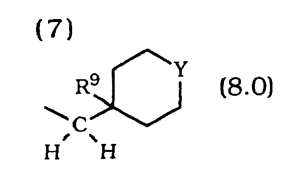

   wherein:
   R⁹ is selected from: -CN, -CO₂H, or -C(O)N(R¹⁰)₂;
   each R¹⁰ is the same or diferent alkyl group (preferably, methyl); 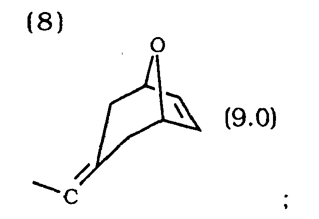 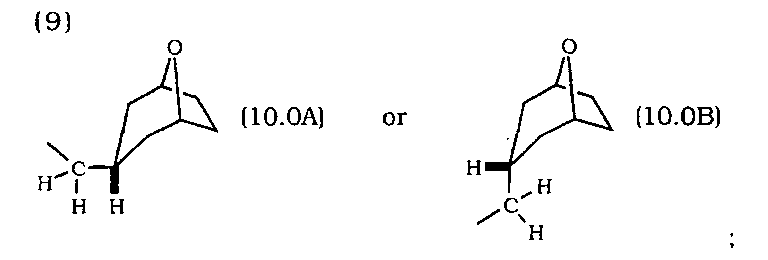 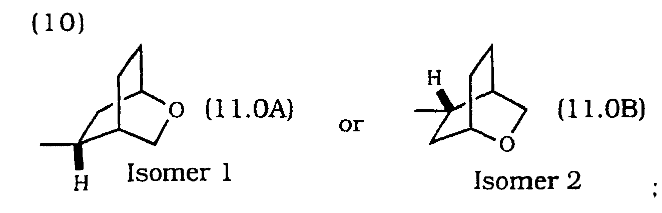 and

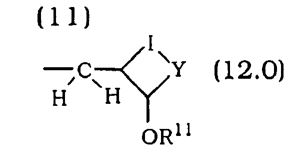

   wherein:
   I represents -(CH₂)ₘ-;
   m represents 2 or 3;
   Y represents O, S, SO, or SO₂; and
   R¹¹ represents alkyl (preferably ethyl).

U.S. Application Serial No. 08/877057 filed June 17, 1997 discloses compounds of the formula: 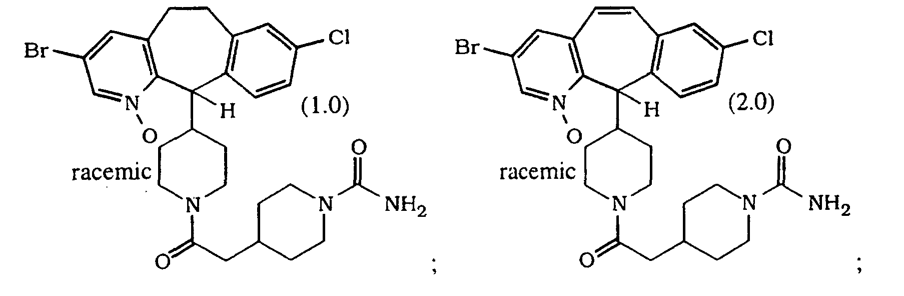 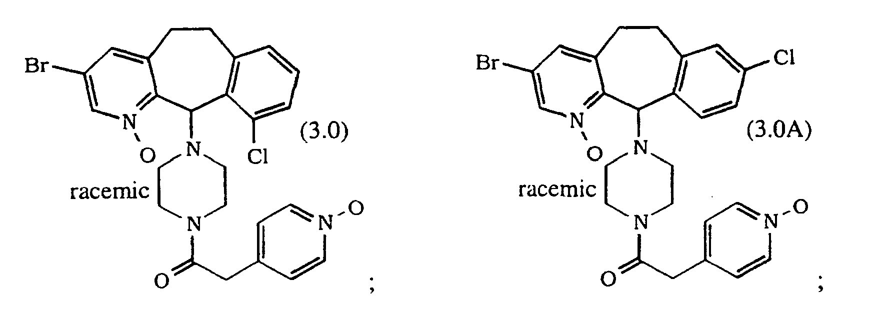 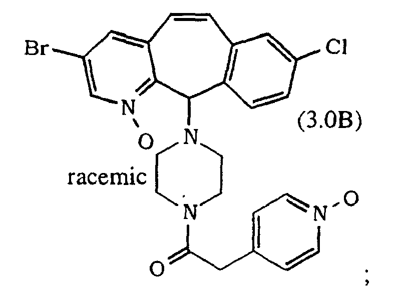 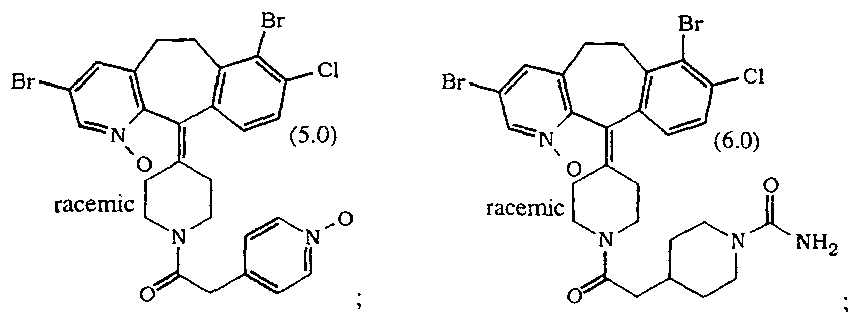 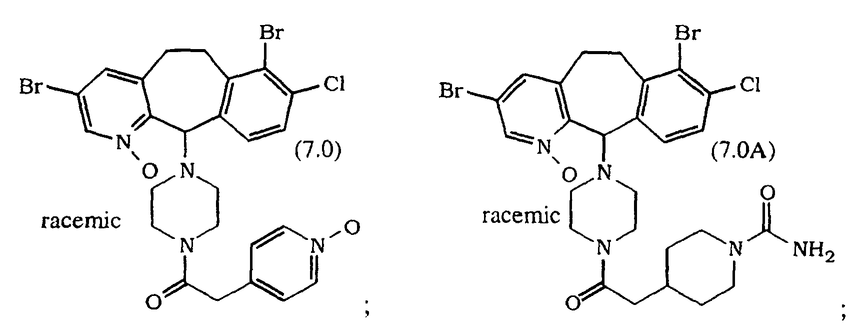 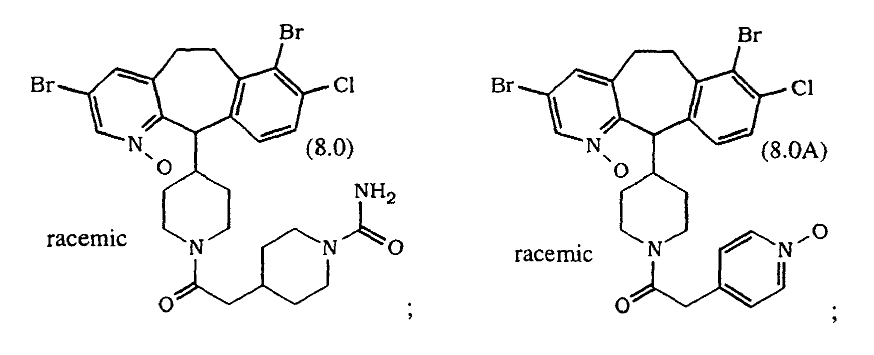 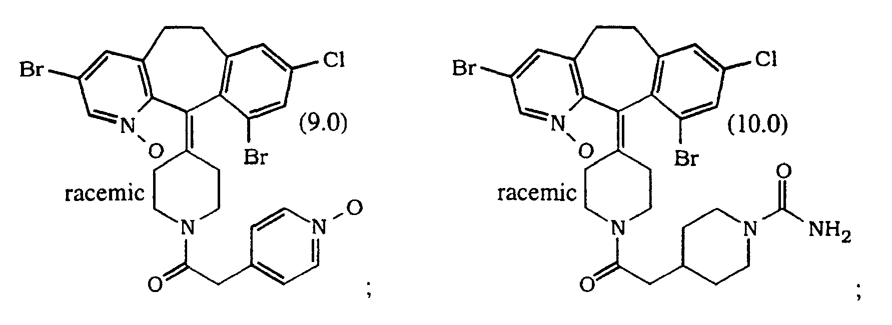 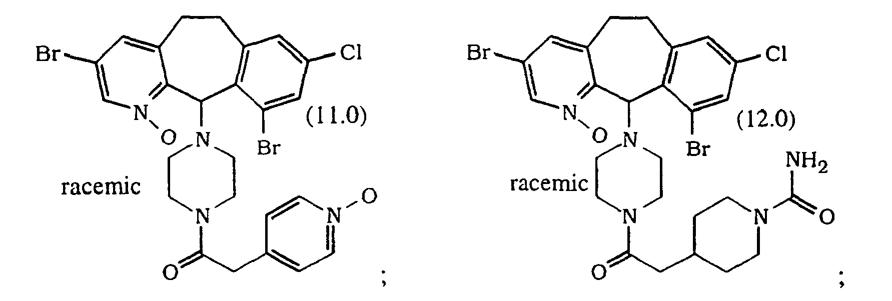 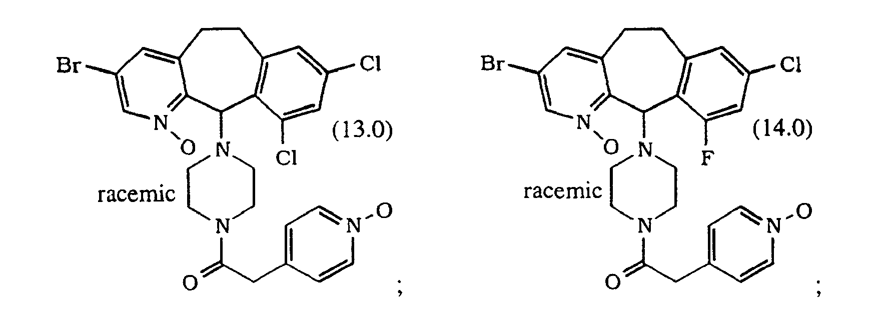 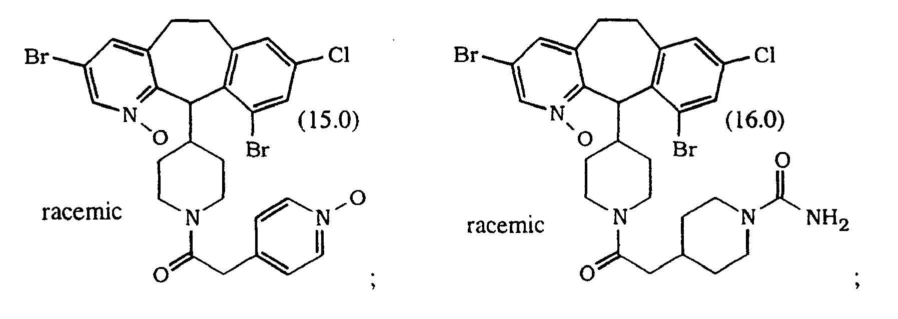 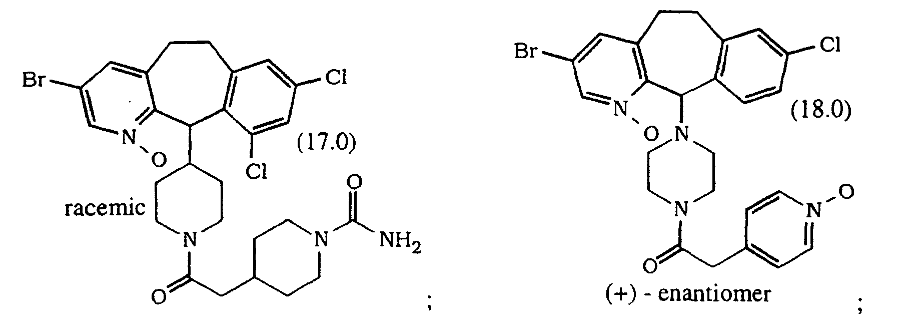 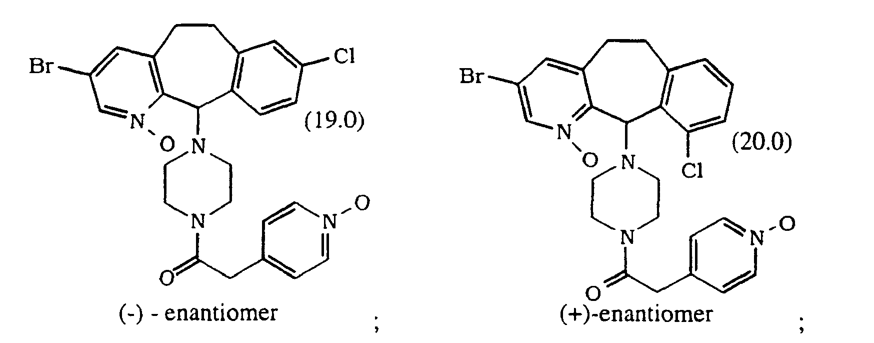 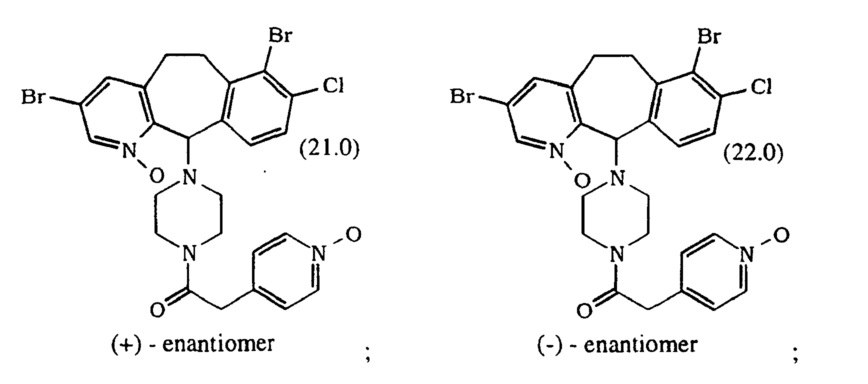 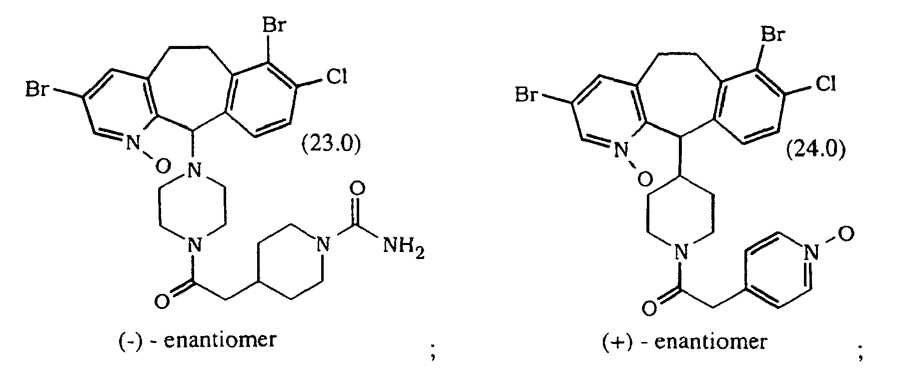 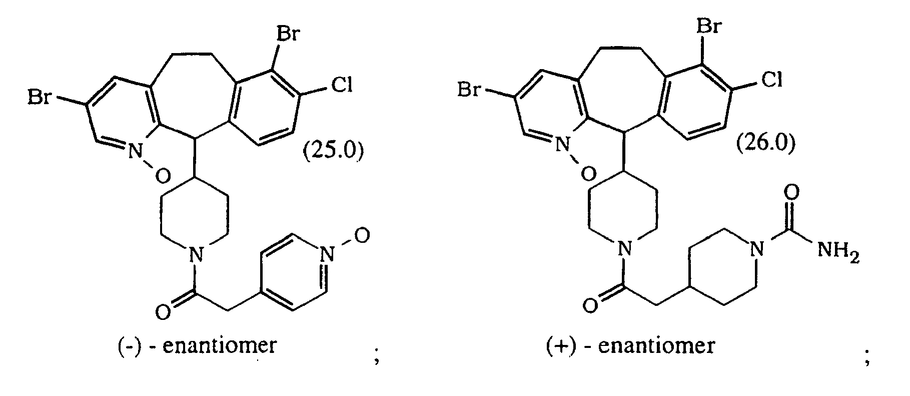 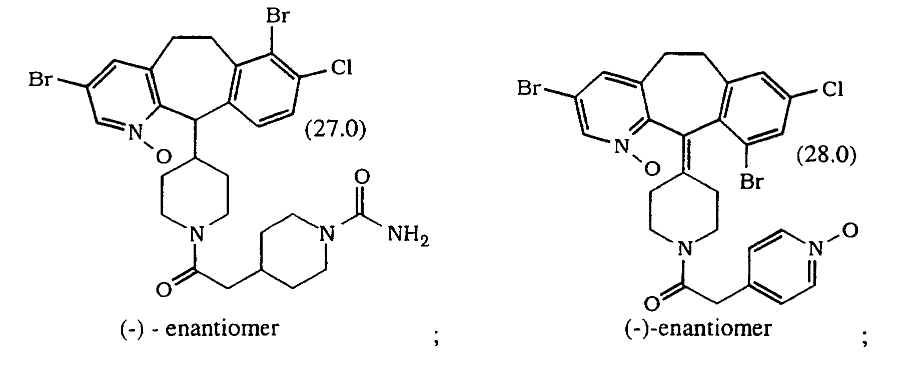 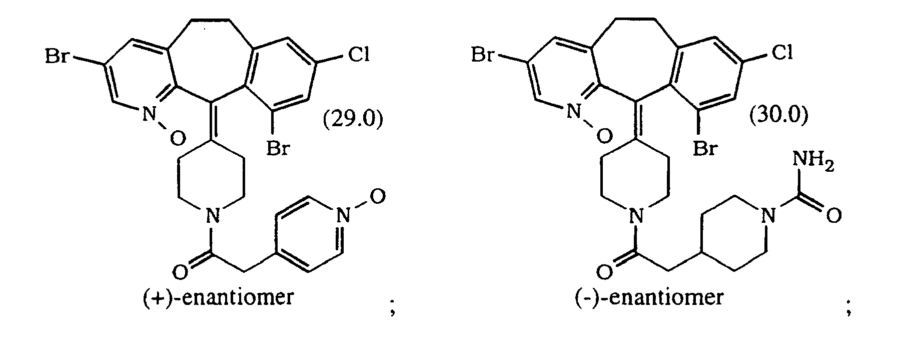 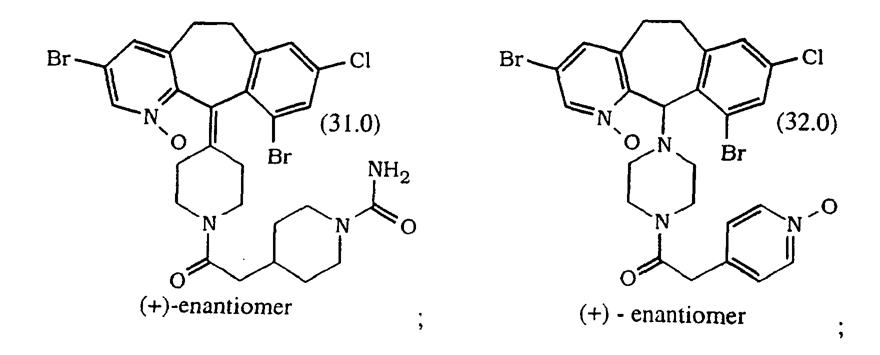 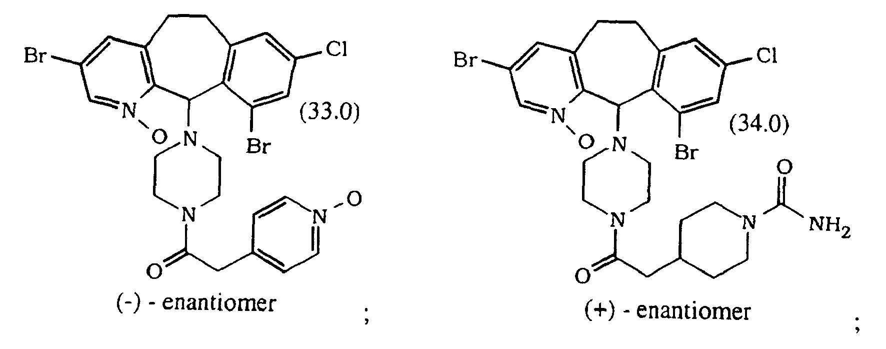 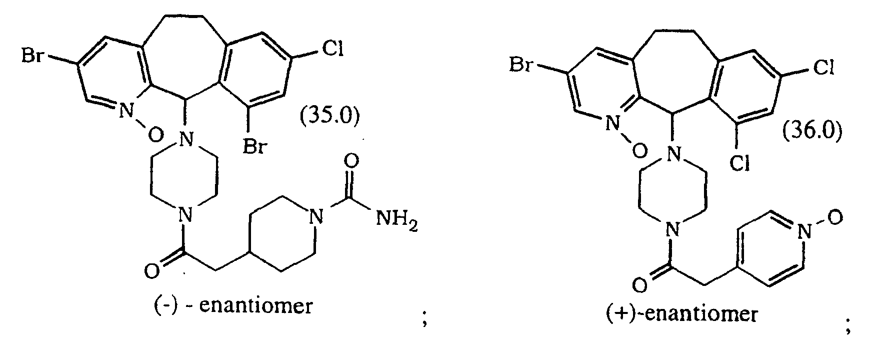 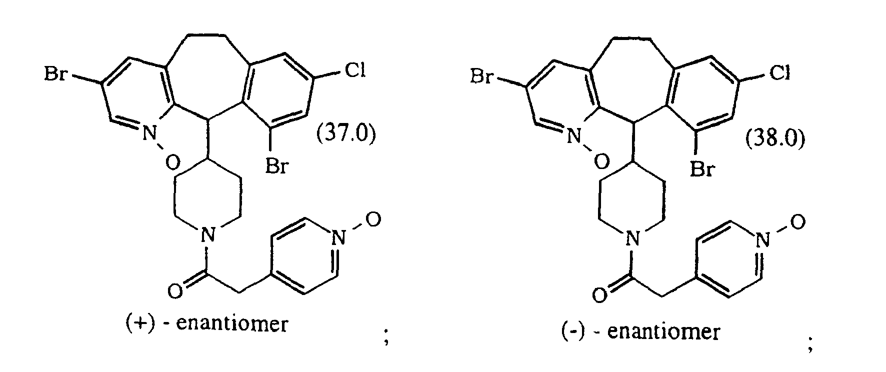 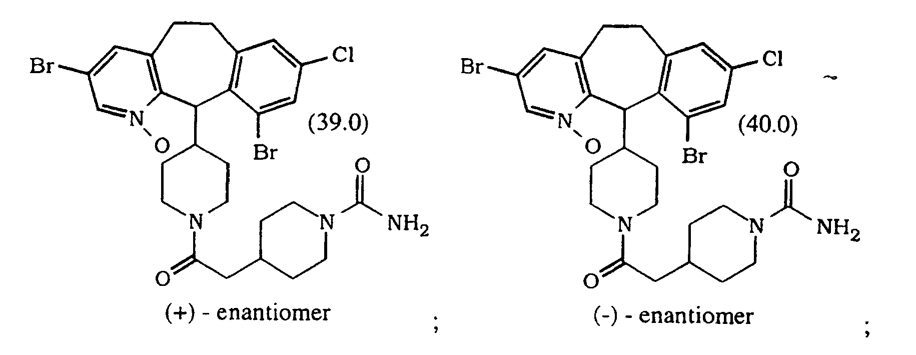 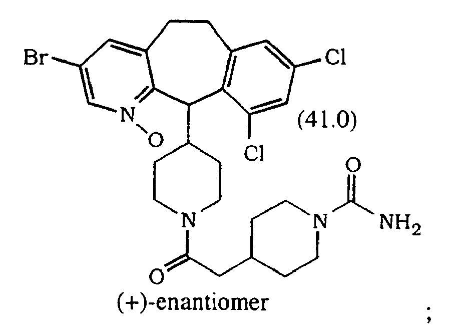 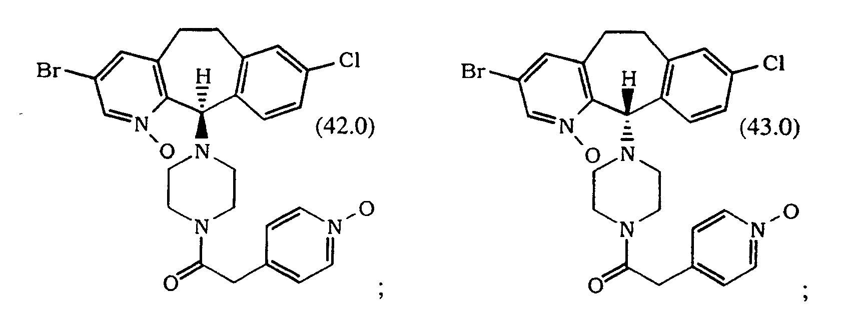 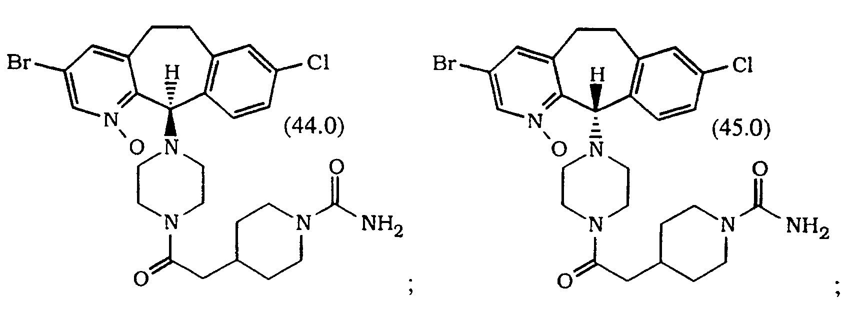 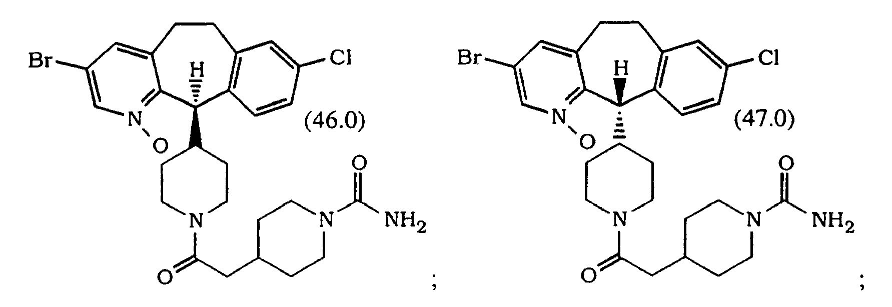 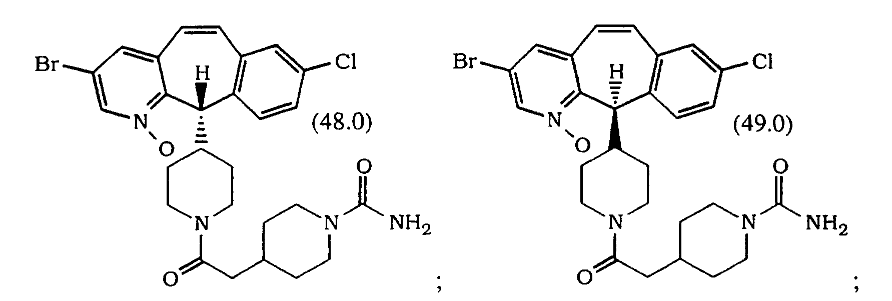 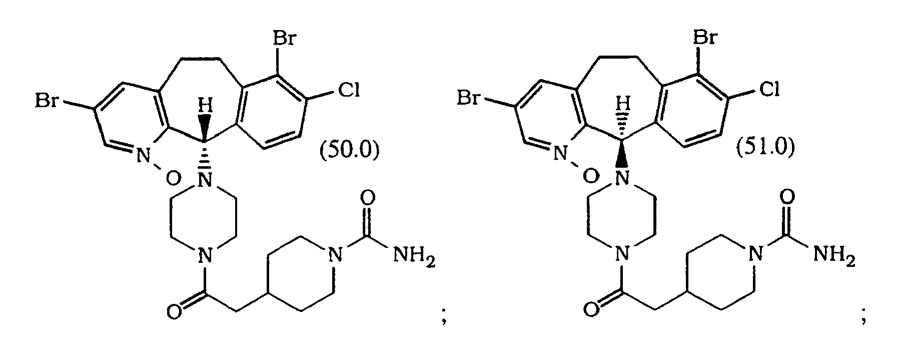 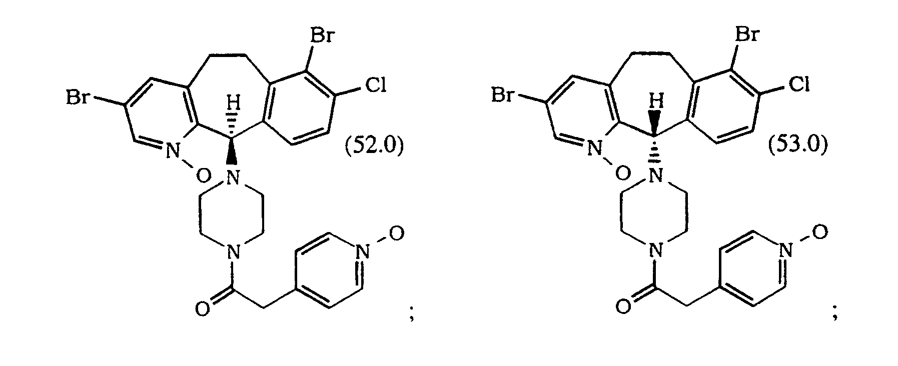 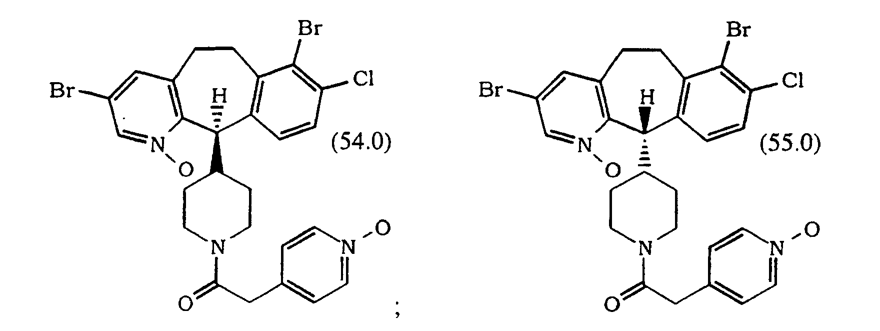 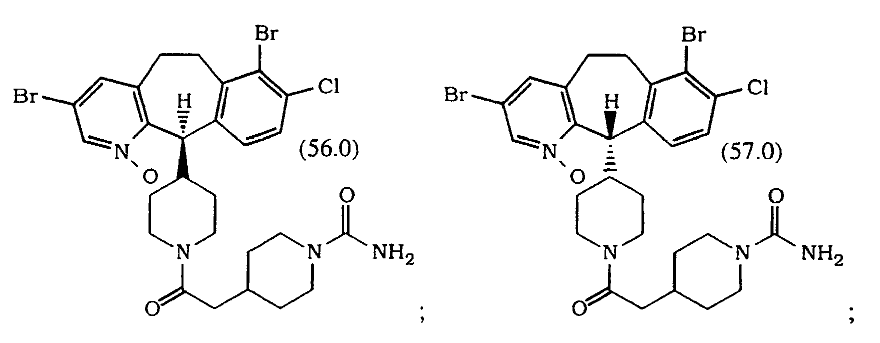 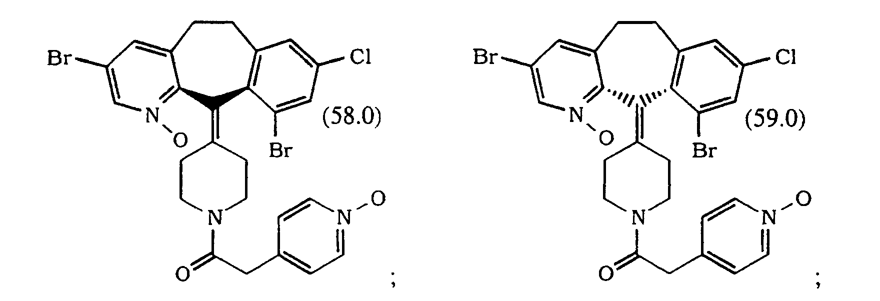 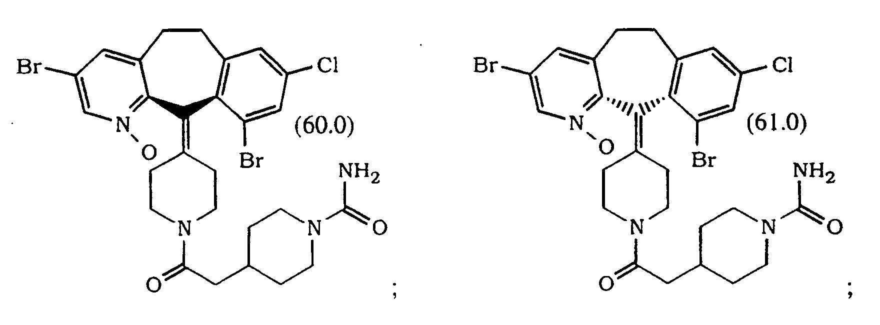 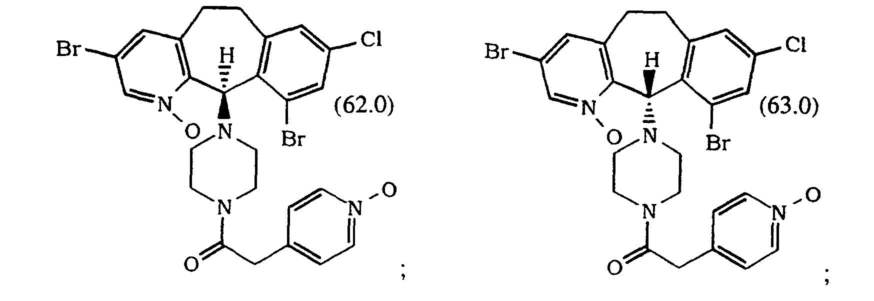 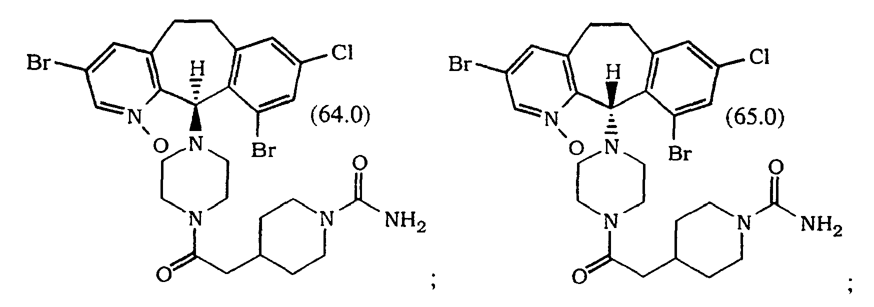 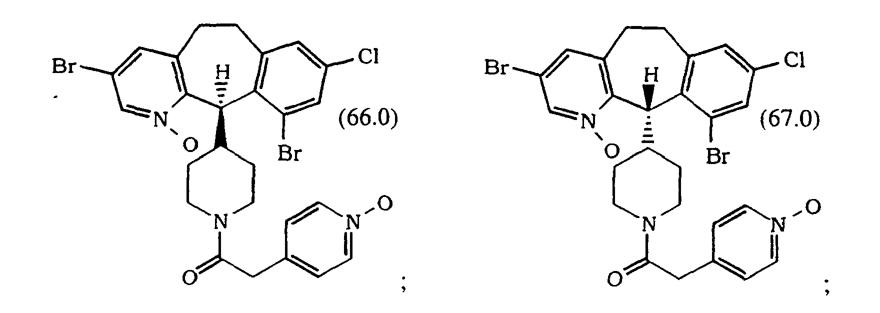 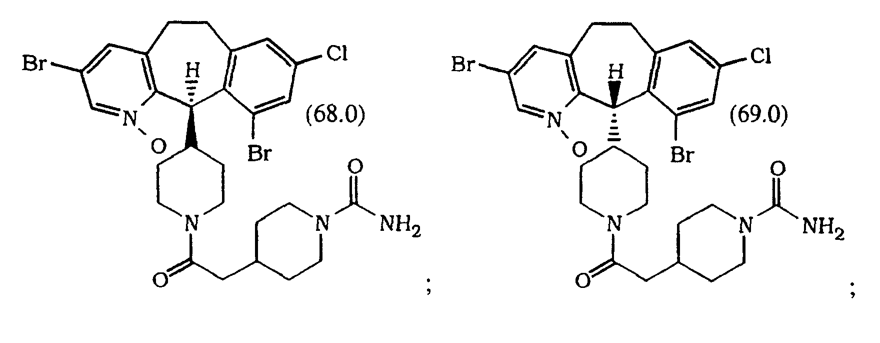 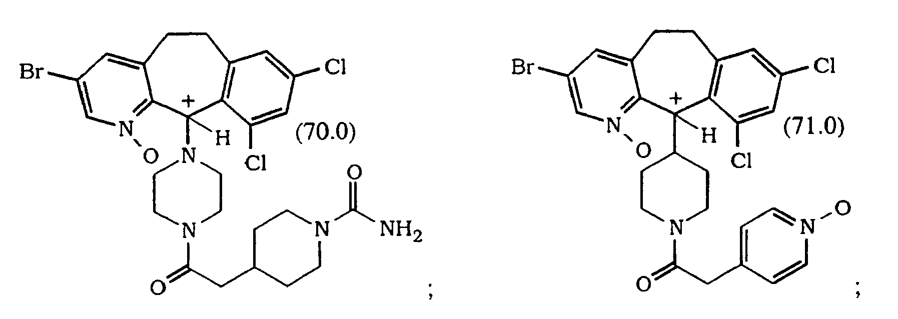 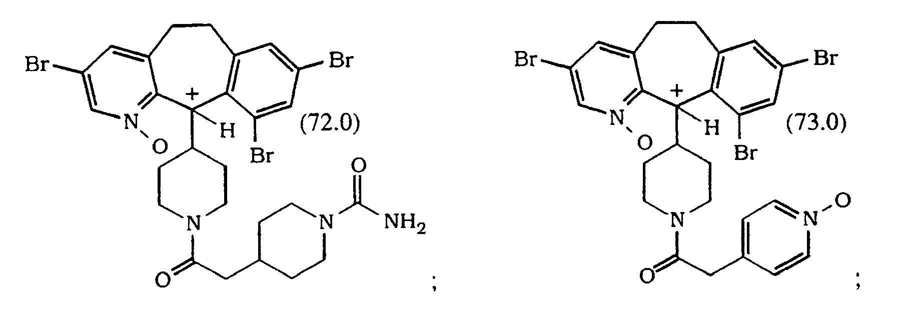 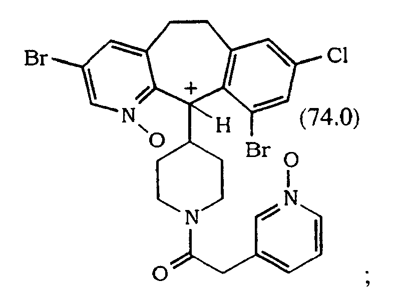 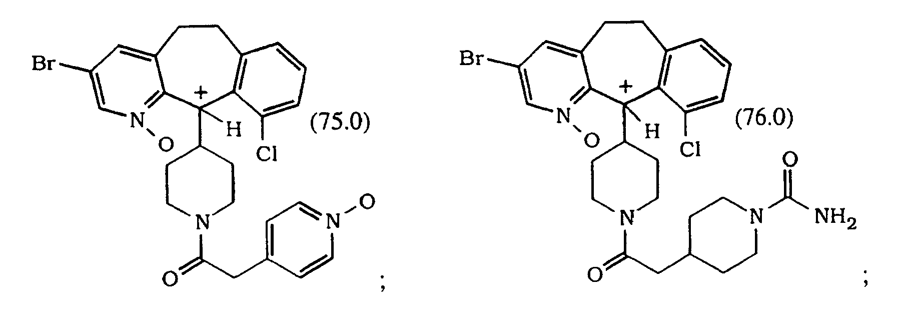 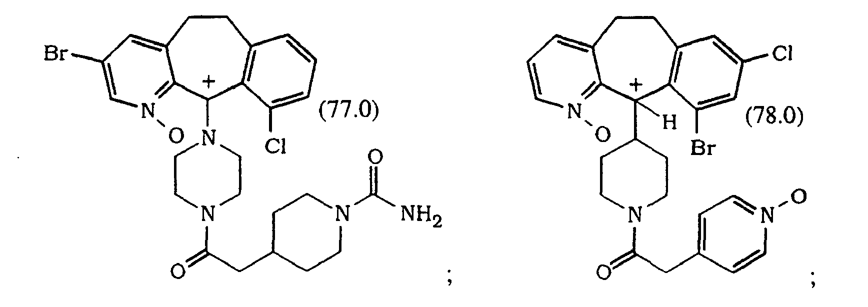 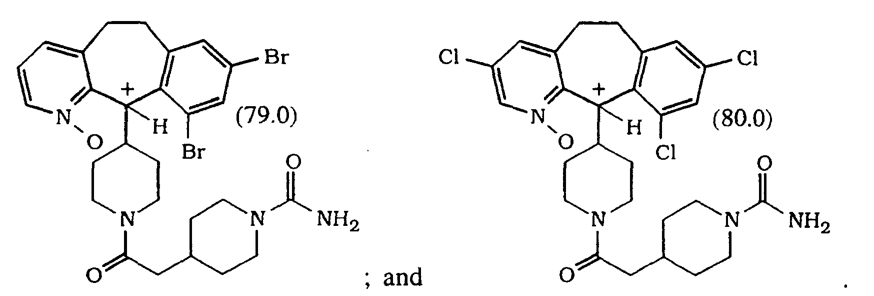
or pharmaceutically acceptable salts or solvates thereof.

U.S. Application Serial No. 08/877739 filed June 17, 1997 discloses compounds of the formula: 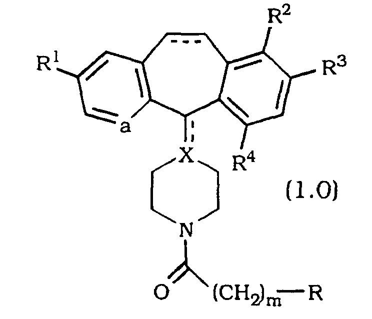
or a pharmaceutically acceptable salt or solvate thereof, wherein:
(A) a represents N or NO⁻;
(B) R¹ and R³ are the same or different halo atom;
(C) R² and R⁴ are selected from H and halo, provided that at least one of R² and R⁴ is H;
(D) the dotted line (- - -) represents an optional bond;
(E) X is N, C when the optional bond to X is present, or CH when the optional bond to X is absent;
(F) m is 0, 1 or 2;
(G) R represents:
   I. a cycloalkyl ring selected from: 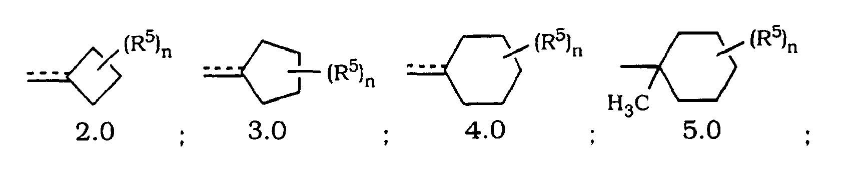 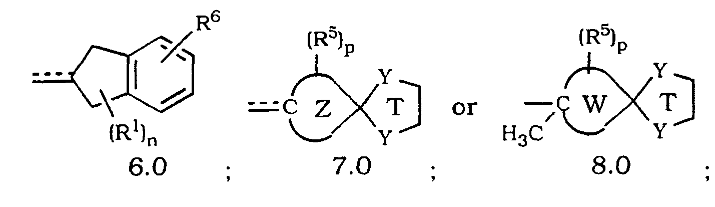 or
   2. a heterocycloalkyl ring selected from: 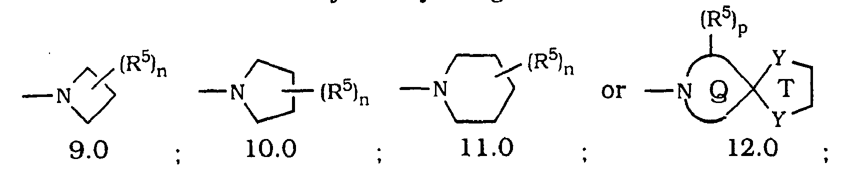
(H) p is 0, 1 or 2;
(I) when n or p is 1 then R⁵ is selected from:
   (1) =O;
   (2) =N-OH;
   (3) =N-OR⁷ wherein R⁷ represents a C₁ to C₆ alkyl group;
   (4) =N-N(H)-C(O)-R⁸ wherein R⁸ represents -NH₂ or C ₁ to C₆ alkyl;
   (5) =N-O-(CH₂)ᵣ-C(O)-R¹¹ wherein r is 1, 2, or 3, and R¹¹ is selected from: -OH, -O-alkyl or -NH₂;
   (6) =N-O-(CH₂)ₛ-O-R¹², wherein s is 2, 3, or 4 and R¹² is selected from: H, alkyl or trialkylsilyl (e.g., Si(CH₃)₂-C(CH₃)₃);
   (7) -NR¹³R¹⁴ wherein R¹³ and R¹⁴ are independently selected from:
      (a) H;
      (b) acyl;
      (c) alkyl;
      (d) aralkyl;
      (d) cycloalkyl;
      (e) heterocycloalkyl;
      (f) heteroaralkyl;
      (g) -S(O)₂R¹⁵ wherein R¹⁵ is C₁ to C₆ alkyl or aryl; or
      (h) an aralkyl, cycloalkyl, heterocycloalkyl, heteroaryl or heteroaralkyl having from 1 to 3 substituents selected from: =O, halo, -OH or -O-alkyl, wherein said substiuents being bound to substitutable ring carbons;

      or
   (8) OR¹⁶ wherein R¹⁶ is selected from:
      (a) H;
      (b) C₁ to C₆ alkyl;
      (c) -C(O)R¹⁷ wherein R¹⁷ is selected from: alkyl, aryl, heteroaryl or aralkyl; or
      (d) -C(O)NHR¹⁸ wherein R¹⁸ is selected from: H, -C(O)R¹⁹ wherein R¹⁹ is selected from: -C(Cl)₃, alkyl or -(CH₂)₂OH;
(J) when n or p is 2, then each R⁵ is the same or different and each R⁵ is selected from:
   (1) -NR¹³R¹⁴ wherein R¹³ and R¹⁴ are independently selected from:
      (a) H;
      (b) acyl;
      (c) alkyl;
      (d) aralkyl;
      (d) cycloalkyl;
      (e) heterocycloalkyl;
      (f) heteroaralkyl;
      (g) -S(O)₂R¹⁵ wherein R¹⁵ is C₁ to C₆ alkyl or aryl; or
      (h) an aralkyl, cycloalkyl, heterocycloalkyl, heteroaryl or heteroaralkyl having from 1 to 3 substituents selected from: =O, halo, -OH or -O-alkyl, wherein said substiuents being bound to substitutable ring carbons; or;
   (2) OR¹⁶ wherein R¹⁶ is selected from:
      (a) H:
      (b) C₁ to C₆ alkyl;
      (c) -C(O)R¹⁷ wherein R¹⁷ is selected from: alkyl, aryl, heteroaryl or aralkyl; or
      (d) -C(O)NHR¹⁸ wherein R¹⁸ is selected from: H, -C(O)R¹⁹ wherein R¹⁹ is selected from: -C(Cl)₃, alkyl or -(CH₂)₂OH; or
(K) provided that R¹ is not bound to a carbon atom adjacent to the nitrogen atom in Rings 9.0, 10.0, 11.0 or 12.0;
(L) Y is selected from O or S, provided that each Y is the same;
(M) Z represents the remainder of cycloalkyl Rings 2.0, 3.0 or 4.0, such that spiro ring T is bound to one of the carbon atoms in said cycloalkyl ring;
(N) W represents the remainder of cycloalkyl Ring 5.0, such that spiro ring T is bound to one of the carbon atoms in said cycloalkyl ring;
(O) Q represents the remainder of heterocycloalkyl Rings 9.0, 10.0 or 11.0, such that spiro ring T is bound to one of the carbon atoms in said heterocycloalkyl ring, provided that spiro Ring T is not bound to a carbon atom adjacent to the nitrogen atom; and
(P) R⁶ is selected from: alkoxy, alkyl or -OH.

U.S. Application Serial No. 08/877677 filed June 17, 1997 discloses compounds of the formula: 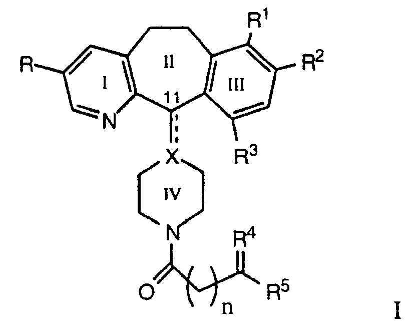
or an N-oxide thereof, or a pharmaceutically acceptable salt or solvate thereof, wherein:
R and R² are independently selected from halo;
R¹ and R³ are independently selected from the group consisting of H and halo, provided that at least one of R¹ and R³ is H;
X is N, CH or C, when the double bond is present at the C-11 position;
R⁴ is =O, -NHOH, -N=NHR⁶, -N=NHSO₂R⁶, -N=NHCOR⁶, -N=NHCONH₂, -N=NHCOCONH₂, (H, OH), (H, -OR⁶), (H, -OCOR⁶), (H, OSO₂R⁶) or -E-(CH₂)ₙ₁-G-, wherein n₁ is 1 to 5, and E and G are independently selected from the group consisting of O, S, and N, and are joined to the same carbon to form a cyclic structure;
R⁵ is H, lower alkyl, aryl, heteroaryl, aralkyl, heteroaralkyl, heterocycloalkyl-alkyl, substituted aryl, substituted heteroaryl, substituted aralkyl, substituted heteroaralkyl or substituted heterocycloalkyl-alkyl, wherein the substituents are 1 to 3 groups independently selected from the group consisting of hydroxy, lower alkyl, halo, -NR⁷R⁸, -COOH, -CONH₂, -COR⁹ and -SOR⁹;
R⁶ is lower alkyl, aryl, heteroaryl, aralkyl, heteroaralkyl, heterocycloalkyl-alkyl, substituted aryl, substituted heteroaryl, substituted aralkyl, substituted heteroaralkyl or substituted heterocycloalkyl-alkyl, wherein the substitution is as defined above for R⁵;
R⁷, R⁸ and R⁹ are independently selected from the group consisting of H, lower alkyl, aryl, and aralkyl; and
n is 0, 1, 2, 3, 4 or 5.

U.S. Application Serial No. 08/877741 filed June 17, 1997 discloses compounds of the formula: 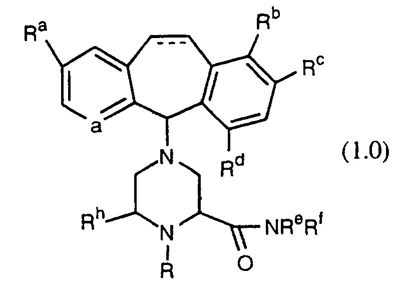
or a pharmaceutically acceptable salt or solvate thereof, wherein:
a represents N or NO⁻;
R^{a}, R^{b}, R^{c}, and R^{d} are the same or different, and are selected from the group consisting of H, halo, alkyl, and alkoxy, with the proviso that at least one, but not more than two of R^{a} , R^{b} ,R^{c} and R^{d} are H;
the dotted line (- - -) represents an optional double bond;
R is selected from the group consisting of H, -S(O)₂R¹,
-S(O)₂NR¹R², -C(O)R¹, and -C(O)NR¹R², wherein R¹ and R² are independently selected from the group consisting of H, alkyl, aryl, arylalkyl, heteroaryl, heteroarylalkyl, (C₃-C₇) cycloalkyl, cycloalkylalkyl, heterocycloalkyl, substituted alkyl, substituted aryl, substituted arylalkyl, substituted heteroaryl, substituted heteroarylalkyl, substituted (C₃-C₇) cycloalkyl, substituted cycloalkylalkyl, substituted heterocycloalkyl, wherein said substituted groups have one or more substituents selected from: alkyl, alkoxy, aralkyl, heteroarylalkyl, -NO₂, alkyloxyalkyl, alkyloxyalkyloxyalkyl, C₃-C₇ cycloalkyl, aryl, -CN, heteroaryl, heterocycloalkyl, =O, -OH, amino, substituted amino, nitro and halo;
R^{e} and R^{f} are independently selected from H, alkyl, alkyloxyalkyl, alkyloxyalkyloxyalkyl, aryl, arylalkyl, heteroaryl, heteroarylalkyl, (C₃-C₇) cycloalkyl, cycloalkylalkyl, heterocycloalkyl, substituted alkyl, substituted alkyloxyalkyl, substituted alkyloxyalkyloxyalkyl, substituted aryl, substituted arylalkyl, substituted heteroaryl, substituted heteroarylalkyl, substituted (C₃-C₇) cycloalkyl, substituted cycloalkylalkyl, substituted heterocycloalkyl, wherein said substituted groups have one or more substituents selected from: alkyl, alkoxy, aralkyl, heteroarylalkyl, -NO₂, alkyloxyalkyl, alkyloxyalkyloxyalkyl, C₃-C₇ cycloalkyl, aryl, -CN, heteroaryl, heterocycloalkyl, =O, -OH, amino, substituted amino, nitro and halo; or R^{e} is selected from the group consisting of H, alkyl and aryl and R^{f} is represented by -(CH₂)ₙ-R¹⁵, wherein n is an integer from 0 to 8 and R¹⁵ is selected from -C(O)NH₂, -SO₂NH₂, aryl, heteroaryl, cycloalkyl, heterocycloalkyl, optionally substituted by alkyl, alkoxy, aralkyl, heteroarylalkyl, -NO₂, alkyloxyalkyl, alkyloxyalkyloxyalkyl, C₃ - C₇ cycloalkyl, aryl, -CN, heterocycloalkyl, =O, -OH, amino, substituted amino, nitro and halo;
or R¹⁵ is 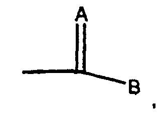 wherein B is OH or NH₂ and A is NH, O, NOH or NCN, or R¹⁵ is NR¹⁶R¹⁷, wherein R¹⁶ is H or alkyl and R¹⁷ is H, alkyl, SO₂CH₃, or C(O)NH₂; or R^{e} and R^{f} together with the nitrogen to which they are bound, form a 5 or 6 membered heterocycloalkyl ring which is optionally substituted by OH, NH₂, NHR¹⁶, NHR¹⁷, NR¹⁶R¹⁷, or (CH₂)ₙR¹⁸R¹⁹, wherein R¹⁶ and R¹⁷ are as defined above, R¹⁸ is H or C₁-C₆ alkyl, and R¹⁹ is selected from H, C₁-C₆ alkyl, substituted alkyl, arylalkyl, acyl (e.g., acetyl, benzoyl, etc.), carboxamido, alkyloxycarbonyl (e.g., methoxycarbonyl), arylalkyloxycarbonyl (e.g., benzyloxycarbonyl), amido derivatives derived from amino acids (e.g., glycine,alnine, serine,etc.), imidate (e.g., phenoxyimidate), cyanide, imidamido (e.g., C(=NH)NH₂, (C=NSO₂NH₂)NH₂, etc.), sulfonamido (e.g., SO₂NH₂, SO₂N(CH₃)₂) sulfonyl (e.g., SO₂CH₃, SO₂C₆H₅, SO₂CH₂C₆H₅, etc.), phosphinate (e.g., P(=O)(CH₃)₂), heterocyclyl and imidamido (e.g., (C=NC₆H₅)C₆H₅), (C=NH)C₆H₅, etc.), wherein n is as defined above; and R^{h} is H or =O; with the further proviso that when R^{h} is H and R^{b} and R^{d} are both H, R^{e} is H and R^{f} is

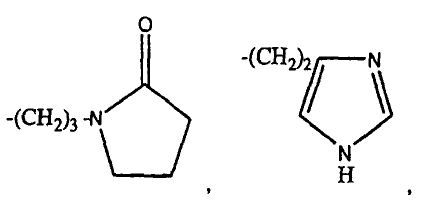

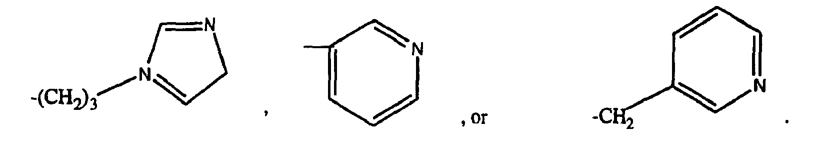

U.S. Application Serial No. 08/877743 filed June 17, 1997 discloses compounds of the formula: 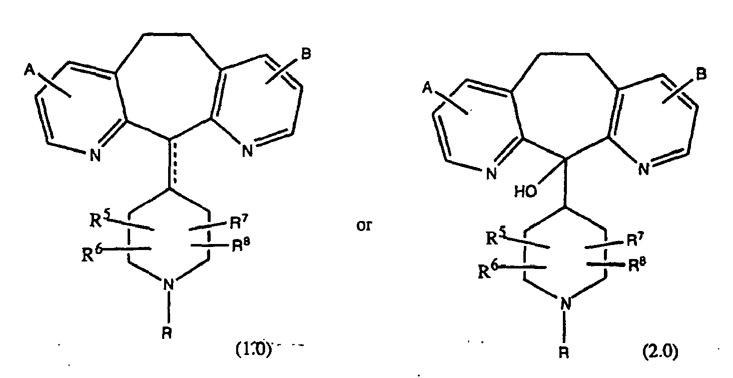
or a pharmaceutically acceptable salt or solvate thereof, wherein:
A is alkyl, halo or H;
B is methyl, halo or H;
the dotted line represents an optional double bond;
R⁵, R⁶, R⁷ and R⁸ are independently selected from the group consisting of H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰,
-OCOR¹⁰, -OCO₂R¹¹, -CO₂R¹⁰, OPO₃R¹⁰ or one of R⁵, R⁶, R⁷ and R⁸ can be taken in combination with R⁴⁰ as defined below to represent -(CH₂)ᵣ- wherein r is 1 to 4 which can be substituted with lower alkyl, lower alkoxy, -CF₃ or aryl, or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
R⁴⁰ represents H, aryl, alkyl, cycloalkyl, alkenyl, alkynyl or -D wherein -D represents 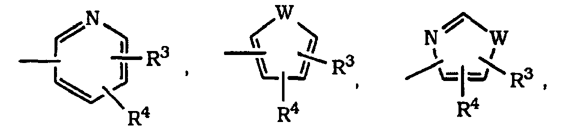 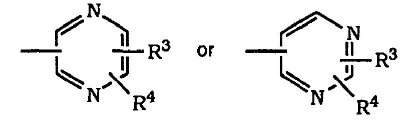
   wherein R³ and R⁴ are independently selected from the group consisting of H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, 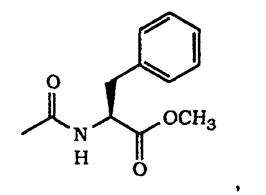
   -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
and W is O, S or NR¹⁰ wherein R¹⁰ is as defined above; said R⁴⁰ cycloalkyl, alkenyl and alkynyl groups being optionally substituted with from 1-3 groups selected from halo, -CON(R¹⁰)₂, aryl, -CO₂R¹⁰, -OR¹², -SR¹², -N(R¹⁰)₂, -N(R¹⁰)CO₂R¹¹, -COR¹², -NO₂ or D, wherein -D, R¹⁰ and R¹¹ are as defined above and R¹² represents R¹⁰, -(CH₂)ₘOR¹⁰ or -(CH₂)_{q}CO₂R¹⁰ wherein R¹⁰ is as previously defined, m is 1 to 4 and q is 0 to 4; said alkenyl and alkynyl R⁴⁰ groups not containing -OH, -SH or
-N(R¹⁰)₂ on a carbon containing a double or triple bond respectively; or
R⁴⁰ represents phenyl substituted with a group selected from -SO₂NH₂, -NHSO₂CH₃, -SO₂NHCH₃, -SO₂CH₃, -SOCH₃, -SCH₃, or -NHSO₂CF₃, preferably, said group is located in the para (p-) position of the phenyl ring; and
R is -C(O)R¹, -C(O)-OR¹, -C(O)NR¹R², -S(O)₂-R¹, or -S(O)₂NR¹R² wherein R¹ and R² are independently selected from the group consisting of H, alkyl, aryl, arylalkyl, heteroaryl, heteroarylalkyl, C3-C6 cycloalkyl, cycloalkylalkyl, heterocycloalkyl, substituted alkyl, substituted aryl, substituted arylalkyl, substituted heteroaryl, substituted heteroarylalkyl, substituted (C3-C6) cycloalkyl, substituted cycloalkylalkyl, substituted heterocycloalkyl, wherein said substituted groups have one or more substituents selected from: C₁-C₆ alkyl, alkoxy, aralkyl, heteroarylalkyl, -NO₂, alkyloxyalkyl, alkyloxyalkyloxyalkyl, C₃-C₆ cycloalkyl, aryl; -CN, heteroaryl, heterocycloalkyl, =O, -OH, amino, substituted amino, nitro and halo, with the proviso that R¹ is not H for -C(O)-OR¹ or for -S(O)²R¹.

U.S. Application Serial No. 08/877457 filed June 17, 1997 discloses compounds of the formula: 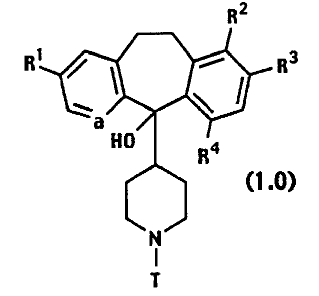
or a pharmaceutically acceptable salt or solvate thereof, wherein:
a represents N or NO-;
R¹ and R³ are the same or different and each represents halo;
R² and R⁴ are the same or different and each is selected from H and halo, provided that at least one of R² and R⁴ is H;
T is a substituent selected from SO₂R or 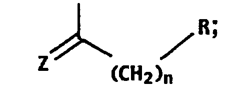
Z is O or S;
n is zero or an integer from 1 to 6;
R is alkyl, aryl, arylalkyl, heteroaryl, heteroarylalkyl, cycloalkyl, heterocycloalkyl, or N(R⁵)₂;
R⁵ is H, alkyl, aryl, heteroaryl or cycloalkyl.

U.S. Application Serial No. 08/877673 filed June 17, 1997 discloses compounds of the formula: 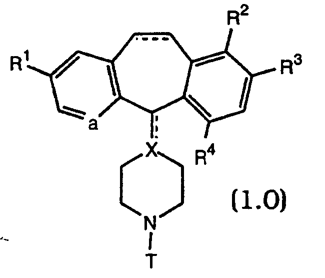
or a pharmaceutically acceptable salt or solvate thereof, wherein:
a represents N or NO⁻;
R¹ and R³ are the same or different and each represents halo;
R² and R⁴ are each independently selected from H and halo, provided that at least one of R² and R⁴ is H;
each dotted line (- - -) represents an optional bond;
X is N, C when the optional bond to X is present, or CH when the optional bond to X is absent;
T is a substituent selected from: 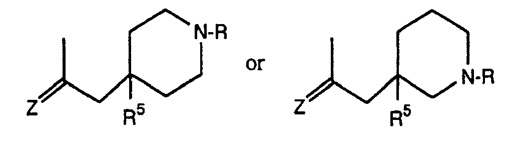
Z represents O or S;
R represents -C(O)N(R¹⁰)₂, -CH²C(O)N(R¹⁰)₂, -SO²R₁₀, -SO²(R₁₀)², -C(O)R¹¹, -C(O)-O-R¹¹, alkyl, aryl, aralkyl, cycloalkyl, heterocycloalkyl or heteroaryl;
R⁵ represents alkyl, aryl, aralkyl, heteroaryl, heteroarylalkyl, cycloalkyl, OR¹², NR¹²H, SR¹², SOR¹² (where R¹² is not H), or SO₂R¹² (where R¹² is not H); and
each R¹⁰ independently represents H, alkyl, aryl, or aralkyl (e.g., benzyl);
R¹¹ is alkyl, aryl, aralkyl, heteroaryl or heterocycloalkyl;
R¹² is selected from H, alkyl, aryl, aralkyl, heteroaryl, heteroarylalkyl, or heterocycloalkyl.

U.S. Application Serial No. 08/876507 filed June 17, 1997 discloses compounds of the formula: 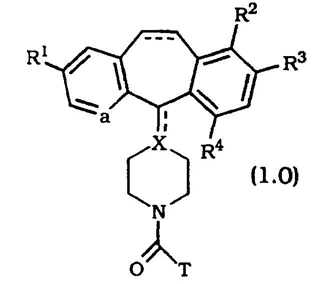 or a pharmaceutically acceptable salt or solvate thereof, wherein:
a represents N or NO-;
R¹ and R³ are the same or different halo atom;
R² and R⁴ are selected from H and halo, provided that at least one of R² and R⁴ is H;
the dotted line (- - -) represents an optional bond;
X is N, C when the optional bond is present, or CH when the optional bond is absent;
T represents 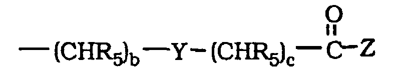
   wherein R₅ represents H, (C₁-C₆)alkyl or a bond; b and c are independently 0 to 3 ; and Y represents 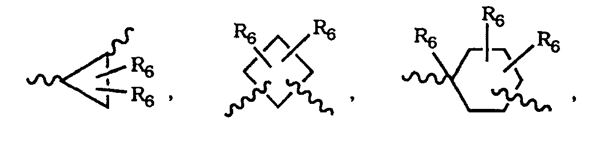 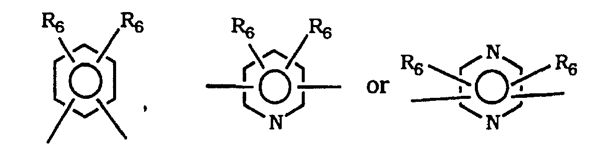
R₆ represents (C₁-C₆)alkyl or H;
Z represents OR₇, R₇ or NR₈R₉;
R₇ represents H, (C₁-C₆)alkyl or (C₁-C₆)alkyl substituted by OR₅, COR₅, phenyl or heteroaryl; and
R₈ and R₉ independently represent H, OH, (C₁-C₆)alkyl or (C₁-C₆) alkyl substituted by OR₅, COR₅, phenyl, heteroaryl or R₈ and R₉ taken together with the nitrogen atom in NR₈R₉ form an unsubstituted or substituted five or six membered heterocyclic ring system containing carbon and one to four heteroatoms selected from N, O and S, SO and SO₂ said heterocyclic substituents being (C₁-C₈) alkanoyl, (C₁-C₆)alkyl or (C₁₋C₆)penthalo alkyl.

U.S. Application Serial No. 08/876507 also discloses compounds represented by the formula: 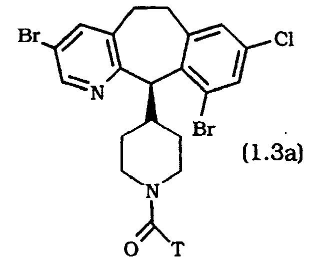
wherein T represents 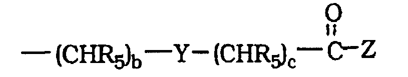
wherein R₅ represents H, (C₁-C₆)alkyl or a bond; b and c are independently 0 to 3 ; and Y represents 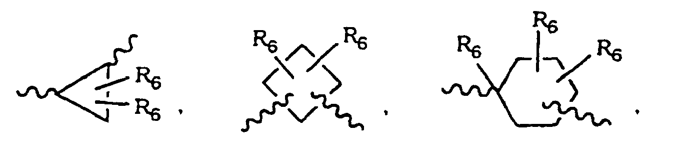 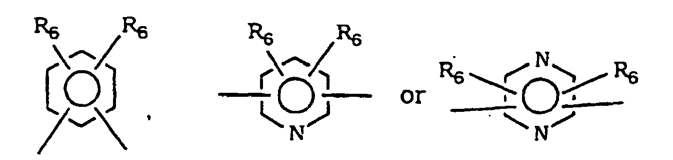
R₆ represents (C₁-C₆)alkyl or H;
Z represents OR₇; R₇ or NR₈R₉: R₇ represents H,(C₁-C₆) alkyl or (C₁-C₆) alkyl substituted by; OR₅, COR₅, phenyl or heteroaryl; and
R₈ and R₉ independently represent H, OH or (C₁-C₆)alkyl, (C₁-C₆)alkyl substituted by OR₅, COR₅, phenyl, heteroaryl or R₈ and R₉ taken together with the nitrogen atom in NR₈R₉ form an unsubstituted or substituted five or six membered heterocyle ring system containing carbon and one to four heteroatoms selected from N, O and S, SO and SO₂ said heterocyclic substituents being (C₁-C₈)alkanoyl, (C₁-C₆)alkyl or (C₁-C₆)perhalo alkyl.

The FPT inhibition and anti-tumor activity of the compound used as FPT inhibitor in this inventioncan be determined by methods known in the art--see, for example, the *in vitro* Enzyme Assays, Cell-Based Assays, Cell Mat Assays, and *in vivo* Anti-Tumor Studies in WO 95/10516 published April 20. 1995, and the soft agar assay in WO 97/23478 published July 3, 1997.

### EXAMPLES

The examples A through G below investigated the *in vitro* effect of combining paclitaxel with the following FPT inhibitory compound (referred to as "Compound X" in the Tables below):

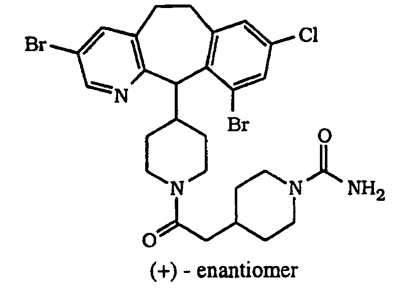

As mentioned previously, figures 1 through 15 show three examples where clear synergy was observed. These three examples are Examples A through C as discussed below. Similar results were observed in the DLD-1 colon, HTB177 lung, PA-1 ovarian, LNCaP prostate, AsPC-1 pancreatic and PANC-1 pancreatic models (summarized in the table below; data not shown). Clear Antagonism was observed in one cell line MDA-MB-231 (Fig. 16-20, Example D). Mixed results were seen in MDA-MB-468 (Figs. 21-35, Examples E through G).

**Analysis of in vitro Drug Interactions between Compound X and Paclitaxel**

| **Cell Line** | **Tumor Type** | **p53 Protein** | **Ras Mutation** | **Isobole Analysis** |
|---|---|---|---|---|
| DLD-1 | Human Colorectal | Mutant | K-ras | Synergy (p=0.0592) |
| NCI-H460 | Human Lung | Wild-type | K-ras | Synergy (p=0.0309) |
| MDA-MB-468 | Human Breast | Mutant | Wild-type | Antagonism (p=0.0001) |
| MDA-MB-468 | Human Breast | Mutant | Wild-type | Synergy (p=0.0237) |
| MDA-MB-468 | Human Breast | Mutant | Wild-type | Synergy (p=0.0094) |
| MDA-MB-231 | Human Breast | Mutant | Mutant | Antagonism (p=0.0093) |
| MidT#2-1 | Mouse Mammary | ? | ? | Synergy (p=0.011) |
| PA-1 | Human Ovarian | Wild-type | Kras | Synergy (p=0.0122) |
| DU-145 | Human Prostate | Mutant | Wild-type | Synergy (p=0.0238) |
| LNCaP | Human Prostate | Wild-type | Wild-type | Synergy (p=0.0021) |
| AsPC-1 | Human Pancreatic | Null | K-ras | Synergy (p=0.0328) |
| MiaPaCa2 | Human Pancreatic | Mutant | K-ras | Synergy (p=0.0002) |
| PANC-1 | Human Pancreatic | Mutant | K-ras | Synergy (p=0.0011) |

To prepare compositions of Compound X for the following examples, the compound was dissolved in 100% DMSO. The final concentration of DMSO within cells was ≤ 0.02% DMSO in cell culture medium. In the case of paclitaxel, stock paclitaxel was dissolved in 100% ethanol. The final concentration of ethanol was ≤ 0.001% in cell culture medium.

### EXAMPLE A: Compound X Synergizes with Paclitaxel to Inhibit Proliferation of MiaPaCa2 Pancreatic Tumor Cells

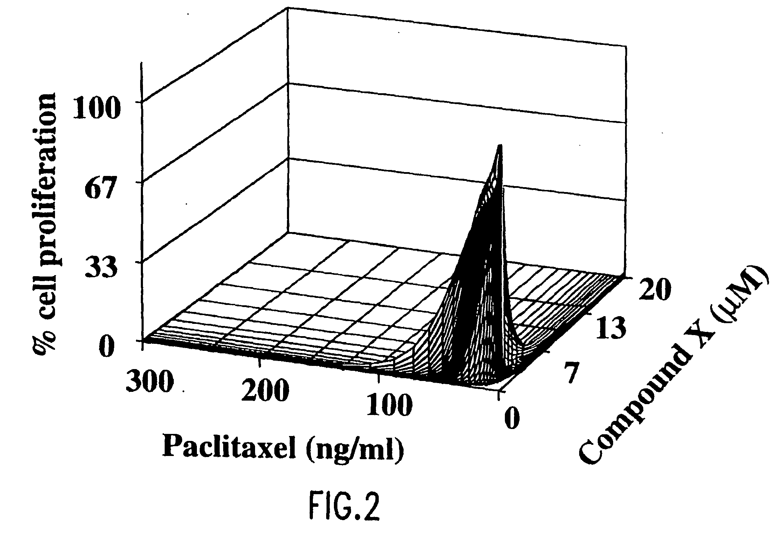

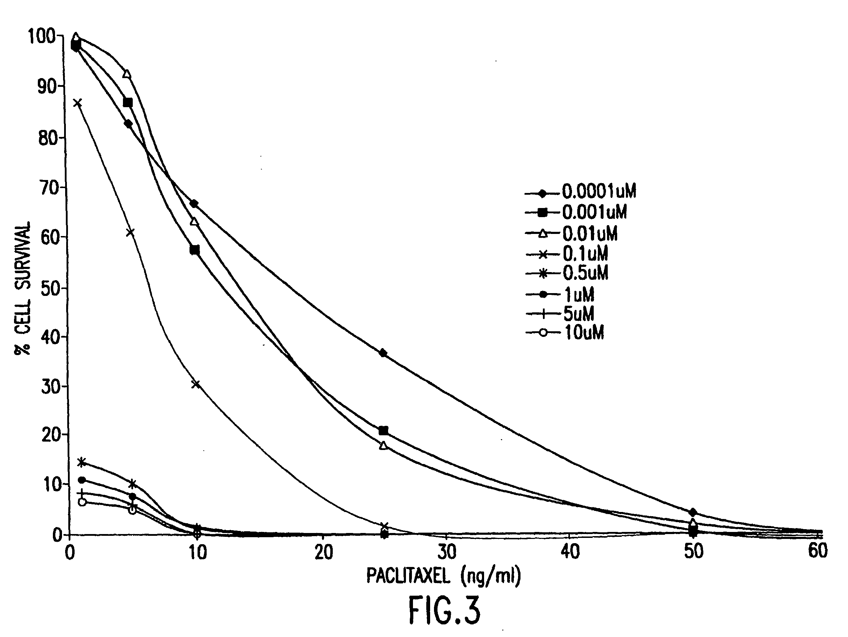

Methods: MiaPaCa2 pancreatic tumor cells were aliquoted into culture wells and allowed to attach for 3 hrs. The cells were incubated with paclitaxel for 4 hrs., washed, then Compound X was added and the incubation continued for 7 days. Cell proliferation was quantified using the MTT assay of Mosmann (see Mosmann, T. (1983) *J. Immunol. Meth., 65*: 55-63). The data was analyzed using the Thin Plate Spline methodology of O'Connell and Wolfinger (1997) (See O'Connell, M.A., and Wolfinger, R.D., *J. Computational and Graphical Statistics 6*: 224-241, 1997).
Results: Compound X and paclitaxel had synergistic efficacy (p=0.0002). Figure 1 shows the Isobole analysis for the interaction of these drugs while Figure 2 shows the 3-dimensional model of cell proliferation from which Figure 1 was derived. Figures 3 4, and 5 show the dose response curves before statistical analysis.

### EXAMPLE B: Compound X Synergizes with Paclitaxel to Inhibit Proliferation of p53^{mut} DU-145 Prostate Tumor Cells

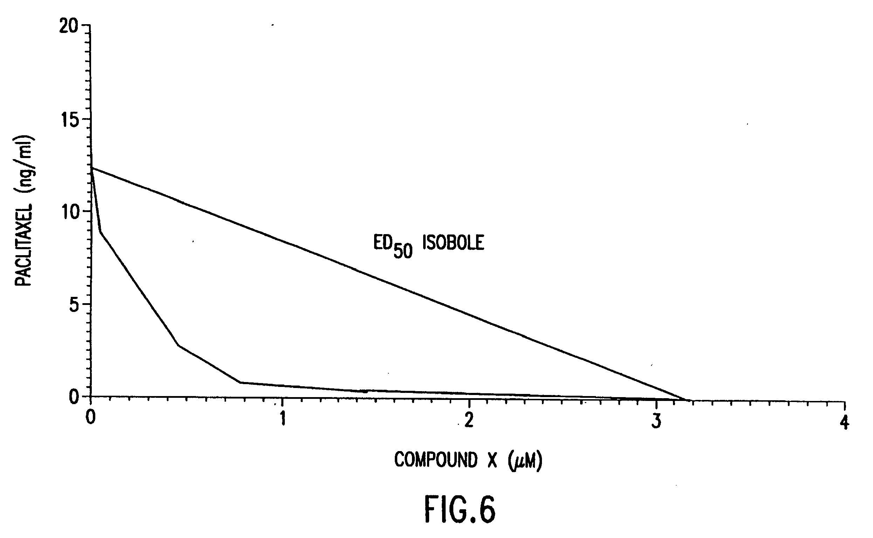

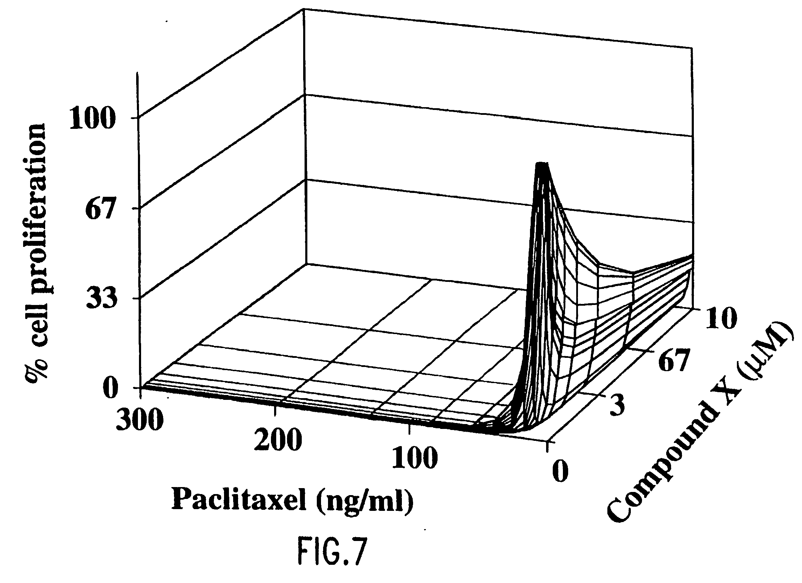

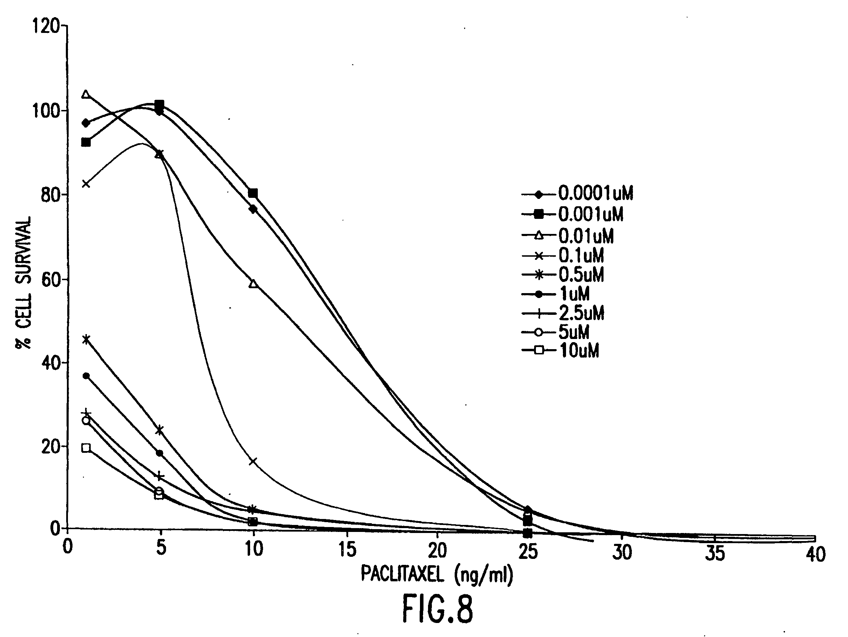

Methods: p53^{mut} DU-145 prostate tumor cells were aliquoted into culture wells and allowed to attach for 3 hrs. The cells were incubated with paclitaxel for 4 hrs., washed, then Compound X was added and the incubation continued for 7 days. Cell proliferation was quantitated using the MTT assay of Mosmann. The data was analyzed using the Thin Plate Spline methodology of O'Connell and Wolfinger (1997).
Results: Compound X and paclitaxel had synergistic efficacy (p=0.0238). Figure 6 shows the Isobole analysis for the interaction of these drugs while Figure 7 shows the three dimensional model of cell proliferation from which Figure 6 was derived. Figures 8, 9, and 10 show the dose response curves before statistical analysis.

### EXAMPLE C: Compound X Synergizes with Paclitaxel to Inhibit Proliferation of MidT#2-1 Transgenic Mouse Mammary Tumor Cells

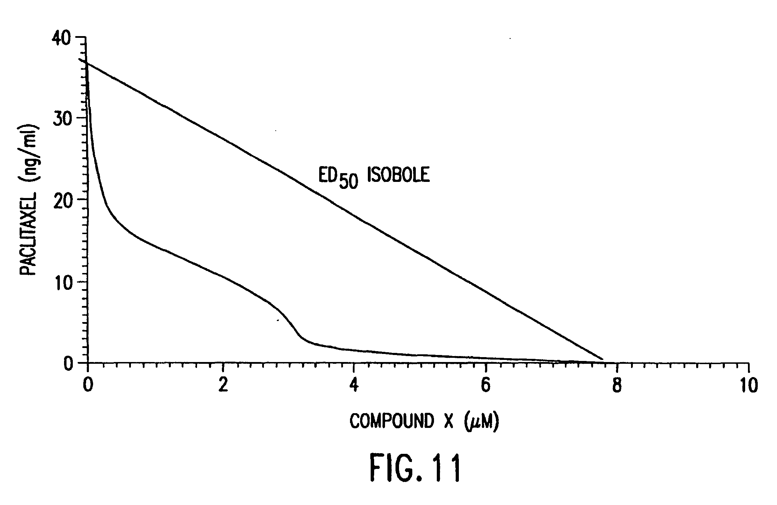

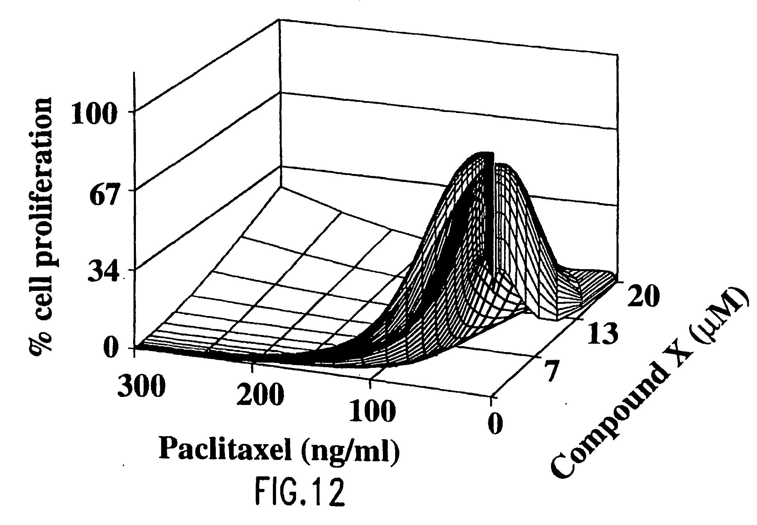

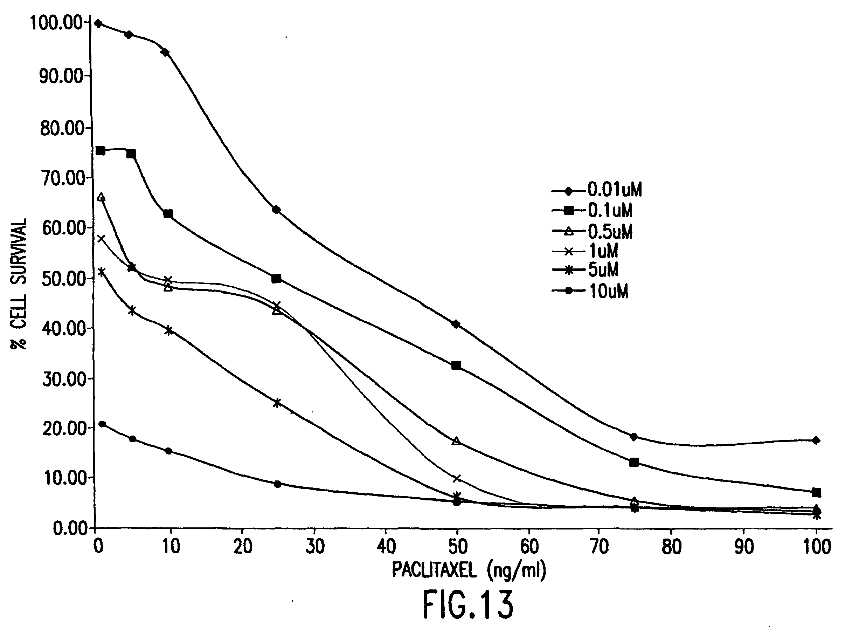

Methods: MidT#2-1 mouse tumor cells were aliquoted into culture wells and allowed to attach for 3 hrs. The cells were incubated with paclitaxel for 4 hrs., washed, then Compound X was added and the incubation continued for 7 days. Cell proliferation was quantitated using the MTT assay of Mosmann. The data was analyzed using the Thin Plate Spline methodology of O'Connell and Wolfinger (1997).
Results: Compound X and paclitaxel had synergistic efficacy (p=0.0110). Figure 11 shows the Isobole analysis for the interaction of these drugs while while Figure 12 shows the 3-dimensional model of cell proliferation from which Figure 11 was derived. Figures 13, 14, and 15 show the dose response curves before statistical analysis.

### EXAMPLE D: Compound X and Paclitaxel Have an Antagonistic Interaction in p53^{mut} MDA-MB-231 Breast Cancer Cells

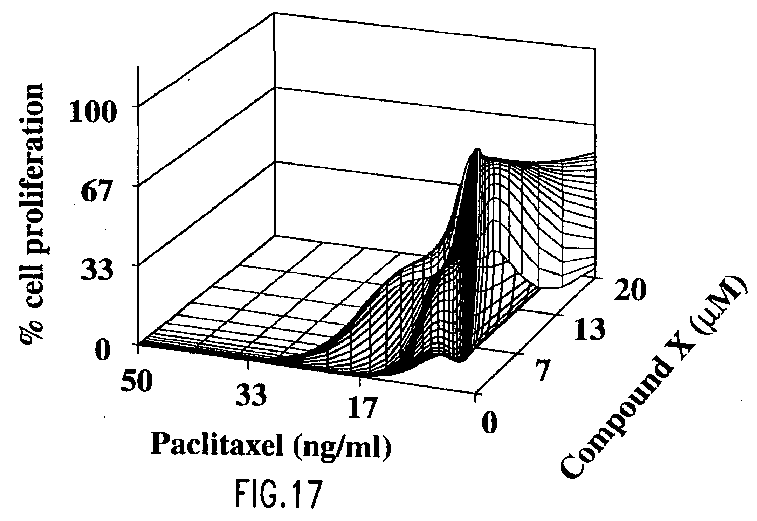

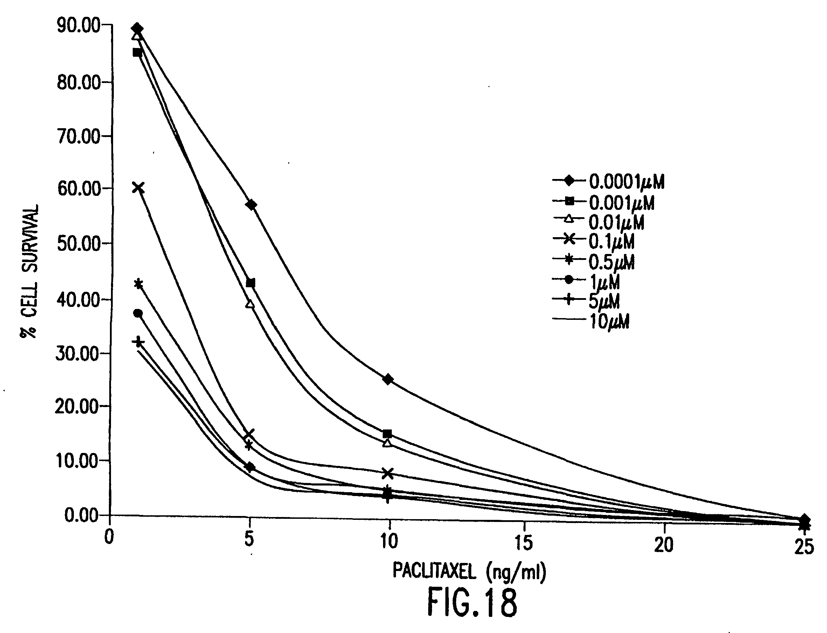

Methods: p53^{*mut*} MDA-MB-231 tumor cells were aliquoted into culture wells and allowed to attach for 3 hrs. The cells were incubated with paclitaxel for 4 hrs., washed, then Compound X was added and the incubation continued for 7 days. Cell proliferation was quantitated using the MTT assay of Mosmann. The data was analyzed using the Thin Plate Spline methodology of O'Connell and Wolfinger (1997).
Results: Compound X and paclitaxel had antagonistic interaction (p=0.0093). Figure 16 shows the Isobole analysis for the interaction of these drugs while Figure 17 shows the 3-dimensional model of cell proliferation from which Figure 16 was derived. Figures 18, 19, and 20 show the dose response curves before statistical analysis.

### EXAMPLES E, F and G: Studies of Compound X and Paclitaxel in p53^{mut} AMA-NM468 Breast Cancer Cells

In the following three Examples (E through G), the effects of Compound X and paclitaxel were studied in p53^{mut} MDA-MB-468 breast cancer cells. In two of the examples (Examples F and G), Compound X and paclitaxel had synergistic interaction, but in Example E the interaction was found to be antagonistic. The cells in Examples F and G appear to have had a better proliferative rate than the cells in Example E, which may have influenced the outcome, although the Isobole curves are atypical in all three of these studies with p53^{mut} MDA-MB-468 breast cancer cells.

Data based on combining a particular peptido-mimetic FTI compound with several different chemotherapeutic agents (*e.g*., taxol (paclitaxel), doxorubicin, cisplatin, and vinblastine) in breast cancer cell lines MDA-MB-468 and MCF-7 are presented in Moasser, MM, *et al*. Proc. Natl. Acad. Sci. USA *95*: 1369-1374, 1998. Results of additional experiments combining the FTI compound with taxol were reported in the Moasser publication (data not shown) for T47D, MDA-MB-231, and MCF-7 breast cancer cells, and for DU-145 prostate cancer cells. Additional results were reported in the publication (data not shown) for fluorouracil in breast cancer cells.

### EXAMPLE E: Compound X and Paclitaxel Have an Antagonistic Interaction in p53^{mut} MDA-MB-468 Breast Cancer Cells (Study #1)

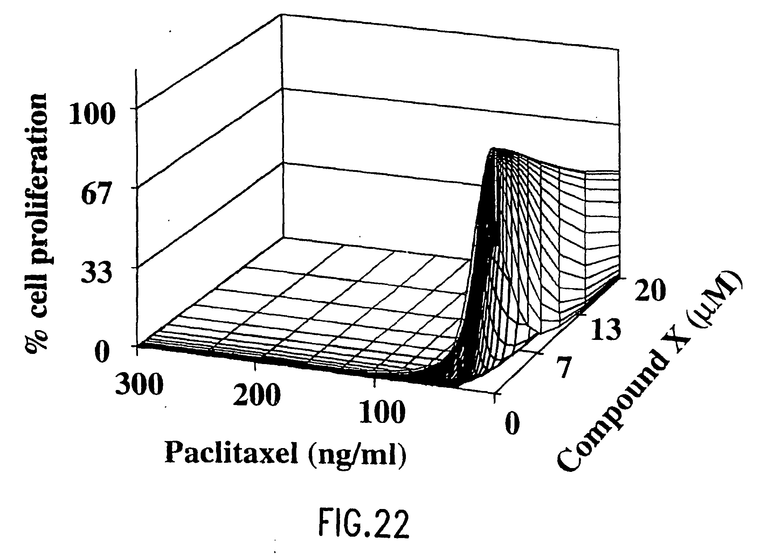

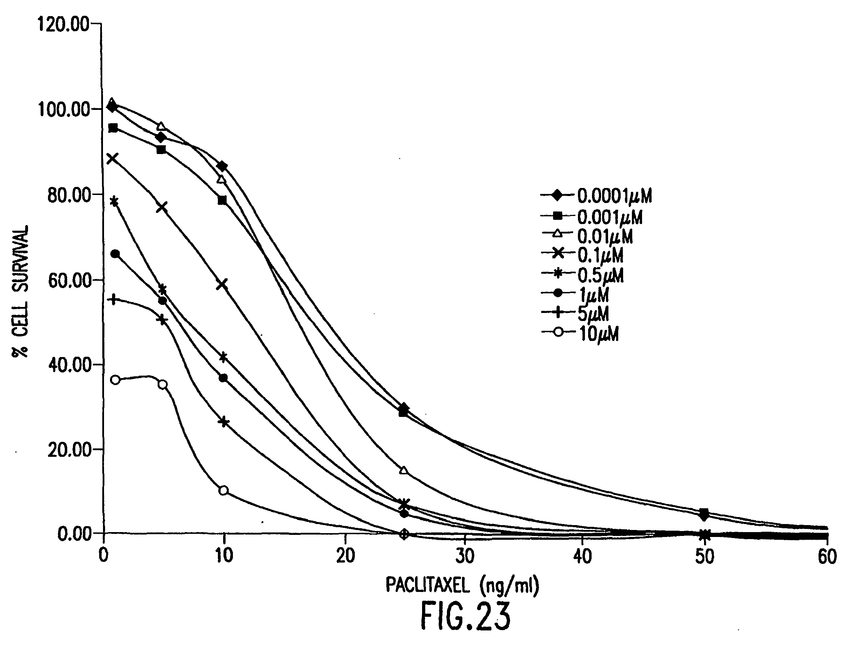

Methods: p53^{mut} MDA-MB-468 tumor cells were aliquoted into culture wells and allowed to attach for 3 hrs. The cells were incubated with paclitaxel for 4 hrs., washed, then Compound X was added and the incubation continued for 7 days. Cell proliferation was quantitated using the MTT assay of Mosmann. The data was analyzed using the Thin Plate Spline methodology of O'Connell and Wolfinger (1997).
Results: Compound X and paclitaxel had antagonistic interaction (p=0.0001). Figure 21 shows the Isobole analysis for the interaction of these drugs while Figure 22 shows the 3-dimensional model of cell proliferation from which Figure 21 was derived. Figures 23, 24, and 25 show the dose response curves before statistical analysis.

### EXAMPLE F: Compound X and Paclitaxel Have a Synergistic Interaction in p53^{mut} MDA-MB-468 Breast Cancer Cells (Study #2)

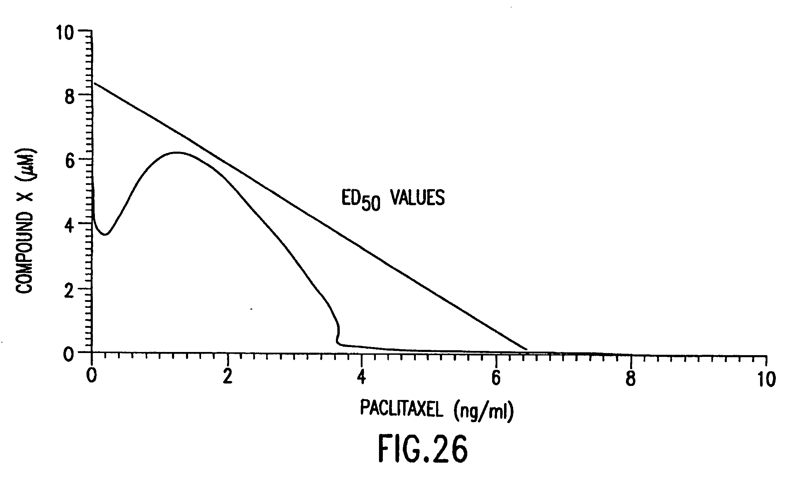

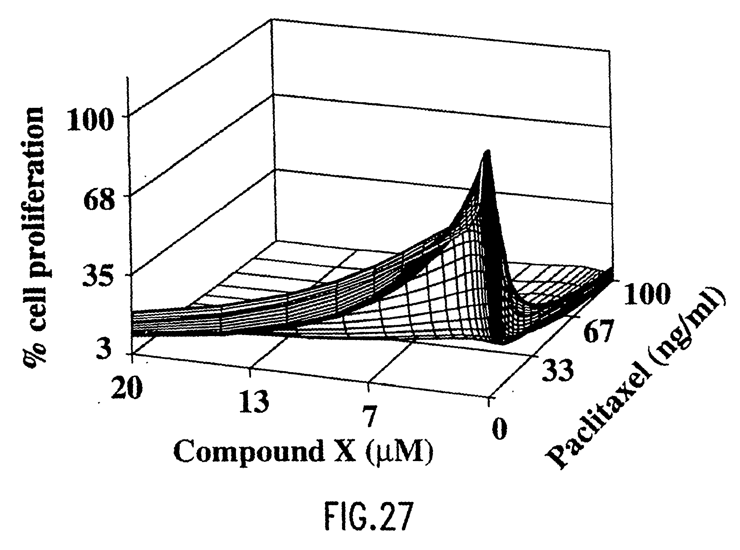

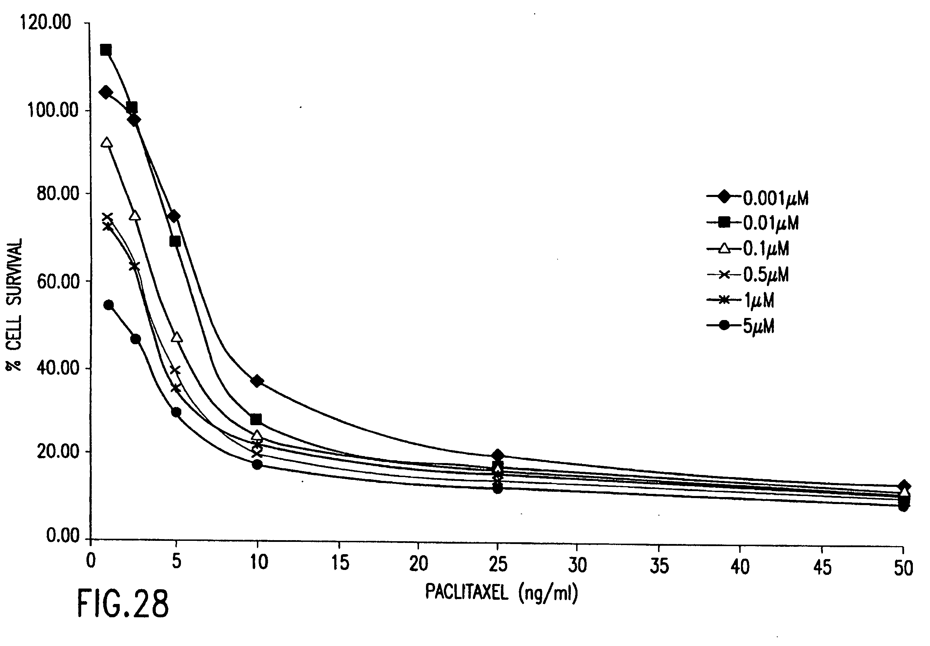

Methods: p53^{mut} MDA-MB-468 tumor cells were aliquoted into culture wells and allowed to attach for 3 hrs. The cells were incubated with paclitaxel for 4 hrs., washed, then Compound X was added and the incubation continued for 7 days. Cell proliferation was quantified using the MTT assay of Mosmann. The data was analyzed using the Thin Plate Spline methodology of O'Connell and Wolfinger (1997).
Results: Compound X and paclitaxel had a synergistic interaction (p=0.0237). Figure 26 shows the Isobole analysis for the interaction of these drugs, while Figure 27 shows the 3-dimensional model of all proliferation from which Figure 26 was derived. Figures 28, 29, and 30 show the dose response curves before statistical analysis. The cells in this study had a better proliferative rate than the cells in the previous study (Example E), which may have influenced the outcome, although the Isobole curves are atypical in both studies.

### EXAMPLE G: Compound X and Paclitaxel Have a Synergistic Interaction in p53^{mut} MDA-MB-468 Breast Cancer Cells (Study #3)

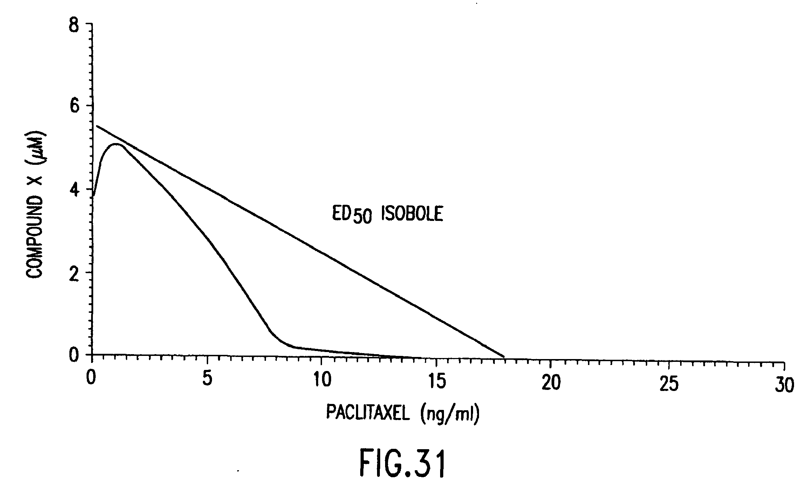

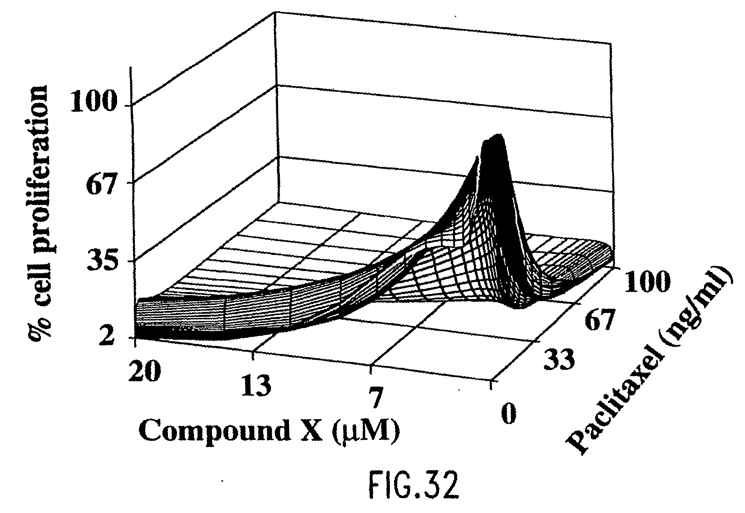

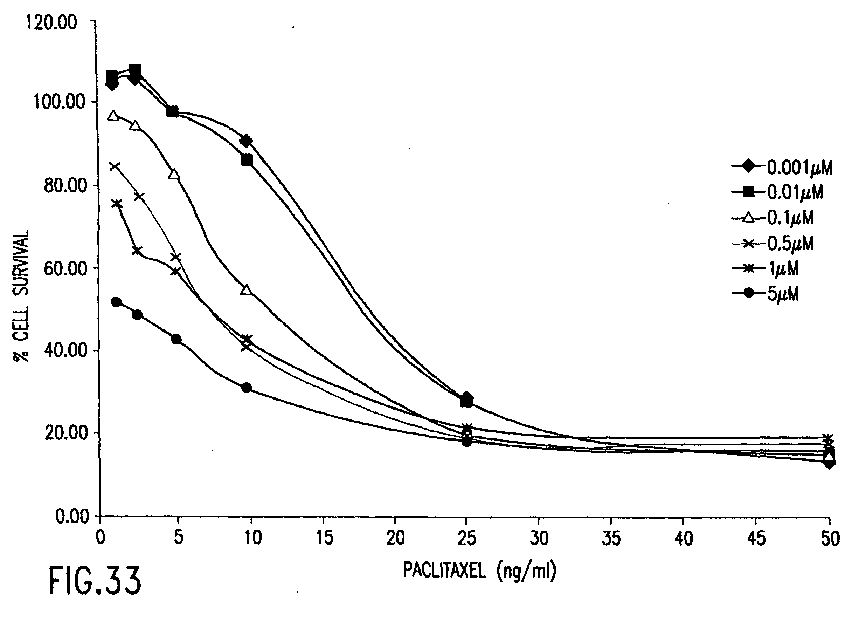

Methods: p53^{mut} MDA-MB-468 tumor cells were aliquoted into culture wells and allowed to attach for 3 hrs. The cells were incubated with paclitaxel for 4 hrs., washed, then Compound X was added and the incubation continued for 7 days. Cell proliferation was quantified using the MTT assay of Mosmann. The data was analyzed using the Thin Plate Spline methodology of O'Connell and Wolfinger (1997).
Results: Compound X and paclitaxel had a synergistic interaction (p=0.0094). Figure 31 shows the Isobole analysis for the interaction of these drugs, while Figure 32 shows the 3-dimensional model of all proliferation from which Figure 31 was derived. Figures 33, 34, and 35 show the dose response curves before statistical analysis. As was the case with Example F, the cells in Example G appear to have had a better proliferative rate than the cells in Example E, which may have influenced the outcome, although the Isobole curves are atypical in all these studies with p53^{mut} MDA-MB-468 breast cancer cells.

### EXAMPLE H: IN VIVO COMBINATION THERAPY - B.I.D.

The effect of *in vivo* combination therapy of an FPT inhibitory compound (referred to as "Compound X" in the Tables below) 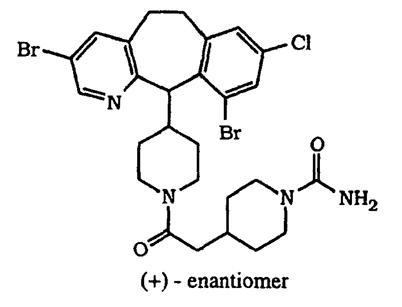
with paclitaxel on HTB 177 xenografts (NCI-H460, a human lung large cell carcinoma) using two times a day dosing was determined.

Athymic nu/nu female mice, 5-6 weeks old were used. On Day 0, HTB 177 cells, 3 x 10⁶, were injected s.c. into the flank of 120 mice. An overview of the groups is set forth below:

| | | | |
|---|---|---|---|
| Group 1 | No treatment | | 10 mice |
| Group 2 | Vehicle control I | p.o. | 10 mice |
| Group 3 | Compound X, 80 mpk/dosing, | p.o. | 10 mice |
| Group 4 | Compound X, 20 mpk/dosing, | p.o. | 10 mice |
| Group 5 | paclitaxel 20 mpk/dosing | i.p. | 10 mice |
| Group 6 | paclitaxel 5 mpk/dosing | i.p. | 10 mice |
| Group 7 | Compound X 80 mpk & paclitxel 20 mpk | | 10 mice |
| Group 8 | Compound X 80 mpk & paclitaxel 5 mpk | | 10 mice |
| Group 9 | Compound X 20 mpk & paclitaxel 20 mpk | | 10 mice |
| Group 10 | Compound X 20 mpk & paclitaxel 5 mpk | | 10 mice |
| Group 11 | Vehicle control II | p.o. & i.p. | 10 mice |

**Formulation:** Compound X for groups 3, 4, 7, 8, 9, and 10 was dissolved in 20% hydroxyl-propyl-betacyclodexatrin (Vehicle I). 0.2 ml of Compound X solution was the dosing volume. Paclitaxel was dissolved in a diluted ethanol/cremophor EL solution (Vehicle II) and the i.p. dosing volume for paclitaxel was 0.1 ml.

The 80 mpk dosing solution of Compound X was made by adding 17 ml of 20% HPBCD to a 50 ml tube containing 136 mg of Compound X to dissolve the compound. The mixture was sonicated until a complete solution was made.

The 20 mpk dosing solution was made by placing 2 ml of the 80 mpk solution into a 15 ml tube, adding 6 ml of 20% HPBCD, and vortexing the solution to mix it.

**Protocol**: Tumor cells were inoculated into 120 mice in the morning of Day 0, and the mice are weighed, randomized, and ear-marked afterwards. Drug treatment began at 7:30 am on Day 4. The animals in groups 2, 3, 4, 7, 8, 9, 10, and 11 were dosed p.o., B.I.D. with Compound X or vehicle I solution (HPBCD), at 7:30 am, and 7:30 pm, 7 days a week. Groups 5, 6, 7, 8, 9, 10 and 11 are dosed i.p., on Day 4 to Day 7, with paclitaxel or vehicle II solution. Tumor growth is quantitated by measuring tumor volume on Day 7 and Day 14.

The results are given in Table 1 below:

**TABLE 1**

| Group | Days of Treatment | Median Tumor Vol. | Mean Tumor Vol. | Standard Deviation | Average Inhibition |
|---|---|---|---|---|---|
| 1. No treatment | 7 | 114.58 | 124.36 | 37.32 | |
| | 14 | 632.81 | 718.82 | 338.44 | |
| 2. Vehicle | 7 | 130.29 | 156.17 | 62.78 | |
| | 14 | 738.8 | 760.87 | 379.45 | |
| 3. Compound X (80 mpk) | 7 | 32.83 | 34.84 | 13.44 | |
| | 14 | 28.08 | 34.68 | 27.95 | 95% |
| 4. Compound X (20 mpk) | 7 | 95.61 | 90.55 | 28.56 | |
| | 14 | 357.72 | 364.59 | 114.83 | 52% |
| 5. paclitaxel (20 mpk) | 7 | 37.75 | 48.52 | 20.34 | |
| | 14 | 75.19 | 91.61 | 57.08 | 88% |
| 6. paclitaxel (5 mpk) | 7 | 78.93 | 91.59 | 35.14 | |
| | 14 | 178.73 | 298.20 | 280.59 | 61% |
| 7. Compound X (80 mpk) & paclitaxel (20 mpk) | 7 | 23.88 | 23.63 | 6.58 | |
| | 14 | 0 | 0 | 0 | 100% |
| 8. Compound X (80 mpk) & paclitaxel (5 mpk) | 7 | 32.76 | 33.19 | 27 | |
| | 14 | 23.60 | 30.75 | 72.78 | 95% |
| 9. Compound X (20 mpk) & paclitaxel (20 mpk) | 7 | 30.03 | 32.49 | 11 | |
| | 14 | 48.32 | 65.18 | 36.18 | 91% |
| 10. Compound X (20 mpk) & paclitaxel (5 mpk) | 7 | 38.49 | 40.37 | 9.66 | |
| | 14 | 97.65 | 103.25 | 46.19 | 86% |
| 11. Ethanol/ Cremaphor | 7 | 194.77 | 190.48 | 61.81 | |
| | 14 | 1147.61 | 1080.01 | 632.87 | |

With regard to Table 1 above, it is particularly noteworthy that while Compound X alone at 20 mpk exhibited only 52% average inhibition (Experiment 4) and paclitaxel alone at 5 mpk exhibited only 61% average inhibition (Experiment 6), the *combination* of Compound X at 20 mpk plus paclitaxel at 5 mpk resulted in 86% average inhibition (see Experiment 10).

### EXAMPLE J: IN VIVO COMBINATION THERAPY - Q.I.D.

The effect of in vivo combination therapy of an FPT inhibitory compound (referred to as "Compound X" in Table 2 below) 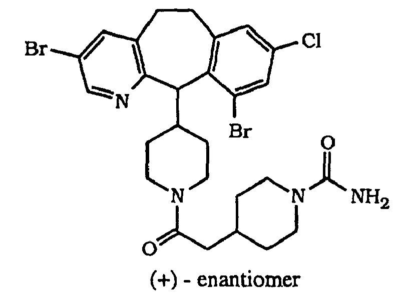
with chemotherapeutic agents on HTB 177 (NCI-H460, a human lung large cell carcinoma) using four times a day dosing was determined.

Athymic nu/nu female mice, 5-6 weeks old were used. On Day 0, HTB 177, 3 x 10⁶, was injected s.c. into the flank of 170 mice. Afterwards the mice were weighed and randomly divided into 17 groups of 10 mice per group. Drug treatment began at about 6:00 am on Day 1. The mice in groups 2, 3, 4, 5, 9, 10, 11, 12, 13, 14, 15, 16, and 17 were dosed p.o., q.i.d., at about 6 am, 12 noon, 6 pm, and 12 midnight, 7 days a week for 4 weeks. The mice in Groups 6-17 were dosed i.p. once with the indicated cytotoxic agent (see Table 1) on Day 13. The primary tumors were measured two times a week. The results are given in Table 2.

**TABLE 2**

| Group | Treatment | % Average Inhibition |
|---|---|---|
| 1 | No treatment - control | ---- |
| 2 | Vehicle, 20% HPBCD, QID, Days 1-26, p.o. | ---- |
| 3 | Compound X, 40 mpk, QID, Days 1-26, p.o. | 68 |
| 4 | Compound X, 40 mpk, QID. Days 1-12, p.o. Vehicle, 40 mpk, QID, Days 13-26, p.o. | 64 |
| 5 | Vehicle, 40 mpk, QID, Days 1-12, p.o. Compound X, 40 mpk, QID, Days 13-26, p.o. | 25 |
| 6 | Cytoxan, 200 mpk, once on Day 13, i.p. | 9 |
| 7 | 5-FU, 50 mpk, once on Day 13, i.p. | 28 |
| 8 | Vincrisitine, 1 mpk, once on Day 13, i.p. | 7 |
| 9 | Compound X, 40 mpk, QID, Days 1-26 Cytoxan, 200 mpk, once on Day 13, i.p. | 81 |
| 10 | Compound X, 40 mpk, QID, Days 1-26 5-FU, 50 mpk, once on Day 13, i.p. | 80 |
| 11 | Compound X, 40 mpk, QID, Days 1-26 Vincristine, 1 mpk, once on Day 13, i.p. | 80 |
| 12 | Vehicle, 40 mpk, QID, Days 1-12 Cytoxan, 200 mpk, once on Day 13, i.p. Compound X, 40 mpk, QID, Days 13-26. p.o. | 36 |
| 13 | Vehicle, 40 mpk, QID, Days 1-12, p.o. 5-FU, 50 mpk, once on Day 13, i.p. Compound, 40 mpk, QID, Days 13-26. p.o. | 25 |
| 14 | Vehicle, 40 mpk, QID, Days 1-12, p.o. Vincristine, 1 mpk, once on Day 13, i.p. Compound X, 40 mpk, QID, Days 13-26. p.o. | 12 |
| 15 | Compound X, 40 mpk, QID, Days 1-12. p.o. Cytoxan, 200 mpk, once on Day 13, i.p. Vehicle, 40 mpk, QID, Days 13-26. p.o. | 83 |
| 16 | Compound X, 40 mpk, QID, Days 1-12, p.o. 5-FU, 50 mpk, once on Day 13, i.p. Vehicle, 40 mpk, QID, Days 13-26. p.o. | 68 |
| 17 | Compound X, 40 mpk, QID, Days 1-12, p.o. Vincristine, 1 mpk, once on Day 13, i.p. Vehicle, 40 mpk, QID, Days 13-26. p.o. | 85 |

The Compound X (FPT Inhibitory Compound) for Groups 3, 4, 5, 9, 10, 11, 12, 13, 14, 15, 16, and 17 was dissolved in 20% hydroxypropylbetacyclodextran (HPBCD). Cytoxan, 5-FU, and Vincristine were dissolved in sterile water.

The 40 mpk dosing solution of Compound X was made by adding 39.6 ml of 20% HPBCD to a 50 ml tube containing 320 mg of Compound X to disssolve the compound. The mixture was sonicated until a complete solution was made. Aliquots of the solution were prepared ahead of time for the needed number of doses for the following 24 hour period. Aliquots of the 20% HPBCD were period ahead of time for the needed number of dosings with Vehicle Control on the specific day of dosing.

The 20 mpk dosing solution was made by placing 2 ml of the 80 mpk solution into a 15 ml tube, adding 6 ml of 40% HPBCD, and vortexing the solution to mix it.

### EXAMPLE K: IN VIVO COMBINATION THERAPY - B.I.D.

The effect of in vivo combination therapy of an FPT inhibitory compound (referred to as "Compound X" in Table 3 below) 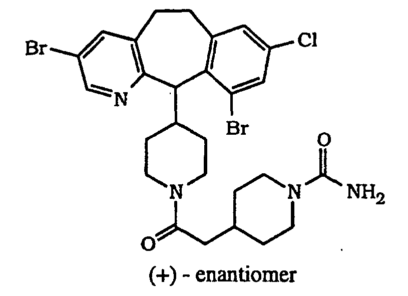
with the chemotherapeutic agent Cytoxan on HTB 177 (NCI-H460, a human lung large cell carcinoma) using twice a day dosing was determined.

Athymic nu/nu female mice, 5-6 weeks old were used. On Day 0, HTB 177, 3 x 10⁶, was injected s.c. into the flank of 100 mice. Afterwards the mice were weighed and randomly divided into 10 groups of 10 mice per group. Drug treatment began at about 8:00 am on Day 1. The mice in groups 2, 5, 6, 7, 8, 9, and 10 were dosed p.o., b.i.d., at about 8 am and 8 pm, 7 days a week for 4 weeks. The mice in Groups 3, 4, 6, 7, 9, and 10 were dosed i.p. with the indicated dosage of Cytoxan (see Table 2) on Days 5, 12, and 19. The primary tumors were measured once a week starting when the average size was about 50-100mm³. The results are given in Table 3.

**TABLE 3**

| Group | Treatment | % Average Inhibition |
|---|---|---|
| 1 | No treatment - control | ---- |
| 2 | Vehicle, 40% HPBCD, BID, p.o. | ---- |
| 3 | Cytoxan, 200 mpk, BID, Days 5, 12, & 19, i.p. | 75.38 |
| 4 | Cytoxan, 100 mpk, BID, Days 5, 12, & 19, i.p. | 61.87 |
| 5 | Compound X, 80 mpk, BID, p.o., | 74.77 |
| 6 | Compound X, 80 mpk, BID, p.o. Cytoxan, 200 mpk, BID, i.p. | 89.29 |
| 7 | Compound X, 80 mpk, BID, p.o. Cytoxan, 100 mpk, BID, i.p. | 89.32 |
| 8 | Compound X, 20 mpk, BID, p.o., | 49.47 |
| 9 | Compound X, 20 mpk, BID, p.o. Cytoxan, 200 mpk, BID, i.p. | 90.29 |
| 10 | Compound X, 20 mpk, BID, p.o. Cytoxan, 100 mpk, BID, i.p. | 68.71 |

The Compound X (FPT Inhibitory Compound) for Groups 5, 6, 7, 8, 9, and 10 was dissolved in 40% hydroxypropylbetacyclodextran (HPBCD). Cytoxan was dissolved in sterile water.

The 80 mpk dosing solution of Compound X was made by adding 10.8 ml of 40% HPBCD to a 50 ml tube containing 176 mg of Compound X to disssolve the compound. The mixture was sonicated until a complete solution was made.

The 20 mpk dosing solution was made by placing 2 ml of the 80 mpk solution into a 15 ml tube, adding 6 ml of 40% HPBCD, and vortexing the solution to mix it.

### EXAMPLE L: IN VIVO COMBINATION THERAPY - B.I.D.

The effect of *in vivo* combination therapy of the following FPT inhibitory compound (referred to as "Compound X" in the Tables below) 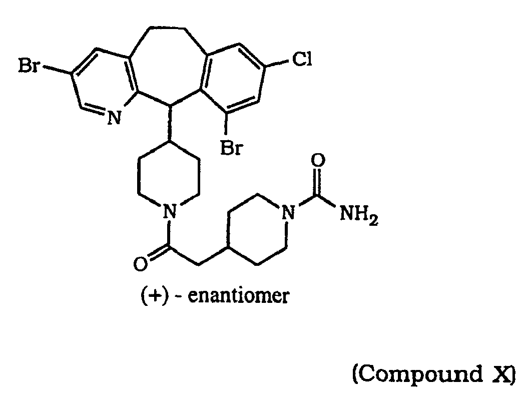
with Gemzar® (Gemcitabine HCl) on MIA PaCa xenografts (a human pancreatic carcinoma) was determined. Athymic nu/nu female mice, 7 weeks old, were used. On day 0, human pancreatic cancer MIA PaCa cells, 6 x 10⁶, were injected s.c. into the flank of each of 50 mice. An overview of the groups is set forth below:

| | | |
|---|---|---|
| Group 1 | Vehicle control I | 10 mice |
| Group 2 | Vehicle control II | 10 mice |
| Group 3 | Compound X (80mpk/dosing) | 10 mice |
| Group 4 | Compound X(80mpk) & Gemzar® (120 mpk qid x 3) | 10 mice |
| Group 5 | Gemzar® 120 mpk qid x 3 | 10 mice |

**Formulation:** Gemzar® was dissolved in Normal Saline (Vehicle I). Compound X was dissolved in 20% hydroxy-propyl-betacyclodexatrin (Vehicle II). 0.2 ml of Compound X solution was the oral dosing volume and the i.p. dosing volume for Gemzar® was 0.1 ml.

The 80 mpk dosing solution of Compound X was made by adding 15 ml of 20% HPBCD to a 50 ml tube containing 120 mg of Compound X to dissolve the compound. The mixture was sonicated until a complete solution was made.

The Gemzar® dosing solution was made by adding 16.6 ml of Saline to a vial of Gemzar® for Injection (200 mg of gemcitabine HCl), and vortexing to mix the solution. (Gemzar® is a commercially available form of gemcitabine (2', 2'-difluoro-deoxycytidine, dFdC, Gemzar®), which is a pyrimidine analogue of deoxycytidine in which the deoxyribose moiety contains two fluorine atoms at the 2'-position. (See Heinemann *et al*. Cancer Res 1988 48:4024). As noted in DeVita *et al*. (Eds.), *Cancer: Principles and Practice of Oncology* (Lippencott-Raven, Phila., Pa., 5th Ed. 1997), gemcitabine is known to have a broad spectrum of antitumor activity against leukemias and solid tumors. (reference: Hertel *et al*., Evaluation of the antitumor activity of gemcitabine (2', 2'-difluoro-2'deoxycytidine). Cancer Res 1990, 50:4417.) According to DeVita *et al*., "The most commonly used clinical schedule is a 30-minute IV infusion weekly for 3 weeks followed by a 1-week rest, and the recommended dose is 1000 mg/m². In phase II trials using this schedule (800 to 1250 dFdC mg/m² per week), response rates in the 16% to 24% range were reported in patients with non-small cell lung cancer (previously untreated) and small cell lung cancer, breast cancer patients who had received no more than one prior regimen for metastatic disease, and patients with refractory ovarian cancer, hormone-refractory prostate cancer, and head and neck cancer." DeVita *et al*. (Eds.), *Cancer: Principles and Practice of Oncology* (Lippencott-Raven, Phila., Pa., 5th Ed. 1997).)

**Protocol**: Tumor cells were inoculated into 50 mice in the morning of Day 0, and the mice were weighed, randomized, and ear-marked afterwards. Drug treatments with Compound X or Vehicle II started on Day 1 and continued twice a day at 7 am and 7 pm until Day 32. Gemzar® and Vehicle I treatments started on Day 7 and continued on every 3rd day (Day 10 and Day 13). Tumor growth was quantitated by measuring tumor volumes in 3 dimensions on Day 10, 15, 21, 26, and 32.

The results are given in Table 4 below:

**TABLE 4**

| Group | Days of Treatment | Median Tumor Vol. | Mean Tumor Vol. | Standard Deviation | Average Inhibition |
|---|---|---|---|---|---|
| 1. Vehicle I | 10 | 6.12 | 13.15 | 17.94 | |
| | 15 | 33.3 | 47.41 | 39.47 | |
| | 21 | 63.9 | 74.32 | 34.38 | |
| | 26 | 90.33 | 83.88 | 30.16 | |
| | 32 | 166.9 | 154.17 | 70.5 | |
| 2. Vehicle II | 10 | 2.46 | 7.51 | 10.0 | |
| | 15 | 63.1 | 63.86 | 21.85 | |
| | 21 | 84.81 | 83.25 | 34.4 | |
| | 26 | 90.9 | 92.61 | 44.43 | |
| | 32 | 139.76 | 125.76 | 48.07 | |
| 3. Compound X | 10 | 4.96 | 8.87 | 10.7 | |
| (80 mpk) | 15 | 12.84 | 16.91 | 8.78 | |
| | 21 | 13.0 | 14.63 | 25.03 | |
| | 26 | 41.21 | 34.53 | 20.07 | |
| | 32 | 43.3 | 54.2 | 44.61 | 57% |
| 4. Compound X | 10 | 4.12 | 5.57 | 6.28 | |
| (80mpk) | 15 | 6.66 | 12.21 | 14.06 | |
| & Gemzar® | 21 | 0.40 | 0.67 | 0.68 | |
| (120mpk) | 26 | 0.53 | 0.76 | 0.48 | |
| | 32 | 0.34 | 15.03 | 23.67 | 88% |
| 5. Gemzar® | 10 | 12.29 | 13.26 | 11.56 | |
| (120 mpk) | 15 | 31.82 | 34.91 | 22.67 | |
| | 21 | 0.84 | 5.95 | 14.56 | |
| | 26 | 20.06 | 20.03 | 17.18 | |
| | 32 | 49.3 | 49.25 | 50.29 | 60% |

With regard to Table 4 above, it is particularly noteworthy that while Compound X alone at 80 mpk exhibited only 57% average inhibition and Gemzar® alone at 120 mpk exhibited only 60% average inhibition, the combination of Compound X at 80 mpk plus Gemzar® at 120 mpk resulted in 88% average inhibition. The percent inhibition observed in the case of the combination of Compound X with Gemzar® is statistically significant over the percentage observed with either agent alone (p < 0.05).

### EXAMPLE M: IN VIVO THERAPY IN THE WAP-RAS TRANSGENIC MODEL

Compound X and Paclitaxel combination efficacy was also evaluated in the Wap-ras transgenic model. This model was used in a therapeutic mode in which treatments were initiated after mice had well developed tumors. An overview of the groups is set forth below:
**Groups:**

| | | |
|---|---|---|
| Group 1 | No treatment | 10 mice |
| Group 2 | Vehicle control I p.o. | 10 mice |
| Group 3 | Compound X, 20 mpk/dosing, p.o. | 10 mice |
| Group 4 | Paclitaxel 5 mpk/dosing i.p. | 10 mice |
| Group 5 | Compound X, 20 mpk & Paclitaxel 5 mpk | 10 mice |
| Group 6 | Vehicle control II p.o. & i.p. | 10 mice |

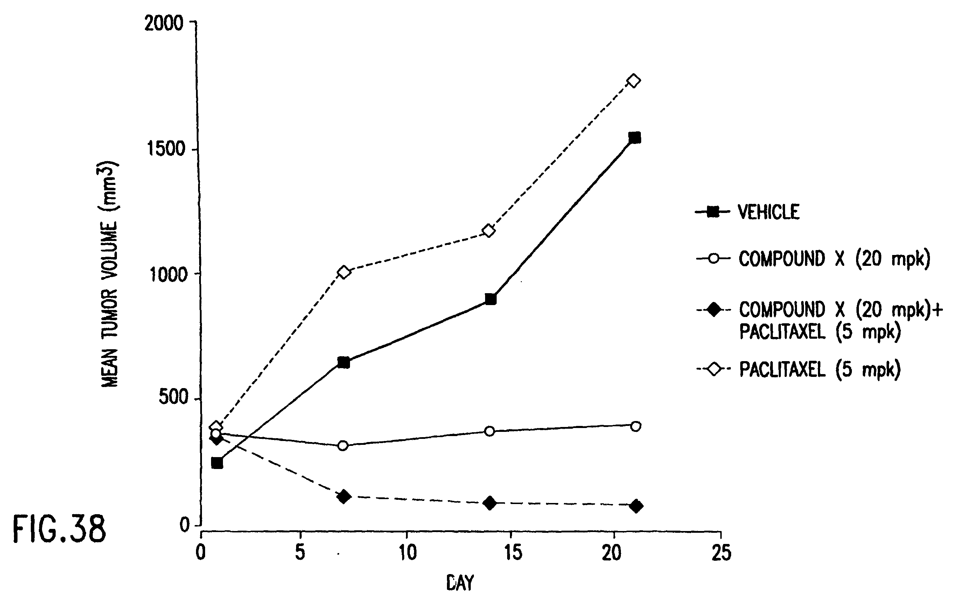

**Formulation:** Compound X was dissolved in 20% hydroxyl-propyl-betacyclodexatrin (Vehicle I). 0.2 ml of Compound X solution is the oral dosing volume. Paclitaxel was dissolved in a diluted ethanol/cremophor EL solution (Vehicle II) and the i.p. dosing volume for paclitaxel was 0.1 ml.
**Protocol:** The mice were weighed, randomized, and ear-marked on Day 0. Compound X treatment and Vehicle I treatment began on Day 1 and continued every 12 hours until Day 21. Paclitaxel and Vehicle II treatments started on Day 4 and continued daily on Day 5, 6, and 7.
**Result**: The results are illustrated in Fig. 38. Wap-ras tumors did not respond to treatment with Paclitaxel. They did respond (89% growth inhibition) to Compound X treatment at 20 mpk alone. When 20 mpk of Compound X and 5 mpk of Paclitaxel were combined, enhanced efficacy (tumor regression, equivalent to 180% growth inhibition) was seen compared to single agent alone. In addition to enhanced efficacy resulting from the combination of Compound X and Paclitaxel, the results also indicated that Compound X was able to sensitize Paclitaxel-resistant tumors.

### PHARMACEUTICAL COMPOSITIONS

Inert, pharmaceutically acceptable carriers used for preparing pharmaceutical compositions of the FPT inhibitor and the chemotherapeutic agents described herein can be either solid or liquid. Solid preparations include powders, tablets, dispersible granules, capsules, cachets and suppositories. The powders and tablets may comprise from about 5 to about 70% active ingredient. Suitable solid carriers are known in the art, e.g., magnesium carbonate, magnesium stearate, talc, sugar, and/or lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into conveniently sized molds, allowed to cool and thereby solidify.

Liquid preparations include solutions, suspensions and emulsions. As an example may be mentioned water or water-propylene glycol solutions for parenteral injection. Liquid preparations may also include solutions for intranasal administration.

Aerosol preparations suitable for inhalation may include solutions and solids in powder form, which may be in combination with a pharmaceutically acceptable carrier, such as an inert compressed gas.

Also included are solid preparations which are intended for conversion, shortly before use, to liquid preparations for either oral or parenteral administration. Such liquid forms include solutions, suspensions and emulsions.

The FPT inhibitor and the chemotherapeutic agents described herein may also be deliverable transdermally. The transdermal compositions can take the form of creams, lotions, aerosols and/or emulsions and can be included in a transdermal patch of the matrix or reservoir type as are conventional in the art for this purpose.

Preferably the compounds are administered orally.

Preferably, the pharmaceutical preparation is in unit dosage form. In such form, the preparation is subdivided into unit doses containing appropriate quantities of the active component, *e.g*., an effective amount to achieve the desired purpose.

The quantity of active compound in a unit dose of preparation may be varied or adjusted from about 0.1 mg to 1000 mg, preferably from about 1 mg to 300 mg, more preferably 10 mg to 200 mg, according to the particular application.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day if desired.

The amount and frequency of administration of the FPT inhibitor and the chemotherapeutic agents and/or radiation therapy will be regulated according to the judgment of the attending clinician (physician) considering such factors as age, condition and size of the patient as well as severity of the disease being treated. A dosage regimen of the FPT inhibitor can be oral administration of from 10 mg to 2000 mg/day, preferably 10 to 1000 mg/day, more preferably 50 to 600 mg/day, in two to four (preferably two) divided doses, to block tumor growth. In a preferred embodiment, the preferred dosage of the inhibitor is oral administration of from 50 to 600 mg/day, more preferably 50 to 400 mg/day, in two divided doses. Intermittant therapy (*e.g*., one week out of three weeks or three out of four weeks) may also be used.

The chemotherapeutic agent and/or radiation therapy can be administered according to therapeutic protocols well known in the art. It will be apparent to those skilled in the art that the administration of the chemotherapeutic agent and/or radiation therapy can be varied depending on the disease being treated and the known effects of the chemotherapeutic agent and/or radiation therapy on that disease. Also, in accordance with the knowledge of the skilled clinician, the therapeutic protocols (e.g., dosage amounts and times of administration) can be varied in view of the observed effects of the administered therapeutic agents (i.e., antineoplastic agent or radiation) on the patient, and in view of the observed responses of the disease to the administered therapeutic agents.

In a preferred example of combination therapy in the treatment of pancreatic cancer, the FPT inhibitor, i.e. "Compound X", as identified previously, is administered orally in a range of from 50 to 400 mg/day, in two divided doses, on a continuous dosing regimen; and the antineoplastic agent is gemcitabine administered at a dosage of from 750 to 1350 mg/m² weekly for three but of four weeks during the course of treatment.

In a preferred example of combination therapy in the treatment of lung cancer, the FPT inhibitor, i.e. "Compound X", as identified previously, is administered orally in a range of from 50 to 400 mg/day, in two divided doses, on a continuous dosing regimen; and the antineoplastic agent is paclitaxel administered at a dosage of from 65 to 175 mg/m² once every three weeks.

In a preferred example of combination therapy in the treatment of gliomas, the FPT inhibitor, i.e. "Compound X", as identified previously, is administered orally in a range of from 50 to 400 mg/day, in two divided doses; and the antineoplastic agent is temozolomide administered at a dosage of from 100 to 250 mg/m².

In another example of combination therapy, the FPT inhibitor, i.e. "Compound X", as identified previously, is administered orally in a range of from 50 to 400 mg/day, in two divided doses, on a continuous dosing regimen: and the antineoplastic agent is 5-Fluorouracil (5-FU) administered either at a dosage of 500 mg/m² per week (once a week), or at a dosage of 200-300 mg/m² per day in the case of continuous infusion of the 5-FU. In the case of 5-FU administration on a weekly injection, 5-FU may be administered in combination with a follate agonist (*e*.*g*., Leucovoran (at a dosage of 20 mg/m²/week).

In the uses of this invention, an FPT inhibitor is in a form for administration concurrently or sequentially with a chemotherapeutic agent and/or radiation. Thus, it is not necessary that, for example, the chemotherapeutic agent and the FPT inhibitor, or the radiation and the FPT inhibitor, should be administered simultaneously or essentially simultaneously. The advantage of a simultaneous or essentially simultaneous administration is well within the determination of the skilled clinician.

Also, in general, the FPT inhibitor and the chemotherapeutic agent do not have to be administered in the same pharmaceutical composition, and may, because of different physical and chemical characteristics, have to be administered by different routes. For example, the FPT inhibitor may be administered orally to generate and maintain good blood levels thereof, while the chemotherapeutic agent may be administered intravenously. The determination of the mode of administration and the advisability of administration, where possible, in the same pharmaceutical composition, is well within the knowledge of the skilled clinician. The initial administration can be made according to established protocols known in the art, and then, based upon the observed effects, the dosage, modes of administration and times of administration can be modified by the skilled clinician.

The particular choice of chemotherapeutic agent and/or radiation will depend upon the diagnosis of the attending physicians and their judgement of the condition of the patient and the appropriate treatment protocol.

The FPT inhibitor, and chemotherapeutic agent and/or radiation may be in form for administration concurrently (e.g., simultaneously, essentially simultaneously or within the same treatment protocol) or sequentially, depending upon the nature of the proliferative disease, the condition of the patient, and the actual choice of chemotherapeutic agent and/or radiation to be administered in conjunction (i.e., within a single treatment protocol) with the FPT inhibitor.

If the FPT inhibitor, and the chemotherapeutic agent and/or radiation are not administered simultaneously or essentially simultaneously, then the initial order of administration of the FPT inhibitor, and the chemotherapeutic agent and/or radiation, may not be important. Thus, the FPT inhibitor may be administered first followed by the administration of the chemotherapeutic agent and/or radiation; or the chemotherapeutic agent and/or radiation may be administered first followed by the administration of the FPT inhibitor. This alternate administration may be repeated during a single treatment protocol. The determination of the order of administration, and the number of repititions of administration of each therapeutic agent during a treatment protocol, is well within the knowledge of the skilled physician after evaluation of the disease being treated and the condition of the patient. For example, the chemotherapeutic agent and/or radiation may be administered first, especially if it is a cytotoxic agent, and then the treatment continued with the administration of the FPT inhibitor followed, where determined advantageous, by the administration of the chemotherapeutic agent and/or radiation, and so on until the treatment protocol is complete.

Thus, in accordance with experience and knowledge, the practising physician can modify each protocol for the administration of a component (therapeutic agent-- *i*.*e*., FPT inhibitor, chemotherapeutic agent or radiation) of the treatment according to the individual patient's needs, as the treatment proceeds.

The attending clinician, in judging whether treatment is effective at the dosage administered, will consider the general well-being of the patient as well as more definite signs such as relief of disease-related symptoms, inhibition of tumor growth, actual shrinkage of the tumor, or inhibition of metastasis. Size of the tumor can be measured by standard methods such as radiological studies, e.g., CAT or MRI scan, and successive measurements can be used to judge whether or not growth of the tumor has been retarded or even reversed. Relief of disease-related symptoms such as pain, and improvement in overall condition can also be used to help judge effectiveness of treatment.

The following are examples (Examples 1-4) of capsule formulations for the FPT Inhibitory Compound:

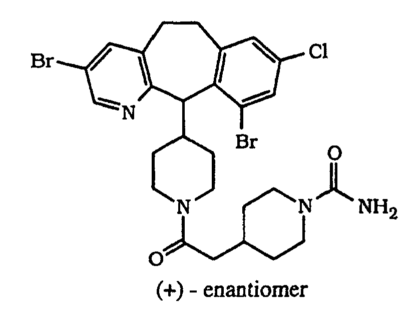

### EXAMPLES 1 and 2

### Capsule Formulation

| Composition | Example 1 mg/capsule | Example 2 mg/capsule | % Composition |
|---|---|---|---|
| Solid Solution | 100 | 400.0 | 84.2 |
| Silicon Dioxide NF⁽¹⁾ | 0.625 | 2.5 | 0.5 |
| Magnesium Stearate NF⁽²⁾ | 0.125 | 0.5 | 0.1 |
| Croscarmellose Sodium NF | 11.000 | 44.0 | 9.3 |
| Pluronic F68 NF | 6.250 | 25.0 | 5.3 |
| Silicon Dioxide NF⁽³⁾ | 0.625 | 2.5 | 0.5 |
| Magnesium Stearate NF⁽⁴⁾ | 0.125 | 0.5 | 0.1 |
| TOTAL | 118.750 | 475.00 | |
| Capsule size | No. 4 | No. 0 | |

### METHOD (Examples 1 and 2)

### Preparation of Solid Solution

| Composition | g/batch | % Composition |
|---|---|---|
| FPT Inhibitory Compound | 80 | 33.3 |
| Povidone NF K29/32 | 160 | 66.6 |
| Methylene Chloride | 5000 mL | evaporates |

Crystalline FPT Inhibitory Compound and the povidone were dissolved in methylene chloride. The solution was dried using a suitable solvent spray dryer. The residue was then reduced to fine particles by grinding. The powder was then passed through a 30 mesh screen. The powder was found to be amorphous by x-ray analysis.

The solid solid solution, silicon dioxide⁽¹⁾ and magnesium stearate⁽²⁾ were mixed in a suitable mixer for 10 minutes. The mixture is compacted using a suitable roller compactor and milled using a suitable mill fitted with 30 mesh screen. Croscarmellose sodium, Pluronic F68 and silicon dioxide⁽³⁾ are added to the milled mixture and mixed further for 10 minutes. A premix was made with magnesium stearate⁽⁴⁾ and equal portions of the mixture. The premix was added to the remainder of the mixture and mixed for 5 minutes, the mixture was encapsulated in hard shell gelatin capsule shells.

### EXAMPLES 3 and 4

### Capsule Formulation

| Composition | Example 3 mg/capsule | Example 4 mg/capsule | % Composition |
|---|---|---|---|
| Solid Solution | 400 | 200.0 | 80.0 |
| Silicon Dioxide NF⁽¹⁾ | 3.75 | 1.875 | 0.75 |
| Magnesium Stearate NF⁽²⁾ | 0.125 | 0.625 | 0.25 |
| Croscarmellose Sodium NF | 40.00 | 20.00 | 8.0 |
| Pluronic F68 NF | 50.00 | 25.00 | 10 |
| Silicon Dioxide NF⁽³⁾ | 3.75 | 1.875 | 0.75 |
| Magnesium Stearate NF⁽⁴⁾ | 1.25 | 0.625 | 0.25 |
| TOTAL | 500.00 | 250.00 | |
| Capsule size | No. 0 | No. 2 | |

### METHOD (Examples 3 and 4)

### Preparation of Solid Solution

| Composition | g/batch | % Composition |
|---|---|---|
| FPT Inhibitory Compound | 15 | 50 |
| Povidone NF K29/32 | 15 | 50 |
| Methylene Chloride | 140 mL | evaporates |
| Methanol | 60 mL | evaporates |

For information on formulations, reference can also be made to U.S. Patent Application Serial Nos. 08/997168 and 60/068387 (filed December 22, 1997), incorporated herein by reference.

Crystalline FPT Inhibitory Compound and the povidone were dissolved in a mixture of methylene chloride and methanol. The solution was dried using a suitable solvent spray dryer. The residue was then reduced to fine particles by grinding. The powder was then passed through a 30 mesh screen. The powder was found to be amorphous by x-ray analysis.

The solid solid solution, silicon dioxide⁽¹⁾ and magnesium stearate⁽²⁾ were mixed in a suitable mixer for 10 minutes. The mixture is compacted using a suitable roller compactor and milled using a suitable mill fitted with 30 mesh screen. Croscarmellose sodium, Pluronic F68 and silicon dioxide⁽³⁾ are added to the milled mixture and mixed further for 10 minutes. A premix was made with magnesium stearate⁽⁴⁾ and equal portions of the mixture. The premix was added to the remainder of the mixture and mixed for 5 minutes. The mixture was encapsulated in hard shell gelatin capsule shells.

## Claims

1. Use of the FPT inhibitor 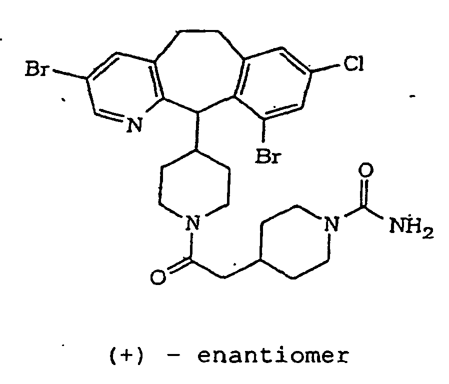
for the manufacture of a medicament for use concurrently or sequentially with an antineoplastic agent and/or radiation therapy in the treatment of proliferative disease in a patient in need of such treatment.

2. Use according to claim 1 **characterized in that** said antineoplastic agent is selected from: Uracil mustard, Chlormethine, Cyclophosphamide, Ifosfamide, Melphalan, Chlorambucil, Pipobroman, Triethylene-melamine, Triethylenethiophosphoramine, Busulfan, Carmustine, Lomustine, Streptozocin, Dacarbazine, Temozolomide, Methotrexate, 5-Fluorouracil, Floxuridine, Cytarabine, 6-Mercaptopurine, 6-Thioguanine, Fludarabine phosphate, Pentostatine, Gemcitabine, Vinblastine, Vincristine, Vindesine, Bleomycin, Dactinomycin, Daunorubicin, Doxorubicin, Epirubicin, Idarubicin, Paclitaxel, Mithramycin, Deoxycoformycin, Mitomycin-C, L-Asparaginase, Interferons, Etoposide, Teniposide, 17α-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, Goserelin, Cisplatin, Carboplatin, Hydroxyurea, Amsacrine, Procarbazine, Mitotane, Mitoxantrone, Levamisole, Navelbene, CPT-11, Anastrazole, Letrazole, Capecitabine, Reloxafine, Droloxafine, or Hexamethylmelamine.

3. Use according to claims 1 and 2 **characterized in that** said antineoplastic agent is selected from: Temozolomide, Cyclophosphamide, 5-Fluorouracil or Vincristine.

4. Use according to anyone of claims 1 to 3 **characterized in that** said proliferative disease is: lung cancer, pancreatic cancer, colon cancer, myeloid leukemia, melanoma, glioma, thyroid follicular cancer, bladder carcinoma, myelodysplastic syndrome, breast cancer or prostate cancer.

5. Use according to claim 1 **characterized in that** the said medicament is in a form for administration concurrently, simultaneously or sequentially with said antineoplastic agent and/or radiation.

6. Use according to claim 1 **characterized in that** the said medicament is in a form for administration first or said antineoplastic agent and/or radiation is in a form for administration first.

7. Use according to claim 1 **characterized in that** the antineoplatic agent is gemcitabine

8. Use according to claim 7 **characterized in that** said proliferative disease is: prostate cancer, lung cancer or pancreatic cancer.

9. Use according to claim 7 **characterized in that** said proliferative disease is pancreatic cancer.

10. Use according to anyone of claims 7 to 9 **characterized in that** said FPT inhibitor is in a form for oral administration in an amount of from 50 to 400 mg/day, and the gemcitabine is in a form for administration in an amount of from 750 to 1350 mg/m² weekly for three out of four weeks.

11. Use of the FPT inhibitor 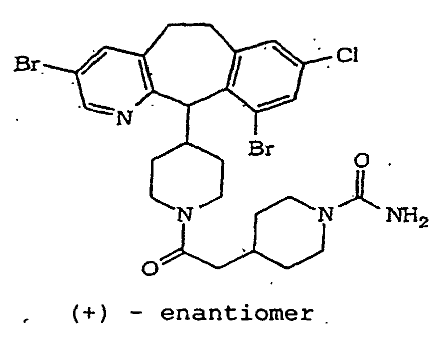
for the manufacture of a medicament for use concurrently or sequentially with a microtubule affecting agent in the treatment of proliferative disease in a patient in need of such treatment.

12. Use according to claim 11 **characterized in that** said microtubule affecting agent is Taxotere.

13. Use according to claim 11 **characterized in that** said microtubule affecting agent is paclitaxel or paclitaxel derivative.

14. Use according to claim 11 **characterized in that** said proliferative disease is: prostate cancer, pancreatic cancer or lung cancer.

## Patentansprüche

1. Verwendung des FPT-Inhibitors 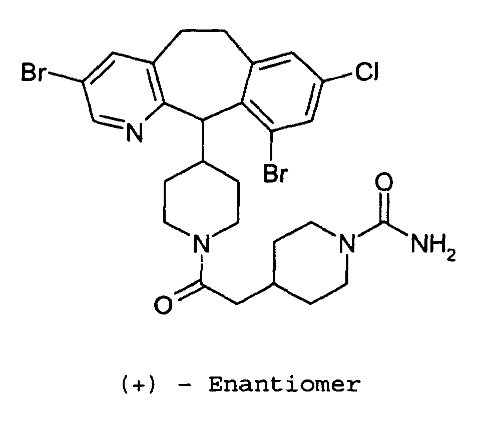
zur Herstellung eines Medikaments zur gleichzeitigen oder aufeinander folgenden Verwendung mit einem antineoplastischen Mittel und/oder Strahlungstherapie bei der Behandlung von proliferativen Erkrankungen bei einem Patienten, der eine solche Behandlung benötigt.

2. Verwendung nach Anspruch 1, **dadurch gekennzeichnet, dass** das antineoplastische Mittel ausgewählt ist aus Uracillost, Chlormethin, Cyclophosphamid, Ifosfamid, Melphalan, Chlorambucil, Pipobroman, Triethylenmelamin, Triethylenthiophosphoramin, Busulfan, Carmustin, Lomustin, Streptozocin, Dacarbazin, Temozolomid, Methotrexat, 5-Fluoruracil, Floxuridin, Cytarabin, 6-Mercaptopurin, 6-Thioguanin, Fludarabinphosphat, Pentostatin, Gemcitabin, Vinblastin, Vincristin, Vindesin, Bleomycin, Dactinomycin, Daunorubicin, Doxorubicin, Epirubicin, Idarubicin, Paclitaxel, Mithramycin, Deoxycoformycin, Mitomycin-C, L-Asparaginase, Interferonen, Etoposid, Teniposid, 17α-Ethinylestradiol, Diethylstilbestrol, Testosteron, Prednison, Fluoxymesteron, Dromostanolonpropionat, Testolacton, Megestrolacetat, Tamoxifen, Methylprednisolon, Methyltestosteron, Prednisolon, Triamcinolon, Chlortrianisen, Hydroxyprogesteron, Aminoglutethimid, Estramustin, Medroxyprogesteronacetat, Leuprolid, Flutamid, Toremifen, Goserelin, Cisplatin, Carboplatin, Hydroxyharnstoff, Amsacrin, Procarbazin, Mitotan, Mitoxantron, Levamisol, Navelben, CPT-11, Anastrazol, Letrazol, Capecitabin, Reloxafin, Droloxafin oder Hexamethylmelamin.

3. Verwendung nach Ansprüchen 1 und 2, **dadurch gekennzeichnet, dass** das antineoplastische Mittel ausgewählt ist aus Temozolomid, Cyclophosphamid, 5-Fluoruracil oder Vincristin.

4. Verwendung nach einem der Ansprüche 1 bis 3, **dadurch gekennzeichnet, dass** die proliferative Erkrankung Lungenkrebs, Bauchspeicheldrüsenkrebs, Dickdarmkrebs, myeloische Leukämie, Melanom, Gliom, Schilddrüsenfollikelkrebs, Blasenkarzinom, myelodysplastisches Syndrom, Brustkrebs oder Prostatakrebs ist.

5. Verwendung nach Anspruch 1, **dadurch gekennzeichnet, dass** das Medikament in einer Form zur gleichzeitigen, simultanen oder aufeinander folgenden Verabreichung mit dem antineoplastischen Mittel und/oder der Strahlung vorliegt.

6. Verwendung nach Anspruch 1, **dadurch gekennzeichnet, dass** das Medikament in einer Form zur ersten Verabreichung vorliegt oder das antineoplastische Mittel und/oder die Strahlung in einer Form zur ersten Verabreichung vorliegt.

7. Verwendung nach Anspruch 1, **dadurch gekennzeichnet, dass** das antineoplastische Mittel Gemcitabin ist.

8. Verwendung nach Anspruch 7, **dadurch gekennzeichnet, dass** die proliferative Erkrankung Prostatakrebs, Lungenkrebs oder Bauchspeicheldrüsenkrebs ist.

9. Verwendung nach Anspruch 7, **dadurch gekennzeichnet, dass** die proliferative Erkrankung Bauchspeicheldrüsenkrebs ist.

10. Verwendung nach einem der Ansprüche 7 bis 9, **dadurch gekennzeichnet, dass** der FPT-Inhibitor in einer Form zur oralen Verabreichung in einer Menge von 50 bis 400 mg/Tag vorliegt und das Gemcitabin in einer Form zur Verabreichung in einer wöchentlichen Menge von 750 bis 1350 mg/m² über drei von vier Wochen vorliegt.

11. Verwendung des FPT-Inhibitors 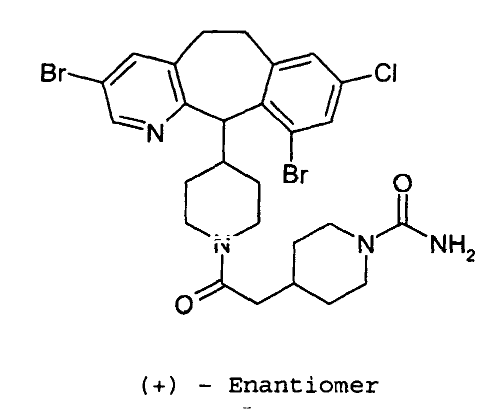
zur Herstellung eines Medikaments zur gleichzeitigen oder aufeinander folgenden Verwendung mit einem Mikrotubulus beeinflussenden Mittel bei der Behandlung einer proliferativen Erkrankung bei einem Patienten, der eine solche Behandlung benötigt.

12. Verwendung nach Anspruch 11, **dadurch gekennzeichnet, dass** das Mikrotubulus beeinflussende Mittel Taxoter ist.

13. Verwendung nach Anspruch 11, **dadurch gekennzeichnet, dass** das Mikrotubulus beeinflussende Mittel Paclitaxel oder ein Paclitaxelderivat ist.

14. Verwendung nach Anspruch 11, **dadurch gekennzeichnet, dass** die proliferative Erkrankung Prostatakrebs, Bauchspeicheldrüsenkrebs oder Lungenkrebs ist.

## Revendications

1. Emploi de l'inhibiteur de farnésyl-transférase de formule : 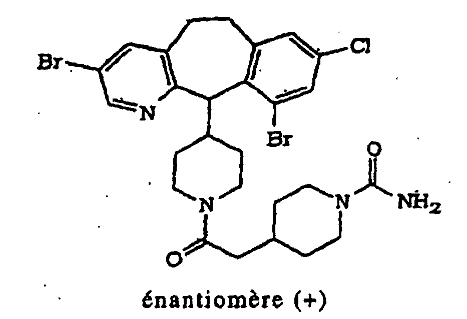
en vue de la fabrication d'un médicament à employer, parallèlement ou séquentiellement, avec un agent antinéoplasique et/ou une radiothérapie dans le traitement d'une maladie proliférative chez un patient qui a besoin d'un tel traitement.

2. Emploi conforme à la revendication 1, **caractérisé en ce que** ledit agent antinéoplasique est choisi parmi les suivants : moutarde uracile, chlorméthine, cyclophosphamide, ifosfamide, melphalan, chlorambucil, pipobromane, triéthylène-mélamine, triéthylènethiophosphoramine, busulfan, carmustine, lomustine, streptozocine, dacarbazine, temozolomide, méthotrexate, 5-fluoro-uracile, floxuridine, cytarabine, 6-mercaptopurine, 6-thioguanine, phosphate de fludarabine, pentostatine, gemcitabine, vinblastine, vincristine, vindésine, bléomycine, dactinomycine, daunorubicine, doxorubicine, épirubicine, idarubicine, paclitaxel, mithramycine, désoxycoformycine, mitomycine C, L-asparaginase, interférons, étoposide, téniposide, 17α-éthynyl-oestradiol, diéthyl-stilbestrol, testostérone, prednisone, fluoxymestérone, propionate de dromostanolone, testolactone, acétate de mégestrol, tamoxifène, méthyl-prednisolone, méthyl-testostérone, prednisolone, triamcinolone, chlorotrianisène, hydroxyprogestérone, aminoglutéthimide, estramustine, acétate de médroxyprogestérone, leuprolide, flutamide, torémifène, goséréline, cisplatine, carboplatine, hydroxyurée, amsacrine, procarbazine, mitotane, mitoxantrone, lévamisole, navelbine, CPT-11, anastrazole, létrazole, capécitabine, réloxafine, droloxafine, hexaméthyl-mélamine.

3. Emploi conforme à la revendication 1 ou 2, **caractérisé en ce que** ledit agent antinéoplasique est choisi parmi les témozolomide, cyclophosphamide, 5-fluorouracile et vincristine.

4. Emploi conforme à l'une des revendications 1 à 3, **caractérisé en ce que** ladite maladie proliférative est un cancer du poumon, un cancer du pancréas, un cancer du côlon, une leucémie myéloïde, un mélanome, un gliome, un cancer vésiculaire de la thyroïde, un cancer de la vessie, un syndrome myélodisplasique, un cancer du sein ou un cancer de la prostate.

5. Emploi conforme à la revendication 1, **caractérisé en ce que** ledit médicament se trouve sous une forme destinée à être administrée parallèlement, simultanément ou séquentiellement avec ledit agent antinéoplasique et/ou ladite radiothérapie.

6. Emploi conforme à la revendication 1, **caractérisé en ce que** ledit médicament se trouve sous une forme destinée à être administrée en premier ou ledit agent antinéoplasique et/ou ladite radiothérapie se trouve(nt) sous une forme destinée à être administrée en premier.

7. Emploi conforme à la revendication 1, **caractérisé en ce que** ledit agent antinéoplasique est de la gemcitabine.

8. Emploi conforme à la revendication 7, **caractérisé en ce que** ladite maladie proliférative est un cancer de la prostate, un cancer du poumon ou un cancer du pancréas.

9. Emploi conforme à la revendication 7, **caractérisé en ce que** ladite maladie proliférative est un cancer du pancréas.

10. Emploi conforme à l'une des revendications 7 à 9, **caractérisé en ce que** ledit inhibiteur de farnésyl-transférase se trouve sous une forme adaptée pour être administrée par voie orale à raison de 50 à 400 mg par jour, et la gemcitabine se trouve sous une forme adaptée pour être administrée à raison de 750 à 1350 mg/m² par semaine, trois semaines sur quatre.

11. Emploi de l'inhibiteur de farnésyl-transférase de formule : 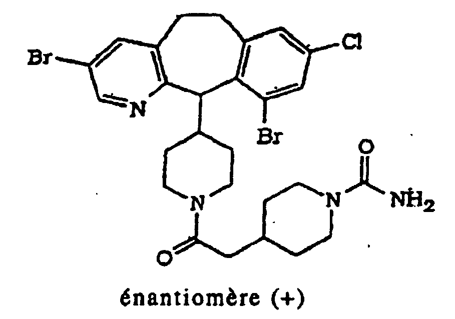
en vue de la fabrication d'un médicament à employer, parallèlement ou séquentiellement, avec un agent agissant sur les microtubules dans le traitement d'une maladie proliférative chez un patient qui a besoin d'un tel traitement.

12. Emploi conforme à la revendication 11, **caractérisé en ce que** ledit agent agissant sur les microtubules est du taxotère.

13. Emploi conforme à la revendication 11, **caractérisé en ce que** ledit agent agissant sur les microtubules est du paclitaxel ou un dérivé de paclitaxel.

14. Emploi conforme à la revendication 11, **caractérisé en ce que** ladite maladie proliférative est un cancer de la prostate, un cancer du pancréas ou un cancer du poumon.
